# EUROPÄISCHE PATENTSCHRIFT

(11) **EP 3 423 446 B1**
(45) Veröffentlichungstag und Bekanntmachung des Hinweises auf die Patenterteilung: **16.09.2020**
(21) Anmeldenummer: 17707049.7
(22) Anmeldetag: 28.02.2017
(51) Int. Cl.: C07D 401/14, C07D 405/14, C07D 413/14, C07D 409/14, C07D 417/14, A61K 31/4439, A61P 29/00, A61P 35/00

(54) **NEUE 2-SUBSTITUIERTE INDAZOLE, VERFAHREN ZU IHRER HERSTELLUNG, PHARMAZEUTISCHE PRÄPARATE DIE DIESE ENTHALTEN, SOWIE DEREN VERWENDUNG ZUR HERSTELLUNG VON ARZNEIMITTELN**
NEW 2-SUBSTITUTED INDAZOLES, METHODS FOR PRODUCING SAME, PHARMACEUTICAL PREPARATIONS THAT CONTAIN SAME, AND USE OF SAME TO PRODUCE DRUGS
NOUVEAUX INDAZOLES SUBSTITUÉS EN 2, LEURS PROCÉDÉS DE PRÉPARATION, PRÉPARATIONS PHARMACEUTIQUES LES CONTENANT, ET LEUR UTILISATION POUR PRODUIRE DES MÉDICAMENTS

(30) Priorität: 03.03.2016 EP 16158542
(43) Veröffentlichungstag der Anmeldung: 09.01.2019
(73) Patentinhaber: Bayer Pharma Aktiengesellschaft, 13353 Berlin (DE)
(72) Erfinder: BOTHE, Ulrich, 13187 Berlin (DE); SIEBENEICHER, Holger, 10557 Berlin (DE); SCHMIDT, Nicole, 42113 Wuppertal (DE); GÜNTHER, Judith, 13187 Berlin (DE); STEUBER, Holger, 10115 Berlin (DE); BÖMER, Ulf, 16548 Glienicke (DE); LANGE, Martin, 10405 Berlin (DE); NUBBEMEYER, Reinhard, 13509 Berlin (DE); RAY, Nicholas, Charles, Harlow, Essex CM19 5TR (GB); SAVY, Pascal, Harlow, Essex CM19 5TR (GB)
(74) Vertreter: BIP Patents
(86) Internationale Anmeldenummer: PCT/EP2017/054577
(87) Internationale Veröffentlichungsnummer: WO 2017/148902

(56) Entgegenhaltungen:
- WO-A1-2015/104662
- WO-A1-2015/193846
- WO-A1-2017/009798

## Beschreibung

Die vorliegende Anmeldung betrifft neue 2-substituierte Indazole, Verfahren zu ihrer Herstellung, die Verwendung der neuen substituierten Indazole zur Behandlung und/ oder Prophylaxe von Krankheiten sowie ihre Verwendung zur Herstellung von Arzneimitteln zur Behandlung und/ oder Prophylaxe von Krankheiten, insbesondere von proliferativen Erkrankungen, von Autoimmunerkrankungen, von metabolischen und von inflammatorischen Erkrankungen wie z.B. Rheumatoider Arthritis, Spondyloarthritiden (insbesondere Spondyloarthritis psoriatica und Morbus Bechterew), chronisch obstruktiver Lungenerkrankung (englisch chronic obstructive pulmonary disease, Abkürzung: COPD), Multipler Sklerose, Systemischem Lupus Erythematodes, Gicht, des metabolischen Syndroms, Fettleberhepatitis, Insulinresistenz, Endometriose und entzündungsinduziertem oder chronischem Schmerz sowie von Lymphomen.

Die vorliegende Erfindung betrifft neue 2-substituierte Indazole der allgemeinen Formel (I), die die Interleukin-1 Receptor-Associated Kinase 4 (IRAK4) hemmen.
Humanes IRAK4 (Interleukin-1 receptor-associated kinase 4) spielt eine Schlüsselrolle bei der Aktivierung des Immunsystems. Deshalb ist diese Kinase ein wichtiges therapeutisches Zielmolekül für die Entwicklung von entzündungshemmenden Substanzen. IRAK4 wird von einer Vielzahl von Zellen exprimiert und vermittelt die Signaltransduktion von Toll-like Rezeptoren (TLR), außer TLR3, sowie Rezeptoren der Interleukin (IL)-1β Familie, bestehend aus dem IL-1R (Rezeptor), IL-18R, IL-33R und IL-36R (Janeway und Medzhitov, Annu. Rev. Immunol., 2002; Dinarello, Annu. Rev. Immunol., 2009; Flannery and Bowie, Biochemical Pharmacology, 2010). Weder IRAK4 Knockout Mäuse noch humane Zellen von Patienten, denen IRAK4 fehlt, reagieren auf die Stimulation von TLRs (außer TLR3) und der IL-1β Familie (Suzuki, Suzuki, et al., Nature, 2002; Davidson, Currie, et al., The Journal of Immunology, 2006; Ku, von Bernuth, et al., JEM, 2007; Kim, Staschke, et al., JEM, 2007).
Die Bindung der TLR Liganden bzw. der Liganden der IL-1β Familie an den jeweiligen Rezeptor führt zur Rekrutierung und Bindung von MyD88 [Myeloid differentiation primary response gene (88)] an den Rezeptor. Infolgedessen tritt MyD88 mit IRAK4 in Interaktion und es kommt zur Bildung eines aktiven Komplexes, welcher mit den Kinasen IRAK1 oder IRAK2 interagiert und diese aktiviert (Kollewe, Mackensen, et al., Journal of Biological Chemistry, 2004; Precious et al., J. Biol. Chem., 2009). Infolgedessen wird der NF (nuclear factor)-KB Signalweg und der MAPK (Mitogen-activated protein kinase) Signalweg aktiviert (Wang, Deng, et al., Nature, 2001). Die Aktivierung des NF-κB Signalweges als auch des MAPK Signalweges führen zu Prozessen, die mit verschiedenen Immunprozessen assoziiert sind. So kommt es beispielsweise zu einer erhöhten Expression von unterschiedlichen inflammatorischen Signalmolekülen und Enzymen, wie z.B. Zytokinen, Chemokinen und COX-2 (Cyclooxygenase-2), und zu einer erhöhten mRNA Stabilität von inflammationsassoziierten Genen wie beispielsweise COX-2, IL-6 (Interleukin-6)-, IL-8 (Holtmann, Enninga, et al., Journal of Biological Chemistry, 2001; Datta, Novotny, et al., The Journal of Immunology, 2004). Des Weiteren können diese Prozesse mit der Proliferation und der Differenzierung von bestimmten Zelltypen, wie z.B. Monozyten, Makrophagen, Dendritischen Zellen, T-Zellen und B-Zellen einhergehen (Wan, Chi, et al., Nat Immunol, 2006; McGettrick and J. O'Neill, British Journal of Haematology, 2007).

Die zentrale Rolle von IRAK4 in der Pathologie von unterschiedlichen inflammatorischen Erkrankungen konnte bereits durch den direkten Vergleich von Wildtyp (WT) Mäusen mit genetisch veränderten Tieren mit einer Kinase-inaktiven Form des IRAK4 (IRAK4 KDKI) gezeigt werden. IRAK4 KDKI Tiere weisen ein verbessertes Krankheitsbild im Tiermodell für Multiple Sklerose, Atherosklerose, Herzinfarkt und Alzheimer auf (Rekhter, Staschke, et al., Biochemical and Biophysical Research Communication, 2008; Maekawa, Mizue, et al., Circulation, 2009; Staschke, Dong, et al., The Journal of Immunology, 2009; Kim, Febbraio, et al., The Journal of Immunology, 2011; Cameron, Tse, et al., The Journal of Neuroscience, 2012). Des Weiteren zeigte sich, dass die Deletion von IRAK4 im Tiermodell vor einer viral-induzierten Myokarditis infolge einer verbesserten anti-viralen Reaktion bei gleichzeitig verringerter systemischer Inflammation schützt (Valaperti, Nishii, et al., Circulation, 2013). Außerdem wurde gezeigt, dass die Expression von IRAK4 mit dem Ausmaß des Vogt-Koyanagi-Harada-Syndroms korreliert (Sun, Yang, et al., PLoS ONE, 2014). Zudem konnte die hohe Relevanz von IRAK4 für die Immunkomplexvermittelte IFNα (Interferon-alpha) Produktion durch plasmazytoide Dendritische Zellen, ein Schlüsselprozess bei der Pathogenese des Systemischen Lupus Erythematodes (SLE), gezeigt werden (Chiang et al., The Journal of Immunology, 2010). Des Weiteren ist der Signalweg mit Fettleibigkeit (Adipositas) assoziiert (Ahmad, R., P. Shihab, et al., Diabetology & Metabolic Syndrome, 2015)
Neben der essentiellen Rolle von IRAK4 bei der angeborenen Immunität gibt es auch Hinweise, dass IRAK4 die Differenzierung der sogenannten Thl7 T-Zellen, Komponenten der adaptiven Immunität, beeinflusst. In Abwesenheit der IRAK4 Kinaseaktivität werden weniger IL-17 produzierende T-Zellen (Th17 T-Zellen) im Vergleich zu WT Mäusen generiert. Durch die Inhibition von IRAK4 ist die Prophylaxe und/oder Behandlung von Atherosklerose, Diabetes mellitus Typ 1, Rheumatoide Arthritis, Spondyloarthritiden (insbesondere Spondyloarthritis psoriatica und Morbus Bechterew), Lupus erythematodes, Psoriasis, Vitiligo, Riesenzellarteriitis, chronisch entzündlicher Darmerkrankung und Viruserkrankungen, wie z.B. HIV (Humane Immundefizienz-Virus), Hepatitis Virus möglich (Staschke, et al., The Journal of Immunology, 2009; Marquez, et al., Ann Rheum Dis, 2014; Zambrano-Zaragoza, et al., International Journal of Inflammation, 2014; Wang, et al., Experimental and Therapeutic Medicine, 2015; Ciccia, et al., Rheumatology, 2015).

Durch die zentrale Rolle von IRAK4 in der MyD88-vermittelten Signalkaskade von TLRs (außer TLR3) und der IL-1 Rezeptorfamilie kann die Inhibition von IRAK4 zur Prophylaxe und/oder Behandlung von durch die genannten Rezeptoren vermittelte Erkrankungen genutzt werden. TLRs als auch Komponenten der IL-1 Rezeptorfamilie sind in der Pathogenese der Rheumatoiden Arthritis, der Psoriasis Arthritis, Myasthenia gravis, der Vaskulitis wie beispielsweise Morbus Behçet, Granulomatose mit Polyangiitis und Riesenzellarteriitis, Pankreatitis, des Systemischen Lupus Erythematodes, der Dermamyositis und Polymyositis, des Metabolischen Syndroms inklusive z.B. Insulinresistenz, Hypertonie, Dyslipoproteinämie und Adipositas, der Diabetes mellitus (Typ 1 und Typ 2), der diabetischen Nephropathie, der Osteoarthritis, des Sjögren-Syndroms, und der Sepsis involviert (Yang, Tuzun, et al., J Immunol, 2005; Candia, Marquez et al., The Journal of Rheumatology, 2007; Scanzello, Plaas, et al. Curr Opin Rheumatol, 2008; Deng, Ma-Krupa, et al., Circ Res, 2009; Roger, Froidevaux, et al, PNAS, 2009; Devaraj, Tobias, et al., Arterioscler Thromb Vasc Biol, 2011; Kim, Cho, et al., Clin Rheumatol, 2010; Carrasco et al., Clinical and Experimental Rheumatology, 2011; Gambuzza, Licata, et al., Journal of Neuroimmunology, 2011; Fresno, Archives Of Physiology And Biochemistry, 2011; Volin and Koch, J Interferon Cytokine Res, 2011; Akash, Shen, et al., Journal of Pharmaceutical Sciences, 2012; Goh and Midwood, Rheumatology, 2012; Dasu, Ramirez, et al., Clinical Science, 2012; Ouziel, Gustot, et al., Am J Patho, 2012; Ramirez and Dasu, Curr Diabetes Rev, 2012, Okiyama et al., Arthritis Rheum, 2012; Chen et al., Arthritis Research & Therapy, 2013; Holle, Windmoller, et al., Rheumatology (Oxford), 2013; Li, Wang, et al., Pharmacology & Therapeutics, 2013; Sedimbi, Hagglof, et al., Cell Mol Life Sci, 2013; Caso, Costa, et al., Mediators of Inflammation, 2014; Cordiglieri, Marolda, et al., J Autoimmun, 2014; Jialal, Major, et al., J Diabetes Complications, 2014; Kaplan, Yazgan, et al., Scand J Gastroenterol, 2014; Talabot-Aye, et al., Cytokine, 2014; Zong, Dorph, et al., Ann Rheum Di, 2014; Ballak, Stienstra, et al., Cytokine, 2015; Timper, Seelig, et al., J Diabetes Complications, 2015). Hauterkrankungen wie Psoriasis, atopische Dermatitis, Kindler Syndrom, bullösen Pemphigoid, allergische Kontaktdermatitis, Alopecia areata, Acne in versa und Acne vulgaris sind mit dem IRAK4-vermittelten TLR-Signalweg bzw. der IL-1R Familie assoziiert (Schmidt, Mittnacht, et al., J Dermatol Sci, 1996; Hoffmann, J Investig Dermatol Symp Proc, 1999; Gilliet, Conrad, et al., Archives of Dermatology, 2004; Niebuhr, Langnickel, et al., Allergy, 2008; Miller, Adv Dermatol, 2008; Terhorst, Kalali, et al., Am J Clin Dermatol, 2010; Viguier, Guigue, et al., Annals of Internal Medicine, 2010; Cevikbas, Steinhoff, J Invest Dermatol, 2012; Minkis, Aksentijevich, et al., Archives of Dermatology, 2012; Dispenza, Wolpert, et al., J Invest Dermatol, 2012; Minkis, Aksentijevich, et al., Archives of Dermatology, 2012; Gresnigt and van de Veerdonk, Seminars in Immunology, 2013; Selway, Kurczab, et al., BMC Dermatology, 2013; Sedimbi, Hagglof, et al., Cell Mol Life Sci, 2013; Wollina, Koch, et al. Indian Dermatol Online, 2013; Foster, Baliwag, et al., The Journal of Immunology, 2014).
Auch bei pulmonalen Erkrankungen wie Lungenfibrose, obstruktiver Lungenerkrankung (COPD), akutem Atemnot-Syndrom (ARDS), akuter Lungenschädigung (ALI), interstitieller Lungenerkrankung (ILD), Sarkoidose und pulmonaler Hypertonie zeigt sich eine Assoziation mit verschiedenen TLR-vermittelten Signalwegen. Bei der Pathogenese der pulmonalen Erkrankungen kann es sich sowohl um infektiös vermittelte als auch um nicht-infektiös vermittelte Prozesse handeln (Ramirez Cruz, Maldonado Bernal, et al., Rev Alerg Mex, 2004; Jeyaseelan, Chu, et al., Infection and Immunity, 2005; Seki, Tasaka, et al., Inflammation Research, 2010; Xiang, Fan, et al., Mediators of Inflammation, 2010; Margaritopoulos, Antoniou, et al., Fibrogenesis & Tissue Repair, 2010; Hilberath, Carlo, et al., The FASEB Journal, 2011; Nadigel, Prefontaine, et al., Respiratory Research, 2011; Kovach and Standiford, International Immunopharmacology, 2011; Bauer, Shapiro, et al., Mol Med, 2012; Deng, Yang, et al., PLoS One, 2013; Freeman, Martinez, et al., Respiratory Research, 2013; Dubaniewicz, A., Human Immunology, 2013). TLRs als auch IL-1R Familienmitglieder sind auch in die Pathogenese anderer inflammatorischer Erkrankungen wie Allergie, Behcet-Krankheit, Gicht, Lupus erythematodes, Adult Morbus Still-Krankheit, Perikarditis und chronisch entzündliche Darmerkrankungen, wie Kolitis ulcerosa und Morbus Crohn, Transplantatabstoßung und Graft-versus-Host-Reaktion involviert, so dass die Inhibition von IRAK4 hier ein geeigneter prophylaktischer und/oder therapeutischer Ansatz ist (Liu-Bryan, Scott, et al., Arthritis & Rheumatism, 2005; Piggott, Eisenbarth, et al., J Clin Inves, 2005; Christensen, Shupe, et al., Immunity, 2006; Cario, Inflammatory Bowel Diseases, 2010; Nickerson, Christensen, et al., The Journal of Immunology, 2010; Rakoff-Nahoum, Hao, et al., Immunity, 2006; Heimesaat, Fischer, et al., PLoS ONE, 2007; Heimesaat, Nogai, et al., Gut, 2010; Kobori, Yagi, et al., J Gastroenterol, 2010; Schmidt, Raghavan, et al., Nat Immunol, 2010; Shi, Mucsi, et al., Immunological Reviews, 2010; Leventhal and Schroppel, Kidney Int, 2012; Chen, Lin, et al., Arthritis Res Ther, 2013; Hao, Liu, et al., Curr Opin Gastroenterol, 2013; Kreisel and Goldstein, Transplant International, 2013; Li, Wang, et al., Pharmacology & Therapeutics, 2013; Walsh, Carthy, et al., Cytokine & Growth Factor Reviews, 2013; Zhu, Jiang, et al., Autoimmunity, 2013; Yap and Lai, Nephrology, 2013; Vennegaard, Dyring-Andersen, et al., Contact Dermatitis, 2014; D'Elia, Brucato, et al., Clin Exp Rheumatol, 2015; Jain, Thongprayoon, et al., Am J Cardiol., 2015; Li, Zhang, et al., Oncol Rep., 2015).
Durch TLR- und IL-1R Familie-vermittelte gynäkologische Erkrankungen wie Adenomyosis, Dysmenorrhoe, Dyspareunie und Endometriose, insbesondere Endometriose-assoziierte Schmerzen und andere Endometriose-assoziierte Symptome wie Dysmenorrhoe, Dyspareunie, Dysurie und Dyschezie, können durch den prophylaktischen und/oder therapeutischen Einsatz von IRAK4 Inhibitoren positiv beeinflusst werden (Akoum, Lawson, et al., Human Reproduction, 2007; Allhorn, Boing, et al., Reproductive Biology and Endocrinology, 2008; Lawson, Bourcier, et al., Journal of Reproductive Immunology, 2008; Sikora, Mielczarek-Palacz, et al., American Journal of Reproductive Immunology, 2012; Khan, Kitajima, et al., Journal of Obstetrics and Gynaecology Research, 2013; Santulli, Borghese, et al., Human Reproduction, 2013). Der prophylaktische und/oder therapeutische Einsatz von IRAK4 Inhibitoren kann außerdem Atherosklerose positiv beeinflussen (Seneviratne, Sivagurunathan, et al., Clinica Chimica Acta, 2012; Falck-Hansen, Kassiteridi, et al., International Journal of Molecular Sciences, 2013; Sedimbi, Hagglof, et al., Cell Mol Life Sci, 2013).

Neben den bereits aufgeführten Erkrankungen werden IRAK4-vermittelte TLR-Prozesse in der Pathogenese von Augenerkrankungen wie retinale Ischämie, Keratitis, allergisch bedingte Konjunktivitis, Keratoconjunctivitis sicca, Makuladegeneration und Uveitis beschrieben (Kaarniranta and Salminen, J Mol Med (Berl), 2009; Sun and Pearlman, Investigative Ophthalmology & Visual Science, 2009; Redfern and McDermott, Experimental Eye Research, 2010; Kezic, Taylor, et al., J Leukoc Biol, 2011; Chang, McCluskey, et al., Clinical & Experimental Ophthalmology, 2012; Guo, Gao, et al., Immunol Cell Biol, 2012; Lee, Hattori, et al., Investigative Ophthalmology & Visual Science, 2012; Qi, Zhao, et al., Investigative Ophthalmology & Visual Science, 2014).
Die Inhibition von IRAK4 ist außerdem ein geeigneter therapeutischer Ansatz für fibrotische Erkrankungen, wie beispielsweise Leberfibrose, Myokarditis, primär biliäre Zirrhose, zystische Fibrose (Zhao, Zhao, et al., Scand J Gastroenterol, 2011; Benias, Gopal, et al., Clin Res Hepatol Gastroenterol, 2012; Yang, L. and E. Seki, Front Physiol, 2012; Liu, Hu, et al., Biochim Biophys Acta., 2015).
Durch die Schlüsselstellung, die IRAK4 in TLR- und IL-1R Familie-vermittelten Erkrankungen hat, können chronische Lebererkrankungen wie beispielsweise Fettleberhepatitis und insbesondere nichtalkoholischen Fettleber¬erkrankungen (NAFLD - non-alcoholic fatty liver disease) und/oder nichtalkoholischer Fettleber¬hepatitis (NASH - non-alcoholic steatohepatitis), alkoholtoxische Hepatitis (ASH - alkoholische Steatohepatitis) präventiv und/oder therapeutisch mit IRAK4 Inhibitoren behandelt werden (Nozaki, Saibara, et al., Alcohol Clin Exp Res, 2004; Csak, T., A. Velayudham, et al., Am J Physiol Gastrointest Liver Physiol, 2011; Miura, Kodama, et al., Gastroenterology, 2010; Kamari, Shaish, et al., J Hepatol, 2011; Ye, Li, et al., Gut, 2012; Roh, Seki, J Gastroenterol Hepatol, 2013; Ceccarelli, S., V. Nobili, et al., World J Gastroenterol, 2014; Miura, Ohnishi, World J Gastroenterol, 2014; Stojsavljevic, Palcic, et al., World J Gastroenterol, 2014).
Aufgrund der zentralen Rolle von IRAK4 in TLR-vermittelten Prozessen ist durch die Inhibition von IRAK4 auch die Behandlung und/oder Prävention von kardiovaskulären und neurologischen Erkrankungen wie z.B. myokardialem Reperfusionsschaden, Myokardinfarkt, Hypertonie, Bluthochdruck (Oyama, Blais, et al., Circulation, 2004; Timmers, Sluijter, et al., Circulation Research, 2008; Fang and Hu, Med Sci Monit, 2011; Bijani, International Reviews of Immunology, 2012; Bomfim, Dos Santos, et al., Clin Sci (Lond), 2012; Christia and Frangogiannis, European Journal of Clinical Investigation, 2013; Thompson and Webb, Clin Sci (Lond), 2013; Hernanz, Martinez-Revelles, et al., British Journal of Pharmacology, 2015; Frangogiannis, Curr Opin Cardiol, 2015; Bomfim, Echem, et al., Life Sciences, 2015) sowie Alzheimer, Schlaganfall, Hirnschlag, Schädel-Hirn-Trauma, Amyotrophe Lateralsklerose (ALS) und Parkinson möglich (Brough, Tyrrell, et al., Trends in Pharmacological Sciences, 2011; Carty and Bowie, Biochemical Pharmacology, 2011; Denes, Kitazawa, Cheng, et al., The Journal of Immunology, 2011; Lim, Kou, et al., The American Journal of Pathology, 2011; Béraud and Maguire-Zeiss, Parkinsonism & Related Disorders, 2012; Denes, Wilkinson, et al., Disease Models & Mechanisms, 2013; Noelker, Morel, et al., Sci. Rep., 2013; Wang, Wang, et al., Stroke, 2013; Xiang, Chao, et al., Rev Neurosci, 2015; Lee, Lee, et al., J Neuroinflammation, 2015).
Aufgrund der Involvierung von TLR-vermittelten Signalen und IL-1 Rezeptorfamilie-vermittelten Signalen über IRAK4 bei Juckreiz und Schmerz inklusive akutem, chronischem, entzündlichem und neuropathischem Schmerz ist von einer therapeutischen Wirkung in den genannten Indikationen durch die Inhibierung von IRAK4 auszugehen. Für Schmerz seien beispielhaft Hyperalgesie, Allodynie, prämenstrueller Schmerz, Endometriose-assoziierter Schmerz, postoperativer Schmerz, interstitielle Zystitis, CRPS (komplexes regionales Schmerzsyndrom), Trigeminusneuralgie, Prostatitis, Schmerz verursacht durch Rückenmarksverletzungen, entzündungsinduzierter Schmerz, Kreuzschmerzen, Krebsschmerzen, Chemotherapie-assoziierter Schmerz, HIV behandlungs-induzierte Neuropathie, Verbrennungs-induzierter Schmerz und chronischer Schmerz zu nennen (Wolf, Livshits, et al., Brain, Behavior, and Immunity, 2008; Kim, Lee, et al., Toll-like Receptors: Roles in Infection and Neuropathology, 2009; del Rey, Apkarian, et al., Annals of the New York Academy of Sciences, 2012; Guerrero, Cunha, et al., European Journal of Pharmacology, 2012; Kwok, Hutchinson, et al., PLoS ONE, 2012; Nicotra, Loram, et al., Experimental Neurology, 2012; Chopra and Cooper, J Neuroimmune Pharmacol, 2013; David, Ratnayake, et al., Neurobiology of Disease, 2013; Han, Zhao, et al., Neuroscience, 2013; Liu and Ji, Pflugers Arch., 2013; Stokes, Cheung, et al., Journal of Neuroinflammation, 2013; Zhao, Zhang, et al., Neuroscience, 2013; Liu, Zhang, et al., Cell Research, 2014; Park, Stokes, et al., Cancer Chemother Pharmacol, 2014; Van der Watt, Wilkinson, et al., BMC Infect Dis, 2014; Won, K. A., M. J. Kim, et al., J Pain, 2014; Min, Ahmad, et al., Photochem Photobiol., 2015; Schrepf, Bradley, et al., Brain Behav Immun, 2015; Wong, L., J. D. Done, et al., Prostate, 2015).
Dies gilt auch für einige onkologische Erkrankungen. Bestimmte Lymphome, wie beispielsweise ABC-DLBCL (Aktivierte B Zellen-Diffuses großzelliges B-Zell-Lymphom), Mantelzelllymphon und Morbus Waldenström als auch chronisch lymphatische Leukämie, Melanoma, Pankreastumor und Leberzellkarzinom sind durch Mutationen in MyD88 oder Veränderungen in der MyD88-Aktivität charakterisiert, die durch einen IRAK4 Inhibitor behandelt werden können (Ngo, Young, et al., Nature, 2011; Puente, Pinyol, et al., Nature, 2011; Ochi, Nguyen, et al., J Exp Med, 2012; Srivastava, Geng, et al., Cancer Research, 2012; Treon, Xu, et al., New England Journal of Medicine, 2012; Choi, Kim, et al., Human Pathology, 2013; (Liang, Chen, et al., Clinical Cancer Research, 2013). Des Weiteren spielt MyD88 eine wichtige Rolle in Ras-abhängigen Tumoren, so dass IRAK4 Inhibitoren auch zu deren Behandlung geeignet sind (Kfoury, A., K. L. Corf, et al., Journal of the National Cancer Institute, 2013). Es ist außerdem von einer therapeutischen Wirkung bei Brustkrebs, Ovarialkarzinom, Kolorektales Karzinom, Kopf-Hals-Karzinom, Lungenkrebs, Prostatakrebs durch die Inhibierung von IRAK4 auszugehen, da die genannten Indikationen mit dem Signalweg assoziiert sind (Szczepanski, Czystowska, et al., Cancer Res, 2009; Zhang, He, et al., Mol Biol Rep, 2009; Wang, Qian, et al., Br J Cancer Kim, 2010; Jo, et al., World J Surg Oncol, 2012; Zhao, Zhang, et al.; Front Immunol, 2014; Chen, Zhao, et al., Int J Clin Exp Pathol, 2015). Inflammatorische Erkrankungen wie CAPS (Cryopyrin-assoziierte periodische Syndrome), inklusive FCAS (familiäre Kälteurtikaria), MWS (Muckle-Wells-Syndrom), NOMID- (neonatalonset multisystem inflammatory disease) und CONCA- (chronic infantile, neurological, cutaneous, and articular) Syndrom; FMF (familiäres Mittelmeerfieber), HIDS (Hyper-IgD-Syndrom), TRAPS (Tumornekrosefaktor-Rezeptor 1-assoziiertes periodisches Syndrom), juvenile idiopathische Arthritis, Adult Morbus Still-Krankheit, Morbus Adamantiades-Behçet, rheumatoide Arthritis, Osteoarthritis, Keratoconjunctivitis sicca, PAPA-Syndrom (Pyogene Arthritis, Pyoderma gangraenosum und Akne), Schnitzler Syndrom und Sjögren Syndrom werden durch die Blockierung des IL-1 Signalweges behandelt, so dass auch hier ein IRAK4 Inhibitor zur Behandlung der genannten Krankheiten geeignet ist (Narayanan, Corrales, et al., Cornea, 2008; Brenner, Ruzicka, et al., British Journal of Dermatology, 2009; Henderson and Goldbach-Mansky, Clinical Immunology, 2010; Dinarello, European Journal of Immunology, 2011; Gul, Tugal-Tutkun, et al., Ann Rheum Dis, 2012; Pettersson, Annals of MedicinePetterson, 2012; Ruperto, Brunner, et al., New England Journal of Medicine, 2012; Nordström, Knight, et al., The Journal of Rheumatology, 2012; Vijmasi, Chen, et al., Mol Vis, 2013; Yamada, Arakaki, et al., Opinion on Therapeutic Targets, 2013; de Koning, Clin Transl Allergy, 2014). Der Ligand des IL-33R, IL-33, ist insbesondere in der Pathogenese von akutem Nierenversagen involviert, so dass die Inhibition von IRAK4 zur Prophylaxe und/oder Behandlung ein geeigneter Therapieansatz ist (Akcay, Nguyen, et al., Journal of the American Society of Nephrology, 2011). Komponenten der IL-1 Rezeptorfamilie sind mit Myokardinfarkt, unterschiedlichen pulmonalen Erkrankungen wie Asthma, COPD, idiopathischer interstitieller Pneumonie, allergische Rhinitis, Lungenfibrose und akutem Atemnot-Syndrom (ARDS) assoziiert, so dass eine prophylaktische und/oder therapeutische Wirkung in den genannten Indikationen durch die Inhibierung von IRAK4 zu erwarten ist (Kang, Homer, et al., The Journal of Immunology, 2007; Imaoka, Hoshino, et al., European Respiratory Journal, 2008; Couillin, Vasseur, et al., The Journal of Immunology, 2009; Abbate, Kontos, et al., The American Journal of Cardiology, 2010; Lloyd, Current Opinion in Immunology, 2010; Pauwels, Bracke, et al., European Respiratory Journal, 2011; Haenuki, Matsushita, et al., Journal of Allergy and Clinical Immunology, 2012; Yin, Li, et al., Clinical & Experimental Immunology, 2012; Abbate, Van Tassell, et al., The American Journal of Cardiology, 2013; Alexander-Brett, et al., The Journal of Clinical Investigation, 2013; Bunting, Shadie, et al., BioMed Research International, 2013; Byers, Alexander-Brett, et al., The Journal of Clinical Investigation, 2013; Kawayama, Okamoto, et al., J Interferon Cytokine Res, 2013; Martínez-González, Roca, et al., American Journal of Respiratory Cell and Molecular Biology, 2013; Nakanishi, Yamaguchi, et al., PLoS ONE, 2013; Qiu, Li, et al., Immunology, 2013; Li, Guabiraba, et al., Journal of Allergy and Clinical Immunology, 2014; Saluja, Ketelaar, et al., Molecular Immunology, 2014; Lugrin, Parapanov, et al., The Journal of Immunology, 2015).

Aus dem Stand der Technik sind eine Vielzahl von IRAK4 Inhibitoren bekannt (siehe beispielsweise Annual Reports in Medicinal Chemistry (2014), 49, 117-133).

US8293923 und US20130274241 offenbaren IRAK4 Inhibitoren mit einer an Position 3 substituierten Indazolstruktur. 2-substituierte Indazole werden nicht beschrieben.

In WO2013106254 und WO2011153588 werden 2,3-disubstituierte Indazolderivate offenbart.
In WO2007091107 werden 2-substituierte Indazolderivate für die Behandlung der Duchenne-Muskeldystrophie beschrieben. 5,6-Disubstituierte Indazolderivate werden in WO2007091107 nicht offenbart.

In WO2009024341 werden Indazole als Insektizide beschrieben, die einen zusätzlichen Substituenten an Position 7 aufweisen.

In WO2013042137 werden Benzothiazole, Benzoxazole und Benzimidazole als IRAK4 Inhibitoren beschrieben, die jeweils an der Position 2 einen Morpholinrest aufweisen, jedoch keinen über ein Kohlenstoff an die 2-Position verknüpften cylischen Substituenten. In WO2013042137 werden keine Indazole beschrieben.

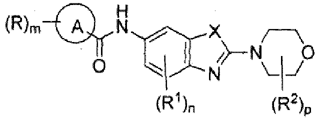

In WO2015104688 wird über weitere bicyclische IRAK4 Inhibitoren mit einem kondensierten 6-gliedrigen und 5-gliedrigen bicylischen heteroaromatischen Ringsystem berichtet. Diese Inhibitoren sind substituiert am 5-gliedrigen heteroaromatischen Ring mit einem gesättigten Stickstoff-haltigen Heterocyclylring, der über das Stickstoffatom an den Bicyclus verknüpft vorliegt. Indazole als Bicyclus werden nicht beschrieben.

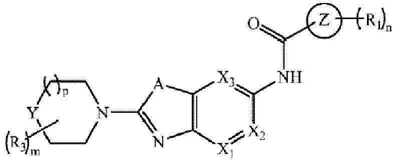

WO2015091426 beschreibt Indazole, die an der Position 2 mit einer Carboxamidseitenkette substituiert sind.

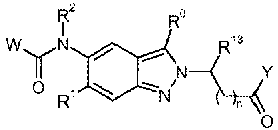

Verbindungen mit cylischen Substituenten, die direkt an die 2-Position des Indazols verknüpft sind, werden nicht offenbart.

In WO2015104662 werden 2-substituierte Indazole der folgenden allgemeinen Formel 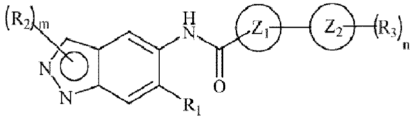 offenbart, in denen R² eine Alkyl- oder Cycloalkylgruppe ist. Explizit beschrieben werden Indazole mit einer Methyl, 2-Methoxyethyl und Cyclopentylgruppe an der 2-Position (Beispiele 1, 4, 7 und 76). 2-substituierte Indazole, die einen heterocyclischen gesättigten Ring verknüpft über einen Kohlenstoffatom an Position 2 des Indazols aufweisen, werden in WO2015104662 nicht beschrieben.

In WO2015193846 werden 2-substituierte Indazole der folgenden allgemeinen Formel 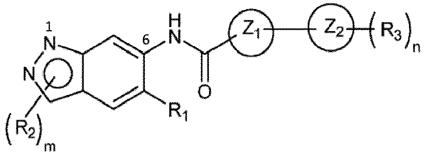 offenbart, in denen Z¹ und Z² jeweils eine gegebenenfalls substituierte Cycloalkyl-, Aryl- oder Heteroarylgruppe sind. R² kann ein Wasserstoff, Halogen, eine Aminogruppe, eine jeweils gegebenenfalls substitutierte Alkyl-, Cycloalkyl-, Aryl-, Heterocyclo-, Arylalkyl- oder Heterocycloalkylgruppe bedeuten. Explizit beschrieben sind Indazolderivate, in denen R² ein Methylrest sowie Z¹ und/ oder Z² Hetarylgruppen bedeuten, wobei der Substituent -NH(C=O)Z¹-Z²-(R³)ₙ an der 6-Position des Indazolgerüstes gebunden ist. Indazolderivate mit einem Substituenten -NH(C=O)Z¹-Z²-(R³)ₙ gebunden an die 5-Postion werden nicht beschrieben. Indazolderivate mit einem gesättigten, über ein Kohlenstoffatom verknüpften Heterocyclus an Postion 2 werden in WO2015193846 nicht beschrieben. Die Aufgabe der vorliegenden Erfindung besteht darin, neue Verbindungen zur Verfügung zu stellen, die als Inhibitoren der Interleukin-1 Receptor Associated Kinase-4 (IRAK4) wirken.

Gegenstand der vorliegenden Erfindung sind Verbindungen der allgemeinen Formel (I), 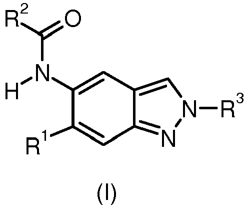 worin
R¹ für Halogen, Cyano, C(=O)OH, C(=O)OR^{a}, C(=O)NH₂, C(=O)N(H)R^{a}, C(=O)N(R^{a})R^{b}, C(=O)R^{d}, Hydroxy oder C₁-C₆-Alkyl steht, wobei der C₁-C₆-Alkylrest gegebenenfalls ein- oder mehrfach, gleich oder verschieden substituiert sein kann mit
   Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- bis sechsfach mit Fluor substituiertem C₁-C₆-Alkoxy oder C₃-C₇-Cycloalkoxy, einem gegebenenfalls ein- bis dreifach, gleich oder verschieden mit R^{c} substituiertem 4- bis 7-gliedrigem Heterocycloalkyl,
   oder für C₁-C₆-Alkoxy steht, wobei der C₁-C₆-Alkoxyrest gegebenenfalls ein- oder mehrfach, gleich oder verschieden substituiert sein kann mit
   Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- bis vierfach mit Fluor substituiertem C₃-C₇-Cycloalkyl, einem gegebenenfalls ein- bis fünffach mit Fluor substituiertem C₁-C₆-Alkoxy, einem gegebenenfalls ein- bis vierfach mit Fluor substituiertem C₃-C₇-Cycloalkoxy, einem gegebenenfalls ein- oder mehrfach, gleich oder verschieden mit R^{c} substituiertem 4- bis 7-gliedrigem Heterocycloalkyl,
   oder für C₃-C₇-Cycloalkyloxy oder 4- bis 7-gliedriges Heterocycloalkyloxy steht, worin C₃-C₇-Cycloalkyloxy und 4- bis 7-gliedriges Heterocycloalkyloxy gegebenenfalls ein- oder mehrfach, gleich oder verschieden substituiert sein können mit Hydroxy, Fluor, Cyano, C₁-C₆-Alkyl oder C₁-C₆-Alkoxy;
R^{a} für C₁-C₆-Alkyl, C₃-C₇-Cycloalkyl, 4- bis 7-gliedriges Heterocycloalkyl steht, worin C₁-C₆-Alkyl, C₃-C₇-Cycloalkyl und 4- bis 7-gliedriges Heterocycloalkyl gegebenenfalls ein- oder mehrfach, gleich oder verschieden substituiert sein können mit Fluor, Hydroxy, Cyano, C₁-C₄-Alkyl, C₁-C₄-Alkoxy oder C₃-C₇-Cycloalkyl;
R^{b} für C₁-C₆-Alkyl oder C₃-C₇-Cycloalkyl steht;
oder R^{a} und R^{b} gemeinsam mit dem Stickstoffatom einen 5- oder 6-gliedrigen Heterocyclus bilden, welcher gegebenenfalls ein- oder zweifach, gleich oder verschieden substituiert sein kann mit Hydroxy, Halogen, Cyano oder C₁-C₆-Alkyl;
R^{c} für Hydroxy, Fluor, Chlor, Cyano, C₁-C₄-Alkyl oder C₁-C₄-Alkoxy steht;
R^{d} für Wasserstoff, C₃-C₇-Cycloalkyl oder für C₁-C₆-Alkyl steht, welches gegebenenfalls mit einer Hydroxygruppe substituiert sein kann;
R² für 5-gliedriges Heteroaryl steht, welches einfach mit R⁴ und einfach mit R⁵ substituiert ist oder
R² für 6-gliedriges Heteroaryl steht, welches einfach mit R⁴ und ein- oder zweifach, gleich oder verschieden mit R⁵ substituiert ist;
R³ für eine Gruppe steht, ausgewählt aus: 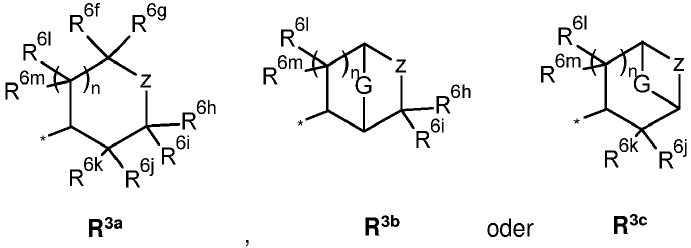
wobei * für die Verknüpfungsstelle der Gruppe mit dem Rest des Moleküls steht;
R⁴ für Wasserstoff, Halogen, Hydroxy, C(=O)OH, Cyano, NH₂, NHR^{a}, N(R^{a})R^{b}, C(=O)R^{a}, N(H)C(=O)R^{a}, C(=O)NH₂, C(=O)N(H)R^{a}, C(=O)N(R^{a})R^{b}, S(=O)R^{a}, S(=O)₂R^{a}, S(=O)₂NH₂, S(=O)₂NHR^{a} oder S(=O)₂N(R^{a})R^{b} steht,
   oder für C₁-C₆-Alkyl steht, wobei
   C₁-C₆-Alkyl gegebenenfalls mit ein bis fünf Fluoratomen substituiert sein kann und gegebenfalls ein- oder zweifach, gleich oder verschieden substituiert sein kann mit Hydroxy, Brom, Chlor, Cyano, C(=O)OH, S(=O)₂-C₁-C₆-Alkyl, NH₂, NHR^{a}, N(R^{a})R^{b}, C₃-C₇-Cycloalkyl, C₁-C₄-Alkoxy, C₃-C₇-Cycloalkoxy, Trifluormethoxy,
   oder für C₁-C₆-Alkoxy steht, wobei
   C₁-C₆-Alkoxy gegebenenfalls mit ein bis fünf Fluoratomen substituiert sein kann und gegebenenfalls ein- oder zweifach, gleich oder verschieden substituiert sein kann mit Hydroxy, Chlor, Brom, Cyano, C(=O)OH, S(=O)₂-C₁-C₆-Alkyl, NH₂, NHR^{a}, N(R^{a})R^{b}, C₃-C₇-Cycloalkyl, C₁-C₄-Alkoxy, C₃-C₇-Cycloalkoxy, Trifluormethoxy,
   oder für C₃-C₇-Cycloalkyl oder für C₃-C₇-Cycloalkyloxy steht, wobei
   C₃-C₇-Cycloalkyl und C₃-C₇-Cycloalkyloxy gegebenenfalls mit ein bis vier Fluoratomen substituiert sein können und gegebenenfalls ein- oder zweifach, gleich oder verschieden substituiert sein können mit Hydroxy, Chlor, Brom, Cyano, C(=O)R^{d}, C(=O)OH, C₁-C₆-Alkyl oder C₁-C₄-Alkoxy,
   oder für 4-7-gliedriges Heterocycloalkyl steht, welches gegebenenfalls mit ein bis vier Fluoratomen substituiert sein kann und gegebenenfalls ein- oder zweifach, gleich oder verschieden substituiert sein kann mit Hydroxy, Chlor, Brom, Cyano, NH₂, NHR^{a}, N(R^{a})R^{b}, C(=O)R^{d}, C(=O)OH, C₁-C₆-Alkyl, Trifluormethyl, 2,2,2-Trifluorethyl, Cyclopropyl, Cyclopropylmethyl oder C₁-C₄-Alkoxy,
   oder für Phenyl oder 5- oder 6-gliedriges Heteroaryl steht, worin
   Phenyl und 5- oder 6-gliedriges Heteroaryl gegebenenfalls ein- bis zweimal, gleich oder verschieden substituiert sein können mit Fluor, Chlor, Brom, Hydroxy, Cyano, C(=O)OH, S(=O)₂-C₁-C₄-Alkyl, NH2, NHR^{a}, N(R^{a})R^{b}, N(H)C(=O)R^{a}, C₁-C₄-Alkoxy, Trifluormethoxy oder C₁-C₄-Alkyl, wobei C₁-C₄-Alkyl gegebenenfalls ein- bis dreifach substituiert sein kann mit Fluor;
R⁵ für Wasserstoff, Halogen, Hydroxy, Cyano, C₁-C₄-Alkoxy, Trifluormethoxy oder C₁-C₆-Alkyl steht, worin C₁-C₆-Alkyl gegebenenfalls mit ein bis fünf Fluoratomen substituiert sein kann,
R^{6f} für Wasserstoff, Fluor, C(=O)OH, C(=O)NH₂, Trifluormethyl, Hydroxymethyl, Methoxymethyl, Cyano oder C₁-C₆-Alkyl steht;
R^{6g} für Wasserstoff, Fluor oder C₁-C₆-Alkyl steht, oder
R^{6f} und R^{6g} bilden gemeinsam mit dem Kohlenstoffatom, an welches sie gebunden sind, ein C₃-C₇-Cycloalkyl, oder
R^{6f} und R^{6g} stehen gemeinsam für eine Oxo-Gruppe;
R^{6h} für Wasserstoff, Trifluormethyl oder C₁-C₆-Alkyl steht;
R⁶ⁱ für Wasserstoff oder C₁-C₆-Alkyl steht, oder
R^{6h} und R⁶ⁱ stehen gemeinsam für eine Oxo-Gruppe;
R^{6j} für Wasserstoff, Fluor, NH₂, N(H)R^{a}, N(R^{a})R^{b}, C₁-C₆-Alkyl, Hydroxy, Cyano, C₁-C₄-Alkoxy, C(=O)OH, C(=O)NH₂, C(=O)N(H)R^{a}, C(=O)N(R^{a})R^{b}, Hydroxymethyl, Dimethylaminomethyl, Trifluormethyl steht;
R^{6k} für Wasserstoff, Fluor oder C₁-C₆-Alkyl steht, oder
R^{6j} und R^{6k} bilden gemeinsam mit dem Kohlenstoffatom ein C₃-C₇-Cycloalkyl;
R^{6l} für Wasserstoff oder Methyl steht;
R^{6m} für Wasserstoff oder Methyl steht;
G für -CH₂- oder -CH₂CH₂- steht;
n in der Formel R^{3a} für 0, 1 oder 2 steht,
n in der Formeln R^{3b} für 1 oder 2 steht,
n in der Formel R^{3c} für 0 oder 1 steht,
z für eine Gruppe steht ausgewählt aus NR⁷, O, S, S(=O), S(=O)₂, S(=O)(=NH);
R⁷ für Wasserstoff, C(=O)R^{e}, C(=O)OR^{a}, C(=O)NH₂, C(=O)N(H)R^{a}, C(=O)N(R^{a})R^{b}, S(=O)₂R^{a}, S(=O)₂NH₂, S(=O)₂N(R^{a})H, S(=O)₂N(R^{a})R^{b}, S(=O)₂NHC(=O)CH₃, S(=O)₂NHC(=O)CH₂CH₃ oder C₁-C₆-Alkyl steht, wobei
   C₁-C₆-Alkyl gegebenenfalls ein- bis fünfmal mit Fluoratomen substituiert sein kann und ein- bis zweifach, gleich oder verschieden substituiert sein kann mit Hydroxy, Chlor, Brom, Cyano, C(=O)R^{a}, C(=O)OH, C(=O)NH₂, C(=O)N(H)R^{a}, C(=O)N(R^{a})R^{b}, S(=O)₂-C₁-C₆-Alkyl, NH2, NHR^{a}, N(R^{a})R^{b}, Morpholin-4-yl, 4-Methylpiperazin-1-yl, C₃-C₇-Cycloalkyl, C₁-C₄-Alkoxy oder C₃-C₇-Cycloalkoxy;
   oder für C₃-C₇-Cycloalkyl, welches gegebenenfalls ein- bis viermal mit Fluoratomen substutiert sein kann und gegebenenfalls ein- bis zweifach, gleich oder verschieden substituiert sein kann mit Hydroxy, Methyl, Ethyl, Trifluormethyl oder Cyano;
   oder für ein über ein Kohlenstoffatom an den Rest des Moleküls gebundenes 4-7-gliedriges Heterocycloalkyl oder für 4-7-gliedriges Heterocycloalkyl-C₁-C₄-Alkyl steht, welche gegebenenfalls ein- bis sechsmal mit Fluoratomen substituiert sein können und ein- bis dreifach, gleich oder verschieden substituiert sein können mit Hydroxy, Chlor, Brom, Cyano, C₁-C₆-Alkyl, C₁-C₆-Alkoxy, Trifluormethyl, 2,2,2-Trifluorethyl, Trifluormethoxy, Cyclopropyl, Cyclopropylmethyl;
R^{e} für C₁-C₆-Alkyl steht, wobei C₁-C₆-Alkyl gegebenenfalls ein- bis dreifach, gleich oder verschieden substituiert sein kann mit Hydroxy, Fluor, Chlor, Cyano, C(=O)R^{a}, C(=O)OH, NH₂, NHR^{a}, N(R^{a})R^{b}, C₃-C₇-Cycloalkyl, C₁-C₄-Alkoxy, Trifluormethoxy oder C₃-C₇-Cycloalkoxy oder
R^{e} für C₃-C₇-Cycloalkyl steht, wobei C₃-C₇-Cycloalkyl gegebenfalls ein- bis vierfach substituiert sein kann mit Fluor und gegebenfalls einfach mit Hydroxy,
und ihre Diastereomere, Enantiomere, ihre Salze, ihre Solvate oder die Solvate ihrer Salze.

Eine Ausführungsform der Erfindung umfasst Verbindungen der allgemeinen Formel (I), 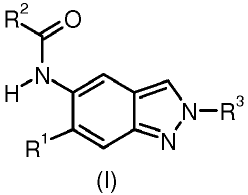 worin
R¹ für Halogen, Cyano, C(=O)OH, C(=O)OR^{a}, C(=O)NH₂, C(=O)N(H)R^{a}, C(=O)N(R^{a})R^{b}, C(=O)R^{d}, Hydroxy oder C₁-C₆-Alkyl steht, wobei der C₁-C₆-Alkylrest gegebenenfalls ein- oder mehrfach, gleich oder verschieden substituiert sein kann mit
   Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- bis sechsfach mit Fluor substituiertem C₁-C₆-Alkoxy oder C₃-C₇-Cycloalkoxy, einem gegebenenfalls ein- bis dreifach, gleich oder verschieden mit R^{c} substituiertem 4- bis 7-gliedrigem Heterocycloalkyl,
   oder für C₁-C₆-Alkoxy steht, wobei der C₁-C₆-Alkoxyrest gegebenenfalls ein- oder mehrfach, gleich oder verschieden substituiert sein kann mit
   Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- bis vierfach mit Fluor substituiertem C₃-C₇-Cycloalkyl, einem gegebenenfalls ein- bis fünffach mit Fluor substituiertem C₁-C₆-Alkoxy, einem gegebenenfalls ein- bis vierfach mit Fluor substituiertem C₃-C₇-Cycloalkoxy, einem gegebenenfalls ein- oder mehrfach, gleich oder verschieden mit R^{c} substituiertem 4- bis 7-gliedrigem Heterocycloalkyl,
   oder für C₃-C₇-Cycloalkyloxy oder 4- bis 7-gliedriges Heterocycloalkyloxy steht, worin C₃-C₇-Cycloalkyloxy und 4- bis 7-gliedriges Heterocycloalkyloxy gegebenenfalls ein- oder mehrfach, gleich oder verschieden substituiert sein können mit Hydroxy, Fluor, Cyano, C₁-C₆-Alkyl oder C₁-C₆-Alkoxy;
R^{a} für C₁-C₆-Alkyl, C₃-C₇-Cycloalkyl, 4- bis 7-gliedriges Heterocycloalkyl steht,
   worin C₁-C₆-Alkyl, C₃-C₇-Cycloalkyl und 4- bis 7-gliedriges Heterocycloalkyl gegebenenfalls ein- oder mehrfach, gleich oder verschieden substituiert sein können mit Fluor, Hydroxy, Cyano, C₁-C₄-Alkyl, C₁-C₄-Alkoxy oder C₃-C₇-Cycloalkyl;
R^{b} für C₁-C₆-Alkyl oder C₃-C₇-Cycloalkyl steht;
oder R^{a} und R^{b} gemeinsam mit dem Stickstoffatom einen 5- oder 6-gliedrigen Heterocyclus bilden, welcher gegebenenfalls ein- oder zweifach, gleich oder verschieden substituiert sein kann mit Hydroxy, Halogen, Cyano oder C₁-C₆-Alkyl;
R^{c} für Hydroxy, Fluor, Chlor, Cyano, C₁-C₄-Alkyl oder C₁-C₄-Alkoxy steht;
R^{d} für Wasserstoff, C₃-C₇-Cycloalkyl oder für C₁-C₆-Alkyl steht, welches gegebenenfalls mit einer Hydroxygruppe substituiert sein kann;
R² für 5-gliedriges Heteroaryl steht, welches einfach mit R⁴ und einfach mit R⁵ substituiert ist oder
R² für 6-gliedriges Heteroaryl steht, welches einfach mit R⁴ und ein- oder zweifach, gleich oder verschieden mit R⁵ substituiert ist;
R³ für eine Gruppe steht, ausgewählt aus: 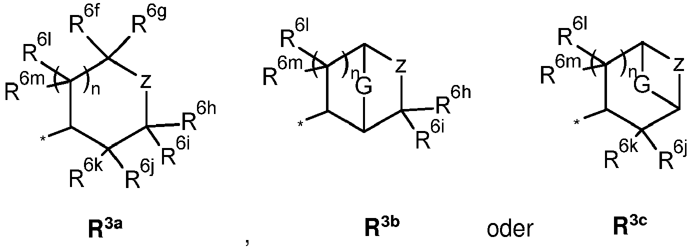
wobei * für die Verknüpfungsstelle der Gruppe mit dem Rest des Moleküls steht;
R⁴ für Wasserstoff, Halogen, Hydroxy, C(=O)OH, Cyano, NH₂, NHR^{a}, N(R^{a})R^{b}, C(=O)R^{a}, N(H)C(=O)R^{a}, C(=O)NH₂, C(=O)N(H)R^{a}, C(=O)N(R^{a})R^{b}, S(=O)R^{a}, S(=O)₂R^{a}, S(=O)₂NH₂, S(=O)₂NHR^{a} oder S(=O)₂N(R^{a})R^{b} steht,
   oder für C₁-C₆-Alkyl steht, wobei
   C₁-C₆-Alkyl gegebenenfalls mit ein bis fünf Fluoratomen substituiert sein kann und gegebenfalls ein- oder zweifach, gleich oder verschieden substituiert sein kann mit Hydroxy, Brom, Chlor, Cyano, C(=O)OH, S(=O)₂-C₁-C₆-Alkyl, NH₂, NHR^{a}, N(R^{a})R^{b}, C₃-C₇-Cycloalkyl, C₁-C₄-Alkoxy, C₃-C₇-Cycloalkoxy, Trifluormethoxy,
   oder für C₁-C₆-Alkoxy steht, wobei
   C₁-C₆-Alkoxy gegebenenfalls mit ein bis fünf Fluoratomen substituiert sein kann und gegebenenfalls ein- oder zweifach, gleich oder verschieden substituiert sein kann mit Hydroxy, Chlor, Brom, Cyano, C(=O)OH, S(=O)₂-C₁-C₆-Alkyl, NH₂, NHR^{a}, N(R^{a})R^{b}, C₃-C₇-Cycloalkyl, C₁-C₄-Alkoxy, C₃-C₇-Cycloalkoxy, Trifluormethoxy,
   oder für C₃-C₇-Cycloalkyl oder für C₃-C₇-Cycloalkyloxy steht, wobei
   C₃-C₇-Cycloalkyl und C₃-C₇-Cycloalkyloxy gegebenenfalls mit ein bis vier Fluoratomen substituiert sein können und gegebenenfalls ein- oder zweifach, gleich oder verschieden substituiert sein können mit Hydroxy, Chlor, Brom, Cyano, C(=O)R^{d}, C(=O)OH, C₁-C₆-Alkyl oder C₁-C₄-Alkoxy,
   oder für 4-7-gliedriges Heterocycloalkyl steht, welches gegebenenfalls mit ein bis vier Fluoratomen substituiert sein kann und gegebenenfalls ein- oder zweifach, gleich oder verschieden substituiert sein kann mit Hydroxy, Chlor, Brom, Cyano, NH₂, NHR^{a}, N(R^{a})R^{b}, C(=O)R^{d}, C(=O)OH, C₁-C₆-Alkyl, Trifluormethyl, 2,2,2-Trifluorethyl, Cyclopropyl, Cyclopropylmethyl oder C₁-C₄-Alkoxy,
   oder für Phenyl oder 5- oder 6-gliedriges Heteroaryl steht, worin
   Phenyl und 5- oder 6-gliedriges Heteroaryl gegebenenfalls ein- bis zweimal, gleich oder verschieden substituiert sein können mit Fluor, Chlor, Brom, Hydroxy, Cyano, C(=O)OH, S(=O)₂-C₁-C₄-Alkyl, NH2, NHR^{a}, N(R^{a})R^{b}, N(H)C(=O)R^{a}, C₁-C₄-Alkoxy, Trifluormethoxy oder C₁-C₄-Alkyl, wobei C₁-C₄-Alkyl gegebenenfalls ein- bis dreifach substituiert sein kann mit Fluor;
R⁵ für Wasserstoff, Halogen, Hydroxy, Cyano, C₁-C₄-Alkoxy, Trifluormethoxy oder C₁-C₆-Alkyl steht, worin C₁-C₆-Alkyl gegebenenfalls mit ein bis fünf Fluoratomen substituiert sein kann,
R^{6f} für Wasserstoff, Fluor, C(=O)OH, C(=O)NH₂, Trifluormethyl, Hydroxymethyl, Methoxymethyl, Cyano oder C₁-C₆-Alkyl steht;
R^{6g} für Wasserstoff, Fluor oder C₁-C₆-Alkyl steht, oder
R^{6f} und R^{6g} bilden gemeinsam mit dem Kohlenstoffatom, an welches sie gebunden sind, ein C₃-C₇-Cycloalkyl, oder
R^{6f} und R^{6g} stehen gemeinsam für eine Oxo-Gruppe;
R^{6h} für Wasserstoff, Trifluormethyl oder C₁-C₆-Alkyl steht;
R⁶ⁱ für Wasserstoff oder C₁-C₆-Alkyl steht, oder
R^{6h} und R⁶ⁱ stehen gemeinsam für eine Oxo-Gruppe;
R^{6j} für Wasserstoff, Fluor, NH₂, N(H)R^{a}, N(R^{a})R^{b}, C₁-C₆-Alkyl, Hydroxy, Cyano, C₁-C₄-Alkoxy, C(=O)OH, C(=O)NH₂, C(=O)N(H)R^{a}, C(=O)N(R^{a})R^{b}, Hydroxymethyl, Dimethylaminomethyl, Trifluormethyl steht;
R^{6k} für Wasserstoff, Fluor oder C₁-C₆-Alkyl steht, oder
R^{6j} und R^{6k} bilden gemeinsam mit dem Kohlenstoffatom ein C₃-C₇-Cycloalkyl;
R^{6l} für Wasserstoff oder Methyl steht;
R^{6m} für Wasserstoff oder Methyl steht;
G für -CH₂- oder -CH₂CH₂- steht;
n in der Formel R^{3a} für 0, 1 oder 2 steht,
n in der Formeln R^{3b} für 1 oder 2 steht,
n in der Formel R^{3c} für 0 oder 1 steht,
z für eine Gruppe steht ausgewählt aus NR⁷, O, S, S(=O), S(=O)₂, S(=O)(=NH);
R⁷ für Wasserstoff, C(=O)R^{e}, C(=O)OR^{a}, C(=O)NH₂, C(=O)N(H)R^{a}, C(=O)N(R^{a})R^{b}, S(=O)₂R^{a}, S(=O)₂NH₂, S(=O)₂N(R^{a})H, S(=O)₂N(R^{a})R^{b}, S(=O)₂NHC(=O)CH₃, S(=O)₂NHC(=O)CH₂CH₃ oder C₁-C₆-Alkyl steht, wobei
   C₁-C₆-Alkyl gegebenenfalls ein- bis fünfmal mit Fluoratomen substituiert sein kann oder
   C₁-C₆-Alkyl gegebenenfalls ein- bis zweifach, gleich oder verschieden substituiert sein kann mit Hydroxy, Chlor, Brom, Cyano, C(=O)R^{a}, C(=O)OH, C(=O)NH₂, C(=O)N(H)R^{a}, C(=O)N(R^{a})R^{b}, S(=O)₂-C₁-C₆-Alkyl, NH₂, NHR^{a}, N(R^{a})R^{b}, Morpholin-4-yl, 4-Methylpiperazin-1-yl, C₃-C₇-Cycloalkyl, C₁-C₄-Alkoxy oder C₃-C₇-Cycloalkoxy oder
   C₁-C₆-Alkyl gegebenenfalls ein- bis fünfmal mit Fluoratomen substituiert sein kann und ein- bis zweifach, gleich oder verschieden substituiert sein kann mit Hydroxy, Chlor, Brom, Cyano, C(=O)R^{a}, C(=O)OH, C(=O)NH₂, C(=O)N(H)R^{a}, C(=O)N(R^{a})R^{b}, S(=O)₂-C₁-C₆-Alkyl, NH₂, NHR^{a}, N(R^{a})R^{b}, Morpholin-4-yl, 4-Methylpiperazin-1-yl, C₃-C₇-Cycloalkyl, C₁-C₄-Alkoxy oder C₃-C₇-Cycloalkoxy;
   oder für C₃-C₇-Cycloalkyl, welches gegebenenfalls ein- bis viermal mit Fluoratomen substutiert sein kann und gegebenenfalls ein- bis zweifach, gleich oder verschieden substituiert sein kann mit Hydroxy, Methyl, Ethyl, Trifluormethyl oder Cyano;
   oder für ein über ein Kohlenstoffatom an den Rest des Moleküls gebundenes 4-7-gliedriges Heterocycloalkyl oder für 4-7-gliedriges Heterocycloalkyl-C₁-C₄-Alkyl steht, welche gegebenenfalls ein- bis sechsmal mit Fluoratomen substituiert sein können und ein- bis dreifach, gleich oder verschieden substituiert sein können mit Hydroxy, Chlor, Brom, Cyano, C₁-C₆-Alkyl, C₁-C₆-Alkoxy, Trifluormethyl, 2,2,2-Trifluorethyl, Trifluormethoxy, Cyclopropyl, Cyclopropylmethyl;
R^{e} für C₁-C₆-Alkyl steht, wobei C₁-C₆-Alkyl gegebenenfalls ein- bis dreifach, gleich oder verschieden substituiert sein kann mit Hydroxy, Fluor, Chlor, Cyano, C(=O)R^{a}, C(=O)OH, NH₂, NHR^{a}, N(R^{a})R^{b}, C₃-C₇-Cycloalkyl, C₁-C₄-Alkoxy, Trifluormethoxy oder C₃-C₇-Cycloalkoxy oder
R^{e} für C₃-C₇-Cycloalkyl steht, wobei C₃-C₇-Cycloalkyl gegebenfalls ein- bis vierfach substituiert sein kann mit Fluor und gegebenfalls einfach mit Hydroxy,
und ihre Diastereomere, Enantiomere, ihre Salze, ihre Solvate oder die Solvate ihrer Salze.

Die neuen IRAK4 Inhibitoren sind insbesondere zur Behandlung und zur Prävention von proliferativen, metabolischen und entzündlichen Erkrankungen geeignet, die durch ein überreagierendes Immunsystem charakterisiert sind. Besonders genannt seien hier entzündliche Hauterkrankungen, Herz-Kreislauf-Erkrankungen, Lungenerkrankungen, Augenerkrankungen, neurologische Erkrankungen, Schmerzerkrankungen und Krebserkrankungen.

Des Weiteren sind die neuen IRAK4 Inhibitoren geeignet zur Behandlung und Prävention
- von Autoimmun- und inflammatorischen Erkrankungen, insbesondere Rheumatoide Arthritis, Multiple Sklerose, Systemischer Lupus Erythematodes, Spondyloarthritiden und Gicht,
- von Stoffwechselerkrankungen, insbesondere Lebererkrankungen wie Fettleber sowie
- von gynäkologischen Erkrankungen, insbesondere von Endometriose sowie von Endometriose-assoziierten Schmerzen und anderen Endometriose-assoziierten Symptomen wie Dysmenorrhoe, Dyspareunie, Dysurie und Dyschezie.

Wenn bei den im Folgenden beschriebenen Syntheseintermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.
Sinngemäß gleiches gilt für den Fall, dass Syntheseintermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung nicht bekannt ist.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.
Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind, aber beispielsweise für die Isolierung oder Reinigung der erfindungsgemäßen Verbindungen verwendet werden können.
Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Procain, Dibenzylamin, N-Methylmorpholin, Arginin, Lysin, Ethylendiamin und N-Methylpiperidin.
Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.
Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie 2H (Deuterium), 3H (Tritium), 13C, 14C, 15N, 17O, 18O, 32P, 33P, 33S, 34S, 35S, 36S, 18F, 36Cl, 82Br, 123I, 124I, 129I und 131I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoffverteilung im Körper; aufgrund der vergleichsweise leichten Herstell- und Detektierbarkeit sind hierfür insbesondere mit 3H- oder 14C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den für die Ausführungsbeispiele angegebenen Vorschriften, indem entsprechende isotopische Modifikationen der jeweiligen Reagentien und/oder Ausgangsverbindungen eingesetzt werden.
Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Im Rahmen der vorliegenden Erfindung haben die Substituenten, soweit nicht anders spezifiziert, die folgenden Bedeutungen:
Alkyl steht im Rahmen der Erfindung für einen linearen oder verzweigten Alkylrest mit der jeweils angegebenen Anzahl an Kohlenstoffatomen. Beispielhaft seien Methyl, Ethyl, n-Propyl, Isopropyl, n-Butyl, iso-Butyl, 1-Methylpropyl, 2-Methylpropyl, tert.-Butyl, n-Pentyl, 1-Ethylpropyl, 1-Methylbutyl, 2-Methylbutyl, 3-Methylbutyl, 2,2-Dimethylpropyl, n-Hexyl, 1-Methylpentyl, 2-Methylpentyl, 3-Methylpentyl, 4-Methylpentyl, 1-Ethylbutyl und 2-Ethylbutyl genannt. Bevorzugt sind Methyl, Ethyl, n-Propyl, Isopropyl, n-Butyl, tert.-Butyl, 2-Methylbutyl, 3-Methylbutyl und 2,2-Dimethylpropyl. Besonders bevorzugt sind Methyl, Ethyl und Isopropyl.
Cycloalkyl steht im Rahmen der Erfindung für einen monocyclischen, gesättigten Alkylrest mit der jeweils angegebenen Anzahl an Kohlenstoffatomen. Beispielhaft seien Cyclopropyl, Cyclobutyl, Cyclopentyl, Cyclohexyl und Cycloheptyl genannt. Bevorzugt sind Cyclopropyl, Cyclobutyl, Cyclopentyl und Cyclohexyl. Besonders bevorzugt ist Cyclopropyl.
Alkoxy steht im Rahmen der Erfindung für einen linearen oder verzweigten Alkoxyrest mit der jeweils angegebenen Anzahl an Kohlenstoffatomen. 1 bis 6 Kohlenstoffatome sind bevorzugt. Beispielhaft seien Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy genannt. Besonders bevorzugt ist ein linearer oder verzweigter Alkoxyrest mit 1 bis 4 Kohlenstoffatomen. Beispielhaft und vorzugsweise seien Methoxy, Ethoxy, n-Propoxy, 1-Methylpropoxy, n-Butoxy und iso-Butoxy genannt. Ganz besonders bevorzugt sind Methoxy und Ethoxy.
Halogen steht im Rahmen der Erfindung für Fluor, Chlor, Brom und Iod. Bevorzugt sind Fluor und Chlor. Besonders bevorzugt ist Fluor.
Hydroxy steht im Rahmen der Erfindung für OH.

### Heterocycloalkyl

Der Begriff "4- bis 7-gliedriges Heterocycloalkyl" bezieht sich auf einen monocyclischen gesättigten Heterocyclus mit insgesamt 4 bis 7 Ringatomen, bei der ein oder zwei Ringkohlenstoffatome durch gleiche oder verschiedene Heteroatome aus der Gruppe N, O und S ersetzt werden; die Heterocycloalkylgruppe kann über ein beliebiges der Kohlenstoffatome oder, falls vorhanden, ein Stickstoffatom an den Rest des Moleküls gebunden sein.

Bei der Heterocycloalkylgruppe kann es sich, ohne dass dies eine Einschränkung darstellen soll, zum Beispiel um einen 4-gliedrigen Ring wie Azetidinyl, Oxetanyl oder Thietanyl; oder einen 5-gliedrigen Ring wie Tetrahydrofuranyl, 1,3-Dioxolanyl, Thiolanyl, Pyrrolidinyl, Imidazolidinyl, Pyrazolidinyl, 1,1-Dioxidothiolanyl, 1,2-Oxazolidinyl, 1,3-Oxazolidinyl oder 1,3-Thiazolidinyl; oder einen 6- gliedrigen Ring wie Tetrahydropyranyl, Tetrahydrothiopyranyl, Piperidinyl, Morpholinyl, Dithianyl, Thiomorpholinyl, Piperazinyl, 1,3-Dioxanyl, 1,4-Dioxanyl oder 1,2-Oxazinanyl, oder einen 7- gliedrigen Ring wie Azepanyl, 1,4-Diazepanyl oder 1,4-Oxazepanyl handeln.

Bevorzugt ist 4- bis 6-gliedriges Heterocycloalkyl.

Besonders bevorzugt sind Oxetanyl, Azetidinyl, Pyrrolidinyl, Morpholinyl, Piperazinyl und Piperidinyl.

### Heterocycloalkyloxy

Der Begriff "4- bis 7-gliedrige Heterocycloalkyloxygruppe" bezieht sich auf eine monocyclische gesättigte Heterocycloalkylgruppe mit insgesamt 4 bis 7 Ringatomen, bei der ein oder zwei Ringkohlenstoffatome durch gleiche oder verschiedene Heteroatome aus der Gruppe N, O und S ersetzt werden. Die Heterocycloalkyloxygruppe kann über ein beliebiges Kohlenstoffatom an das Sauerstoffatom, welches die Heterocycloalkyloxygruppe mit dem Rest des Moleküls verknüpft, gebunden sein. Bevorzugt als Heteroatom im Ring ist ein Stickstoffatom oder ein Sauerstoffatom.
Bevorzugt sind 4- bis 6-gliedrige Heterocycloalkyloxygruppen. Beispielhaft genannt sind Oxetan-3-yloxy, Azetidin-3-yloxy, Tetrahydrofuran-3-yloxy, Tetrahydro-2H-pyran-4-yloxy und Piperidin-4-yloxy.

Bevorzugt sind Oxetan-3-yloxy und Tetrahydrofuran-3-yloxy.

### Heteroaryl

Der Begriff Heteroaryl ist als ein einwertiges monocyclisches aromatisches Ringsystem mit 5 oder 6 Ringatomen zu verstehen, das mindestens ein Ringheteroatom und gegebenenfalls ein, zwei oder drei weitere Ringheteroatome aus der Gruppe N, O und S enthält und das über ein Ringkohlenstoffatom oder gegebenenfalls (wenn es die Wertigkeit erlaubt) über ein Ringstickstoffatom an den Rest des Moleküls gebunden ist.

Für eine 5-gliedrige Heteroarylgruppe ("5-gliedriges Heteroaryl") seien zum Beispiel Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl oder Tetrazolyl genannt. Für eine 6-gliedrige Heteroarylgruppe ("6-gliedriges Heteroaryl") seien zum Beispiel Pyridinyl, Pyridazinyl, Pyrimidinyl, Pyrazinyl oder Triazinyl genannt.

Im Allgemeinen, und wenn nicht anders erwähnt, schließen die Heteroarylreste alle möglichen isomeren Formen davon ein, z. B. Tautomere und Positionsisomere in Bezug auf den Anbindungspunkt zum Rest des Moleküls. Beispielsweise schließt der Begriff Pyridinyl sowohl Pyridin-2-yl, Pyridin-3-yl als auch Pyridin-4-yl ein. Als weiteres erläuterndes Beispiel sei der Begriff Thiazolyl genannt, welcher sowohl 1,3-Thiazol-4-yl, 1,3-Thiazol-5-yl als auch 1,3-Thiazol-2-yl einschließt. Die genannten Beispiele sind zur Illustration der Definition angeführt und sollen keinesfalls als Limitierung auf die genannten Begriffe verstanden werden.

Bevorzugt als 5-gliedriges Heteroaryl sind Oxazolyl, Thiazolyl, und Pyrazolyl. Besonders bezorzugt sind Pyrazol-3-yl, 1,3-Thiazol-4-yl und 1,3-Thiazol-2-yl. Ganz besonders bevorzugt sind Pyrazol-3-yl und 1,3-Thiazol-4-yl.

Bevorzugt als 6-gliedriges Heteroaryl ist Pyridinyl. Besonders bevorzugt sind Pyridin-2-yl und Pyridin-4-yl. Ganz besonders bevorzugt ist Pyridin-2-yl.

Ein Symbol * an einer Bindung bedeutet die Verknüpfungsstelle im Molekül.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Wenn R⁷ für C₁-C₆-Alkyl steht, welches gegebenenfalls ein- bis fünfmal mit Fluoratomen substituiert sein kann und ein- bis zweifach, gleich oder verschieden substituiert sein kann mit Hydroxy, Chlor, Brom, Cyano, C(=O)R^{a}, C(=O)OH, C(=O)NH₂, C(=O)N(H)R^{a}, C(=O)N(R^{a})R^{b}, S(=O)₂-C₁-C₆-Alkyl, NH₂, NHR^{a}, N(R^{a})R^{b}, Morpholin-4-yl, 4-Methylpiperazin-1-yl, C₃-C₇-Cycloalkyl, C₁-C₄-Alkoxy oder C₃-C₇-Cycloalkoxy, dann ist die Substitution des C₁-C₆-Alkyl folgendermaßen zu verstehen:
Wenn R⁷ für C₁-C₆-Alkyl steht, dann kann
   C₁-C₆-Alkyl entweder unsubstituiert oder ein- bis fünfmal mit Fluoratomen substituiert sein, oder
   C₁-C₆-Alkyl kann unsubstituiert oder ein- bis zweifach, gleich oder verschieden mit Hydroxy, Chlor, Brom, Cyano, C(=O)R^{a}, C(=O)OH, C(=O)NH₂, C(=O)N(H)R^{a}, C(=O)N(R^{a})R^{b}, S(=O)₂-C₁-C₆-Alkyl, NH₂, NHR^{a}, N(R^{a})R^{b}, Morpholin-4-yl, 4-Methylpiperazin-1-yl, C₃-C₇-Cycloalkyl, C₁-C₄-Alkoxy oder C₃-C₇-Cycloalkoxy substituiert sein, oder
   C₁-C₆-Alkyl kann unsubstituiert oder ein- bis fünfmal mit Fluoratomen und ein- bis zweifach, gleich oder verschieden mit Hydroxy, Chlor, Brom, Cyano, C(=O)R^{a}, C(=O)OH, C(=O)NH₂, C(=O)N(H)R^{a}, C(=O)N(R^{a})R^{b}, S(=O)₂-C₁-C₆-Alkyl, NH₂, NHR^{a}, N(R^{a})R^{b}, Morpholin-4-yl, 4-Methylpiperazin-1-yl, C₃-C₇-Cycloalkyl, C₁-C₄-Alkoxy oder C₃-C₇-Cycloalkoxy substituiert sein.
Bevorzugte Ausführungsformen der vorliegenden Erfindung sind Verbindungen der Formel (I), in denen
R¹ ein mit einer Hydroxygruppe substituierter C₁-C₃-Alkylrest ist. Besonders bevorzugt sind Hydroxymethyl, 1-Hydroxyethyl und 2-Hydroxypropan-2-yl. Ganz besonders bevorzugt ist 2-Hydroxypropan-2-yl.

Weitere bevorzugte Ausführungsformen der vorliegenden Erfindung sind Verbindungen, in denen R¹ ein C₁-C₆-Alkoxyrest ist, welcher gegebenenfalls mit C₃-C₇-Cycloalkyl substituiert sein kann. Hierbei sind ein C₁-C₄-Alkoxyrest oder ein Cyclopropylmethoxyrest bevorzugt. Besonders bevorzugt sind Cyclopropylmethoxy, Ethoxy und Methoxy. Ganz besonders bevorzugt sind Cyclopropylmethoxy und Methoxy.

In weiteren bevorzugten Ausführungsformen der vorliegenden Erfindung ist R¹ ein 2,2,2-Trifluorethoxyrest oder 2,2-Difluorethoxyrest.

In weiteren bevorzugten Ausführungsformen der vorliegenden Erfindung ist R¹ C(=O)NH₂.

In weiteren bevorzugten Ausführungsformen der vorliegenden Erfindung ist R¹ C(=O)OH.

In weiteren bevorzugten Ausführungsformen der vorliegenden Erfindung ist R¹ eine C(=O)(C₁-C₄-Alkyl)-Gruppe. Besonders bevorzugt ist C(=O)CH₃.

Bevorzugte Ausführungsformen der vorliegenden Erfindung sind Verbindungen, in denen R² ein Pyridin-2-ylrest ist, der an der 6-Position mit C₁-C₆-Alkyl subsituiert ist, wobei C₁-C₆-Alkyl gegebenfalls mit bis zu 5 Fluoratomen substituiert sein kann. Alternativ steht R² für einen Pyridin-2-ylrest, der an der 6-Position mit Cyano, Chlor, Cyclopropyl, Cyclopropylmethyl, NH₂, NH(C₁-C₄-Alkyl), N(C₁-C₄-Alkyl)₂, C₁-C₄-Alkoxy, 2,2,2-Trifluorethoxy, 2-Hydroxypropan-2-yl, Morpholin-4-yl, 4-Methylpiperazin-1-yl oder Piperazin-1-yl substituiert ist.
Besonders bevorzugt ist für R² ein Pyridin-2-ylrest, der an der 6-Position mit Trifluormethyl, Difluormethyl, Methyl, 2,2,2-Trifluorethyl, 1,1-Difluorethyl, Ethyl, Isopropyl, tert-Butyl, Cyano, Chlor, Cyclopropyl, Cyclopropylmethyl, NH₂, NH(C₁-C₄-Alkyl), N(C₁-C₄-Alkyl)₂, C₁-C₄-Alkoxy, 2,2,2-Trifluorethoxy, 2-Hydroxypropan-2-yl, Morpholin-4-yl, 4-Methylpiperazin-1-yl oder Piperazin-1-yl substituiert ist. Ganz besonders bevorzugt steht R² für 6-(Trifluormethyl)pyridin-2-yl, 6-(Difluormethyl)pyridin-2-yl, 6-(1,1-Difluorethyl)pyridin-2-yl, 6-Amino-pyridin-2-yl und 6-(2-Hydroxypropan-2-yl)-pyridin-2-yl. Davon bevorzugt ist 6-(Trifluormethyl)pyridin-2-yl.

Außerdem ist für R² ein gegebenenfalls substituierter 1,3-Thiazolrest bevorzugt, insbesondere ein gegebenenfalls substituiertes 1,3-Thiazol-2-yl oder 1,3-Thiazol-4-yl. Besonders bevorzugt sind 4-Cyclopropyl-1,3-thiazol-2-yl und 1,3-Thiazol-2-yl, wobei der 1,3-Thiazol-2-ylrest an der 4-Position mit C₁-C₆-Alkyl substituiert ist und der C₁-C₆-Alkylrest gegebenenfalls mit 1 bis 5 Fluoratomen substituiert sein kann.
Besonders bevorzugt ist zusätzlich 2-Cyclopropyl-1,3-thiazol-4-yl und 1,3-Thiazol-4-yl, wobei der 1,3-Thiazol-4-ylrest an der 2-Position mit C₁-C₆-Alkyl substituiert ist und der C₁-C₆-Alkylrest gegebenenfalls mit 1 bis 5 Fluoratomen substituiert sein kann.
Insbesondere bevorzugt für R² sind 4-Methyl-1,3-thiazol-2-yl, 2-Methyl-1,3-thiazol-4-yl, 4-(Trifluormethyl)-1,3-thiazol-2-yl oder 2-(Trifluormethyl)-1,3-thiazol-4-yl. Ganz besonders bevorzugt ist 4-(Trifluormethyl)-1,3-thiazol-2-yl.

Eine weitere bevorzugte Ausführungsform für R² ist ein Pyrazolrest, insbesondere Pyrazol-3-yl. Besonders bevorzugt ist 1-(C₁-C₆-Alkyl)-1H-pyrazol-3-yl, wobei der C₁-C₆-Alkyl-Substituent 1 bis 5 Fluoratome enthalten kann. Außerdem besonders bevorzugt sind 1-Cyclopropyl-1H-pyrazol-3-yl und 1-Cyclopropylmethyl-1H-pyrazol-3-yl. Ganz besonders bevorzugt sind 1-Methyl-1H-pyrazol-3-yl, 1-Ethyl-1H-pyrazol-3-yl, 1-(2,2,2-Trifluorethyl)-1H-pyrazol-3-yl und 1-(Difluormethyl)-1H-pyrazol-3-yl. Hiervon ist 1-(Difluormethyl)-1H-pyrazol-3-yl besonders bevorzugt.

Bevorzugte Ausführungsformen für R³ sind Tetrahydro-2H-pyran-4-yl, 2,6-Dimethyltetrahydro-2H-pyran-4-yl, Tetrahydrofuran-3-yl, (3S)-Tetrahydrofuran-3-yl, (3R)-Tetrahydrofuran-3-yl und 5,5-Dimethyltetrahydrofuran-3-yl. Besonders bevorzugt sind Tetrahydro-2H-pyran-4-yl, Tetrahydrofuran-3-yl, (3S)-Tetrahydrofuran-3-yl und (3R)-Tetrahydrofuran-3-yl.

Weitere bevorzugte Ausführungsformen von R³ sind 1-Oxidotetrahydro-2H-thiopyran-4-yl, 1,1-Dioxidotetrahydro-2H-thiopyran-4-yl, 1-Oxidotetrahydrothiophen-3-yl, 1,1-Dioxidotetrahydrothiophen-3-yl, (3S)-1,1-Dioxidotetrahydrothiophen-3-yl und (3R)-1,1-Dioxidotetrahydrothiophen-3-yl. Besonders bevorzugt sind 1,1-Dioxidotetrahydro-2H-thiopyran-4-yl, 1,1-Dioxidotetrahydrothiophen-3-yl, (3S)-1,1-Dioxidotetrahydrothiophen-3-yl und (3R)-1,1-Dioxidotetrahydrothiophen-3-yl.

Weitere bevorzugte Ausführungsformen von R³ sind Tetrahydro-2H-thiopyran-4-yl und Tetrahydrothiophen-3-yl.

Im Rahmen einer weiteren bevorzugten Ausführungsform steht R³ für eine Gruppe 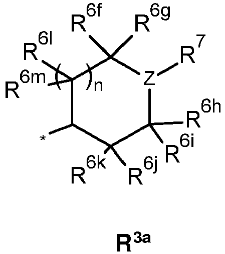 wobei
z Stickstoff bedeutet;
R^{6f} Wasserstoff oder Methyl, bevorzugt Wasserstoff bedeutet;
R^{6g} Wasserstoff oder Methyl, bevorzugt Wasserstoff bedeutet;
R^{6h} Wasserstoff oder Methyl, bevorzugt Wasserstoff bedeutet;
R⁶ⁱ Wasserstoff oder Methyl, bevorzugt Wasserstoff bedeutet;
R^{6j} Wasserstoff oder Methyl, bevorzugt Wasserstoff bedeutet;
R^{6k} Wasserstoff oder Methyl, bevorzugt Wasserstoff bedeutet;
R^{6l} Wasserstoff oder Methyl, bevorzugt Wasserstoff bedeutet;
R^{6m} Wasserstoff oder Methyl, bevorzugt Wasserstoff bedeutet;
R⁷ für Wasserstoff, C(=O)CH₂OH, C(=O)C(CH₃)₂OH, S(=O)₂NH₂, S(=O)₂NHC(=O)CH₃, C₁-C₄-Alkyl, 2,2,2-Trifluorethyl, Cyclopropyl, Cyclopropylmethyl, 2-(Dimethylamino)ethyl, 2-Aminoethyl, 2-(Diethylamino)ethyl, 2-(Methylamino)ethyl, 3-(Dimethylamino)propyl, 2-Hydroxyethyl, 3-Hydroxypropyl, 2-Hydroxypropyl, 3-Hydroxy-3-methylbutyl, 2-Methoxyethyl, Oxetan-3-yl, Oxetan-3-ylmethyl, Tetrahydrofuran-3-yl, 1-Methylpiperidin-4-yl, 1-Ethylpiperidin-4-yl, Pyrrolidin-3-yl, 1-Methylpyrrolidin-3yl, Azetidin-3-yl, 1-Methylazetidin-3-yl, Azetidin-3-yl, Azetidin-3-ylmethyl, 1-Methylazetidin-3-ylmethyl steht;
bevorzugt R⁷ für Wasserstoff, C(=O)CH₂OH, S(=O)₂NH₂, S(=O)₂NHC(=O)CH₃, Methyl, Ethyl, 2,2,2-Trifluorethyl, 2-(Dimethylamino)ethyl, 2-Hydroxyethyl, 3-Hydroxy-3-methylbutyl, Oxetan-3-yl, 1-Methylpiperidin-4-yl steht;
ganz besonders bevorzugt R⁷ für Wasserstoff, Methyl, 2,2,2-Trifluorethyl, 2-(Dimethylamino)ethyl, 2-Hydroxyethyl, 3-Hydroxy-3-methylbutyl, Oxetan-3-yl oder 1-Methylpiperidin-4-yl steht;
n die Bedeutung n = 0, 1 hat und bevorzugt n = 1 bedeutet.

Im Rahmen einer weiteren Ausführungsform steht R³ für eine Gruppe 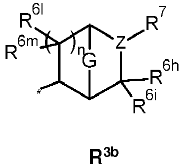 worin
z Stickstoff bedeutet;
R^{6h}, R⁶ⁱ, R^{6l} R^{6m} für Wasserstoff stehen;
G die Bedeutung -CH₂CH₂- oder -CH₂- hat und bevorzugt -CH₂- bedeutet;
n die Bedeutung n = 1, 2 hat und bevorzugt n =1 hat;
R⁷ für Wasserstoff, C(=O)CH₂OH, S(=O)₂NH₂, S(=O)₂NHC(=O)CH₃, Methyl, Ethyl, 2,2,2-Trifluorethyl, 2-(Dimethylamino)ethyl, 2-Hydroxyethyl, 3-Hydroxy-3-methylbutyl, Oxetan-3-yl, 1-Methylpiperidin-4-yl steht und bevorzugt für Wasserstoff oder Methyl steht.

Im Rahmen einer weiteren Ausführungsform steht R³ für eine Gruppe 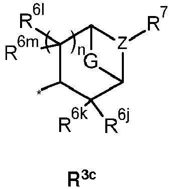 worin
z Stickstoff bedeutet;
R^{6j}, R^{6k}, R^{6l} R^{6m} für Wasserstoff stehen;
G die Bedeutung -CH₂CH₂- hat;
n die Bedeutung n = 1 hat;
R⁷ für Wasserstoff, C(=O)CH₂OH, S(=O)₂NH₂, S(=O)₂NHC(=O)CH₃, Methyl, Ethyl, 2,2,2-Trifluorethyl, 2-(Dimethylamino)ethyl, 2-Hydroxyethyl, 3-Hydroxy-3-methylbutyl, Oxetan-3-yl, 1-Methylpiperidin-4-yl und bevorzugt für Wasserstoff oder Methyl steht.

Bevorzugte Ausführungsformen für R⁴ sind Cyclopropyl, C₁-C₆-Alkyl und ein mit ein bis fünf Fluoratomen substituiertes C₁-C₆-Alkyl. Besonders bevorzugt sind Methyl, Ethyl, 2,2,2-Trifluorethyl, 1,1-Difluorethyl, Difluormethyl und Trifluormethyl. Ganz besonders bevorzugt ist Trifluormethyl.

Bevorzugte Ausführungsformen für R⁵ sind Wasserstoff, Fluor, Chlor, Cyano, Methoxy, C₁-C₆-Alkyl und mit ein bis fünf Fluoratomen substituiertes C₁-C₆-Alkyl. Besonders bevorzugt sind Wasserstoff, Fluor, Chlor, Cyano, Methyl, Ethyl und Trifluormethyl. Ganz besonders bevorzugt sind Wasserstoff, Fluor und Methyl. Insbesondere Wasserstoff ist bevorzugt.

Bevorzugte Ausführungsformen für R^{a} sind C₁-C₆-Alkyl, Cyclopropyl, Cyclopropylmethyl, Oxetan-3-yl, Azetidin-3-yl, 1-Methylazetidin-3-yl, Tetrahydrofuran-3-yl, Pyrrolidin-3-yl, 1-Methylpyrrolidin-3-yl, Piperidin-4-yl, 1-Methylpiperidin-4-yl, 2,2,2-Trifluorethyl, 2-Hydroxyethyl. Besonders bevorzugt ist C₁-C₄-Alkyl. Ganz besonders bevorzugt ist Methyl.

Bevorzugte Ausführungsformen für R^{b} sind C₁-C₄-Alkyl und Cyclopropyl. Besonders bevorzugt sind Methyl und Ethyl.

Bevorzugte Ausführungsformen für R^{c} sind Hydroxy, Fluor, Methyl, Ethyl, Methoxy und Ethoxy. Besonders bevorzugt sind Fluor und Methyl.

Bevorzugte Ausführungsformen für R^{d} sind Cyclopropyl und C₁-C₆-Alkyl, welches gegebenenfalls einfach oder zweifach mit Hydroxy substituiert sein kann. Besonders bevorzugt ist C₁-C₄-Alkyl, welches gegebenenfalls einfach mit Hydroxy substituiert sein kann. Ganz besonders bevorzugt steht R^{d} für Methyl oder Hydroxymethyl.

Eine bevorzugte Ausführungsform für R^{e} ist C₁-C₆-Alkyl, welches gegebenenfalls einfach oder zweifach mit Hydroxy substituiert sein kann. Besonders bevorzugt ist C₁-C₄-Alkyl einfach mit Hydroxy substituiert. Ganz besonders bevorzugt ist R^{e} Hydroxymethyl.

Weiterhin bevorzugt sind Verbindungen der Formel (I), in der
R¹ für Fluor, Chlor, Cyano, C(=O)OH, C(=O)OR^{a}, C(=O)NH₂, C(=O)N(H)R^{a}, C(=O)N(R^{a})R^{b}, Hydroxy oder C₁-C₆-Alkyl steht, wobei der C₁-C₆-Alkylrest gegebenenfalls substituiert sein kann mit Hydroxy
oder für C₁-C₆-Alkoxy steht, wobei der C₁-C₆-Alkoxyrest gegebenenfalls ein- bis dreifach mit Fluor und gegebenenfalls mit Hydroxy, einem gegebenenfalls ein- bis zweifach mit Fluor substituierten C₃-C₆-Cycloalkyl, mit einem gegebenenfalls ein- oder zweifach mit Fluor substituiertem Oxetan oder Tetrahydrofuran substituiert sein kann
oder für C₃-C₆-Cycloalkyloxy, Oxetan-3-yloxy oder für Tetrahydrofuran-3-yloxy steht;
R^{a} für C₁-C₆-Alkyl steht;
R^{b} für C₁-C₆-Alkyl steht;
R² für eine Gruppe steht, ausgewählt aus den folgenden allgemeinen Formeln II bis VII, IX und X, worin R² gegebenenfalls einfach mit R⁵ substituiert ist und 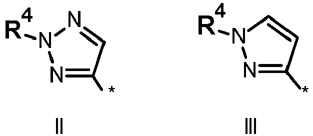 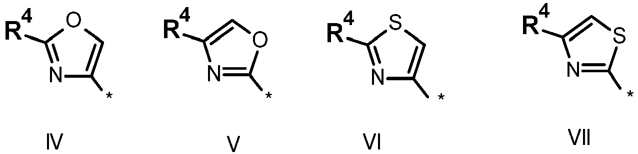 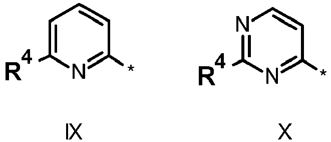 * für die Verknüpfungsstelle der Gruppe mit dem Rest des Moleküls steht und
R⁴ in den Formeln II und III
für Wasserstoff, C(=O)R^{a} oder C₁-C₆-Alkyl steht, wobei C₁-C₆-Alkyl gegebenenfalls einbis dreifach mit Fluor und gegebenenfalls einfach mit Hydroxy oder einfach mit Cyclopropyl substituiert sein kann,
oder für C₃-C₆-Cycloalkyl steht,
oder für Pyridinyl steht, welches gegebenenfalls ein- oder zweimal, gleich oder verschieden mit Fluor, Chlor oder C₁-C₄-Alkyl substituiert ist und
R⁴ in den Formeln IV bis VII
für Wasserstoff, Fluor, Chlor, Hydroxy, Cyano, C(=O)R^{a}, NH₂, NHR^{a}, N(R^{a})R^{b}, oder C₁-C₆-Alkyl steht, wobei C₁-C₆-Alkyl gegebenenfalls ein- bis dreifach substituiert sein kann mit Fluor und gegebenenfalls einfach mit Hydroxy oder Cyclopropyl,
oder für C₁-C₆-Alkoxy steht, welches gegebenenfalls ein- bis vierfach substituiert sein kann mit Fluor,
oder für C₃-C₆-Cycloalkyl, C₃-C₆-Cycloalkyloxy oder für Pyridinyl steht, wobei der Pyridinylrest gegebenenfalls ein- oder zweimal, gleich oder verschieden mit Fluor, Chlor oder C₁-C₄-Alkyl substituiert sein kann und
R⁴ in den Formeln IX und X
für Wasserstoff, Cyano, NH₂, NHC₁-C₄-Alkyl, N(C₁-C₄-Alkyl)(C₁-C₄-Alkyl), Cyclopropyl oder C₁-C₆-Alkyl steht, wobei C₁-C₆-Alkyl gegebenenfalls ein- oder dreifach substituiert sein kann mit Fluor und gegebenenfalls einfach mit Hydroxy oder Cyclopropyl, oder für Pyridinyl steht, welches gegebenenfalls ein- oder zweimal, gleich oder verschieden substituiert mit Fluor, Chlor oder C₁-C₄-Alkyl oder für Morpholin-1-yl, 4-Methylpiperazin-1-yl, Piperazin-1-yl, Piperidin-1-yl, Pyrrolidin-1-yl steht;
R⁵ für Wasserstoff, Fluor, Chlor, Cyano, C₁-C₄-Alkyl steht;
R³ für eine Gruppe R^{3a} steht 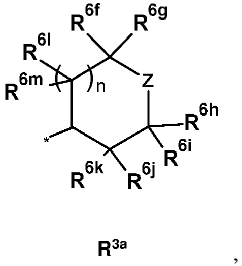 wobei * für die Verknüpfungsstelle der Gruppe mit dem Rest des Moleküls steht;
R^{6f} für Wasserstoff oder C₁-C₄-Alkyl steht;
R^{6g} für Wasserstoff oder C₁-C₄-Alkyl steht;
R^{6h} für Wasserstoff oder C₁-C₄-Alkyl steht;
R⁶ⁱ für Wasserstoff oder C₁-C₄-Alkyl steht;
oder R^{6h} und R⁶ⁱ stehen gemeinsam für eine Oxo-Gruppe;
R^{6j} für Wasserstoff oder Methyl steht;
R^{6k} für Wasserstoff oder Methyl steht;
R^{6l} für Wasserstoff steht;
R^{6m} für Wasserstoff steht;
n für 0 oder 1 steht;
z für eine Gruppe steht ausgewählt aus NR⁷, O, S, S(=O), S(=O)₂, S(=O)(=NH) steht;
R⁷ für Wasserstoff, C(=O)R^{e}, S(=O)₂R^{a}, S(=O)₂NH₂, S(=O)₂N(R^{a})H, S(=O)₂N(R^{a})R^{b}, S(=O)₂NHC(=O)CH₃, S(=O)₂NHC(=O)CH₂CH₃ oder C₁-C₆-Alkyl steht, wobei C₁-C₆-Alkyl gegebenenfalls ein- bis dreimal mit Fluor substituiert sein kann und gegebenenfalls ein- bis zweimal substituiert sein kann mit Hydroxy und gegebenenfalls substituiert sein kann mit N(R^{a})R^{b}, Cyclopropyl, Methoxy oder Ethoxy,
oder für C₃-C₆-Cycloalkyl oder Oxetan-3yl, Tetrahydrofuran-3-yl, Tetrahydro-2H-pyran-4-yl, 1-Methylazetidin-3-yl, 1-Methylpyrrolidin-3-yl, 1-Methylpiperidin-4-yl steht;
R^{e} für C₁-C₃-Alkyl steht, wobei C₁-C₃-Alkyl gegebenenfalls substituiert sein kann mit Hydroxy;
oder R³ steht für eine Gruppe R^{3b} 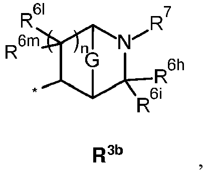 worin G für -CH₂- steht;
n für 1 steht;
R^{6h}, R⁶ⁱ, R^{6m}, R^{6l} für Wasserstoff stehen;
R⁷ für Wasserstoff, C(=O)CH₂OH, S(=O)₂NH₂, S(=O)₂NHC(=O)CH₃, Methyl, Ethyl, 2,2,2-Trifluorethyl, 2-(Dimethylamino)ethyl, 2-Hydroxyethyl, 3-Hydroxy-3-methylbutyl, Oxetan-3-yl, 1-Methylpiperidin-4-yl steht.

Besonders bevorzugt sind Verbindungen der Formel (I), in der
R¹ für Chlor, C(=O)OH, C(=O)OMe, C(=O)NH₂, Hydroxy, mit einer Hydroxygruppe substituiertes C₁-C₃-Alkyl, unsubstituiertes C₁-C₃-Alkoxy oder Cyclopropylmethoxy steht,
R² für eine Gruppe steht, ausgewählt aus den folgenden allgemeinen Formeln III, VI, VII, VIII, IX oder X 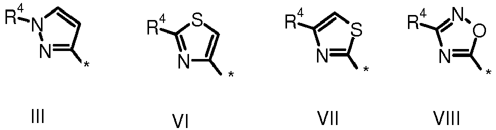 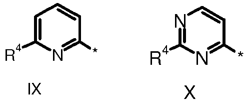 und
* für die Verknüpfungsstelle der Gruppe mit dem Rest des Moleküls steht und
R⁴ in der Formel III für Wasserstoff oder C₁-C₄-Alkyl steht, wobei C₁-C₄-Alkyl gegebenenfalls mit bis zu drei Fluoratomen oder einfach mit Hydroxy substituiert sein kann,
oder für Pyridin-4-yl steht, und
R⁴ in den Formeln VI, VII und VIII
für Wasserstoff, Cyano, C₃-C₆-Cycloalkyl oder C₁-C₄-Alkyl steht, wobei C₁-C₄-Alkyl gegebenenfalls mit bis zu drei Fluoratomen oder einfach mit Hydroxy substituiert sein kann oder für Pyridin-4-yl steht, und
R⁴ in den Formeln IX und X
für Wasserstoff, Cyano, NH₂, NHC₁-C₄-Alkyl, N(C₁-C₄-Alkyl)(C₁-C₄-Alkyl), Cyclopropyl oder C₁-C₄-Alkyl steht, wobei C₁-C₄-Alkyl gegebenenfalls mit bis zu 3 Fluoratomen oder einfach mit Hydroxy subsituiert sein kann oder für Pyridinyl, Morpholin-1-yl, 4-Methylpiperazin-1-yl oder Piperazin-1-yl steht;
R³ für Tetrahydro-2H-pyran-4-yl, 1,1-Dioxidotetrahydro-2H-thiopyran-4-yl, 1-Imino-1-oxidohexahydro-1λ⁴-thiopyran-4-yl, 5-Oxopyrrolidin-3-yl, Tetrahydrofuran-3-yl, (3S)-Tetrahydrofuran-3-yl, (3R)-Tetrahydrofuran-3-yl, (3S)-Tetrahydrothiophen-3-yl, 1,1-Dioxidotetrahydrothiophen-3-yl, (3S)-1,1-Dioxidotetrahydrothiophen-3-yl, (3R)-1,1-Dioxidotetrahydrothiophen-3-yl oderTetrahydro-2H-thiopyran-4-y steht, oder
R³ steht für eine Gruppe R^{3d} 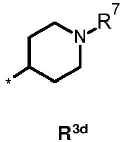 wobei * für die Verknüpfungsstelle der Gruppe mit dem Rest des Moleküls steht;
R⁷ für Wasserstoff, C(=O)CH₂OH, S(=O)₂NH₂, S(=O)₂NHC(=O)CH₃, Methyl, Ethyl, 2,2,2-Trifluorethyl, 2-(Dimethylamino)ethyl, 2-Hydroxyethyl, 3-Hydroxy-3-methylbutyl oder Oxetan-3-yl, 1-Methylpiperidin-4-yl steht.
Im Weiteren sind sind Verbindungen der Formel (I) bevorzugt, in der
R¹ für Chlor, C(=O)NH₂, 2-Hydroxypropan-2-yl, Methoxy, Cyclopropylmethoxy steht,
R² für 6-(Trifluormethyl)pyridin-2-yl, 6-(Difluormethyl)pyridin-2-yl, 6-(1,1-Difluorethyl)-pyridin-2-yl, 6-(Morpholin-4-yl)pyridin-2-yl, 2-Methyl-1,3-thiazol-4-yl, 6-Amino-pyridin-2-yl, 2-Isopropyl-pyrimidin-4-yl, 6-(2-Hydroxypropan-2-yl)-pyridin-2-yl, 4-(Trifluormethyl)-1,3-thiazol-2-yl, 3-(Pyridin-4-yl)-1,2,4-oxadiazol-5-yl, 3-Methyl-1,2,4-oxadiazol-5-yl oder 1-(Difluormethyl)-1H-pyrazol-3-yl steht,
R³ für Tetrahydro-2H-pyran-4-yl, 1,1-Dioxidotetrahydro-2H-thiopyran-4-yl, 1-Imino-1-oxidohexahydro-1λ⁴-thiopyran-4-yl, 5-Oxopyrrolidin-3-yl, Tetrahydrofuran-3-yl, (3S)-Tetrahydrofuran-3-yl, (3R)-Tetrahydrofuran-3-yl, 1,1-Dioxidotetrahydrothiophen-3-yl, (3S)-1,1-Dioxidotetrahydrothiophen-3-yl oder (3R)-1,1-Dioxidotetrahydrothiophen-3-yl steht,
oder
R³ steht für Piperidin-4-yl, 1-(2,2,2-Trifluorethyl)piperidin-4-yl, 1-Methylpiperidin-4-yl, 1-Glycoloylpiperidin-4-yl, 1'-Methyl-1,4'-bipiperidin-4-yl, 1-(Acetylsulfamoyl)piperidin-4-yl, [2-(Dimethylamino)ethyl]piperidin-4-yl, 1-(Oxetan-3-yl)piperidin-4-yl, 1-(2-Hydroxyethyl)piperidin-4-yl, 1-(3-Hydroxy-3-methylbutyl)piperidin-4-yl.

Ebenfalls ganz besonders bevorzugt sind Verbindungen der Formel (I), in der
R¹ für C(=O)NH₂, 2-Hydroxypropan-2-yl oder Methoxy steht,
R² für 6-(Trifluormethyl)pyridin-2-yl, 6-(Difluormethyl)pyridin-2-yl, 6-Amino-pyridin-2-yl, 4-(Trifluormethyl)-1,3-thiazol-2-yl, 1-(Difluormethyl)-1H-pyrazol-3-yl,
R³ für Tetrahydro-2H-pyran-4-yl, 1,1-Dioxidotetrahydro-2H-thiopyran-4-yl, Tetrahydrofuran-3-yl, (3S)-Tetrahydrofuran-3-yl, (3R)-Tetrahydrofuran-3-yl oder (3S)-1,1-Dioxidotetrahydrothiophen-3-yl steht
oder
R³ steht für Piperidin-4-yl, 1-(2,2,2-Trifluorethyl)piperidin-4-yl, 1-Methylpiperidin-4-yl, 1-Glycoloylpiperidin-4-yl, 1'-Methyl-1,4'-bipiperidin-4-yl, 1-(Acetylsulfamoyl)piperidin-4-yl, [2-(Dimethylamino)ethyl]piperidin-4-yl, 1-(Oxetan-3-yl)piperidin-4-yl, 1-(2-Hydroxyethyl)piperidin-4-yl oder 1-(3-Hydroxy-3-methylbutyl)piperidin-4-yl steht.

Ganz besonders bevorzugt sind die folgenden erfindungsgemäßen Verbindungen:
(1) N-[6-Methoxy-2-(tetrahydro-2H-pyran-4-yl)-2H-indazol-5-yl]-6-(trifluormethyl)pyridin-2-carboxamid
(2) N-[6-Methoxy-2-(piperidin-4-yl)-2H-indazol-5-yl]-6-(trifluormethyl)pyridin-2-carboxamid
(3) N-{6-Methoxy-2-[1-(2,2,2-trifluorethyl)piperidin-4-yl] -2H-indazol-5-yl} -6-(trifluormethyl)-pyridin-2-carboxamid
(4) N-[6-Methoxy-2-(1-methylpiperidin-4-yl)-2H-indazol-5-yl]-6-(trifluormethyl)pyridin-2-carboxamid
(5) N-[2-(1-Glycoloylpiperidin-4-yl)-6-methoxy-2H-indazol-5-yl]-6-(trifluormethyl)pyridin-2-carboxamid
(6) N-[6-Methoxy-2-(1'-methyl-1,4'-bipiperidin-4-yl)-2H-indazol-5-yl]-6-(trifluoromethyl)-pyridin-2-carboxamid
(7) N-[6-Methoxy-2-(1-sulfamoylpiperidin-4-yl)-2H-indazol-5-yl]-6-(trifluormethyl)pyridin-2-carboxamid
(8) N-{2-[1-(Acetylsulfamoyl)piperidin-4-yl]-6-methoxy-2H-indazol-5-yl}-6-(trifluormethyl)-pyridin-2-carboxamid
(9) N-(2-{1-[2-(Dimethylamino)ethyl]piperidin-4-yl}-6-methoxy-2H-indazol-5-yl)-6-(trifluormethyl)pyridin-2-carboxamid
(10) N-{6-Methoxy-2-[1-(oxetan-3-yl)piperidin-4-yl]-2H-indazol-5-yl}-6-(trifluormethyl)pyridin-2-carboxamid
(11) N-[2-(1,1-Dioxidotetrahydro-2H-thiopyran-4-yl)-6-methoxy-2H-indazol-5-yl]-6-(trifluormethyl)pyridin-2-carboxamid
(12) N-[6-Methoxy-2-(1-oxidotetrahydro-2H-thiopyran-4-yl)-2H-indazol-5-yl]-6-(trifluormethyl)pyridin-2-carboxamid
(13) N-{2-[1-(2-Hydroxyethyl)piperidin-4-yl] -6-methoxy-2H-indazol-5-yl}-6-(trifluormethyl)-pyridin-2-carboxamid
(14) rel-N-{2-[(1R,4R,5S)-2-Azabicyclo[2.2.1]hept-5-yl]-6-methoxy-2H-indazol-5-yl}-6-(trifluormethyl)pyridin-2-carboxamid
(15) rel-N-{2-[(1R,4R,5R)-2-Azabicyclo[2.2.1]hept-5-yl]-6-methoxy-2H-indazol-5-yl}-6-(trifluormethyl)pyridin-2-carboxamid
(16) N-[2-(1,1-Dioxidotetrahydro-2H-thiopyran-4-yl)-6-hydroxy-2H-indazol-5-yl]-6-(trifluormethyl)pyridin-2-carboxamid
(17) N-[6-(Cyclopropylmethoxy)-2-(1,1-dioxidotetrahydro-2H-thiopyran-4-yl)-2H-indazol-5-yl]-6-(trifluormethyl)pyridin-2-carboxamid
(18) rel-N-{6-Methoxy-2-[(1R,4R,5S)-2-methyl-2-azabicyclo[2.2.1]hept-5-yl]-2H-indazol-5-yl}-6-(trifluormethyl)pyridin-2-carboxamid
(19) rel-N-{6-Methoxy-2-[(1R,4R,5R)-2-methyl-2-azabicyclo[2.2.1]hept-5-yl]-2H-indazol-5-yl}-6-(trifluormethyl)pyridin-2-carboxamid
(20) N-[2-(1-Imino-1-oxidohexahydro-1λ⁴-thiopyran-4-yl)-6-methoxy-2H-indazol-5-yl]-6-(trifluormethyl)pyridin-2-carboxamid (Isomer 1)
(21) N-[2-(1-Imino-1-oxidohexahydro-1λ⁴-thiopyran-4-yl)-6-methoxy-2H-indazol-5-yl]-6-(trifluormethyl)pyridin-2-carboxamid (Isomer 2)
(22) N-[6-Methoxy-2-(5-oxopyrrolidin-3-yl)-2H-indazol-5-yl]-6-(trifluormethyl)pyridin-2-carboxamid
(23) 6-(Difluormethyl)-N-[6-methoxy-2-(tetrahydro-2H-pyran-4-yl)-2H-indazol-5-yl]pyridin-2-carboxamid
(24) N-[6-Methoxy-2-(tetrahydro-2H-pyran-4-yl)-2H-indazol-5-yl]-6-(morpholin-4-yl)pyridin-2-carboxamid
(25) N-[6-Methoxy-2-(tetrahydro-2H-pyran-4-yl)-2H-indazol-5-yl]-2-methyl-1,3-thiazol-4-carboxamid
(26) 6-Amino-N-[6-methoxy-2-(tetrahydro-2H-pyran-4-yl)-2H-indazol-5-yl]pyridin-2-carboxamid
(27) 2-Isopropyl-N-[6-methoxy-2-(tetrahydro-2H-pyran-4-yl)-2H-indazol-5-yl]pyrimidin-4-carboxamid
(28) 6-(2-Hydroxypropan-2-yl)-N-[6-methoxy-2-(tetrahydro-2H-pyran-4-yl)-2H-indazol-5-yl]pyridin-2-carboxamid
(29) N-[6-Methoxy-2-(tetrahydrofuran-3-yl)-2H-indazol-5-yl]-6-(trifluormethyl)pyridin-2-carboxamid
(30) N-[6-Chlor-2-(tetrahydro-2H-pyran-4-yl)-2H-indazol-5-yl]-6-(trifluormethyl)pyridin-2-carboxamid
(31) N-[6-Chlor-2-(tetrahydro-2H-pyran-4-yl)-2H-indazol-5-yl]-6-(difluormethyl)pyridin-2-carboxamid
(32) N-[6-Chlor-2-(1,1-dioxidotetrahydro-2H-thiopyran-4-yl)-2H-indazol-5-yl]-6-(2-hydroxypropan-2-yl)pyridin-2-carboxamid
(33) Methyl-2-(tetrahydro-2H-pyran-4-yl)-5-({[6-(trifluormethyl)pyridin-2-yl]carbonyl}amino)-2H-indazol-6-carboxylat
(34) N-[6-(2-Hydroxypropan-2-yl)-2-(tetrahydro-2H-pyran-4-yl)-2H-indazol-5-yl]-6-(trifluormethyl)pyridin-2-carboxamid
(35) Methyl-2-[(3S)-tetrahydrofuran-3-yl]-5-({[6-(trifluormethyl)pyridin-2-yl]carbonyl}amino)-2H-indazol-6-carboxylat
(36) N-{6-(2-Hydroxypropan-2-yl)-2-[(3S)-tetrahydrofuran-3-yl]-2H-indazol-5-yl}-6-(trifluormethyl)pyridin-2-carboxamid
(37) Methyl-2-[(3R)-tetrahydrofuran-3-yl]-5-({[6-(trifluormethyl)pyridin-2-yl]carbonyl}amino)-2H-indazol-6-carboxylat
(38) N-{6-(2-Hydroxypropan-2-yl)-2-[(3R)-tetrahydrofuran-3-yl]-2H-indazol-5-yl}-6-(trifluormethyl)pyridin-2-carboxamid
(39) Methyl-2-[(3S)-tetrahydrothiophen-3-yl]-5-({[6-(trifluormethyl)pyridin-2-yl]carbonyl}-amino)-2H-indazol-6-carboxylat
(40) N-{6-(2-Hydroxypropan-2-yl)-2-[(3S)-tetrahydrothiophen-3-yl]-2H-indazol-5-yl}-6-(trifluormethyl)pyridin-2-carboxamid
(41) N-{2-[(3S)-1,1-Dioxidotetrahydrothiophen-3-yl]-6-(2-hydroxypropan-2-yl)-2H-indazol-5-yl}-6-(trifluormethyl)pyridin-2-carboxamid
(42) Methyl-2-(tetrahydro-2H-thiopyran-4-yl)-5-({[6-(trifluormethyl)pyridin-2-yl]carbonyl}-amino)-2H-indazol-6-carboxylat
(43) N-[6-(2-Hydroxypropan-2-yl)-2-(tetrahydro-2H-thiopyran-4-yl)-2H-indazol-5-yl]-6-(trifluormethyl)pyridin-2-carboxamid
(44) N-[2-(1,1-Dioxidotetrahydro-2H-thiopyran-4-yl)-6-(2-hydroxypropan-2-yl)-2H-indazol-5-yl]-6-(trifluormethyl)pyridin-2-carboxamid
(45) Methyl-2-(piperidin-4-yl)-5-({[6-(trifluormethyl)pyridin-2-yl]carbonyl}amino)-2H-indazol-6-carboxylat
(46) N-[6-(2-Hydroxypropan-2-yl)-2-(piperidin-4-yl)-2H-indazol-5-yl]-6-(trifluormethyl)pyridin-2-carboxamid
(47) N-[6-Methoxy-2-(tetrahydro-2H-pyran-4-yl)-2H-indazol-5-yl]-4-(trifluormethyl)-1,3-thiazol-2-carboxamid
(48) N-[6-Methoxy-2-(tetrahydro-2H-pyran-4-yl)-2H-indazol-5-yl]-3-(pyridin-4-yl)-1,2,4-oxadiazol-5-carboxamid
(49) N-[6-Methoxy-2-(tetrahydro-2H-pyran-4-yl)-2H-indazol-5-yl]-3-methyl-1,2,4-oxadiazol-5-carboxamid
(50) N-[6-Chlor-2-(tetrahydro-2H-pyran-4-yl)-2H-indazol-5-yl]-4-(trifluormethyl)-1,3-thiazol-2-carboxamid
(51) 1-(Difluormethyl)-N-[6-methoxy-2-(tetrahydro-2H-pyran-4-yl)-2H-indazol-5-yl]-1H-pyrazol-3-carboxamid
(52) N-{2-[1-(3-Hydroxy-3-methylbutyl)piperidin-4-yl]-6-methoxy-2H-indazol-5-yl}-6-(trifluormethyl)pyridin-2-carboxamid
(53) 2-(Piperidin-4-yl)-5-({[6-(trifluormethyl)pyridin-2-yl]carbonyl}amino)-2H-indazol-6-carboxamid
(54) 2-(Piperidin-4-yl)-5-({[6-(trifluormethyl)pyridin-2-yl]carbonyl}amino)-2H-indazol-6-carbonsäure
(55) Methyl-2-(1-methylpiperidin-4-yl)-5-({[6-(trifluormethyl)pyridin-2-yl]carbonyl}amino)-2H-indazol-6-carboxylat
(56) 2-[1-(3-Hydroxy-3-methylbutyl)piperidin-4-yl]-5-({[6-(trifluormethyl)pyridin-2-yl]carbonyl}-amino)-2H-indazol-6-carboxamid
(57) 2-(1-Methylpiperidin-4-yl)-5-({[6-(trifluormethyl)pyridin-2-yl]carbonyl}amino)-2H-indazol-6-carboxamid
(58) N-{6-Methoxy-2-[1-(2-methoxyethyl)piperidin-4-yl]-2H-indazol-5-yl}-6-(trifluormethyl)pyridin-2-carboxamid.

Insbesondere sind sind folgende Verbindungen bevorzugt:
(34) N-[6-(2-Hydroxypropan-2-yl)-2-(tetrahydro-2H-pyran-4-yl)-2H-indazol-5-yl]-6-(trifluormethyl)pyridin-2-carboxamid
(44) N-[2-(1,1-Dioxidotetrahydro-2H-thiopyran-4-yl)-6-(2-hydroxypropan-2-yl)-2H-indazol-5-yl]-6-(trifluormethyl)pyridin-2-carboxamid
(46) N-[6-(2-Hydroxypropan-2-yl)-2-(piperidin-4-yl)-2H-indazol-5-yl]-6-(trifluormethyl)pyridin-2-carboxamid

Die erfindungsgemäßen Verbindungen wirken als Inhibitoren der IRAK4 Kinase, und zeigen ein nicht vorhersehbares, wertvolles pharmakologisches Wirkspektrum.

Daher ist neben den weiter oben genannten ein weiterer Gegenstand der vorliegenden Erfindung die Verwendung der erfindungsgemäßen Verbindungen zur Behandlung und/oder Prophylaxe von Krankheiten bei Menschen und Tieren.

Die Behandlung und/oder Prophylaxe von gynäkologischen Erkrankungen, entzündlichen Hauterkrankungen, Herz-Kreislauf-Erkrankungen, Lungenerkrankungen, Augenerkrankungen, Autoimmunerkrankungen, Schmerzerkrankungen, Stoffwechselerkrankungen, Gicht, Lebererkrankungen, des metabolischen Syndroms, der Insulinresistenz und von Krebserkrankungen mit den erfindungsgemäßen IRAK4 Inhibitoren ist besonders bevorzugt.

Die erfindungsgemäßen Verbindungen sind geeignet für die Prophylaxe und/oder Behandlung von verschiedenen Erkrankungen und krankheitsbedingten Zuständen, insbesondere von TLR- (außer TLR3) und/oder IL-1-Rezeptorfamilie-vermittelten Erkrankungen bzw. Erkrankungen, dessen Pathologie direkt durch IRAK4 vermittelt ist. Als IRAK4-assoziierte Erkrankungen sind Multiple Sklerose, Atherosklerose, Herzinfarkt, Alzheimer, viral-induzierte Myokarditis, Gicht, Vogt-Koyanagi-Harada-Syndrom, Lupus erythematodes, Psoriasis, Spondyloarthritiden und Arthritis zu benennen.

Die erfindungsgemäßen Verbindungen können ferner für die Prophylaxe und/oder Behandlung von MyD88- und TLR (außer TLR3)-vermittelte Erkrankungen eingesetzt werden. Dies umfasst Multiple Sklerose, Rheumatoide Arthritis, Spondyloarthritiden (insbesondere Spondyloarthritis psoriatica und Morbus Bechterew), Metabolisches Syndrom inklusive Insulinresistenz, Diabetes mellitus, Osteoarthritis, Sjögren-Syndrom, Riesenzellarteriitis, Sepsis, Poly- und Dermatomyositis, Hauterkrankungen wie Psoriasis, atopische Dermatitis, Alopecia areata, Acne inversa und Acne vulgaris, pulmonale Erkrankungen wie Lungenfibrose, chronisch obstruktive Lungenerkrankung (COPD), akutes Atemnot-Syndrom (ARDS), akute Lungenschädigung (ALI), interstitielle Lungenerkrankung (ILD), Sarkoidose und pulmonale Hypertonie.

Aufgrund des Wirkmechanismus der erfindungsgemäßen Verbindungen sind sie zur Prophylaxe und/oder Behandlung der TLR-vermittelten Erkrankungen Behcet-Krankheit, Gicht, Endometriose sowie von Endometriose-assoziierten Schmerzen und anderen Endometriose-assoziierten Symptomen wie Dysmenorrhoe, Dyspareunie, Dysurie und Dyschezie geeignet. Des Weiteren sind die erfindungsgemäßen Verbindungen zur Prophylaxe und/oder Behandlung bei Transplantatabstoßung, von Lupus erythematodes, Adult Morbus Still-Krankheit und chronisch entzündlichen Darmerkrankungen wie Kolitis ulcerosa und Morbus Crohn geeignet.

Neben den bereits aufgeführten Erkrankungen ist die Verwendung der erfindungsgemäßen Verbindungen auch zur Behandlung und/oder Prävention folgender Erkrankungen geeignet: Augenerkrankungen wie Keratitis, allergisch bedingte Konjunktivitis, Keratoconjunctivitis sicca, Makuladegeneration und Uveitis; kardiovaskuläre Erkrankungen wie Atherosklerose, myokardialer Reperfusionsschaden, Myokardinfarkt, Hypertonie und neurologische Erkrankungen wie Alzheimer, Schlaganfall und Parkinson.

Der Wirkmechanismus der erfindungsgemäßen Verbindungen ermöglicht ferner die Prophylaxe und/oder Behandlung von TLR- und IL-1 Rezeptorfamilie vermittelten Lebererkrankungen, insbesondere NAFLD, NASH, ASH, Leberfibrose und Leberzirrhose.

Weiterhin ist die Prophylaxe und/oder Behandlung von Juckreiz und Schmerz, insbesondere von akutem, chronischem, entzündlichem und neuropathischem Schmerz durch die erfindungsgemäßen Verbindungen gegeben.

Aufgrund des Wirkmechanismus der erfindungsgemäßen Verbindungen sind sie zur Prophylaxe und/oder Behandlung von onkologischen Erkrankungen wie Lymphome, chronisch lymphatische Leukämie, Melanoma und Leberzellkarzinom, Brustkrebs, Prostatakrebs und Ras-abhängige Tumore geeignet.

Außerdem sind die erfindungsgemäßen Verbindungen geeignet zur Behandlung und/oder Prävention von Erkrankungen, die über die IL-1 Rezeptorfamilie vermittelt sind. Diese Erkrankungen umfassen CAPS (Cryopyrin-assoziierte periodische Syndrome) inklusive FCAS (familiäre Kälteurtikaria), MWS (Muckle-Wells-Syndrom), NOMID- (neonatal-onset multisystem inflammatory disease) und CONCA- (chronic infantile, neurological, cutaneous, and articular) Syndrom, FMF (familiäres Mittelmeerfieber), HIDS (Hyper-IgD-Syndrom), TRAPS (Tumornekrosefaktor-Rezeptor 1-assoziiertes periodisches Syndrom), juvenile idiopathische Arthritis, Adult Morbus Still-Krankheit, Morbus Adamantiades-Behçet, Rheumatoide Arthritis, Psoriasis Arthritis, Morbus Bechterew, Osteoarthritis, Keratoconjunctivitis sicca und Sjögren Syndrome, Multiple Sklerose, Lupus erythematodes, Alopecia areata, Diabetes mellitus Typ 1, Diabetes mellitus Typ 2 und die Folgen eines Myokardinfarktes. Pulmonale Erkrankungen wie Asthma, COPD, idiopathische interstitielle Pneumonie und ARDS, gynäkologische Erkrankungen wie Endometriose sowie Endometriose-assoziierte Schmerzen und andere Endometriose-assoziierte Symptome wie Dysmenorrhoe, Dyspareunie, Dysurie und Dyschezie, chronisch-entzündliche Darmerkrankungen wie Morbus Crohn und Kolitis ulcerosa sind mit einer Dysregulation der IL-1 Rezeptorfamilie assoziiert und für den therapeutischen und/oder prophylaktischen Einsatz der erfindungsgemäßen Verbindungen geeignet.

Die erfindungsgemäßen Verbindungen können ferner für Behandlung und/oder Prävention von IL1-Rezeptorfamilie-vermittelten neurologischen Erkrankungen wie Hirnschlag, Alzheimer, Schlaganfall, Schädel-Hirn-Trauma und dermatologische Erkrankungen wie Psoriasis, atopische Dermatitis, Acne inversa, Alopecia areata und allergische Kontaktdermatitis eingesetzt werden.

Weiterhin sind die erfindungsgemäßen Verbindungen geeignet für die Behandlung und/oder Prophylaxe von Schmerzerkrankungen, insbesondere von akutem, chronischem, entzündlichem und neuropathischem Schmerz. Hierfür seien vorzugsweise Hyperalgesie, Allodynie, Schmerz bei Arthritis (wie Osteoarthritis, rheumatoider Arthritis und Spondyloarthritis), prämenstrueller Schmerz, Endometriose-assoziierter Schmerz, postoperativer Schmerz, Schmerz bei interstitieller Zystitis, CRPS (komplexes regionales Schmerzsyndrom), Trigeminusneuralgie, Schmerz bei Prostatitis, Schmerzen verursacht durch Rückenmarksverletzungen, entzündungsinduzierter Schmerz, Kreuzschmerzen, Krebsschmerzen, Chemotherapie-assoziierter Schmerz, HIV behandlungsinduzierte Neuropathie, Verbrennungs-induzierter Schmerz und chronischer Schmerz genannt.

Im Weiteren ist Gegenstand der vorliegenden Erfindung auch ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als Synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
Allgemein sind Wirkstoffe wie antibakterielle (z.B. Penicilline, Vancomycin, Ciprofloxacin), antivirale (z.B. Aciclovir, Oseltamivir) und antimykotische (z.B. Naftifin, Nystatin) Substanzen und Gammaglobuline, immunomodulatorische und immunosuppressive Verbindungen wie Cyclosporin, Methotrexat®, TNF-Antagonisten (z.B. Humira®,, Etanercept, Infliximab), IL-1 Inhibitoren (z.B. Anakinra, Canakinumab, Rilonacept), Phosphodiesterasehemmer (z.B Apremilast), Jak/STAT Inhibitoren (z.B. Tofacitinib, Baricitinib, GLPG0634), Leflunomid, Cyclophosphamid, Rituximab, Belimumab, Tacrolimus, Rapamycin, Mycophenolate mofetil, Interferone, Corticosteroide (z.B. Prednisone, Prednisolone, Methylprednisolone, Hydrocortisone, Betamethason), Cyclophophamide, Azathioprine und Sulfasalazine; Paracetamol, non-steroidale anti-inflammatorische Substanzen (NSAIDS) (z.B. Aspirin, Ibuprofen, Naproxen, Etodolac, Celecoxib, Colchicine) zu nennen.

Für die Tumortherapie seien zu nennen: Immunotherapie (z.B. Aldesleukin, Alemtuzumab, Basiliximab, Catumaxomab, Celmoleukin, Denileukin-Diftitox, Eculizumab, Edrecolomab, Gemtuzumab, Ibritumomab-Tiuxetan, Imiquimod, Interferon-alpha, Interferon-beta, Interferongamma, Ipilimumab, Lenalidomid, Lenograstim, Mifamurtid, Ofatumumab, Oprelvekin, Picibanil, Plerixafor, Polysaccharid-K, Sargramostim, Sipuleucel-T, Tasonermin, Teceleukin, Tocilizumab), antiproliferative Substanzen wie beispielsweise aber nicht ausschließlich Amsacrin, Arglabin, Arsentrioxid, Asparaginase, Bleomycin, Busulfan, Dactinomycin, Docetaxel, Epirubicin, Peplomycin, Trastuzumab, Rituximab, Obinutuzumab, Ofatumumab, Tositumomab, Aromatase Inhibitoren (z.B. Exemestan, Fadrozol, Formestan, Letrozol, Anastrozol, Vorozol), Antiestrogene (z.B. Chlormadinon, Fulvestrant, Mepitiostan, Tamoxifen, Toremifen), Estrogene (z.B. Estradiol, Polyestradiolphosphat, Raloxifen), Gestagene (z.B. Medroxyprogesteron, Megestrol), Topoisomerase I Inhibitoren (z.B. Irinotecan, Topotecan), Topoisomerasen II Inhibitoren (z.B. Amrubicin, Daunorubicin, Elliptiniumacetat, Etoposid, Idarubicin, Mitoxantron, Teniposid), Mikrotubuli-aktive Substanzen (z.B. Cabazitaxel, Eribulin, Paclitaxel, Vinblastin, Vincristin, Vindesin, Vinorelbin), Telomeraseinhibitoren (z.B. Imetelstat), alkylierende Substanzen und Histon-Deacetylasen Inhibitoren (z.B. Bendamustin, Carmustin, Chlormethin, Dacarbazin, Estramustin, Ifosfamid, Lomustin, Mitobronitol, Mitolactol, Nimustin Prednimustin, Procarbazin, Ranimustin, Streptozotocin, Temozolomid, Thiotepa, Treosulfan, Trofosfamid, Vorinostat, Romidepsin, Panobinostat); Substanzen, die Prozesse der Zelldifferenzierung beeinflussen wie Abarelix, Aminoglutethimid, Bexaroten, MMP Inhibitoren (Peptidmimetika, Nicht-Peptidmimetika und Tetracycline wie z.B. Marimastat, BAY 12-9566, BMS-275291, Clodronate, Prinomastat, Doxycycline), mTOR Inhibitoren (z.B. Sirolimus, Everolimus, Temsirolimus, Zotarolimus), Antimetabolite (z.B. Clofarabin, Doxifluridin, Methotrexat, 5-Fluoruracil, Cladribin, Cytarabin, Fludarabin, Mercaptopurin, Methotrexat, Pemetrexed, Raltitrexed, Tegafur, Tioguanin), Platin verbindungen (z.B. Carboplatin, Cisplatin, Cisplatinum, Eptaplatin, Lobaplatin, Miriplatin, Nedaplatin, Oxaliplatin); Antiangiogene Verbindungen (z.B. Bevacizumab), antiandrogene Verbindungen (z.B. Bevacizumab, Enzalutamid, Flutamid, Nilutamid, Bicalutamid, Cyproteron, Cyproteronacetat), Proteasomeinhibitoren (z.B. Bortezomib, Carfilzomib, Oprozomib, ONYX0914), Gonadoliberin-Agonisten und -Antagonisten (z.B. Abarelix, Buserelin, Deslorelin, Ganirelix, Goserelin, Histrelin, Triptorelin, Degarelix, Leuprorelin), Methionine Aminopeptidase Inhibitoren (z.B. Bengamid-Derivate, TNP-470, PPI-2458), Heparanaseinhibitoren (z.B. SST0001, PI-88); Inhibitoren gegen genetisch verändertes Ras-Protein (z.B. Farnesyl-Transferase Inhibitoren wie Lonafarnib, Tipifarnib), HSP90 Inhibitoren (z.B. Geldamycin-Derivate wie 17-Allylaminogeldanamycin, 17-demethoxygeldanamycin (17AAG), 17-DMAG, Retaspimycin Hydrochloride, IPI-493, AUY922, BIIB028, STA-9090, KW-2478), Kinesin Spindleprotein Inhibitoren (z.B. SB715992, SB743921, Pentamidine/Chlorpromazine), MEK (mitogen-activated protein kinase) Inhibitoren (z.B. Trametinib, BAY 86-9766 (Refametinib), AZD6244), Kinase-Inhibitoren (z.B.: Sorafenib, Regorafenib, Lapatinib, Sutent, Dasatinib, Cetuximab, BMS-908662, GSK2118436, AMG 706, Erlotinib, Gefitinib, Imatinib, Nilotinib, Pazopanib, Roniciclib, Sunitinib, Vandetanib, Vemurafenib), Hedgehog Signalinhibitoren (z.B. Cyclopamin, Vismodegib), BTK (Bruton's Tyrosine Kinase) Inhibitor (z.B. Ibrutinib), JAK/pan-JAK (Janus Kinase) Inhibitor (z.B. SB-1578, Baricitinib, Tofacitinib, Pacritinib, Momelotinib, Ruxolitinib, VX-509, AZD-1480, TG-101348), PI3K Inhibitor (z.B. BAY 1082439, BAY 80-6946 (Copanlisib), ATU-027, SF-1126, DS-7423, GSK-2126458, Buparlisib, PF-4691502, BYL-719, XL-147, XL-765, Idelalisib), SYK (Spleen Tyrosine Kinase) Inhibitor (z.B. Fostamatinib, Excellair, PRT-062607), p53-Gentherapie, Bisphosphonate (z.B. Etridonat, Clodronat, Tiludronat, Pamidronat, Alendronsäure, Ibandronat, Risedronat, Zoledronat). Zur Kombination sind außerdem beispielhaft, aber nicht ausschließlich folgende Wirkstoffe zu nennen: Rituximab, Cyclophosphamide, Doxorubicin, Doxorubicin in Kombination mit Estron, Vincristine, Chlorambucil, Fludarabin, Dexamethasone, Cladribin, Prednisone, 131I-chTNT, Abirateron, Aclarubicin, Alitretinoin, Bisantren, Calciumfolinat, Calciumlevofolinat, Capecitabin, Carmofur, Clodronsäure, Romiplostim, Crisantaspase, Darbepoetin-alfa, Decitabin, Denosumab, Dibrospidiumchlorid, Eltrombopag, Endostatin, Epitiostanol, Epoetin-alfa, Filgrastim, Fotemustin, Galliumnitrat, Gemcitabin, Glutoxim, Histamindihydrochlorid, Hydroxycarbamid, Improsulfan, Ixabepilon, Lanreotid, Lentinan, Levamisol, Lisurid, Lonidamin, Masoprocol, Methyltestosteron, Methoxsalen, Methylaminolevulinat, Miltefosin, Mitoguazon, Mitomycin, Mitotan, Nelarabin, Nimotuzumab, Nitracrin, Omeprazol, Palifermin, Panitumumab, Pegaspargase, PEG-epoetin-beta (Methoxy-PEG-epoetin-beta), Pegfilgrastim, Peg-interferon-alfa-2b, Pentazocin, Pentostatin, Perfosfamid, Pirarubicin, Plicamycin, Poliglusam, Porfimer-Natrium, Pralatrexat, Quinagolid, Razoxan, Sizofiran, Sobuzoxan, Natriumglycididazol, Tamibaroten, die Kombination von Tegafur und Gimeracil und Oteracil, Testosteron, Tetrofosmin, Thalidomid, Thymalfasin, Trabectedin, Tretinoin, Trilostan, Tryptophan, Ubenimex, Vapreotid, Yttrium-90-Glasmikrokugeln, Zinostatin, Zinostatin-Stimalamer.

Für die Tumortherapie geeignet ist auch eine Kombination aus nicht-medikamentöser Therapie wie Chemotherapie (z.B. Azacitidin, Belotecan, Enocitabine, Melphalan, Valrubicin, Vinflunin, Zorubicin), Radiotherapie (z.B. I-125-Seeds, Palladium-103-Seed, Radium-223-chlorid) oder Phototherapie (z.B. Temoporfin, Talaporfin), die von einer medikamentösen Behandlung mit den erfindungsgemäßen IRAK4 Inhibitoren begleitet werden oder die nach Beendigung der nichtmedikamentösen Tumortherapie wie Chemotherapie, Radiotherapie oder Phototherapie durch eine medikamentöse Behandlung mit den erfindungsgemäßen IRAK4 Inhibitoren ergänzt werden.

Die erfindungsgemäßen IRAK4 Inhibitoren können außer mit den bereits genannten auch mit folgenden Wirkstoffen kombiniert werden:
Wirkstoffe für die Alzheimertherapie wie beispielsweise Acetylcholinesterase-Hemmern (z.B. Donepezil, Rivastigmine, Galantamin, Tacrin), NMDA (N-Methyl-D-Aspartat)-Rezeptorantagonisten (z.B. Memantine); L-DOPA/Carbidopa (L-3,4-Dihydroxyphenylalanin), COMT (Catechol-O-Methyltransferase)-Hemmer (z.B. Entacapon), Dopaminagonisten (z.B. Ropinrol, Pramipexol, Bromocriptin), MAO-B (Monoaminooxidase-B)-Hemmer (z.B. Selegilin), Anticholinergika (z.B. Trihexyphenidyl) und NMDA-Antagonisten (z.B. Amantadin) zur Behandlung von Parkinson; Beta-Interferon (IFN-beta) (z.B. IFN beta-lb, IFN beta-la Avonex® und Betaferon®), Glatirameracetat, Immunglobuline, Natalizumab, Fingolimod und Immunsuppressiva wie Mitoxantron, Azathioprin und Cyclophosphamid zur Behandlung der Multiplen Sklerose; Substanzen zur Behandlung von pulmonalen Erkrankungen wie beispielsweise Beta-2-Sympathomimetika (z.B. Salbutamol), Anticholinergika (z.B. Glycopyrronium), Methylxanthine (z.B. Theophyllin), Leukotrienrezeptor-Antagonist (z.B. Montelukast), PDE-4 (Phosphodiesterase Typ 4)-Hemmer (z.B. Roflumilast), Methotrexat, IgE Antikörper, Azathioprin und Cyclophosphamid, Kortisolhaltige Präparate; Substanzen zur Behandlung von Osteoarthritis wie non-steroidale anti-inflammatorische Substanzen (NSAIDs). Neben den zwei genannten Therapien sind für rheumatoide Erkrankungen wie beispielsweise rheumatoide Arthritis, Spondyloarthritiden und juvenile idiopathische Arthritis Methotrexat und Biologika zur B-Zell- und T-Zell-Therapie (z.B. Rituximab, Abatacept) zu nennen. Neurotrophe Substanzen wie Acetylcholinesterase Inhibitoren (z.B. Donepezil), MAO (Monoaminooxidase) Inhibitoren (z.B. Selegilin), Interferone und Antikonvulsivum (z.B. Gabapentin); Wirkstoffe zur Behandlung von kardiovaskulären Erkrankungen wie beta-Blocker (z.B. Metoprolol), ACE Inhibitoren (z.B. Benazepril), Angiotensin-Rezeptorblocker (z.B. Losartan, Valsartan), Diuretika (z.B. Hydrochlorothiazid), Calciumkanal-Blocker (z.B. Nifedipin), Statine (z.B. Simvastatin, Fluvastatin); Anti-Diabetika wie z.B. Metformin, Glinide (z.B. Nateglinid), DPP-4 (Dipeptidyl-Peptidase-4)-Inhibitoren (z.B. Linagliptin, Saxagliptin, Sitagliptin, Vildagliptin), SGLT2 (sodium/glucose cotransporter 2)-Inhibitoren/ Gliflozin (z.B. Dapagliflozin, Empagliflozin), Inkretinmimetika (Hormone Glukoseabhängiges insulinotropes Peptid (GIP)- und Glucagon-like Peptid 1(GLP-1)-Analoga/Agonisten) (z.B. Exenatid, Liraglutid, Lixisenatide), α-Glucosidase-Hemmer (z.B. Acarbose, Miglitol, Voglibiose) und Sulfonylharnstoffe (z.B. Glibenclamid, Tolbutamid), Insulin-Sensitizer (z.B. Pioglitazon) und Insulintherapie (z.B. NPH-Insulin, Insulin lispro), Substanzen zur Behandlung einer Hypoglykämie zur Behandlung von Diabetes und metabolischem Syndrom. Lipidsenker wie beispielsweise Fibrate (z.B. Bezafibrat, Etofibrat, Fenofibrat, Gemfibrozil), Nikotinsäurederivate (z.B. Nicotinsäure/Laropiprant), Ezetimib, Statine (z.B. Simvastatin, Fluvastatin), Anionenaustauscher (z.B. Colestyramin, Colestipol, Colesevelam). Wirkstoffe wie Mesalazin, Sulfasalazin, Azathioprin, 6-Mercaptopurin oder Methotrexat, probiotische Bakterien (Mutaflor, VSL#3®, Lactobacillus GG, Lactobacillus plantarum, L. acidophilus, L. casei, Bifidobacterium infantis 35624, Enterococcus fecium SF68, Bifidobacterium longum, Escherichia coli Nissle 1917), Antibiotika wie beispielsweise Ciprofloxacin und Metronidazol, Antidiarrhoika wie z.B. Loperamid oder Laxativa (Bisacodyl) zur Behandlung von chronisch-entzündlichen Darmerkrankungen. Immunsuppressiva wie Glucocorticoide und non-steroidale anti-inflammatorische Substanzen (NSAIDs), Cortison, Chloroquin, Cyclosporin, Azathioprin, Belimumab, Rituximab, Cyclophosphamid zur Behandlung von Lupus erythematodes. Beispielhaft aber nicht ausschließlich Calcineurinhemmer (z.B. Tacrolimus und Ciclosporin), Zellteilungshemmer (z.B. Azathioprin, Mycophenolat Mofetil, Mycophenolsäure, Everolimus oder Sirolimus), Rapamycin, Basiliximab, Daclizumab, Anti-CD3-Antikörper, Anti-T-Lymphozytenglobulin/Anti-Lymphozytenglobulin bei Organtransplantation. Vitamin D3-Analoga wie beispielsweise Calcipotriol, Tacalcitol oder Calcitriol; Salicylsäure, Harnstoff, Ciclosporin, Methotrexat, Efalizumab bei dermatologischen Erkrankungen.

Weiterhin seien Arzneimittel genannt, die mindestens eine der erfindungsgemäßen Verbindungen und einen oder mehrere weitere Wirkstoffe enthalten, insbesondere EP4-Inhibitoren (Prostaglandin E2 Receptor 4 Inhibitoren), P2X3- Inhibitoren (P2X Purinoceptor 3), PTGES-Inhibitoren (Prostaglandin E Synthase Inhibitoren) oder AKR1C3-Inhibitoren (Aldo-keto reductase family 1 member C3 Inhibitoren), zur Behandlung und/oder Prävention der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, über das Ohr oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorpher und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Ein weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Herstellung der erfindungsgemäßen Verbindungen

Die Herstellung der erfindungsgemäßen Verbindungen wird durch die folgenden Syntheseschemata verdeutlicht.

Als Ausgangsmaterial zur Synthese einiger der erfindungsgemäßen Verbindungen werden Carbonsäuren R²-CO₂H verwendet, in denen R² die unter der Formel (I) angegebene Bedeutung hat, welche kommerziell erhältlich sind oder auf literaturbekannten oder analog zu literaturbekannten Wegen (siehe zum Beispiel European Journal of Organic Chemistry 2003, 8, 1559-1568, Chemical and Pharmaceutical Bulletin, 1990, 38, 9, 2446-2458, Synthetic Communications 2012, 42, 658-666, Tetrahedron, 2004, 60, 51, 11869-11874) hergestellt werden können. Einige Carbonsäuren R²-CO₂H, in denen R² die unter der Formel (I) angegebene Bedeutung hat, können ausgehend von Carbonsäureestern durch Verseifung (vergleiche zum Beispiel die Umsetzung von Ethyl-6-(hydroxymethyl)pyridin-2-carboxylat mit wässriger Natriumhydroxidlösung in Methanol, WO2004113281) oder - in dem Fall, dass es sich um einen *tert-*Butylester handelt - durch Reaktion mit einer Säure wie zum Beispiel Chlorwasserstoff oder Trifluoressigsäure (vergleiche zum Beispiel Dalton Transactions, 2014 , 43, 19, 7176-7190) hergestellt werden. Die Carbonsäuren R²-CO₂H können auch in Form ihrer Alkalimetallsalze in Reaktionen eingesetzt werden. Die Herstellung von Carbonsäureestern als Startmaterialien für die Herstellung der Carbonsäuren R²-CO₂H kann gegebenenfalls aus halogenierten Bausteinen R²-I, R²-Br oder R²-Cl mit R² wie in Formel (I) definiert erfolgen, durch Umsetzung in einer Kohlenmonoxid-Atmosphäre gegebenenfalls unter Überdruck in Gegenwart eines Phosphinliganden wie zum Beispiel 1,3-Bis(diphenylphoshino)propan, einer Palladium-Verbindung wie zum Beispiel Palladium(II)acetat und einer Base wie zum Beispiel Triethylamin unter Zusatz von Ethanol oder Methanol in einem Lösungsmittel wie zum Beispiel Dimethylsulfoxid (für Herstellungsmethoden vergleiche zum Beispiel WO2012112743, WO 2005082866, Chemical Communications (Cambridge, England), 2003, 15, 1948-1949, WO200661715). Die Ausgangsmaterialien R²-I, R²-Br oder R²-Cl sind entweder kommerziell erhältlich oder können auf literaturbekannten Wegen hergestellt werden. Beispielhafte Herstellungsmethoden sind in
WO2012061926, European Journal of Organic Chemistry, 2002, 2, 327-330, Synthesis, 2004, 10, 1619-1624, Journal of the American Chemical Society, 2013, 135, 32, 12122-12134, Bioorganic and Medicinal Chemistry Letters, 2014, 24, 16, 4039-4043, US2007185058, WO2009117421 aufgeführt.

Einige für die Synthese der erfindungsgemäßen Verbindungen benötigten Azidverbindungen (Intermediate 2) können gemäß dem Syntheseschema 1 hergestellt werden. Ausgehend von geeigneten, literaturbekannten (beispielhafte Synthesemethoden: Synthesis, 2009, 12, 2040-2060; Journal of Fluorine Chemistry, 1995, 70, 39-44) oder kommerziell erhältlichen Aldehyden können Intermediate 1 durch Nitrierung hergestellt werden. Hierbei können dem Fachman bekannte Nitrierungsmethoden benutzt werden (beispielhafte Synthesemethoden: WO2013174744, Journal of Medicinal Chemistry, 2013, 56, 4343-4356). Bevorzugt ist die Verwendung von Nitriersäure oder Kaliumnitrat in konzentrierter Schwefelsäure. Die Intermediate 1 können dann mit Aziden wie zum Beispiel Natriumazid in einem Lösungsmittel wie zum Beispiel Dimethylsulfoxid in die Intermediate 2 umgewandelt werden.

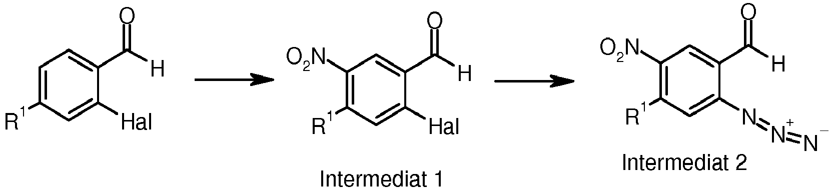

Hal bedeutet Fluor oder Chlor, bevorzugt ist Fluor.
Der Substituent R¹ hat die in der allgemeinen Formel (I) angegebene Definition.

### Syntheseschema 1

Ausgehend von geeigneten Azidverbindungen (Intermediate 2) können durch Umsetzung mit primären Aminen Indazole (Intermediate 3) hergestellt werden (Syntheseschema 2). Hierfür kommen beispielhaft Methoden aus Chemical Communications 2011, 47, 10133-10135 und RSC Adv., 2014, 4, 34232-34236 in Betracht. Alternativ können jedoch auch verwandte Methoden wie zum Beispiel in Organic Process Research and Development, 2011, 15, 4, 831-840, Chemistry of Heterocyclic Compounds, 2001, 37, 504-505 und Organic Letters, 2011, 13, 3542-3545 beschrieben verwendet werden. Bevorzugt ist die Umsetzung des Intermediates 2 mit einem Amin R³-NH₂ mit R³ wie für Formel (I) definiert in einem Lösungsmittel wie zum Beispiel Dichlormethan in Gegenwart von aktiviertem Molsieb. Alternativ kann auch Trimethoxymethan (CAS 149-73-5) verwendet werden und als Lösungsmittel Toluol.

Gegebenenfalls können die verwendeten Amine R³-NH₂ funktionelle Gruppen aufweisen, die vorher gegebenenfalls mit einer Schutzgruppe geschützt wurden. Diese Schutzgruppe kann nach der Reaktion oder auch in einem späteren Schritt der Synthese der erfindungsgemäßen Verbindungen abgespalten werden (vergleiche für die Einführung und Abspaltung von geeigneten Schutzgruppen auch P. G. M. Wuts, T. W. Greene, Greene's Protective Groups in Organic Synthesis, Fourth Edition, ISBN: 9780471697541).

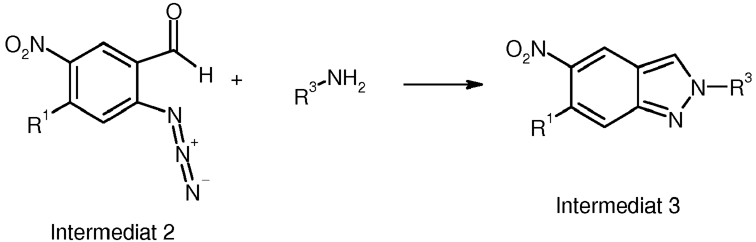

Die Substituenten R¹ und R³ haben die in der allgemeinen Formel (I) angegebenen Definitionen.

### Syntheseschema 2

Alternativ können Intermediate 3 auch ausgehend von den Intermediaten 7 hergestellt werden (siehe Syntheseschema 3). Hierfür kommen Reaktionen mit Chlorverbindungen (R³-Cl), Bromidverbindungen (R³-Br), Iodverbindungen (R³-I), Methansulfonaten (R³-OMs) oder 4-Methylbenzolsulfonaten (R³-OTs) in Betracht. Die verwendeten Halogenidverbindungen oder 4-Methylbenzolsulfonate sind kommerziell erhältlich oder können analog zu literaturbekannten Wegen hergestellt werden (für die Herstellung von 4-Methylbenzolsulfonaten sei zum Beispiel die Umsetzung eines entsprechenden Alkoholes mit 4-Methylbenzolsulfonylchlorid in Gegenwart von Triethylamin oder Pyridin genannt, siehe zum Beispiel Bioorganic and Medicinal Chemistry, 2006, 14, 12 4277-4294). Gegebenenfalls kann bei der Verwendung von Chlorverbindungen oder Bromverbindungen noch ein Alkalimetalliodid zugegeben werden wie Kaliumiodid oder Natriumiodid. Als Basen können beispielsweise Kaliumcarbonat, Cäsiumcarbonat oder Natriumhydrid verwendet werden. Als Lösungsmittel kommen zum Beispiel 1-Methylpyrrolidin-2-on, DMF, DMSO oder THF in Betracht. Gegebenenfalls können die verwendeten Halogenverbindungen oder 4-Methylbenzolsulfonate funktionelle Gruppen aufweisen, die vorher gegebenenfalls mit einer Schutzgruppe geschützt wurden (vergleiche auch P. G. M. Wuts, T. W. Greene, Greene's Protective Groups in Organic Synthesis, Fourth Edition, ISBN: 9780471697541). Kommen beispielsweise Halogenverbindungen oder Alkyl-4-methylbenzolsulfonate zum Einsatz, die eine oder mehrere Hydroxygruppen aufweisen, so können diese Hydroxygruppen gegebenenfalls durch eine *tert*-Butyl(dimethyl)silyl-Gruppe oder eine ähnliche dem Fachmann geläufige Silicium-haltige Schutzgruppe geschützt vorliegen. Alternativ können die Hydroxygruppen aber auch mit der Tetrahydro-2H-pyran (THP)-Gruppe oder mit der Acetyl oder Benzoylgruppe geschützt vorliegen. Die verwendeten Schutzgruppen können dann nachfolgend der Synthese von Intermediat 3, aber auch als letzte Stufe zur Freisetzung der erfindungsgemäßen Verbindungen abgespalten werden. Wird zum Beispiel eine *tert-*Butyl(dimethylsilyl)gruppe als Schutzgruppe verwendet, so kann diese unter Verwendung von Tetrabutylammoniumfluorid in einem Lösungsmittel wie zum Beispiel THF abgespalten werden. Eine THP-Schutzgruppe kann zum Beispiel unter Verwendung von 4-Methylbenzolsulfonsäure (ggf. als Monohydrat) abgespalten werden. Acetylgruppen oder Benzoylgruppen können durch Behandlung mit wässriger Natronlauge abgespalten werden.

Alternativ kann die Herstellung von Intermediat 3 durch Mitsunobu Reaktion (siehe zum Beispiel K. C. K. Swamy et. al. Chem. Rev. 2009, 109, 2551-2651) von Intermediat 7 mit Alkoholverbindungen (R³-OH) erfolgen. Verschiedene Phosphine wie Triphenylphosphin, Tributylphosphin oder 1,2-Diphenylphosphinoethan in Kombination mit Azodicarbonsäurediisopropylester (CAS 2446-83-5) oder weiteren in der Literatur genannten Diazenderivaten (K. C. K. Swamy et. al., Chem. Rev. 2009, 109, 2551-2651) können benutzt werden. Bevorzugt ist die Verwendung von Triphenylphosphin und Azodicarbonsäurediisopropylester. Trägt die Alkoholverbindung (R³-OH) eine funktionelle Gruppe, so können - wie bei den oben genannten Reaktionen mit Halogenverbindungen - bekannte Schutzgruppenstrategien verwendet werden (weitere Hinweise sind P. G. M. Wuts, T. W. Greene, Greene's Protective Groups in Organic Synthesis, Fourth Edition, ISBN: 9780471697541 zu entnehmen).

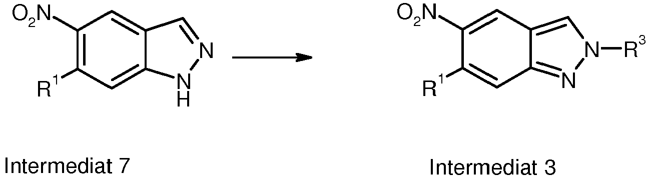

Die Substituenten R¹ und R³ haben die in der allgemeinen Formel (I) angegebenen Definitionen.

### Syntheseschema 3

Ausgewählte Intermediate 7 können durch Nitrierung von geeigneten 6-substituierten Indazolen erhalten werden (siehe Syntheseschema 4). Hierfür kommen dem Fachmann bekannte Nitrierungsmethoden wie zum Beispiel die Verwendung von Salpetersäure in Kombination mit konzentrierter Schwefelsäure in Betracht. Einige Intermediate 7 sind literaturbekannt oder kommerziell erhältlich oder können analog zu literaturbekannten Wegen synthetisiert werden (vergleiche zum Beispiel Bioorganic and Medicinal Chemistry, 2004, 12, 2115-2137).

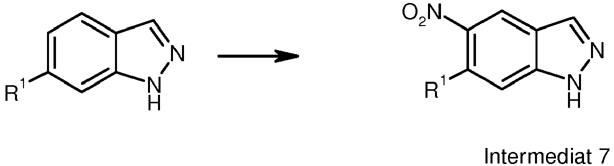

Der Substituent R¹ hat die in der allgemeinen Formel (I) angegebenen Definitionen.

### Syntheseschema 4

Ausgehend von den Intermediaten 3 können Intermediate 4 hergestellt werden durch Reduktion der Nitrogruppe (siehe Syntheseschema 5). Beispielsweise kann die Nitrogruppe mit Palladium auf Kohlenstoff unter einer Wasserstoffatmosphäre reduziert werden (vergleiche zum Beispiel WO2013174744 für die Reduktion von 6-Isopropoxy-5-nitro-1H-indazol zu 6-Isopropoxy-1H-indazol-5-amin) oder durch die Verwendung von Eisen und Ammoniumchlorid in Wasser und Ethanol (siehe zum Beispiel auch Journal of the Chemical Society, 1955, 2412-2419) oder durch die Verwendung von Zinn(II)-chlorid (CAS 7772-99-8) (vergleiche zum Beispiel Bioorganic and Medicinal Chemistry, 2004, 12, 2115-2137). Die Verwendung von Eisen und Ammoniumchlorid in Wasser und Ethanol sowie die Verwendung von Palladium auf Kohlenstoff unter einer Wasserstoffatmosphäre sind bevorzugt.

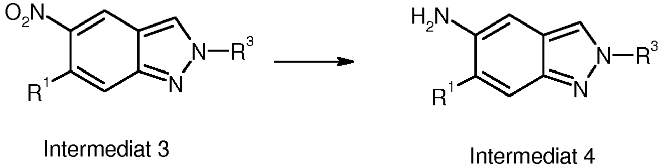

Die Substituenten R¹ und R³ haben die in der allgemeinen Formel (I) angegebenen Definitionen.

### Syntheseschema 5

Ausgehend von den Intermediaten 4 können erfindungsgemäße Verbindungen der allgemeinen Formel (I) hergestellt werden (siehe Syntheseschema 6). Hierfür können verschiedene in der Literatur bekannte Kupplungsreagenzien eingesetzt werden (Amino Acids, Peptides and Proteins in Organic Chemistry. Vol.3-Building Blocks, Catalysis and Coupling Chemistry, Andrew B. Hughes, Wiley, Kapitel 12 - Peptide-Coupling Reagents, 407-442; Chem. Soc. Rev., 2009, 38, 606). Beispielsweise können 1-(3-Dimethylaminopropyl)-3-ethylcarbodiimidhydrochlorid in Kombination mit 1-Hydroxy-1H-benzotriazol Hydrat (HOBt, WO2012107475; Bioorg. Med. Chem. Lett., 2008, 18, 2093), (1H-Benzotriazol-1-yloxy)(dimethylamino)-N,N-dimethylmethaniminiumtetrafluoroborat (TBTU, CAS 125700-67-6), (Dimethylamino)-N,N-dimethyl(3H-[1,2,3]triazolo[4,5-b]pyridin-3-yloxy)methaneiminiumhexafluorophosphat (HATU, CAS 148893-10-1), Propanphosphonsäureanhydrid (als Lösung in Ethylacetat oder DMF, CAS68957-94-8) oder Di-1H-imidazol-1-ylmethanon (CDI) als Kupplungsreagenzien verwendet werden, wobei jeweils zur Reaktionsmischung noch eine Base wie Triethylamin oder N-Ethyl-N-isopropylpropan-2-amin gegeben wird. Bevorzugt sind die Kupplungsreagenzien HATU, N-[3-(Dimethylamino)propyl]-N'-ethylcarbodiimid (EDC, CAS1892-57-5) in Kombination mit 1H-Benzotriazol-1-olhydrat (1:1) (HOBt, CAS123333-53-9) und TBTU. Bevorzugt als Base ist die Verwendung von N-Ethyl-N-isopropylpropan-2-amin. Als Lösungsmittel bevorzugt sind THF oder DMF.

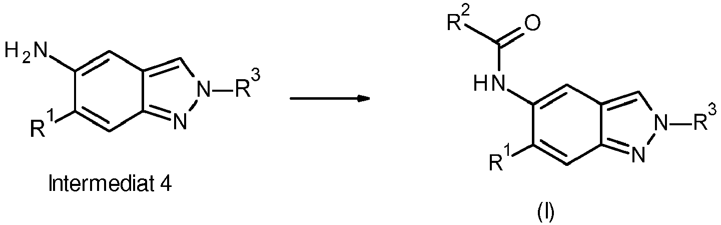

Herstellung von Verbindungen der allgemeinen Formel (I) aus Intermediat 4. Die Substituenten R¹, R² und R³ haben die in der allgemeinen Formel (I) angegebenen Definitionen

### Syntheseschema 6

Ausgehend von literaturbekannten oder kommerziell erhältlichen 6-substituierten Indazolen können ausgewählte Intermediate 6 hergestellt werden durch die Mitsubobu Reaktion mit Alkoholen (R³-OH) oder durch Alkylierung mit den entsprechenden Halogenid-Reagenzien R³-Cl, R³-Br, R³-I oder Methansulfonaten (R³-OMs) oder 4-Methylbenzolsulfonaten (R³-OTs) (vergleiche Syntheseschema 7). Für die Mitsunobu-Reaktion und die Alkylierung kommen Methoden wie bei Syntheseschema 3 beschrieben in Betracht. Zur Synthese von Intermediat 3 ausgehend von Intermediat 6 können Nitrierungsmethoden wie bei Syntheseschema 4 beschrieben Anwendung finden.

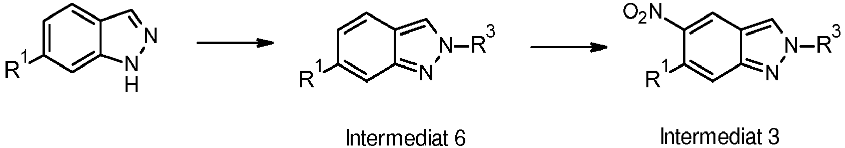

Alternative Herstellung von Intermediat 3: Die Substituenten R¹ und R³ haben die in der allgemeinen Formel (I) angegebenen Definitionen.

### Syntheseschema 7

Alternativ können die Verbindungen der allgemeinen Formel (I) ausgehend von Intermediat 5 erhalten werden. Intermediat 5 kann hierfür mit Halogenverbindungen R³-I, R³-Br, R³-Cl, Methansulfonaten (R³-OMs) oder 4-Methylbenzolsulfonaten (R³-OTs) umgesetzt werden wie bei Syntheseschema 3 beschrieben. Intermediat 5 lässt sich herstellen aus dem entsprechenden 5-Amino-indazol durch eine Amidsynthese mit einer Carbonsäure R²-CO₂H in Gegenwart eines Kupplungsreagenzes wie bei Syntheseschema 6 beschrieben. Geeignete 5-Amino-indazole sind kommerziell erhältlich oder können auf literaturbekannten Wegen hergestellt werden.

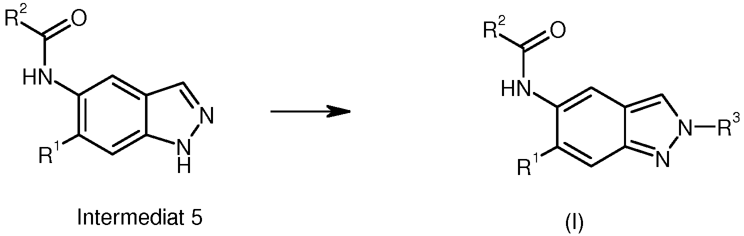

Herstellung vonVerbindungen der allgemeinen Formel (I) aus Intermediat 5. Die Substituenten R¹, R² und R³ haben die in der allgemeinen Formel (I) angegebenen Definitionen.

### Syntheseschema 8

Hat R¹ in der allgemeinen Formel (I) dieBedeutung -CO₂Me (siehe Formel (I)-a), so kann diese Gruppe -CO₂Me in alternative funktionelle Gruppen wie dem Fachmann bekannt umgesetzt werden. Insbesondere können durch Reaktion mit Methylmagnesiumbromid Verbindungen der allgemeinen Formel (I) mit der Bedeutung R¹ = -C(OH)(CH₃)₂ erhalten werden. Des Weiteren können durch Reaktion mit Ammoniak-Verbindungen der allgemeinen Formel (I), in denen R¹ = - CO₂NH₂ bedeutet, hergestellt werden.

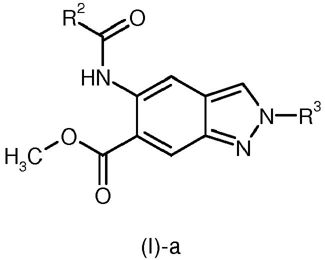

Die Substituenten R² und R³ haben die in der allgemeinen Formel (I) angegebenen Definitionen.

Weist R³ geeignete funktionelle Gruppen auf, so können diese mit dem Fachmann bekannten Methoden derivatisiert werden (vergleiche zum Beispiel Science of Synthesis, Georg Thieme Verlag). Zum Beispiel können sekundäre Amine im Rahmen einer Alkylierung (vergleiche zum Beispiel die Alkylierung von 1-(Piperidin-4-yl)-1H-indazol mit 1-Brom-2-(2-methoxyethoxy)ethan in WO2007142584 oder die Alkylierung eines Pyrazol-Piperidinderivates mit 2-Bromethanol in US2015133422) oder im Rahmen einer reduktiven Aminierung zu tertiären Aminogruppen umgesetzt werden (vergleiche zum Beispiel WO20148992 für eine reduktive Aminierung mit Oxetan-3-on oder US2002156081 für die reduktive Aminierung eines Piperidinderivats mit Acetaldehyd). Bevorzugt ist für die reduktive Aminierung die Verwendung von Natriumtriacetoxyborohydrid in Gegenwart von Essigsäure. Für die Alkylierung bevorzugt ist die Verwendung von Kaliumcarbonat als Base. Darüberhinaus können sekundäre Amine mit Carbonsäuren im Rahmen einer Amidkupplung (zum Beispiel durch Verwendung von Bedingungen wie bei Syntheseschema 6) umgesetzt werden.
Handelt es sich beispielsweise bei der funktionellen Gruppe um eine Sulfidgruppe, so kann diese durch literaturbekannte Methoden zu einer Sulfoxid oder Sulfongruppe oxidiert werden. Handelt es sich um eine Sulfoxidgruppe so kann diese ebenfalls zu einer Sulfongruppe oder Sulfoximingruppe (vgl. Angewandte Chemie, 2013, 125, 9570) oxidiert werden. Beispielsweise kann für die Oxidationsschritte 3-Chloroperbenzoesäure (CAS 937-14-4) verwendet werden (vergleiche hierzu zum Beispiel auch US201094000 für die Oxidation eines 2-(Methylsulfanyl)ethyl-1H-pyrazol-Derivates zu einem 2-(Methylsulfinyl)ethyl-1H-pyrazol-Derivat und die Oxidation eines weiteren 2-(Methylsulfanyl)ethyl-1H-pyrazol-Derivates zu einem 2-(Methylsulfonyl)ethyl-1H-pyrazol-Derivat). Handelt es sich bei der funktionellen Gruppe um eine Ketogruppe, so kann diese durch dem Fachmann bekannte Reduktionsmethoden zu einer Alkoholgruppe reduziert werden (vergleiche zum Beispiel Chemische Berichte, 1980, 113, 1907-1920 für die Verwendung von Natriumborhydrid).

### Synthese der Beispielverbindungen

### Abkürzungen und Erläuterungen

| | |
|---|---|
| DMF | *N*,*N*-Dimethylformamid |
| DMSO | Dimethylsulfoxid |
| THF | Tetrahydrofuran |
| RT | Raumtemperatur |
| d. Th. | Der Theorie |
| HPLC | Hochleistungsflüssigkeitschromatographie |
| h | Stunde(n) |
| min | Minute(n) |
| UPLC | Ultra-Hochleistungsflüssigchromatographie |
| DAD | Diodenarraydetektor |
| ELSD | Verdampfungslichtstreudetektor |
| ESI | Elektronenspray- Ionisation |
| SQD | Single Quadrupole Detektor |
| KPG | Kerngezogenes Präzisions-Glasgerät |

Der Begriff *Kochsalzlösung* bedeutet eine gesättigte wässrige Natriumchloridlösung.

Die chemische Bezeichnung der Intermediate und Beispiele wurde unter Verwendung der Software von ACD / LABS (Batch Version 12.01.) erstellt.

### Methoden

In einigen Fällen wurden die erfindungsgemäßen Verbindungen sowie deren Vor- und/oder Zwischenstufen durch LC-MS analysiert:

### LC-MS Methoden (analytisch):

### Methode A:

MS Instrument: Waters ZMD Massenspektrometer; HPLC Instrument: Agilent 1100; Säule: Phenomenex Luna C18 (2) 3.0 micron 30mm x 4.6mm; Mobile Phase A: Wasser 0.1% Ameisensäure, Mobile Phase B: Acetonitril 0.1% Ameisensäure; Gradient: 0.0 min 95% A -> 0.5 min 95% A -> 4.5 min 5% A -> 5.5 min 5% A; Flussrate: 2.0 ml/min; UV Detektion: 190 - 450nM.

### Methode B:

MS Instrument: Waters Micromass ZQ2000; HPLC Instrument: Waters Acquity UPLC System; Säule: Acquity UPLC BEH C18 1.7micron 100 mm x 2.1 mm; Mobile Phase A: Wasser 0.1% Ameisensäure, Mobile Phase B: Acetonitril 0.1% Ameisensäure; Gradient: 0.0 min 95% A -> 0.4 min 95% A -> 6.0 min 5% A -> 6.8 min 5% A; Flussrate: 0.4 ml/min; UV Detektion: PDA.

### Methode C: UPLC (MeCN-HCOOH):

Instrument: Waters Acquity UPLC-MS SQD 3001; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C.; Injektion: 2 µl; DAD scan: 210-400 nm; MS ESI+, ESI-, scan range 160-1000 m/z; ELSD.

### Methode D: UPLC (MeCN-NH₃):

Instrument: Waters Acquity UPLC-MS SQD 3001; Säule: Acquity UPLC BEH C18 1.7 50x2.1mm; Eluent A: Wasser + 0.2% Vol. Ammoniak (32%), Eluent B: Acetonitril; Gradient: 0-1.6 min 1-99% B, 1.6-2.0 min 99% B; Fluss 0.8 ml/min; Temperatur: 60 °C; Injektion: 2µl; DAD scan: 210-400 nm; MS ESI+, ESI-, scan range 160-1000 m/z; ELSD.

In einigen Fällen wurden die erfindungsgemäßen Verbindungen sowie deren Vor- und/oder Zwischenstufen mit folgenden beispielhaften präparativen HPLC-Methoden gereinigt:

### LC-MS Methode (präparativ):

### Methode E:

MS Instrument: Agilent 1260 infinity Reinigungssystem. Agilent 6100 series single Quadrupole LC/MS; Säule: XSEELECT CSH Prep C18 5µm OBD, 30X150mm; Mobile Phase A: 0.1% wässrige Ameisensäure, Mobile Phase B: 0.1% Ameisensäure in Acetonitril; Gradient: 10%-95%, 22min, zentriert um einen spezifisch fokussierten Gradienten; Flussrate: 60 ml/min. Probe: Injektion von 20-60 mg/ml Lösung in DMSO (+ optional Ameisenäure und Wasser)

### Methode F:

MS Instrument: Agilent 1260 Infinity Reinigungssystem. Agilent 6100 series single Quadrupole LC/MS; Säule: XBridge Prep C18 5µm OBD, 30X150mm; Mobile Phase A: 0.1% wässriges Ammoniak, Mobile Phase B: 0.1% Ammoniak in Acetonitril; Gradient: 10%-95%, 22min, zentriert um einen spezifisch fokussierten Gradienten; Flussrate: 60 ml/min. Probe: Injektion einer 20-60mg/ml Lösung in DMSO (+ optional Ameisensäure und Wasser)

### Methode G:

System: Waters Autopurificationsystem: Pump 2545, Sample Manager 2767, CFO, DAD 2996, ELSD 2424, SQD; Säule: XBrigde C18 5µm 100x30 mm; Eluent: A: Wasser + 0.1% Vol. Ameisensäure, Eluent B: Acetonitril; Gradient: 0-8 min 10-100% B, 8-10 min 100% B; Flow: 50 mL/min; Temperatur: Raumtemperatur; Lösung: Max. 250 mg / max. 2.5 mL DMSO o. DMF; Injektion: 1 x 2.5 mL; Detection: DAD scan range 210-400 nm; MS ESI+, ESI-, scan range 160-1000 m/z.

### Methode H:

Waters Autopurificationsystem: Pump 254, Sample Manager 2767, CFO, DAD 2996, ELSD 2424, SQD 3100; Säule: XBrigde C18 5µm 100x30 mm; Eluent: A: Wasser + 0.2% Vol. Ammoniak (32%), Eluent B: Methanol; Gradient: 0-8 min 30-70% B; Fluss: 50 mL/min; Temperatur: Raumtemperatur; Detektion: DAD scan range 210-400 nm; MS ESI+, ESI-, scan range 160-1000 m/z; ELSD.

In einigen Fällen erfolgte die Aufreinigung von Substanzgemischen durch Säulenchromatographie an Kieselgel.

Zur Herstellung einiger der erfindungsgemäßen Verbindungen sowie deren Vorstufen und/oder Zwischenstufen wurde eine säulenchromatographische Reinigung ("Flash-Chromatographie") an Kieselgel durchgeführt unter Verwendung von Geräten Isolera® der Firma Biotage. Hierbei kamen Kartuschen der Firma Biotage wie zum Beispiel die Kartusche "SNAP Cartridge, KP_SIL" unterschiedlicher Größe sowie Kartuschen "Interchim Puriflash Silica HP 15UM flash column" der Firma Interchim unterschiedlicher Größe zum Einsatz.

### Ausgangsverbindungen

### Intermediat V2-1

### Methyl-6-(2-hydroxypropan-2-yl)pyridin-2-carboxylat

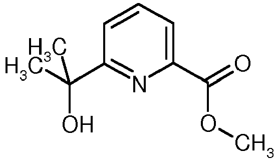

2.00 g (9.26 mmol) 2-(6-Bromopyridin-2-yl)propan-2-ol (CAS 638218-78-7) wurden in 20 ml Methanol und 20 ml DMSO gelöst. Anschließend wurden 250 mg 1,3-Bis(diphenylphoshino)-propan, 130 mg Palladium(II)acetat und 3 ml Triethylamin zugegeben. Das Reaktionsgemisch wurde bei Raumtemperatur dreimal mit Kohlenmonoxid gespült und unter 13 bar Kohlenmonoxidatmosphäre 30 min gerührt. Man entfernte die Kohlenmonoxidatmosphäre durch Anlegen eines Vakuums und rührte unter 14 bar Kohlenmonoxidatmosphäre bei 100°C 24 h. Man entspannte den Autoklaven, versetzte die Reaktionsmischung mit Wasser, extrahierte dreimal mit Ethylacetat, wusch mit gesättigter wässriger Natriumhydrogencarbonatlösung, Kochsalzlösung, filtrierte über einen wasserabweisenden Filter und engte ein. Man erhielt 1.60 g eines Rohproduktes.
UPLC-MS (Methode C): Rt = 0.76 min (UV Detektor: TIC), gefundene Masse 195.00.

### Intermediat V3-1

### Kalium-6-(2-hydroxypropan-2-yl)pyridin-2-carboxylat

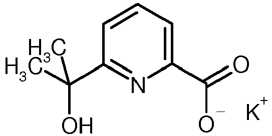

1.60 g des Rohproduktes Intermediat 0-1 wurden in 15 ml Methanol vorgelegt, man addierte 0.74 g Kaliumhydroxid und rührte 16.5 h bei 50°C. Nach Einengen erhielt man 2.1 g eines Rückstandes, welcher ohne weitere Reinigung eingesetzt wurde.
UPLC-MS (Methode C): Rₜ = 0.47 min (UV Detektor: TIC), gefundene Masse 181.00.

### Intermediat 1A

### 2-Fluor-4-methoxy-5-nitrobenzaldehyd

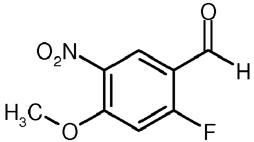

Zu 21 ml gekühlter Schwefelsäure wurden 3 g (19.5 mmol) 2-Fluor-4-methoxybenzaldehyd CAS: [331-64-6] gegeben und die Lösung wurde gekühlt gehalten in einem Temperaturbereich zwischen -25°C und -15°C. 1.83 g 70% Nitriersäure wurde tropfenweise zugegeben und die Mischung wurde 45 Minuten lang gerührt zwischen -25°C und -15°C. Die Mischung wurde auf Eiswasser gegeben (100 ml), 30 Minuten stehen gelassen und der Feststoff wurde abfiltriert und mit Wasser gewaschen. Der Feststoff wurde in Dichlormethan gelöst, die Lösung mit Natriumhydrogencarbonatlösung gewaschen und eingeengt. Nach Reinigung durch Flash-Chromatographie (Biotage Isolera, 100g Kieselgelsäule, Cyclohexan/Ethylacetat-Gradient) wurden 2.73 g (13.7 mmol) der Titelverbindung erhalten.
LC-MS (Methode A): Rt = 3.19 min; m/z = 200 (M+H)+
¹H-NMR (300 MHz, CDCl₃): δ = 4.06 (s, 3H), 6.87 (d, 1H), 8.45 (d, 1H), 10.22 (s, 1H)

### Intermediat 1B

### 4-Chlor-2-fluor-5-nitrobenzaldehyd

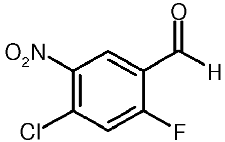

Zu einer auf 0°C abgekühlten Lösung aus 2.81 g Kaliumnitrat in 21 ml Schwefelsäure wurden 4.0 g 4-Chlor-2-fluorbenzaldehyd gegeben und man rührte 0.5 h bei 0°C und 1h bei Raumtemperatur. Die Mischung wurde auf Eiswasser gegeben und zweimal mit Dichlormethan extrahiert. Die vereinigten organischen Phasen wurden mit gesättigter Natriumhydrogencarbonatlösung gewaschen, die organische Phase wurde abgetrennt und eingeengt. Man erhielt 5.1 g der Titelverbindung.
¹H-NMR (300 MHz, CDCl3): δ = 7.47 (d, 1H), 8.46 (d, 1H), 10.31 (s, 1H)

### Intermediat 2A

### 2-Azido-4-methoxy-5-nitrobenzaldehyd

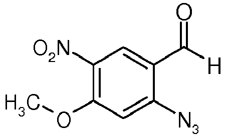

Zu einer Lösung aus 2.73 g (13.7 mmol) 2-Fluor-4-methoxy-5-nitrobenzaldehyd (Intermediat 1A) in 50 ml of Dimethylsulfoxid wurden 1.78 g (27 mmol) Natriumazid gegeben und die Mischung wurde bei Raumtemperatur 0.5 h gerührt. Die Mischung wurde mit 500 ml Ethylacetat verdünnt, dreimal mit Wasser und Kochsalzlösung gewaschen, getrocknet über Natriumsulfat, filtriert und eingeengt. Man erhielt 2.91 g (13.1 mmol) der Titelverbindung.
LC-MS (Methode A): Rt = 3.41 min; keine Ionisation (M+H)+
¹H-NMR (300 MHz, CDCl₃): δ = 4.09 (s, 3H), 6.80 (s, 1H), 8.46 (s, 1H), 10.20 (s, 1H)

### Intermediat 2B

### 2-Azido-4-chlor-5-nitrobenzaldehyd

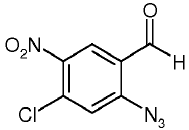

5.1 g 4-Chlor-2-fluor-5-nitrobenzaldehyd (Intermediat 1B) wurden analog zur Herstellung von Intermediat 1B mit 1.63 g Natriumazid in 80 ml DMSO innerhalb von 1 h umgesetzt. Man erhielt 5.35 g der Titelverbindung.
¹H-NMR (300 MHz, CDCl3): δ = 7.43 (s, 1H), 8.46 (s, 1H), 10.28 (s, 1H)

### Intermediat 3A

### 6-Methoxy-5-nitro-2-(tetrahydro-2H-pyran-4-yl)-2H-indazol

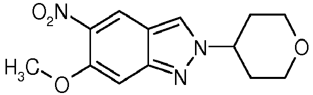

Zu einer Lösung aus 2.4 g (10.8mmol) 2-Azido-4-methoxy-5-nitrobenzaldehyd (Intermediat 2A) in 70 ml Dichlormethan wurden 1.12 ml (10.8 mmol) Tetrahydro-2H-pyran-4-amin [38041-19-9] und 5 g aktiviertes 4 Angstrom Molsieb gegeben. Die Reaktion wurde 3 h gerührt bei Raumtemperatur. Die Lösung wurde durch Celite filtriert und die Celite wurde mit Dichlormethan nachgewaschen und man entfernte das Lösungsmittel im Vakuum. Das Rohprodukt wurde in 50 ml trockenen Toluol gelöst und 1 h erhitzt bei 120°C. Man ließ auf Raumtemperatur kommen und entfernte das Lösungsmittel im Vakuum. Der Rückstand wurde gereinigt durch Flashchromatograpie (Biotage Isolera, Cyclohexan / Ethylacetat). Man erhielt 2.22 g (8.0 mmol) der Titelverbindung.
LC-MS (Methode A): Rt = 3.15 min; m/z = 278 (M+H)+
¹H-NMR (300 MHz, CDCl₃): δ = 2.19 - 2.29 (m, 4H), 3.55 - 3.66 (m, 2H), 3.97 (s, 3H), 4.11 - 4.22 (m, 2H), 4.56 - 4.68 (m, 1H), 7.12 (s, 1H), 8.08 (s, 1H), 8.21 (s, 1H)

### Intermediat 3B

### tert-Butyl-4-(6-methoxy-5-nitro-2H-indazol-2-yl)piperidin-1-carboxylat

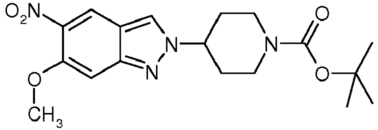

Zu einer Lösung aus 2.65 g (11.9 mmol) of 2-Azido-4-methoxy-5-nitrobenzaldehyd (Intermediat 2A) in 50 ml Dichlormethan wurden eine Lösung aus 2.38 g (11.9 mmol) tert-Butyl-4-aminopiperidin-1-carboxylat [87120-72-7] in 20 ml Dichlormethan und 5 g aktiviertes Molsieb (4 Angstrom) zugesetzt. Danach wurde bei Raumtemperatur 3.5 h gerührt. Eine weitere Lösung aus 0.79 g (4.0 mmol) tert-Butyl-4-aminopiperidin-1-carboxylat in 20 ml Dichlormethan und 5 g aktiviertes 4 Angstrom Molsieb wurden zugesetzt und die Mischung wurde gerührt bei Raumtemperatur 19 h. Die Lösung wurde durch Celite filtriert, mit Dichlormethan wurde nachgewaschen und das Filtrat wurde eingeengt im Vakuum, gelöst in 50 ml wasserfreien Toluol und dann wurde die Mischung 1 h erhitzt bei 120°C. Man ließ auf Raumtemperatur kommen, entfernte das Lösungsmittel im Vakuum und reinigte den Rückstand durch Flashchromatograpie (Biotage Isolera, 100 g Kieselgel-Säule, Cyclohexan / Ethylacetat) und erhielt 4.25 g (11.3 mmol) der Titelverbindung.
LC-MS (Methode A): Rt = 3.93 min; m/z = 399 (M+Na)+
¹H-NMR (300 MHz, CDCl3): δ = 1.49 (s, 9H), 2.08 (ddd, 2H), 2.24 (d, 2H), 2.95 (dd, 2H), 3.97 (s, 3H), 4.33 (d, 2H), 4.47 - 4.59 (m, 1H), 7.11 (s, 1H), 8.06 (s, 1H), 8.21 (s, 1H)

### Intermediat 3C

### 2-(1,1-Dioxidotetrahydro-2H-thiopyran-4-yl)-6-methoxy-5-nitro-2H-indazol

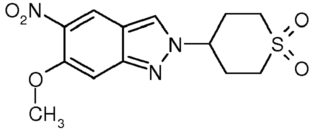

2.5 g (13.5 mmol) Tetrahydro-2H-thiopyran-4-amin-1,1-dioxid [116529-31-8] wurden in 100 ml Dichlormethan gelöst. Dann wurden 3.0 g (13.5 mmol) 2-Azido-4-methoxy-5-nitrobenzaldehyd (Intermediat 2A) und 12 g aktiviertes 4 Angstrom Molsieb zugesetzt. Die Mischung wurde 18 h gerührt bei Raumtemperatur unter Argon, dann filtriert durch Celite und die Celite gewaschen mit Dichlormethan. Das Filtrat wurde eingeengt im Vakuum, 60 ml wasserfreies Toluol wurde zugegeben, dann wurde die Mischung 1.5 h erhitzt bei 120°C. Die Reaktion wurde auf Raumtemperatur abgekühlt, die Lösungsmittel wurden eingeengt im Vakuum. Man erhielt 4.39 g (13.5 mmol) der Titelverbindung.
LC-MS (Methode A): Rt = 3.04 min; m/z = 326 (M+H)+
¹H-NMR (300 MHz, DMSO-d6): δ = 2.41 - 2.48 (m, 2H), 2.53 - 2.67 (m, 2H), 3.27 (s, 2H), 3.38 - 3.51 (m, 2H), 3.91 (s, 3H), 4.90 - 5.02 (m, 1H), 7.31 (s, 1H), 8.39 (s, 1H), 8.67 (s, 1H)

### Intermediat 3D

### 6-Methoxy-5-nitro-2-(tetrahydro-2H-thiopyran-4-yl)-2H-indazol

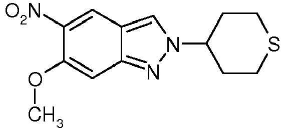

0.949 g (4.3 mmol) 2-Azido-4-methoxy-5-nitrobenzaldehyd (Intermediat 2A) in 75 ml Dichlormethan wurden mit 0.5 g (4.3 mmol) Tetrahydro-2H-thiopyran-4-amin [21926-00-1] und 2 g aktivierten 4 Angstrom Molsieb versetzt. Danach wurde bei Raumtemperatur 72 h gerührt. Weitere 75 mg (0.64 mmol) Tetrahydro-2H-thiopyran-4-amin [21926-00-1] und 2 g aktiviertes 4 Angstrom Molsieb wurden zugesetzt und es wurde bei Raumtemperatur 4 h gerührt. Die Mischung wurde durch Celite filtriert, man engte das Filtrat bei reduziertem Druck ein, man addierte 20 ml wasserfreies Toluol und erhitzte 1.5 h bei 120°C. Die Reaktion wurde auf Raumtemperatur abgekühlt und das Lösungsmittel entfernt bei reduziertem Druck. Nach Reinigung des Rückstandes (Biotage Isolera (100 g Kieselgel-Säule), Cyclohexan/Ethylacetat-Gradient) wurden 0.938 g (3.2 mmol) der Titelverbindung erhalten.
LC-MS (Methode A): Rt = 3.64 min; m/z = 294 (M+H)+
¹H-NMR (300 MHz, CDCl₃): δ = 2.23 - 2.38 (m, 2H), 2.51 - 2.60 (m, 2H), 2.81 - 2.94 (m, 4H), 3.97 (s, 3H), 4.34 - 4.46 (m, 1H), 7.11 (s, 1H), 8.06 (s, 1H), 8.21 (s, 1H)

### Intermediat 3E

### 6-Methoxy-5-nitro-2-(10-oxidotetrahydro-2H-thiopyran-4-yl)-2H-indazol (Isomerenmischung)

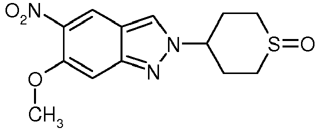

Zu einer Lösung aus 850 mg (2.9 mmol) 6-Methoxy-5-nitro-2-(tetrahydro-2H-thiopyran-4-yl)-2H-indazol (Intermediat 3D) in 51 ml 2:1 Tetrahydrofuran / Wasser bei 0°C wurden 445 mg (0.724 mmol) Oxon addiert und die Reaktion wurde 0.5 h gerührt. Zweimal wurden je 445 mg (0.724 mmol) Oxon zugesetzt innerhalb von 1 h. Dann wurden 0.5 g Natriummetabisulfit zugegeben und die Reaktion wurde zwischen Wasser und Ethylacetat verteilt, man trennte die organische Phase ab, extrahierte die wässrige Phase dreimal mit Ethylacetat, vereinigte die organischen Phasen, wusch mit Kochsalzlösung, Trocknete über Natriumsulfat, filtrierte und engte im Vakuum ein. Nach Reinigung (Biotage Isolera, 50 g Kieselgel-Säule, Dichlormethan/Methanol) wurden 710 mg (2.3 mmol) der Titelverbindung erhalten.
LC-MS (Methode A): Rt = 2.76 & 2.83 min; m/z = 310 (M+H)+
¹H-NMR (300 MHz, CDCl₃): δ = 2.29 - 2.43 (m, 2H), 2.71 - 3.03 (m, 4H), 3.28 (d, 1H) 3.40 - 3.48 (m, 1H), 3.96 - 4.03 (m, 3H), 4.57 - 4.77 (m, 1H), 7.10 (s, 1H), 8.09 - 8.22 (m, 2H)

### Intermediat 3F

### tert-Butyl-rel-(1S,4S,5R)-5-(6-methoxy-5-nitro-2H-indazol-2-yl)-2-azabicyclo[2.2.1]heptan-2-carboxylat

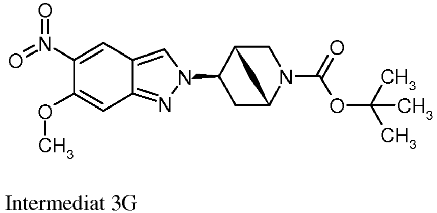

### tert-Butyl-rel-(1S,4S,5S)-5-(6-methoxy-5-nitro-2H-indazol-2-yl)-2-azabicyclo[2.2.1]heptan-2-carboxylat

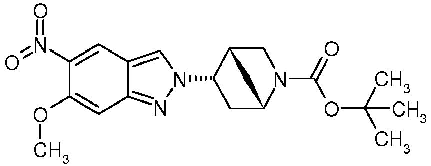

Zu einer Lösung aus 314 mg (1.4 mmol) 2-Azido-4-methoxy-5-nitrobenzaldehyd (Intermediat 2A) in 20 ml Dichlormethan wurde eine Lösung aus 330 mg (1.55 mmol) tert-Butyl-5-amino-2-azabicyclo[2.2.1]heptan-2-carboxylat [207405-62-7] in 10 ml Dichlormethan und 0.773 ml (7.0 mmol) Trimethoxymethan [149-73-5] gegeben und die Mischung wurde bei Raumtemperatur 24 h gerührt. Zur Mischung wurden 30 ml wasserfreies Toluol gegeben, Dichlormethan wurde zum Teil abgedampft und die Lösung wurde dann bei 120°C 1 h erhitzt. Man ließ auf Raumtemperatur kommen, entfernte die Lösungsmittel im Vakuum und reinigte säulenchromatographisch an Kieselgel (Biotage Isolera (50 g Kieselgel-Säule), Cyclohexan / Ethylacetat) und erhielt 193 mg (0.5 mmol) Intermediat 3F und 336 mg (0.87 mmol) Intermediat 3G.
LC-MS (Methode A): Rt = 4.03 min; m/z = 389 (M+H)+
¹H-NMR (300 MHz, CDCl3): δ = 1.48 (s, 9H), 1.78 (dd, 1H), 2.31 - 2.52 (m, 3H), 2.89 - 2.93 (m, 1H), 3.18 (dd, 1H), 3.38 (dd, 1H), 3.95 (s, 3H), 4.42 (d, 1H), 4.67 (dd, 1H), 7.10 (s, 1H), 8.08 (s, 1H), 8.18 (s, 1H). (Intermediat 3F)
LC-MS (Method A): Rt = 3.88 min; m/z = 333 (M-tBu+H)+
¹H-NMR (300 MHz, CDCl3): δ = 1.47 (d, 9H), 1.80 - 1.94 (m, 2H), 2.34 - 2.57 (m, 2H), 2.78 (dd, 1H), 3.13 - 3.23 (m, 2H), 3.96 (s, 3H), 4.39 (d, 1H), 5.07 - 5.14 (m, 1H), 7.08 (s, 1H), 8.05 (d, 1H), 8.19 (s, 1H) (Intermediat 3G)

### Intermediat 3H

### 6-Chlor-5-nitro-2-(tetrahydro-2H-pyran-4-yl)-2H-indazol

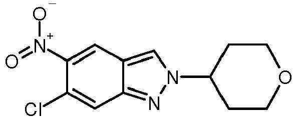

Zu einer Lösung aus 2.5 g (11.0 mmol) 2-Azido-4-chlor-5-nitrobenzaldehyd (Intermediat 2B) und 1.1 ml 4-Aminotetrahydro-2H-pyran in 50 ml Dichlormethan wurden 6 ml (55 mmol) Trimethoxymethan (CAS 149-73-5) gegeben und die Mischung wurde bei Raumtemperatur 17 h gerührt. 50 ml wasserfreies Toluol wurden zugegeben und das Lösungsmittel zum Teil evaporiert und der Rückstand bei 120°C 1 h erhitzt. Man engte ein und reinigte den Rückstand säulenchromatographisch an Kieselgel (Biotage Isolera, Cyclohexan / Ethylacetat). Man erhielt 2.06 g der Titelverbindung als gelben Feststoff.
¹H-NMR (300 MHz, CDCl3): δ = 2.20 - 2.30 (m, 4H), 3.56 - 3.66 (m, 2H), 4.14 - 4.23 (m, 2H), 4.62 - 4.75 (m, 1H), 7.85 (s, 1H), 8.19 (s, 1H), 8.34 (s, 1H).

### Intermediat 3I

### 6-Chlor-2-(1,1-dioxidotetrahydro-2H-thiopyran-4-yl)-5-nitro-2H-indazol

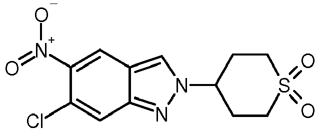

2.13 g Tetrahydro-2H-thiopyran-4-amin-1,1-dioxidhydrochlorid (1:1) wurden in einer Mischung aus Wasser und Methanol gelöst und gereinigt durch eine SCX Kartusche (Laufmittel 2M Ammoniak in Methanol). Das resultierende Amin wurde in 50 ml Dichlormethan gelöst und man addierte diese Lösung zu 2-Azido-4-chlor-5-nitrobenzaldehyd (Intermediat 2B) in 50 ml Dichlormethan. Man rührte 17 h bei Raumtemperatur, addierte 50 ml wasserfreies Toluol und das Lösungsmittel wurde zum Teil evaporiert. Danach wurde 1.5 h bei 120°C erhitzt, man ließ auf Raumtemperatur kommen, filtrierte den Feststoff ab und wusch den Feststoff mit Diethylether. Man erhielt 3 g der Titelverbindung.
¹H-NMR (300 MHz, DMSO-d6): δ = 2.42 - 2.45 (m, 2H), 2.53 - 2.69 (m, 2H), 3.20 - 3.28 (m, 2H), 3.40 - 3.53 (m, 2H), 4.99 - 5.11 (m, 1H), 8.08 (s, 1H), 8.67 (s, 1H), 8.87 (d, 1H)

### Intermediat 3J

### 4-(6-Methoxy-5-nitro-2H-indazol-2-yl)pyrrolidin-2-on

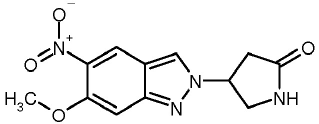

Analog zur Herstellung von Intermediat 3I wurden 122 mg (0.9 mmol) 4-Aminopyrrolidin-2-onhydrochlorid (1:1) mit 0.2 g 2-Azido-4-methoxy-5-nitrobenzaldehyd (Intermediat 2A) umgesetzt. Man erhielt 200 mg der Titelverbindung als gelben Feststoff.
¹H-NMR (300 MHz, DMSO): δ = 2.65 (dd, 1H), 2.89 (dd, 1H), 3.48 - 3.53 (m, 1H), 3.86 (dd, 1H), 3.91 (s, 3H), 5.46 - 5.55 (m, 1H), 7.31 - 7.32 (m, 1H), 7.87 (s, 1H), 8.41 (s, 1H), 8.66 (d, 1H)

### Intermediat 3K

### Methyl-5-nitro-2-(tetrahydro-2H-pyran-4-yl)-2H-indazol-6-carboxylat

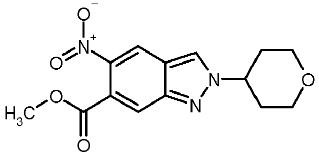

### Herstellungsmethode a), Nitrierung:

Eine eiskalte Lösung aus 985 mg g Methyl-2-(tetrahydro-2H-pyran-4-yl)-2H-indazol-6-carboxylat (Intermediat 6A, Rohprodukt, enthielt Anteile Triphenylphosphinoxid) in 8 ml Schwefelsäure wurde portionsweise mit 528 mg Kaliumnitrat versetzt. Man ließ 2 h im Eiswasser-Kältebad rühren und 17 h bei Raumtemperatur. Das Gemisch wurde auf Eiswasser gegeben, dreimal mit Ethylacetat extrahiert, die vereinigten organischen Phasen wurden mit Kochsalzlösung gewaschen, durch einen wasserabweisenden Filter filtriert und eingeengt. Man erhielt 1.14 g der Titelverbindung.
UPLC-MS (Methode C): Rt = 0.96 min; gefundene Masse 305.00.
¹H-NMR (400MHz, DMSO-d₆): δ [ppm]= 2.00 - 2.21 (m, 4H), 3.31 (s, 1H), 3.43 - 3.62 (m, 2H), 4.03 (dt, 2H), 4.85- 4.99 (m, 1H), 8.07 - 8.12 (m, 1H), 8.69 (s, 1H), 8.89 - 8.91 (m, 1H).

### Herstellungsmethode b), Mitsunobu-Reaktion:

2.00 g Methyl-5-nitro-1H-indazol-6-carboxylat (Intermediat 7A) wurden in 15 ml THF vorgelegt. Danach gab man 3.56 g Triphenylphosphin zu, 1.20 g Tetrahydro-2H-pyran-4-ol, 1.9 ml N-Ethyl-N-isopropylpropan-2-amin und 1.9 ml Diisopropylazodicarboxylat zu und die Mischung wurde 20 h bei Raumtemperatur gerührt. Danach wurden erneut 0.3 Äquivalente Tetrahydro-2H-pyran-4-ol, 0.5 Äquivalente Triphenylphosphin und 0.5 Äquivalente Diisopropylazodicarboxylat zugegeben und die Mischung wurde 4.5 h bei Raumtemperatur gerührt. Man versetzte mit Wasser, extrahierte dreimal mit Ethylacetat, wusch mit Kochsalzlösung, filtrierte durch einen wasserabweisenden Filter und engte ein. Der Rückstand wurde säulenchromatographisch an Kieselgel gereinigt (Isolera, Hexan/Ethylacetat). Man erhielt 1.92 g der Titelverbindung (enthielt Triphenylphopshinoxid als Begleitkomponente).
UPLC-MS (Methode C): Rt = 0.96 min; gefundene Masse 305.00.

### Intermediat 3L

### Methyl-5-nitro-2-[(3S)-tetrahydrofuran-3-yl]-2H-indazol-6-carboxylat

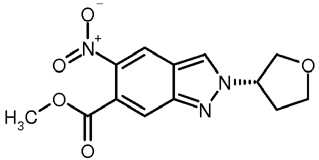

3.00 g Methyl-5-nitro-1H-indazol-6-carboxylat (Intermediat 7A) wurden in 25 ml THF vorgelegt. Danach gab man 5.34 g Triphenylphosphin zu, 1.55 g (3R)-Tetrahydrofuran-3-ol, 3.5 ml N-Ethyl-N-isopropylpropan-2-amin und 4.0 ml Diisopropylazodicarboxylat zu und die Mischung wurde 17 h bei Raumtemperatur gerührt. Danach wurden erneut 0.3 Äquivalente (3R)-Tetrahydrofuran-3-ol, 0.5 Äquivalente Triphenylphosphin und 0.5 Äquivalente Diisopropylazodicarboxylat zugegeben und die Mischung wurde 4 h bei Raumtemperatur gerührt. Man versetzte mit Wasser, extrahierte dreimal mit Ethylacetat, wusch mit Kochsalzlösung, filtrierte durch einen wasserabweisenden Filter und engte ein. Der Rückstand wurde säulenchromatographisch an Kieselgel gereinigt (Isolera, Hexan/Ethylacetat). Man erhielt 1.85 g der Titelverbindung (enthielt Triphenylphopshinoxid als Begleitkomponente).
UPLC-MS (Methode C): Rt = 0.90 min; gefundene Masse 291.00.

### Intermediat 3M

### Methyl-5-nitro-2-[(3R)-tetrahydrofuran-3-yl]-2H-indazol-6-carboxylat

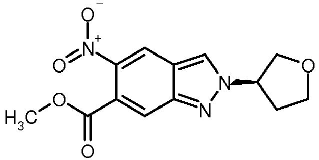

Analog zur Herstellung von Intermediat 3L wurden 3.00 g Methyl-5-nitro-1H-indazol-6-carboxylat (Intermediat 7A) mit 1.55 g (3S)-Tetrahydrofuran-3-ol umgesetzt. Man erhielt 3.41 g der Titelverbindung (enthielt Triphenylphosphinoxid als Begleitkomponente)
UPLC-MS (Methode C): Rt = 0.90 min; gefundene Masse 291.00.

### Intermediat 3N

### Methyl-5-nitro-2-[(3S)-tetrahydrothiophen-3-yl]-2H-indazol-6-carboxylat

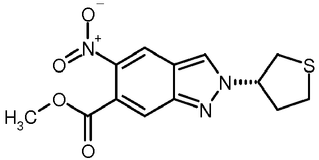

### Stufe A: Herstellung von (3R)-Tetrahydrothiophen-3-yl-4-methylbenzolsulfonat

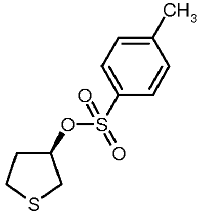

Eine Lösung aus 1.00 g (3R)-Tetrahydrothiophen-3-ol in 20 ml Dichlormethan wurde mit einem Eiswasser-Kältebad abgekühlt und portionsweise wurden 1.92 g 4-Methylbenzolsulfonylchlorid zugegeben. Danach wurden 2.7 ml Triethylamin und 56 mg N,N-Dimethylpyridin-4-amin (DMAP) zugegeben und man rührte bei Raumtemperatur 20 h. Man gab auf gesättigte Natriumhydrogencarbonatlösung, rührte um und trennte die organische Phase ab. Die wässrige Phase wurde zweimal mit Dichlormethan extrahiert, die vereinigten organischen Phasen mit 1M Salzsäurelösung und gesättigter Natriumcarbonatlöung gewaschen, durch einen wasserabweisenden Filter filtriert und eingeengt. Das Rohprodukt wurde durch Säulenchromatographie an Kieselgel (Isolera, Hexan/Ethylacetat) gereinigt. Man erhielt 443 mg eines farblosen Öles.
¹H-NMR (400MHz, DMSO-d₆): δ [ppm]= 1.81 - 1.92 (m, 1H), 2.08 - 2.16 (m, 1H), 2.44 (s, 3H), 2.73 - 2.90 (m, 3H), 3.02 (dd, 1H), 5.22 (tt, 1H), 7.50 (d, 2H), 7.79 - 7.86 (m, 2H).

### Stufe B:

304 mg Methyl-5-nitro-1H-indazol-6-carboxylat (Intermediat 7A) und 338 mg (3R)-Tetrahydrothiophen-3-yl-4-methylbenzolsulfonat wurden in 10 ml 2-Methyltetrahydrofuran vorgelegt. 0.54 g Kaliumcarbonat wurde zugegeben und die Mischung wurde 41.5 h bei 70°C gerührt. Man gab 2 ml DMSO zu und rührte weitere 21 h bei 70°C. Die Mischung wurde mit Wasser verdünnt und dreimal mit Ethylacetat extrahiert. Die vereinigten organischen Phasen wurden eingeengt und durch präparative HPLC gereinigt. Man erhielt 175 mg Methyl-5-nitro-2-[(3S)-tetrahydrothiophen-3-yl]-2H-indazol-6-carboxylat.
¹H-NMR (500MHz, DMSO-d₆): δ [ppm]= 2.52 - 2.56 (m, 1H, verdecktes Signal), 2.60 - 2.68 (m, 1H), 2.92 - 3.05 (m, 2H), 3.33 - 3.45 (m, 2H, verdecktes Signal), 3.85 (s, 3H), 5.48 (quin, 1H), 8.09 (s, 1H), 8.71 (s, 1H), 8.94 - 8.96 (m, 1H).

### Intermediat 30

### Methyl-5-nitro-2-(tetrahydro-2H-thiopyran-4-yl)-2H-indazol-6-carboxylat

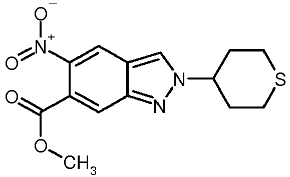

### Herstellungsmethode a):

2.71 g Methyl-5-nitro-1H-indazol-6-carboxylat (Intermediat 7A) wurden analog zur Herstellung von Intermediat 3N, Stufe B mit 5.00 g Tetrahydro-2H-thiopyran-4-yl-4-methylbenzolsulfonat in Gegenwart von 5.07 g Kaliumcarbonat in 20 ml DMF bei 70°C innerhalb von 18 h umgesetzt. Nach säulenchromatographischer Reinigung an Kieselgel (Isolera, Hexan/Ethylacetat) wurden 1.09 g der Titelverbindung erhalten.
UPLC-MS (Methode C): Rt = 1.12 min; gefundene Masse 321.00.

### Herstellungsmethode b):

Analog zur Herstellung von Intermediat 3L wurden 2.50 g Methyl-5-nitro-1H-indazol-6-carboxylat (Intermediat 7A) mit 1.74 g Tetrahydro-2H-thiopyran-4-ol umgesetzt. Nach säulenchromatographischer Reinigung an Kieselgel (Isolera, Hexan/Ethylacetat) wurden 1.42 g der Titelverbindung als Rohprodukt erhalten.
UPLC-MS (Methode C): Rt = 1.12 min; gefundene Masse 321.00.

### Intermediat 3P

### Methyl-2-[1-(tert-butoxycarbonyl)piperidin-4-yl]-5-nitro-2H-indazol-6-carboxylat

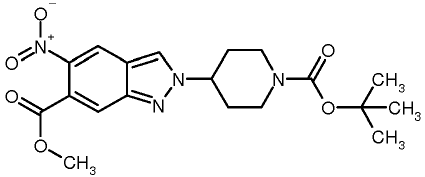

Analog zur Herstellung von Intermediat 3L wurden 2.00 g Methyl-5-nitro-1H-indazol-6-carboxylat (Intermediat 7A) mit 2.37 g tert-Butyl-4-hydroxypiperidin-1-carboxylat, 3.56 g Triphenylphosphin, 2.3 ml N-Ethyl-N-isopropylpropan-2-amin und 2.6 ml Diisopropylazodicarboxylat in 20 ml THF innerhalb von 20.5 h bei Raumtemperatur umgesetzt. Nach säulenchromatographischer Reinigung an Kieselgel wurden 1.44 g der Titelverbindung als Rohprodukt (enthielt neben weiteren Bestandteilen Anteile an Triphenylphosphinoxid) erhalten.
¹H-NMR (500MHz, DMSO-d₆, ausgewählte Signale): δ [ppm]= 1.43 (s), 2.12-2.19 (m, 2H), 2.97 (breites Singulett, 2H), 3.84 (s, 3H), 4.03 (d, 3H), 4.06-4.19 (breites Signal, 2H), 4.82-4.91 (m, 1H), 7.52 - 7.67 (m, 13H), 8.07 (s, 1H), 8.68 (s, 1H), 8.89 - 8.91 (m, 1H).

### Intermediat 4A

### 6-Methoxy-2-(tetrahydro-2H-pyran-4-yl)-2H-indazol-5-amin

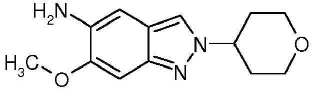

222 mg 10% Palladium auf Kohle (50% w/w) wurden unter Stickstoff vorgelegt, dann wurde eine Lösung aus 2.22 g (8.0 mmol) 6-Methoxy-5-nitro-2-(tetrahydro-2H-pyran-4-yl)-2H-indazol (Intermediat 3A) in 120 ml Ethanol zugegeben. Die Reaktion wurde dann unter Normaldruck in einer Wasserstoffatmosphäre 17 h gerührt. Die Lösung wurde filtriert durch Celite, dann wurde die Celite mit Ethanol und 20% Ethanol / Dichlormethan nachgewaschen. Das Filtrat wurde eingeengt im Vakuum, mit Ethanol versetzt und der Feststoff abfiltriert und getrocknet. Man erhielt 1.5 g (6.0 mmol) der Titelverbindung.
LC-MS (Methode A): Rt = 0.43 min; m/z = 248 (M+H)+
¹H-NMR (300 MHz, CDCl₃): δ = 2.14 - 2.24 (m, 4H), 3.53 - 3.63 (m, 2H), 3.82 - 3.90 (m, 2H), 3.92 (s, 3H), 4.10 - 4.17 (m, 2H), 4.45 - 4.57 (m, 1H), 6.75 (s, 1H), 6.95 (s, 1H), 7.63 (d, 1H)

### Intermediat 4B

### tert-Butyl-4-(5-amino-6-methoxy-2H-indazol-2-yl)piperidin-1-carboxylat

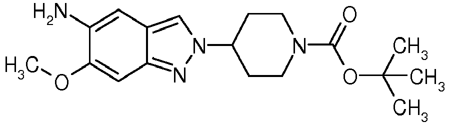

220 mg 10% Palladium auf Kohle (50% w/w) wurden vorgelegt, man evaporierte und spülte mit Stickstoff. Dann wurde eine Lösung aus 2.1 g (5.6 mmol) tert-Butyl-4-(6-methoxy-5-nitro-2H-indazol-2-yl)piperidin-1-carboxylat (Intermediat 3B) in 80 ml Ethanol zugegeben. Die Mischung wurde in einer Wasserstoffatmosphäre bei Normaldruck 24 h gerührt. Die Lösung wurde durch Celite filtriert, man wusch mit Ethanol nach und engte das Filtrat bei reduziertem Druck ein. Danach setzte man Dichlormethan und Cyclohexan zu und engte ein. Man erhielt 1.81 g (5.2 mmol) der Titelverbindung.
LC-MS (Methode A): Rt = 2.53 min; m/z = 347 (M+H)+
¹H NMR (300 MHz, CDCl₃): δ = 1.48 (9H, s), 2.04 (2H, ddd), 2.19 (2H, d), 2.92 (2H, t), 3.84 (2H, s), 3.91 (3H, s), 4.21 - 4.34 (2H, m), 4.36 - 4.48 (1H, m), 6.75 (1H, s), 6.94 (1H, s), 7.60 (1H, s)

### Intermediat 4C

### 2-(1,1-Dioxidotetrahydro-2H-thiopyran-4-yl)-6-methoxy-2H-indazol-5-amin

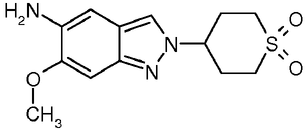

4.39 g (13.5 mmol) 2-(1,1-Dioxidotetrahydro-2H-thiopyran-4-yl)-6-methoxy-5-nitro-2H-indazol (Intermediat 3C) in 150 ml Ethanol und 439 mg 10% Palladium auf Kohle wurden unter einer Wasserstoffatmosphäre bei Raumtemperatur 21 h gerührt. Die Wasserstoffatomosphäre wurde entfernt, man addierte erneut 439 mg 10% Palladium auf Kohle und rührte in einer Wasserstoffatmosphäre 71 h. Die Wasserstoffatomosphäre wurde entfernt, weitere 50 ml Ethanol und 439 mg 10% Palladium auf Kohle wurde zugegeben und man rührte 16 h unter einer Wasserstoffatmosphäre. Die Mischung wurde durch Celite filtriert, die Celite mit Ethanol nachgewaschen mit 10% Ethanol / Dichlormethan und 25% Ethanol / Dichlormethan. Man erhielt nach Einengen 2.55 g (8.6 mmol) der Titelverbindung.
LC-MS (Methode A): Rt = 0.43 min; m/z = 296 (M+H)+
¹H-NMR (300 MHz, DMSO-d6): δ = 2.32 - 2.39 (m, 2H), 2.44 -2.58 (m, 2H), 3.22 - 3.29 (m, 2H), 3.37 - 3.45 (m, 2H), 3.82 (s, 3H), 4.62 (s, 2H), 4.68 - 4.79 (m, 1H), 6.62 (s, 1H), 6.86 (s, 1H), 7.91 (s, 1H)

### Intermediat 4D

### 6-Methoxy-2-(1-oxidotetrahydro-2H-thiopyran-4-yl)-2H-indazol-5-amin (Isomerenmischung).

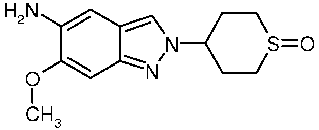

Eine Mischung aus 710 mg (2.3 mmol) 6-Methoxy-5-nitro-2-(1-oxidotetrahydro-2H-thiopyran-4-yl)-2H-indazol (Intermediat 3E) in 50 ml Ethanol und 71 mg 10% Palladium auf Kohlenstoff wurde 24 h mit einer Wasserstoffatomosphäre versetzt. Dann gab man erneut 71 mg 10% Palladium auf Kohlenstoff zu und rührte unter einer Wasserstoffatmosphäre 5 h. Die Reaktion wurde durch Celite filtriert, die Celite wurde mit Ethanol gewaschen und das Filtrat wurde eingeengt im Vakuum. Es wurden 426 mg (1.5 mmol) der Titelverbindung erhalten.
LC-MS (Methode A): Rt = 0.44 min; m/z = 280 (M+H)+
¹H-NMR (300 MHz, CDCl3): δ = 2.26 - 2.44 (m, 2H), 2.63 - 2.96 (m, 4H), 3.20 - 3.28 (m, 1H), 3.35 - 3.46 (m, 1H), 3.91 - 3.93 (m, 3H), 4.50 - 4.62 (m, 1H), 6.74 - 6.77 (m, 1H), 6.92 (s, 1H), 7.61 - 7.71 (m, 1H)

### Intermediat 4E

### tert-Butyl-rel-(1S,4S,5R)-5-(5-amino-6-methoxy-2H-indazol-2-yl)-2-azabicyclo[2.2.1]heptan-2-carboxylat

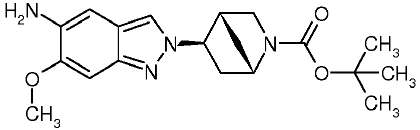

Eine Mischung aus 193 mg (0.497mmol) tert-Butyl-rel-(1S,4S,5R)-5-(6-methoxy-5-nitro-2H-indazol-2-yl)-2-azabicyclo[2.2.1]heptan-2-carboxylat (Intermediat 3F) und 40 mg 10% Palladium auf Kohlenstoff in 20 ml Ethanol wurde 5 h mit einer Wasserstoffatmosphäre behandelt. Die Mischung wurde durch Celite filtriert, man wusch mit Ethanol nach und das Lösungsmittel wurde entfernt im Vakuum. Man erhielt 150 mg (0.418 mmol) der Zielverbindung.
LC-MS (Methode A): Rt = 2.64 min; m/z = 359 (M+H)+
¹H-NMR (300 MHz, CDC13): δ = 1.49 (s, 9H), 1.72 (t, 1H), 2.23 - 2.42 (m, 3H), 2.88 (s, 1H), 3.11 - 3.21 (m, 1H), 3.35 (dd, 1H), 3.85 (s, 2H), 3.91 (s, 3H), 4.40 (d, 1H), 4.58 (dd, 1H), 6.73 (s, 1H), 6.94 (s, 1H), 7.63 (s, 1H).

### Intermediat 4F

### tert-Butyl-rel-(1S,4S,5S)-5-(5-amino-6-methoxy-2H-indazol-2-yl)-2-azabicyclo[2.2.1]heptan-2-carboxylat

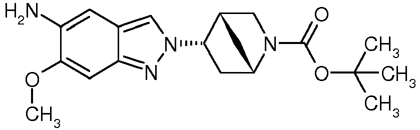

Eine Mischung aus 336 mg (0.865 mmol) tert-Butyl-rel-(1S,4S,5S)-5-(6-methoxy-5-nitro-2H-indazol-2-yl)-2-azabicyclo[2.2.1]heptan-2-carboxylat (Intermediat 3G) und 72 mg 10% Palladium auf Kohle in 20 ml Ethanol wurde mit einer Wasserstoffatmosphäre 19 h behandelt. Die Mischung wurde durch Celite filtriert, man wusch mit Ethanol nach und entfernte das Lösungsmittel im Vakuum. Man erhielt 297 mg (0.829 mmol) der Titelverbindung.
LC-MS (Methode A): Rt = 2.50 & 2.53 min; m/z = 359 (M+H)+
¹H-NMR (300 MHz, CDCl3): δ = 1.45 (d, 9H), 1.74 - 1.91 (m, 2H), 2.27 - 2.51 (m, 2H), 2.72 - 3.01 (m, 1H), 3.08 - 3.18 (m, 2H), 3.84 - 3.85 (m, 2H), 3.91 (s, 3H), 4.29 - 4.40 (m, 1H), 5.00 - 5.07 (m, 1H), 6.74 (s, 1H), 6.89 - 6.94 (m, 1H), 7.60 - 7.65 (m, 1H).

### Intermediat 4G

### 6-Chlor-2-(tetrahydro-2H-pyran-4-yl)-2H-indazol-5-amin

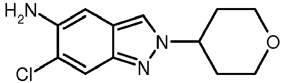

1.39 g 6-Chlor-5-nitro-2-(tetrahydro-2H-pyran-4-yl)-2H-indazol (Intermediat 3H) wurden in 20 ml Ethanol und 3 ml Wasser vorgelegt. Danach gab man 132 mg Ammoniumchlorid und 2.76 g Eisen zu und rührte die Mischung bei 90°C 1.5 h. Man filtrierte durch Celite, wusch mit Ethanol nach und engte ein. Dabei fiel ein Feststoff aus, der abgesaugt wurde und mit Wasser und Diethylether gewaschen wurde. Nach Trocknen des Feststoffes wurden 183 mg der Titelverbindung erhalten. Erneutes Waschen der Celite mit einer Dichlormethan/THF-Mischung, nachfolgendes Einengen und Ausrühren des Rückstandes mit Diethylether und Trocknen führte zu weiteren 617 mg der Titelverbindung.
¹H-NMR (400MHz, DMSO-d₆): δ [ppm]= 1.99 - 2.12 (m, 4H), 3.43 - 3.55 (m, 2H), 3.93 - 4.03 (m, 2H), 4.54 - 4.67 (m, 1H), 4.93 (s, 2H), 6.87 (s, 1H), 7.60 (s, 1H), 8.09 (s, 1H).

### Intermediat 4H

### 6-Chlor-2-(1,1-dioxidotetrahydro-2H-thiopyran-4-yl)-2H-indazol-5-amin

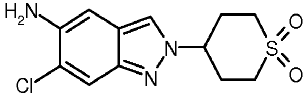

Zu einer Mischung aus 2.78 g (8.4 mmol) 6-Chlor-2-(1,1-dioxidotetrahydro-2H-thiopyran-4-yl)-5-nitro-2H-indazol (Intermediat 3I) und 2.35 g (42mmol) Eisenpulver in 44 ml Ethanol wurde eine Lösung aus 1.35 g (25.3 mmol) Ammoniumchlorid in 22 ml Wasser gegeben und die Mischung wurde 40 min bei 70°C erhitzt. Nach Abkühlen auf Raumtemperatur wurde filtriert (Whatmen Filter Cups, Waschen mit Ethanol) und das Filtrat eingeengt. Danach verteilte man zwischen gesättigter wässriger Natriumhydrogencarbonatlösung und einer Mischung aus 10% Methanol in Dichlormethan. Die wässrige Phase wurde nachextrahiert und die vereinigten organischen Phasen wurden filtriert. Nach Einengen im Vakuum wurden 2.22 g der Titelverbindung erhalten.
¹H-NMR (300 MHz, CDC13): δ = 2.60 - 2.76 (m, 4H), 3.06 - 3.18 (m, 2H), 3.51 - 3.63 (m, 2H), 4.00 (s, 2H), 4.62 - 4.71 (m, 1H), 6.87 (s, 1H), 7.70 (s, 2H).

### Intermediat 4I

### Rac-4-(5-Amino-6-methoxy-2H-indazol-2-yl)pyrrolidin-2-on

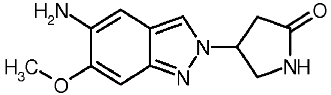

200 mg (0.724 mmol) 4-(6-Methoxy-5-nitro-2H-indazol-2-yl)pyrrolidin-2-on (Intermediat 3J) in 20 ml Ethanol wurden mit 20 mg 10% Palladium auf Kohle (50% Wasser-feucht) versetzt und die Mischung wurde unter einer Wasserstoffatmosphäre 18 h gerührt. 20 mg 10% Palladium auf Kohle (50% Wasser-feucht) wurden hinzugefügt und man rührte unter einer Wasserstoffatmosphäre 21 h. Die Mischung wurde durch Celite filtriert, man wusch die Celite mit Ethanol und engte das Filtrat ein. Man erhielt 119 mg der Titelverbindung (als Rohprodukt).
¹H-NMR (300 MHz, CDC13): d = 2.93 - 2.99 (m, 2H), 3.84 - 3.98 (m, 7H), 5.23 - 5.33 (m, 1H), 5.73 - 5.77 (m, 1H), 6.72 (s, 1H), 6.93 (s, 1H), 7.65 (s, 1H)

### Intermediat 4J

### Methyl-5-amino-2-(tetrahydro-2H-pyran-4-yl)-2H-indazol-6-carboxylat

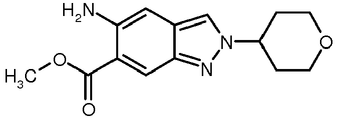

1.13 g Methyl-5-nitro-2-(tetrahydro-2H-pyran-4-yl)-2H-indazol-6-carboxylat (Intermediat 3K) wurden in 10 ml Ethanol und 2 ml Wasser vorgelegt. Danach gab man 84 mg Ammoniumchlorid und 1.76 g Eisen zu und rührte die Mischung bei 90°C 2 h. Man filtrierte durch Celite, wusch mit Ethanol nach und entfernte zum Teil das Lösungsmittel am Rotationsverdampfer. Danach extrahierte man mit Ethylacetat, wusch mit Kochsalzlösung, filtrierte durch einen wasserabweisenden Filter und engte ein. Es wurden 892 mg der Titelverbindung als Rohprodukt erhalten.
¹H-NMR (400MHz, DMSO-d₆): δ [ppm]= 2.01 - 2.14 (m, 4H), 3.44 - 3.55 (m, 2H), 3.84 (s, 3H), 3.95 - 4.03 (m, 2H), 4.61 - 4.75 (m, 1H), 5.81 (s, 2H), 6.79 - 6.82 (m, 1H), 8.11 (d, 1H), 8.18 - 8.21 (m, 1H).

### Intermediat 4K

### Methyl-5-amino-2-[(3S)-tetrahydrothiophen-3-yl]-2H-indazol-6-carboxylat

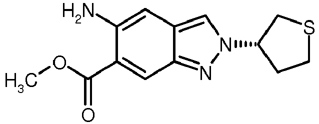

Analog zur Herstellung von Intermediat 4J wurden 167 mg Methyl-5-nitro-2-[(3S)-tetrahydrothiophen-3-yl]-2H-indazol-6-carboxylat (Intermediat 3N) mit 303 mg Eisen, 15 mg Ammoniumchlorid in 7.5 ml Ethanol und 2.5 ml Wasser bei 90°C innerhalb von 17.5 h umgesetzt. Nach Reinigung des Rohproduktes durch präparative HPLC wurden 91 mg der Titelverbindung erhalten.
UPLC (Methode C): Rt = 0.83 min; gefundene Masse 277.00.

### Intermediat 4L

### Methyl-5-amino-2-[(3S)-tetrahydrofuran-3-yl]-2H-indazol-6-carboxylat

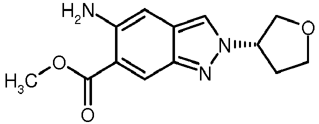

1.85 g Methyl-5-nitro-2-[(3S)-tetrahydrofuran-3-yl]-2H-indazol-6-carboxylat (Intermediat 3L, Charge enthielt Triphenylphosphinoxid) in 4.9 ml Wasser und 28 ml Ethanol wurden mit 1.24 g Eisenpulver und 119 mg Ammoniumchlorid versetzt. Man erhitzte 3 h unter Rückfluß, filtrierte über Celite und wusch mit Ethylacetat nach. Man engte das Filtrat zum Teil ein, addierte Ethylacetat und säuerte mit 1N wässriger Salzsäure auf pH = 3 an. Die Phasen wurden getrennt und die wässrige Phase mit Ethylacetat extrahiert. Die wässrige Phase wurde mit 1M Natronlauge auf pH = 8 gebracht und mit Ethylacetat extrahiert. Die vereinigten organischen Phasen wurden eingeengt. Man erhielt 759 mg der Titelverbindung mit geringen Anteilen an Triphenylphenylphosphinoxid.
¹H-NMR (500MHz, DMSO-d₆): δ [ppm]= 2.35 - 2.42 (m, 1H), 2.44-2.49 (m), 3.83 - 3.90 (m, 4H), 3.99 - 4.10 (m, 3H), 5.26 - 5.32 (m, 1H), 5.83 (s, 2H), 6.81 (d, 1H), 8.08 (d, 1H), 8.21 (s, 1H).

### Intermediat 4M

### Methyl-5-amino-2-[(3R)-tetrahydrofuran-3-yl]-2H-indazol-6-carboxylat

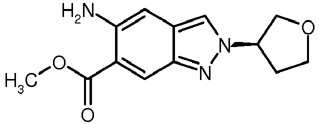

200 mg Methyl-5-nitro-2-[(3R)-tetrahydrofuran-3-yl]-2H-indazol-6-carboxylat (Intermediat 3M, Charge enthielt Triphenylphosphinoxid) wurden analog zur Herstellung von Intermediat 4L mit 383 mg Eisen und 37 mg Ammoniumchlorid in 4 ml Ethanol und 0.7 ml Wasser innerhalb von 3 h unter Rückfluß umgesetzt. Im Rahmen eines zweiten Reaktionsansatzes wurden 3.21 g Methyl-5-nitro-2-[(3R)-tetrahydrofuran-3-yl]-2H-indazol-6-carboxylat (Intermediat 3M, Charge enthielt Triphenylphosphinoxid) mit 3.99 g Eisen und 383 mg Ammoniumchlorid in 42 ml Ethanol und 7 ml Wasser umgesetzt. Die beiden Reaktionsansätze wurden vereint und wie bei der Herstellung von Intermediat 4L aufgearbeitet. Man erhielt 552 mg der Titelverbindung (Rohprodukt) als braunen Feststoff.
¹H-NMR (400MHz, DMSO-d₆): δ [ppm]= 2.31 - 2.49 (m), 3.83 - 3.90 (m, 5H), 3.98 - 4.12 (m, 3H), 5.26 - 5.33 (m, 1H), 5.84 (s, 2H), 6.80 - 6.83 (m, 1H), 8.11 (d, 1H), 8.21 (s, 1H).

### Intermediat 4N

### Methyl-5-amino-2-(tetrahydro-2H-thiopyran-4-yl)-2H-indazol-6-carboxylat

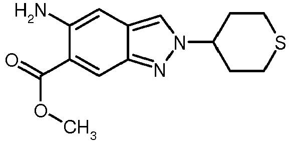

Analog zur Herstellung von Intermediat 4J wurden 1.09 g Methyl-5-nitro-2-(tetrahydro-2H-thiopyran-4-yl)-2H-indazol-6-carboxylat (Intermediat 30) mit 1.27 g Eisen, 61 mg Ammoniumchlorid in 10 ml Ethanol und 2 ml Wasser bei 85°C innerhalb von 20.5 h umgesetzt. Es wurden 992 mg eines Rohproduktes nach analoger Aufarbeitung erhalten.

### Intermediat 40

### Methyl-5-amino-2-[1-(tert-butoxycarbonyl)piperidin-4-yl]-2H-indazol-6-carboxylat

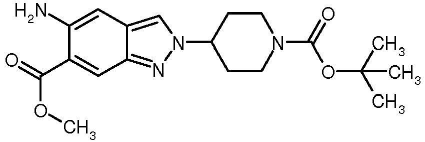

Analog zur Herstellung von Intermediat 4J wurden 6.09 g Methyl-2-[1-(tert-butoxycarbonyl)piperidin-4-yl]-5-nitro-2H-indazol-6-carboxylat (Intermediat 3P) mit 6.06 g Eisen, 299 mg Ammoniumchlorid in 35 ml Ethanol und 7 ml Wasser bei 85°C innerhalb von 19.5 h umgesetzt. Es wurden 5.52 g eines Rohproduktes nach analoger Aufarbeitung erhalten.

### Intermediat 5A

### N-(6-Methoxy-1H-indazol-5-yl)-6-(trifluoromethyl)pyridin-2-carboxamid

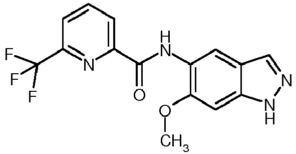

3.84 g (23.5 mmol) 6-Methoxy-1H-indazol-5-amin (CAS Nr.: 749223-61-8) und 4.95 g (25.9 mmol) 6-(Trifluoromethyl)pyridine-2-carbonsäure wurden in 150 ml Tetrahydrofuran gelöst und bei 25°C mit 3.60 g (23.5 mmol) 1-Hydroxy-1H-benzotriazol Hydrat, 9.02 g (47.1 mmol) 1-(3-Dimethylaminopropyl)-3-ethylcarbodiimidhydrochlorid und 9.84 ml (70.6 mmol) Triethylamin versetzt. Die Lösung wurde 24 h bei 25°C gerührt. Nach dem Einengen der Lösung wurde in Essigsäureethylester aufgenommen, mit Wasser versetzt und die wässrige Phase drei Mal mit Essigsäureethylester extrahiert. Die vereinigten, organischen Phasen wurden mit gesättigter Natriumchloridlösung gewaschen, über Natriumsulfat getrocknet und nach Filtration wurde die Lösung eingeengt. Der Rückstand wurde durch säulenchromatographische Reinigung an Kieselgel (Isolera Flash-Aufreinigungssystem (Biotage), Hexan/Ethylacetat) gereinigt. Man erhielt 3.75 g der Titelverbindung.
UPLC-MS (Methode C): Rₜ = 1.12 min
MS (ESIpos): m/z = 337 (M+H)⁺
¹H-NMR (400 MHz, DMSO-d6): δ = 4.01 (s, 3 H), 7.13 (s, 1 H), 8.02 (s, 1 H), 8.21 (dd, 1 H), 8.40 (t, 1 H), 8.47 (d, 1 H), 8.74 (s, 1 H), 10.42 (s, 1 H), 12.91 (s, 1 H).

### Intermediat 6A

### Methyl-2-(tetrahydro-2H-pyran-4-yl)-2H-indazol-6-carboxylat

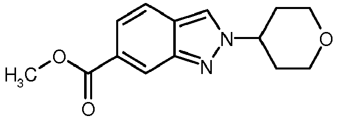

### Herstellungsmethode A: Mitsunobu-Reaktion:

Eine Suspension aus 7.00 g Methyl-1H-indazol-6-carboxylat in 80 ml THF wurde durch ein Eiswasser-Kältebad abgekühlt. Man gab 4.5 ml Tetrahydro-2H-pyran-4-ol, 3.1 g Triphenylphosphin und 2.3 ml Azodicarbonsäureiisopropylester (DIAD, CAS 2446-83-5) zu und ließ 19 h bei Raumtemperatur rühren. Man gab nochmals 3.1 g Triphenylphosphin und 2.3 ml Diisopropylazodicarboxylat zu und rührte 71 h bei Raumtemperatur und 5 h bei 70°C. Man versetzte mit Wasser, extrahierte dreimal mit Ethylacetat, wusch die vereinigten organischen Phasen mit Kochsalzlösung und engte ein. Der Rückstand wurde säulenchromatographisch an Kieselgel (Isolera, Laufmittel Hexan / Ethylacetat) gereinigt. Man erhielt 11.0 g eines Feststoffes, der mit Diethylether ausgerührt wurde. Nach Trocknen erhielt man 8.95 g eines Rohproduktes (enthielt laut UPLC-Analytik die Titelverbindung zusammen Triphenylphosphinoxid). Die Diethylether-Waschphase wurde eingeengt. Dabei wurden 1.78 g eines Feststoffes erhalten (Titelverbindung als Rohprodukt, enthielt Triphenylphosphinoxid).
UPLC-MS (Methode C): Rt = 0.94 min; gefundene Masse 260.00

### Herstellungsmethode B: Alkylierung

5.00 g Methyl-1H-indazol-6-carboxylat wurden in 50 ml DMF vorgelegt. Man addierte 7.0 g 4-Bromtetrahydro-2H-pyran, 11.8 Kaliumcarbonat und 7.07 g Kaliumiodid und rührte 16.5 h bei 100°C. Man versetzte mit Wasser, extrahierte fünfmal mit Ethylacetat, wusch mit Kochsalzlösung und engte ein. Man erhielt 8.99 g eines Öles, welches in 30 ml DMF vorgelegt wurde. Man addierte 6.0 g 4-Bromtetrahydro-2H-pyran, 10.2 Kaliumcarbonat und rührte 20.5 h bei 120°C. Man addierte Wasser, extrahierte mit Ethylacetat, wusch mit Kochsalzlösung und engte ein. Man erhielt 5.73 g eines Rückstandes (enthielt Produkt laut UPLC, Rt = 0.94 min). Man vereinigte mit 8.95 g des Rohproduktes aus der Herstellungsmethode A und reinigte durch Säulenchromatographie an Kieselgel (Isolera, Hexan/Ethylacetat). Man erhielt 1.41 g eines Feststoffes, der mit Diethylether ausgerührt wurde. Nach Trocknen wurden 991 mg der Titelverbindung erhalten.
¹H-NMR (500MHz, DMSO-d₆): δ [ppm]= 2.08 - 2.19 (m, 4H), 3.54 (td, 2H), 3.88 (s, 3H), 3.99 - 4.06 (m, 2H), 4.83 (tt, 1H), 7.57 (dd, 1H), 7.81 (dd, 1H), 8.31 (q, 1H), 8.58 (d, 1H).

### Intermediat 7A

### Methyl-5-nitro-1H-indazol-6-carboxylat

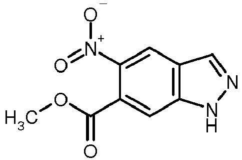

4.60 g (26.1 mmol) Methyl-1H-indazol-6-carboxylat (CAS Nr: 170487-40-8) wurden in 120 ml Schwefelsäure (96%ig) gelöst und in einem Dreihalskolben mit KPG-Rührer, Tropftrichter und Innenthermometer auf -15°C gekühlt. Zu dieser Lösung wurde innerhalb von 15 min die vorher hergestellte und gekühlte Nitriersäure (10 ml Schwefelsäure 96%ig in 5 ml Salpetersäure 65%ig) zugetropft. Nach Beendigung des Zutropfens wurde 1 h nachgerührt (Innentemperatur bei -13°C). Die Reaktionsmischung wurde auf Eis gegeben, der Niederschlag abgesaugt, mit Wasser gewaschen und im Trockenschrank bei 50°C im Vakuum getrocknet. Es wurden 5.49 g der Titelverbindung erhalten.
¹H NMR (400 MHz, DMSO-d6): δ [ppm] = 3.87 (s, 3 H), 7.96 (s, 1 H), 8.44 (s, 1 H), 8.70 (s, 1 H), 13.98 (br. s., 1 H).

### Ausführungsbeispiele

### Beispiel 1

### N-[6-Methoxy-2-(tetrahydro-2H-pyran-4-yl)-2H-indazol-5-yl]-6-(trifluormethyl)pyridin-2-carboxamid

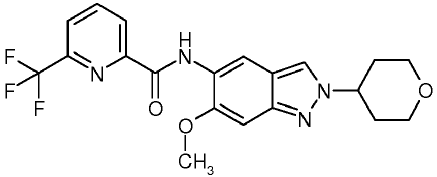

Eine Mischung aus 260 mg (1.05 mmol) 6-Methoxy-2-(tetrahydro-2H-pyran-4-yl)-2H-indazol-5-amin (Intermediat 4A), 201 mg (1.05 mmol) 6-(Trifluormethyl)pyridin-2-carbonsäure [CAS 131747-42-7] und 440 mg (1.2 mmol) O-(7-Azabenzotriazol-1-yl)-N,N,N',N'-tetramethyluronium-hexafluorophosphat (HATU) [148893-10-1] in 5 ml wasserfreien Dimethylformamid wurde mit 0.366 ml (2.1 mmol) N,N-Diisopropylethylamin versetzt und 64 h gerührt bei Raumtemperatur. Die Reaktion wurde mit Ethylacetat und Wasser versetzt, die organische Phase wurde abgetrennt und die wässrige Phase zweimal mit Ethylacetat extrahiert. Die vereinigten organischen Phasen wurden mit Kochsalzlösung gewaschen, über Natriumsulfat getrocknet, filtriert und eingeengt im Vakuum. Nach Reinigung (Biotage Isolera, 50 g Kieselgel-Säule, Cyclohexan/Ethylacetat) wurden 290 mg (0.69 mmol) der Titelverbindung erhalten.
LC-MS (Methode B): Rt = 4.51 min; m/z = 421 (M+H)+
¹H-NMR (400 MHz, DMSO-d6): δ = 2.02 - 2.11 (m, 4H), 3.47 - 3.54 (m, 2H), 3.95 - 4.00 (m, 5H), 4.59 - 4.69 (m, 1H), 7.15 (s, 1H), 8.19 (dd, 1H), 8.35 - 8.45 (m, 3H), 8.67 (s, 1H), 10.48 (s, 1H)

### Beispiel 2

### N-[6-Methoxy-2-(piperidin-4-yl)-2H-indazol-5-yl]-6-(trifluormethyl)pyridin-2-carboxamid

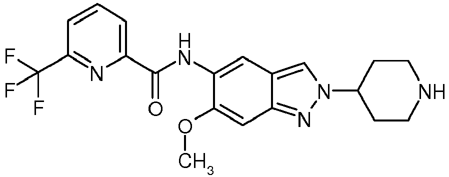

### Stufe A

### tert-Butyl-4-[6-methoxy-5-({[6-(trifluormethyl)pyridin-2-yl]carbonyl}amino)-2H-indazol-2-yl]piperidin-1-carboxylat

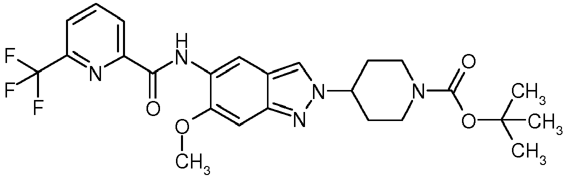

Zu einer Mischung aus 913 mg (2.6 mmol) tert-Butyl-4-(5-amino-6-methoxy-2H-indazol-2-yl)piperidin-1-carboxylat (Intermediat 4B), 500 mg (2.6 mmol), 6-(Trifluormethyl)pyridin-2-carbonsäure [131747-42-7] und 1.1 g (2.9 mmol) O-(7-Azabenzotriazol-1-yl)-N,N,N',N'-tetramethyluronium-hexafluorophosphat (HATU) [148893-10-1] in 10 ml wasserfreien Dimethylformamid wurden 0.918 ml (5.3 mmol) of N,N-Diisopropylethylamin gegeben und die Mischung 16 h bei Raumtemperatur gerührt. Man addierte Ethylacetat und Wasser, trennte die organische Phase ab, extrahierte zweimal mit Ethylacetat, vereinigte die organischen Phasen, wusch mit Kochsalzlösung, trocknete über Natriumsulfat, filtrierte und engte im Vakuum ein. Nach Reinigung durch Säulenchromatographie (Biotage Isolera, 50 g Kieselgel-Säule, Cyclohexan / Ethylacetat) wurden 1.13 g (2.2 mmol) tert-Butyl-4-[6-methoxy-5-({[6-(trifluormethyl)pyridin-2-yl]carbonyl}amino)-2H-indazol-2-yl]piperidin-1-carboxylat erhalten.
LC-MS (Methode A): Rt = 4.49 min; m/z = 464 (M-(tBu+H))+
¹H-NMR (300 MHz, CDCl₃): δ = 1.49 (s, 9H), 2.05 - 2.26 (m, 4H), 2.88 - 2.99 (m, 2H), 4.03 (s, 3H), 4.26 - 4.35 (m, 2H), 4.46 - 4.56 (m, 1H), 7.07 (s, 1H), 7.83 - 7.88 (m, 2H), 8.12 (dd, 1H), 8.49 (d, 1H), 8.82 (s, 1H), 10.70 (s, 1H)

### Stufe B

1.13 g (2.18 mmol) tert-Butyl-4-[6-methoxy-5-({[6-(trifluormethyl)pyridin-2-yl]carbonyl}amino)-2H-indazol-2-yl]piperidin-1-carboxylat wurden mit 10 ml 4M Chlorwasserstoff in Dioxan und 2 ml Methanol versetzt und man rührte bei Raumtemperatur 45 Minuten. Das Lösungsmittel wurde eingeengt im Vakuum. Der Rückstand wurde gereinigt (SCX Kartusche, Laufmittel 2M Ammoniak in Methanol) und man erhielt 820 mg (2.0 mmol) N-[6-Methoxy-2-(piperidin-4-yl)-2H-indazol-5-yl]-6-(trifluormethyl)pyridin-2-carboxamid.
LC-MS (Methode B): Rt = 3.23 min; m/z = 420 (M+H)+
¹H-NMR (300 MHz, CDCl3): δ = 2.00 - 2.15 (m, 2H), 2.23 - 2.32 (m, 2H), 2.79 - 2.90 (m, 2H), 3.31 (td, 2H), 4.03 (s, 3H), 4.40 - 4.52 (m, 1H), 7.07 (s, 1H), 7.83 - 7.90 (m, 2H), 8.12 (dd, 1H), 8.49 (d, 1H), 8.83 (s, 1H), 10.68 - 10.72 (m, 1H)

### Beispiel 3

### N-{6-Methoxy-2-[1-(2,2,2-trifluorethyl)piperidin-4-yl]-2H-indazol-5-yl}-6-(trifluormethyl)pyridin-2-carboxamid

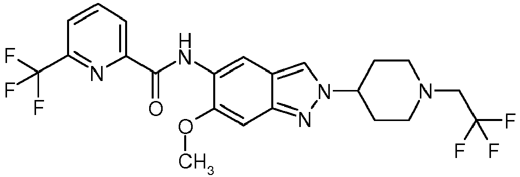

Zu einer Lösung aus 132 mg (0.315 mmol) N-[6-Methoxy-2-(piperidin-4-yl)-2H-indazol-5-yl]-6-(trifluormethyl)pyridin-2-carboxamid (Beispiel 2) in 5 ml Dichlormethan wurden 0.088 ml (0.63 mmol) Triethylamin und 0.091 ml (0.63 mmol) 2,2,2-Trifluorethyl-trifluormethansulfonat [6226-25-1] gegeben und die Reaktion wurde bei Raumtemperatur 2 h gerührt. Dann wurde wasserfreies Tetrahydrofuran zugegeben und die Reaktion wurde 19.5 h erhitzt bei 55°C. Die Reaktion wurde auf Raumtemperatur abgekühlt und dann wurden Dichlormethan und Wasser zugegeben, die organische Phase wurde abgetrennt, die wässrige Phase zweimal mit Dichlormethan extrahiert und die vereinigten organischen Phasen eingeengt im Vakuum. Nach Reinigung (Biotage Isolera, 25 g Kieselgel-Säule, Cyclohexan/Ethylacetat) wurden 107 mg (0.213 mmol) der Titelverbindung erhalten.
LC-MS (Methode B): Rt = 5.11 min; m/z = 502 (M+H)+
¹H-NMR (400 MHz, DMSO-d6): δ = 2.02 - 2.12 (m, 4H), 2.52 - 2.61 (m, 2H), 3.03 (d, 2H), 3.19 - 3.26 (m, 2H), 3.96 (s, 3H), 4.35 - 4.45 (m, 1H), 7.13 (s, 1H), 8.19 (dd, 1H), 8.35 - 8.45 (m, 3H), 8.66 (s, 1H), 10.47 (s, 1H)

### Beispiel 4

### N-[6-Methoxy-2-(1-methylpiperidin-4-yl)-2H-indazol-5-yl]-6-(trifluormethyl)pyridin-2-carboxamid

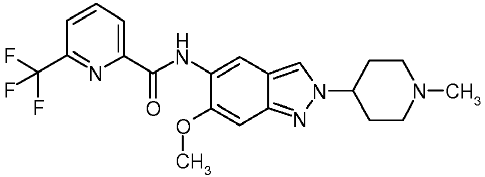

Zu einer Lösung aus 150 mg (0.358 mmol) N-[6-Methoxy-2-(piperidin-4-yl)-2H-indazol-5-yl]-6-(trifluormethyl)pyridin-2-carboxamid (Beispiel 2) in 0.5 ml Tetrahydrofuran und 0.5 ml Methanol wurde 0.1 ml 37% wässriges Formaldehyd [50-00-0] gegeben und dann wurde bei Raumtemperatur 20 Minuten gerührt. 108 mg (0.508 mmol) Natriumtriacetoxyborohydrid wurden zugegeben und die Mischung wurde bei Raumtemperatur 1 h gerührt. Man säuerte mit 1M wässriger Salzsäurelösung tropfenweise an und engte im Vakuum ein. Nach Vorreinigung (SCX Kartusche, 2M Ammoniak in Methanol) wurde zusätzlich durch Säulenchromatographie (Biotage Isolera, 25 g Kieselgel-Säule, Dichlormethan/Methanol) gereinigt. Nach Gefriertrocknung wurden 113 mg (0.261 mmol) der Titelverbindung erhalten.
LC-MS (Methode B): Rt = 3.24 min; m/z = 434 (M+H)+
¹H-NMR (400 MHz, DMSO-d6): δ = 2.05 (dd, 6H), 2.20 (s, 3H), 2.84 - 2.90 (m, 2H), 3.95 (s, 3H), 4.29 - 4.38 (m, 1H), 7.15 (s, 1H), 8.19 (dd, 1H), 8.33 - 8.45 (m, 3H), 8.66 (s, 1H), 10.47 (s, 1H)

### Beispiel 5

### N-[2-(1-Glycoloylpiperidin-4-yl)-6-methoxy-2H-indazol-5-yl]-6-(trifluormethyl)pyridin-2-carboxamid

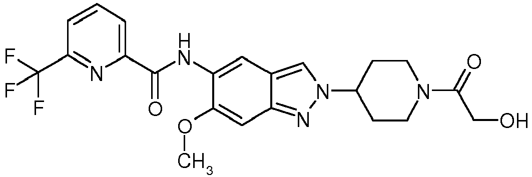

Eine Mischung aus 75 mg (0.179 mmol) N-[6-Methoxy-2-(piperidin-4-yl)-2H-indazol-5-yl]-6-(trifluormethyl)pyridin-2-carboxamid (Beispiel 2), 15 mg (0.197 mmol) Hydroxyessigsäure [79-14-1], 75 mg (0.197 mmol) O-(7-Azabenzotriazol-1-yl)-N,N,N',N'-tetramethyluronium-hexafluorophosphat (HATU) [148893-10-1] in 2 ml N,N-Dimethylformamid wurde mit 0.062 ml (0.358 mmol) N,N-Diisopropylethylamin versetzt und man rührte 22 h bei Raumtemperatur. Die Mischung wurde mit Ethylacetat und Wasser versetzt, man trennte die organische Phase ab, extrahierte zweimal mit Ethylacetat, vereinigte die organischen Phasen, wusch mit Kochsalzlösung, trocknete über Natriumsulfat, filtrierte und engte ein im Vakuum. Nach Reinigung (Biotage Isolera, 25 g Kieselgel-Säule, Ethylacetat/Methanol, danach Massen-basierte automatisierte Reinigung (Methode E)) wurden 19.2 mg (0.040 mmol) der Titelverbindung erhalten.
LC-MS (Methode B): Rt = 4.03 min; m/z = 478 (M+H)+
¹H-NMR (400 MHz, DMSO-d6): δ = 1.85 - 2.05 (m, 2H), 2.12 (dd, 2H), 2.83 (dd, 1H), 3.16 (dd, 1H), 3.84 (d, 1H), 3.95 (s, 3H), 4.13 (d, 2H), 4.46 (d, 1H), 4.55 (s, 1H), 4.63 - 4.73 (m, 1H), 7.13 (s, 1H), 8.19 (dd, 1H), 8.34 - 8.45 (m, 3H), 8.66 (s, 1H), 10.47 (s, 1H)

### Beispiel 6

### N-[6-Methoxy-2-(1'-methyl-1,4'-bipiperidin-4-yl)-2H-indazol-5-yl]-6-(trifluoromethyl)pyridin-2-carboxamid

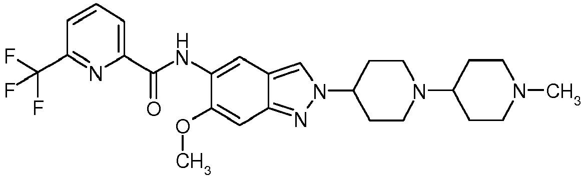

14 mg (0.119 mmol) 1-Methylpiperidin-4-on [1445-73-4] in 2 ml Dichlormethan wurden versetzt mit 50 mg (0.119 mmol) N-[6-Methoxy-2-(piperidin-4-yl)-2H-indazol-5-yl]-6-(trifluormethyl)pyridin-2-carboxamid (Beispiel 2) und 7 Microliter Essigsäure. Die Mischung wurde 45 Minuten bei Raumtemperatur gerührt, dann wurden 38 mg (0.179 mmol) Natriumtriacetoxyborohydrid zugegeben und die Mischung wurde gerührt 19 h bei Raumtemperatur. Weitere 14 mg (0.119 mmol) 1-Methyl-4-piperidinon in 1 ml Dichlormethan und 7 Microliter Essigsäure wurden addiert und die Mischung wurde bei Raumtemperatur 45 Minuten gerührt. Dann wurden 38 mg (0.179 mmol) Natriumtriacetoxyborhydrid zugegeben und die Mischung wurde 24 h gerührt bei Raumtemperatur. Das Lösungsmittel wurde entfernt bei reduziertem Druck, man partitionierte zwischen einer gesättigten NatriumhydrogencarbonatLösung und einer Dichlormethan/Methanol-Mischung, trennte die organische Phase ab und engte im Vakuum ein. Nach Reinigung (Biotage Isolera, 25 g Kieselgel-Säule, 2M Ammoniak in Methanol/Dichlormethan, dann Biotage Isolera (11 g KP-SI NH2, Gradient Cyclohexan/Ethylacetat und Ethylacetat/ Methanol) und Lyophilisierung wurden 21 mg (0.041 mmol) der Titelverbindung erhalten.
LC-MS (Methode B): Rt = 2.78 min; m/z = 517 (M+H)+
¹H-NMR (400 MHz, DMSO-d6): δ = 1.38 - 1.50 (m, 2H), 1.67 (d, 2H), 1.81 (dd, 2H), 1.97 - 2.09 (m, 4H), 2.10 (s, 3H), 2.18 - 2.35 (m, 3H), 2.77 (dd, 2H), 2.97 (d, 2H), 3.95 (s, 3H), 4.27 - 4.37 (m, 1H), 7.13 (s, 1H), 8.19 (dd, 1H), 8.33 - 8.45 (m, 3H), 8.65 (s, 1H), 10.47 (s, 1H)

### Beispiel 7

### N-[6-Methoxy-2-(1-sulfamoylpiperidin-4-yl)-2H-indazol-5-yl]-6-(trifluormethyl)pyridin-2-carboxamid

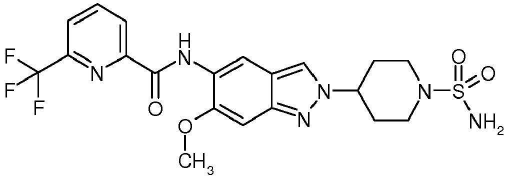

50 mg (0.119 mmol) N-[6-Methoxy-2-(piperidin-4-yl)-2H-indazol-5-yl]-6-(trifluormethyl)pyridin-2-carboxamid (Beispiel 2) in 2 ml Dioxan wurden mit 23 mg (0.239 mmol) Sulfamid [7803-58-9] versetzt. Man erhitzte 10 h auf 110°C. Man kühlte ab, addierte Dichlormethan, Methanol und Wasser, trennte die organische Phase ab und filtrierte. Das Lösungsmittel wurde eingeengt im Vakuum und der Rückstand gereinigt durch Flashchromatograpie (Biotage Isolera, 25 g Kieselgel-Säule, Dichlormethan / Methanol). Man erhielt 45 mg (0.090 mmol) der Titelverbindung.
LC-MS (Methode A): Rt = 3.69 min; m/z = 499 (M+H)+ ¹H-NMR (300 MHz, DMSO-d6): 2.13 - 2.24 (m, 4H), 2.72 -2.85 (m, 2H), 3.58 - 3.64 (m, 2H), 3.99 (s, 3H), 4.52 - 4.57 (m, 1H), 6.84 (s, 2H), 7.18 (s, 1H), 8.22 (dd, 1H), 8.37 - 8.49 (m, 3H), 8.70 (s, 1H), 10.51 (s, 1H)

### Beispiel 8

### N-{2-[1-(Acetylsulfamoyl)piperidin-4-yl]-6-methoxy-2H-indazol-5-yl}-6-(trifluormethyl)pyridin-2-carboxamid

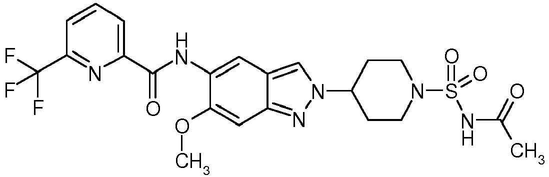

44 mg (0.088 mmol) N-[6-Methoxy-2-(1-sulfamoylpiperidin-4-yl)-2H-indazol-5-yl]-6-(trifluormethyl)pyridin-2-carboxamid (Beispiel 7) in 2 ml Acetonitril wurden mit 0.031ml (0.177 mmol) N,N-Diisopropylethylamin, 11 mg (0.088 mmol) N,N-Dimethylpyridin-4-amin und 0.1 ml einer Lösung aus 0.1 ml Acetylchlorid in 1.0 ml Acetonitril versetzt. Die Mischung wurde bei Raumtemperatur 1 h gerührt. Man versetzte mit Wasser und Ethylacetat, trennte die organische Phase ab, extrahierte zweimal mit Ethylacetat, vereinigte die organischen Phasen, wusch mit Kochsalzlösung, filtrierte und engte ein bei reduziertem Druck. Nach Reinigung (Biotage Isolera, 25 g Kieselgel-Säule, Cyclohexan/Ethylacetat) und Lyophilisieren über Nacht erhielt man 21.8 mg (0.04 mmol) der Titelverbindung.
LC-MS (Methode B): Rt = 4.36 min; m/z = 541 (M+H)+
¹H-NMR (400 MHz, DMSO-d6): 1.97 (s, 3H), 2.05 - 2.21 (m, 4H), 3.06 (t, 2H), 3.74 (d, 2H), 3.96 (s, 3H), 4.51- 4.61 (m, 1H), 7.15 (s, 1H), 8.19 (dd, 1H), 8.34 - 8.45 (m, 3H), 8.67 (s, 1H), 10.48 (s, 1H), 11.48 (s, 1H)

### Beispiel: 9

### N-(2-{1-[2-(Dimethylamino)ethyl]piperidin-4-yl}-6-methoxy-2H-indazol-5-yl)-6-(trifluormethyl)pyridin-2-carboxamid

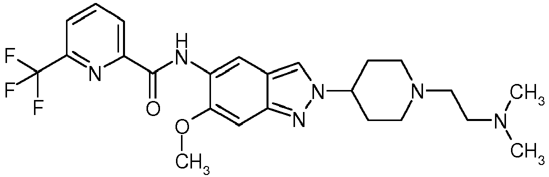

50 mg (0.119 mmol) N-[6-Methoxy-2-(piperidin-4-yl)-2H-indazol-5-yl]-6-(trifluormethyl)pyridin-2-carboxamid (Beispiel 2) in 2 ml wasserfreien Dimethylformamid wurden mit 41 mg (0.298 mmol) Kaliumcarbonat und 24 mg (0.167 mmol) 2-Chlor-N,N-dimethylethanaminhydrochlorid (1:1) [4584-46-7] versetzt. Die Mischung wurde 18 h gerührt bei Raumtemperatur und 8 h bei 60°C und dann auf Raumtemperatur abgekühlt. Die Mischung wurde mit Ethylacetat und Wasser versetzt, die organische Phase abgetrennt, die wässrige Phase dreimal mit Ethylacetat extrahiert und die vereinigten organischen Phasen wurden mit Kochsalzlösung gewaschen, getrocknet (Natriumsulfat), dann filtriert und eingeengt. Nach Reinigung des Rückstandes (Biotage Isolera (11 g KP-NH2), Cyclohexan/Ethylacetat-Gradient, Methanol/Ethylacetat-Gradient, dann Lyophilisieren über Nacht) erhielt man 6.9 mg (0.014 mmol) der Titelverbindung.
LC-MS (Methode B): Rt = 2.91 min; m/z = 491 (M+H)+
¹H-NMR (400 MHz, DMSO-d6): δ = 2.00 - 2.10 (m, 12H), 2.31 - 2.44 (m, 4H), 2.99 (d, 2H), 3.95 (s, 3H), 4.30 - 4.39 (m, 1H), 7.14 (s, 1H), 8.19 (dd, 1H), 8.34 - 8.45 (m, 3H), 8.66 (s, 1H), 10.47 (s, 1H)

### Beispiel: 10

### N-{6-Methoxy-2-[1-(oxetan-3-yl)piperidin-4-yl]-2H-indazol-5-yl}-6-(trifluormethyl)pyridin-2-carboxamid

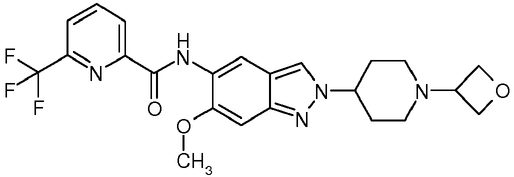

100 mg (0.24 mmol) N-[6-Methoxy-2-(piperidin-4-yl)-2H-indazol-5-yl]-6-(trifluormethyl)pyridin-2-carboxamid (Beispiel 2) in 3 ml Dichlormethan wurden mit einer Lösung aus 26 mg (0.36 mmol) 3-Oxetanon [6704-31-0] in 1 ml Dichlormethan und 0.027 ml (0.48 mmol) Essigsäure versetzt. Die Mischung wurde 0.5 h gerührt bei Raumtemperatur. 76 mg (0.36 mmol) Natriumtriacetoxyborhydrid wurden zugesetzt und die Mischung wurde 67 h bei Raumtemperatur gerührt. Durch Zugabe einer Dichlormethan/Methanol-Mischung und gesättigter Natriumhydrogencarbonatlösung wurden zwei Phasen erzeugt, man trennte die organische Phase ab und entfernte das Lösungsmittel in Vakuum. Nach Reinigung (Biotage Isolera, 25 g Kieselgel-Säule, Dichlormethan/Methanol) wurden 36.5 mg (0.077 mmol) der Titelverbindung erhalten.
LC-MS (Methode B): Rt = 3.27 min; m/z = 476 (M+H)+
¹H-NMR (400 MHz, DMSO-d6): δ = 1.95 - 2.12 (m, 6H), 2.81 (d, 2H), 3.40 - 3.48 (m, 1H), 3.96 (s, 3H), 4.41 - 4.46 (m, 3H), 4.54 (dd, 2H), 7.14 (s, 1H), 8.19 (dd, 1H), 8.35 - 8.45 (m, 3H), 8.66 (s, 1H), 10.48 (s, 1H)

### Beispiel: 11

### N-[2-(1,1-Dioxidotetrahydro-2H-thiopyran-4-yl)-6-methoxy-2H-indazol-5-yl]-6-(trifluormethyl)pyridin-2-carboxamid

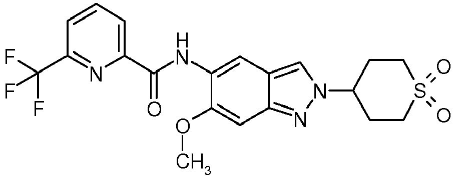

Eine Mischung aus 2.55 g (8.6 mmol) 2-(1,1-Dioxidotetrahydro-2H-thiopyran-4-yl)-6-methoxy-2H-indazol-5-amin (Intermediat 4C), 1.73 g (9.1 mmol) 6-(Trifluormethyl)pyridin-2-carbonsäure [131747-42-7] und 3.45 g (9.1 mmol) *O*-(7-Azabenzotriazol-1-yl)-*N,N,N',N*'-tetramethyluronium-hexafluorophosphat (HATU) [148893-10-1] in 30 ml DMF wurde mit 3 ml (17.3 mmol) N-Ethyl-N-isopropylpropan-2-amin versetzt und die Mischung wurde bei Raumtemperatur 15 h gerührt. Wasser und Ethylacetat wurden zugegeben, man trennte die organische Phase ab, extrahierte dreimal mit Ethylacetat, vereinigte die organischen Phasen, wusch mit Kochsalzlösung, trocknete über Natriumsulfat, filtrierte und engte ein im Vakuum. Der Rückstand wurde mit Dichlormethan versetzt und der entstandene Feststoff mit 100 ml Ethylacetat ausgerührt. Das Lösungsmittel wurde eingeengt bei reduziertem Druck, der Rückstand wurde getrocknet bei 60°C für 24 h und bei 90°C für 24 h. Man erhielt 2.57 g (5.5 mmol) der Titelverbindung.
LC-MS (Methode B): Rt = 4.29 min; m/z = 469 (M+H)+ ¹H-NMR (400 MHz, DMSO-d6): δ = 2.40 (dd, 2H), 2.49 - 2.61 (m, 2H), 3.21 - 3.25 (m, 2H), 3.44 (t, 2H), 3.96 (s, 3H), 4.80 - 4.89 (m, 1H), 7.17 (s, 1H), 8.19 (dd, 1H), 8.37 - 8.45(m, 3H), 8.67 (s, 1H), 10.48 (s, 1H)

### Beispiel 12

### Rac-N-[6-Methoxy-2-(1-oxidotetrahydro-2H-thlopyran-4-yl)-2H-indazol-5-yl]-6-(trifluormethyl)pyridin-2-carboxamid

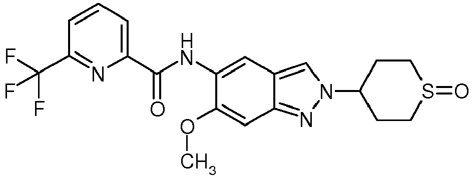

Zu einer Mischung aus 426 mg (1.52 mmol) Rac-6-Methoxy-2-(1-oxidotetrahydro-2H-thiopyran-4-yl)-2H-indazol-5-amin (Intermediat 4D), 306 mg (1.6 mmol) 6-(Trifluormethyl)pyridin-2-carbonsäure [131747-42-7] und 609 mg (1.6 mmol) O-(7-Azabenzotriazol-1-yl)-N,N,N',N'-tetramethyluronium-hexafluorophosphat (HATU) [148893-10-1] in 10 ml Dimethylformamid wurden 0.531 ml (3.0 mmol) N,N-Diisopropylethylamin zugegeben und dann wurde gerührt bei Raumtemperatur 23 h. Die Reaktion wurde mit Ethylacetat und Wasser versetzt, man trennte die organische Phase ab, extrahierte dreimal mit Ethylacetat, vereinigte die organischen Phasen, wusch mit Kochsalzlösung, trocknete über Natriumsulfat, filtrierte und engte das Lösungsmittel bei reduziertem Druck ein. Nach Reinigung (Biotage Isolera, 50 g Kieselgel-Säule, Dichlormethan/Methanol) wurden 560 mg (1.2 mmol) der Titelverbindung erhalten.
LC-MS (Methode A): Rt = 3.42 & 3.47 min; m/z = 453 (M+H)+
¹H-NMR (300 MHz, CDCl3): δ = 2.31 - 2.47 (m, 2H), 2.66 - 3.02 (m, 4H), 3.24 - 3.29 (m, 1H), 3.39 - 3.49 (m, 1H), 4.04 (d, 3H), 4.62 - 4.70 (m, 1H), 7.05 (d, 1H), 7.85 - 7.89 (m, 2H), 8.12 (dd, 1H), 8.49 (d, 1H), 8.84 (d, 1H), 10.71 (s, 1H)

### Beispiel: 13

### N-{2-[1-(2-Hydroxyethyl)piperidin-4-yl]-6-methoxy-2H-indazol-5-yl}-6-(trifluormethyl)pyridin-2-carboxamid

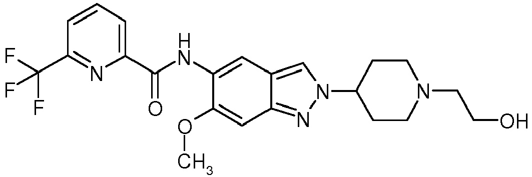

### Stufe A:

### N-{2-[1-(2-{[tert-Butyl(dimethyl)silyl]oxy}ethyl)piperidin-4-yl]-6-methoxy-2H-indazol-5-yl}-6-(trifluormethyl)pyridin-2-carboxamid

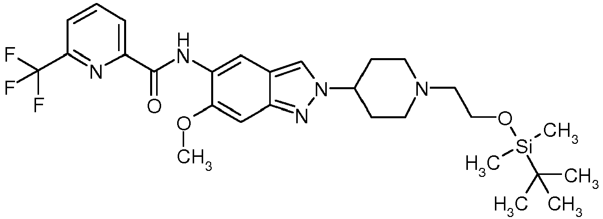

Zu einer Lösung aus 85 mg (0.2 mmol) N-[6-Methoxy-2-(piperidin-4-yl)-2H-indazol-5-yl]-6-(trifluormethyl)pyridin-2-carboxamid (Beispiel 2) in 5 ml Dichlormethan wurden 0.155 ml (0.81 mmol) {[tert-Butyl(dimethyl)silyl]oxy}acetaldehyd [102191-92-4] und 0.046 ml (0.81 mmol) Essigsäure zugesetzt. Die Mischung wurde 5 Minuten gerührt, dann wurden 86 mg (0.41 mmol) Natriumtriacetoxyborohydrid zugesetzt und man rührte 1 Stunde. Man addierte gesättigte Natriumhydrogencarbonat-Lösung, rührte 5 Minuten, filtrierte und entfernte das Lösungsmittel im Vakuum. Nach Reinigung an Kieselgel (Biotage Isolera, 25 g Kieselgel-Säule, Cyclohexan/Ethylacetat-Gradient, dann ein Ethylacetat/Methanol-Gradient) wurden 57 mg (0.099 mmol) N-{2-[1-(2-{[tert-Butyl(dimethyl)silyl]oxy}ethyl)piperidin-4-yl]-6-methoxy-2H-indazol-5-yl} -6-(trifluormethyl)pyridin-2-carboxamid erhalten.
LC-MS (Methode A): Rt = 3.41 min; m/z = 578 (M+H)+
¹H-NMR (300 MHz, CDCl3): δ = 0.08 (s, 6H), 0.91 (s, 9H), 2.24 - 2.26 (m, 6H), 2.57 - 2.66 (m, 2H), 3.06 - 3.18 (m, 2H), 3.75 - 3.83 (m, 2H), 4.03 (s, 3H), 4.30 - 4.40 (m, 1H), 7.06 (s, 1H), 7.83 - 7.90 (m, 2H), 8.11 (dd, 1H), 8.47 - 8.51 (m, 1H), 8.82 (s, 1H), 10.69 -10.71 (m, 1H)

### Stufe B:

57 mg (0.099 mmol) N-{2-[1-(2-{[tert-Butyl(dimethyl)silyl]oxy}ethyl)piperidin-4-yl]-6-methoxy-2H-indazol-5-yl}-6-(trifluormethyl)pyridin-2-carboxamid (Stufe A) wurden mit 3 ml einer 4M Chlorwasserstoff-Lösung in Dioxan versetzt und man rührte 1 h bei Raumtemperatur. Das Lösungsmittel wurde eingeengt im Vakuum und der Rückstand gereinigt durch eine SCX Kartusche (Lösungmittel 2M Ammoniak in Methanol). Nach Entfernen des Lösungsmittels im Vakuum und Lyophilisieren über Nacht wurden 41 mg (0.088 mmol) N-{2-[1-(2-Hydroxyethyl)piperidin-4-yl]-6-methoxy-2H-indazol-5-yl}-6-(trifluormethyl)pyridin-2-carboxamid erhalten.
LC-MS (Methode B): Rt = 3.22 min; m/z = 464 (M+H)+
¹H-NMR (400 MHz, DMSO-d6): δ = 2.01- 2.19 (m, 6H), 2.42 (t, 2H), 2.98 (d, 2H), 3.50 (ddd, 2H), 3.96 (s, 3H), 4.34 - 4.39 (m, 2H), 7.14 (s, 1H), 8.19 (dd, 1H), 8.33 - 8.45 (m, 3H), 8.66 (s, 1H), 10.47 (s, 1H)

### Beispiel 14

### rel-N-{2-[(1S,4S,5R)-2-Azabicyclo[2.2.1]hept-5-yl]-6-methoxy-2H-indazol-5-yl}-6-(trifluormethyl)pyridin-2-carboxamid

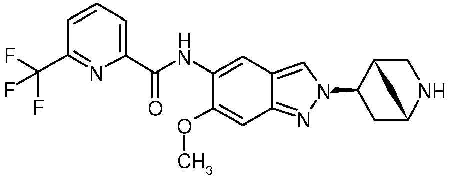

### Stufe A:

### tert-Butyl-rel-(1S,4S,5R)-5-[6-methoxy-5-({[6-(trifluormethyl)pyridin-2-yl]carbonyl}amino)-2H-indazol-2-yl]-2-azabicyclo[2.2.1]heptan-2-carboxylat

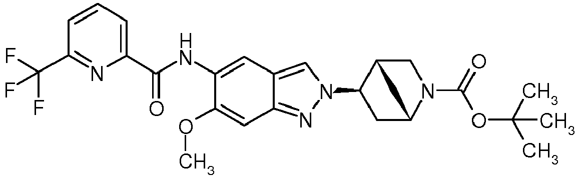

Zu einer Mischung aus 150 mg (0.418mmol) tert-Butyl-rel-(1S,4S,5R)-5-(5-amino-6-methoxy-2H-indazol-2-yl)-2-azabicyclo[2.2.1]heptan-2-carboxylat (Intermediat 4E), 84 mg (0.439 mmol) 6-(Trifluormethyl)pyridin-2-carbonsäure [131747-42-7] und 167 mg (0.439mmol) 0-(7-Azabenzotriazol-1-yl)-N,N,N',N'-tetramethyluronium-hexafluorophosphat (HATU) [148893-10-1] in 3 ml DMF gab man 0.146 ml (0.84 mmol) N-Ethyl-N-isopropylpropan-2-amin und rührte 16 h bei Raumtemperatur. Man addierte Wasser und Ethylacetat, trennte die organische Phase ab, extrahierte zweimal mit Ethylacetat, wusch die vereinigten organischen Phasen mit Kochsalzlösung, trocknete über Natriumsulfat, filtrierte und engte ein. Nach Reinigung (Biotage Isolera (25g Kieselgelsäule), Cyclohexan / Ethylacetat) erhielt man 151 mg (0.284 mmol) tert-Butyl-rel-(1S,4S,5R)-5-[6-methoxy-5-({[6-(trifluormethyl)pyridin-2-yl]carbonyl}amino)-2H-indazol-2-yl]-2-azabicyclo[2.2.1]heptan-2-carboxylat.
LC-MS (Methode A): Rt = 4.62 min; m/z = 532 (M+H)+
¹H-NMR (300 MHz, CDCl3): δ = 1.50 (s, 9H), 1.81 - 1.72 (m, 1H), 2.28 - 2.43 (m, 3H), 2.93 (d, 1H), 3.14 - 3.40 (m, 2H), 4.03 (s, 3H), 4.41 (d, 1H), 4.64 - 4.69 (m, 1H), 7.06 (s, 1H), 7.83 - 7.91 (m, 2H), 8.11 (dd, 1H), 8.49 (d, 1H), 8.81 (s, 1H), 10.70 (s, 1H).

### Stufe B:

151 mg (0.284 mmol) tert-Butyl-rel-(1S,4S,5R)-5-[6-methoxy-5-({[6-(trifluormethyl)pyridin-2-yl]carbonyl}amino)-2H-indazol-2-yl]-2-azabicyclo[2.2.1]heptan-2-carboxylat wurden mit 3 ml 4M Chlorwasserstofflösung in Dioxan und 1 ml Methanol versetzt und man rührte 1 h bei Raumtemperatur, entfernte das Lösungsmittel im Vakuum, reinigte mit einer SCX Kartusche (Laufmittel 2M Ammoniak in Methanol), entfernte das Lösungsmittel im Vakuum und erhielt 116 mg (0.269 mmol) der Titelverbindung.
LC-MS (Methode A): Rt = 2.73 & 2.82 min; m/z = 432 (M+H)+
¹H-NMR (300 MHz, CDCl3): δ = 1.59 (s, 1H), 2.14 - 2.31 (m, 2H), 2.39 - 2.48 (m, 1H), 2.78 - 2.89 (m, 2H), 3.05 (dd, 1H), 3.68 - 3.72 (m, 1H), 4.02 (s, 3H), 4.64 (dd, 1H), 7.07 (s, 1H), 7.83 - 7.92 (m, 2H), 8.11 (dd, 1H), 8.49 (d, 1H), 8.81 (s, 1H), 10.70 (s, 1H).

### Beispiel 15

### rel-N-{2-[(1S,4S,5S)-2-Azabicyclo[2.2.1]hept-5-yl]-6-methoxy-2H-indazol-5-yl}-6-(trifluormethyl)pyridin-2-carboxamid

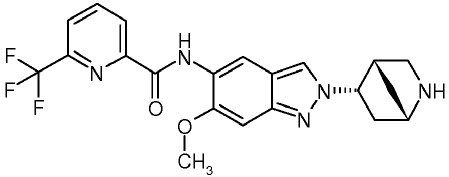

### Stufe A:

### tert-Butyl-rel-(1S,4S,5S)-5-[6-methoxy-5-({[6-(trifluormethyl)pyridin-2-yl]carbonyl}amino)-2H-indazol-2-yl]-2-azabicyclo[2.2.1]heptan-2-carboxylat

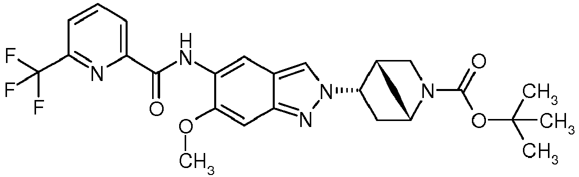

Eine Mischung aus 297 mg (0.829 mmol) tert-Butyl-rel-(1S,4S,5S)-5-(5-amino-6-methoxy-2H-indazol-2-yl)-2-azabicyclo[2.2.1]heptan-2-carboxylat (Intermediat 4F), 166 mg (0.87 mmol) 6-(Trifluormethyl)pyridin-2-carbonsäure [131747-42-7] und 331 mg (0.87 mmol) 0-(7-Azabenzotriazol-1-yl)-N,N,N',N'-tetramethyluronium-hexafluorophosphat (HATU) [148893-10-1] in 3 ml DMF wurde mit 0.289 ml (1.66 mmol) N-Ethyl-N-isopropylpropan-2-amin versetzt und bei Raumtemperatur 17 h gerührt. Man gab Ethylacetat und Wasser zu, trennte die organische Phase ab, extrahierte zweimal die wässrige Phase mit Ethylacetat, vereinigte die organischen Phasen, wusch mit Kochsalzlösung, trocknete über Natriumsulfat, filtrierte und entfernte die Lösungsmittel im Vakuum. Nach Reinigung (Biotage Isolera (25g Kieselgelsäule), Cyclohexan / Ethylacetat) wurden 383 mg (0.721 mmol) tert-Butyl-rel-(1S,4S,5S)-5-[6-methoxy-5-({[6-(trifluormethyl)pyridin-2-yl]carbonyl}amino)-2H-indazol-2-yl]-2-azabicyclo[2.2.1]heptan-2-carboxylat erhalten.
LC-MS (Methode A): Rt = 4.47 min; m/z = 532 (M+H)+
¹H-NMR (300 MHz, CDCl3): δ = 1.50 (d, 9H), 1.80 (d, 1H), 1.88 - 1.95 (m, 1H), 2.32 - 2.42 (m, 2H), 2.53 (d, 0.5H), 2.98 (d, 0.5H), 3.13 - 3.21 (m, 2H), 4.04 (s, 3H), 4.38 (d, 1H), 5.07 - 5.07 (m, 1H), 7.04 (s, 1H), 7.83 - 7.91 (m, 2H), 8.12 (dd, 1H), 8.50 (d, 1H), 8.81 (s, 1H), 10.68 - 10.73 (m, 1H)

### Stufe B:

383 mg (0.72 mmol) tert-Butyl-rel-(lS,4S,5S)-5-[6-methoxy-5-({[6-(trifluormethyl)pyridin-2-yl]carbonyl}amino)-2H-indazol-2-yl]-2-azabicyclo[2.2.1]heptan-2-carboxylat wurden mit 3 ml 4M Chlorwasserstoff in Dioxan und 1 ml Methanol versetzt und man ließ 1 h bei Raumtemperatur rühren. Das Lösungsmittel wurde entfernt bei reduziertem Druck und der Rückstand gereinigt mit einer SCX Kartusche mit 2M Ammoniak in Methanol. Das Lösungsmittel wurde im Vakuum entfernt und man erhielt 245 mg (0.568 mmol) der Titelverbindung.
LC-MS (Methode A): Rt = 2.74 & 2.84 min; m/z = 432 (M+H)+
¹H-NMR (300 MHz, CDCl3): δ =1.79 - 1.89 (m, 2H), 2.27 - 2.39 (m, 2H), 2.65 (d, 1H), 2.78 (d, 1H), 2.97 (s, 1H), 3.69 (d, 1H), 4.03 (s, 3H), 4.96 - 5.02 (m, 1H), 7.07 (s, 1H), 7.85 (dd, 1H), 8.00 (s, 1H), 8.11 (dd, 1H), 8.49 (d, 1H), 8.82 (s, 1H), 10.70 (s, 1H).

### Beispiel 16

### N-[2-(1,1-Dioxidotetrahydro-2H-thiopyran-4-yl)-6-hydroxy-2H-indazol-5-yl]-6-(trifluormethyl)pyridin-2-carboxamid

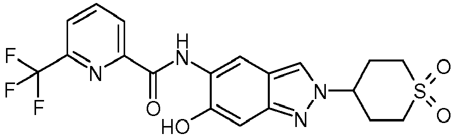

Eine Mischung aus 0.216 g (0.461 mmol) N-[2-(1,1-Dioxidotetrahydro-2H-thiopyran-4-yl)-6-methoxy-2H-indazol-5-yl]-6-(trifluormethyl)pyridin-2-carboxamid (Beispiel 11) und 0.187 g (0.507 mmol) Tetra-n-butylammoniumiodid in 10 mL Dichlormethan unter Argon wurde auf -70°C abgekült Es wurden 3.7 mL (3.7 mmol) 1M Bortrichlorid in Dichlormethan langsam zugegeben und man ließ 10 Minuten bei -70°C, dann 1 h bei Raumtemperatur rühren. Man kühlte auf -70°C ab und addierte weitere 0.92 ml (0.92 mmol) 1M Bortrichlorid in Dichlormethan und ließ 7 h bei Raumtemperatur rühren. Die Mischung wurde auf Wasser gegossen und man extrahierte mit Dichlormethan:Methanol (5 x 25 ml 9:1, dann 4 x 25 ml 4:1). Die wässrige Phase wurde mit Natriumhydrogencarbonatlösung basisch gestellt und dann extrahiert mit Dichlormethan:Methanol (10 x 25 ml 4:1). Die vereinigten organischen Phasen wurden getrocknet, eingeengt und durch Flashchromatographie (Biotage Isolera (50 g Kieselgel), Methanol in Dichlormethan) gereinigt. Man erhielt 0.110 g der Titelverbindung als Feststoff.
LC-MS (Methode B); Rt = 3.41 min; m/z = 455 (M+H)+
1H-NMR (300 MHz, DMSO-d6): δ [ppm] = 2.35 - 2.65 (m, 4H, under DMSO), 3.2 - 3.36 (m, 2H, unter HOD), 3.38 - 3.52 (m, 2H), 4.76 - 4.89 (m, 1H), 6.95 (s, 1H), 8.21 (dd, 1H), 8.34 (s, 1H), 8.37 - 8.49 (m, 2H), 8.68 (s, 1H), 10.55 (S, 1H), 10.71 (s, 1H).

### Beispiel 17

### N-[6-(Cyclopropylmethoxy)-2-(1,1-dioxidotetrahydro-2H-thiopyran-4-yl)-2H-indazol-5-yl]-6-(trifluormethyl)pyridin-2-carboxamid

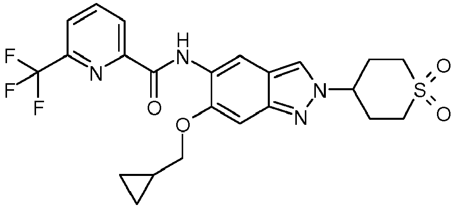

Zu einer Mischung aus 0.107 g (0.235 mmol) N-[2-(1,1-Dioxidotetrahydro-2H-thiopyran-4-yl)-6-hydroxy-2H-indazol-5-yl]-6-(trifluormethyl)pyridin-2-carboxamid (Beispiel 16), 0.080 g (0.307 mmol) Triphenylphosphin und 0.022 g (0.307 mmol) Cyclopropylmethanol in 4 ml Tetrahydrofuran wurden 0.060 ml (0.307 mmol) Diisopropylazodicarboxylat gegeben. Die Mischung wurde unter Argon bei Raumtemperatur 3 h gerührt. Triphenylphosphin (ca. 40 mg) und Diisopropylazodicarboxylat (ca. 0.03 mL) wurden in 0.5 ml Tetrahydrofuran gelöst und diese Lösung wurde zur Reaktionsmischung hinzugegeben. Die Mischung wurde mit Wasser verdünnt, dreimal mit Dichlormethan extrahiert und die vereinigten organischen Phasen wurden eingeengt. Der Rückstand wurde durch Flashchromatographie (Biotage Isolera (50 g Kieselgel), Ethylacetat / Cyclohexan) und zusätzlich noch durch präparative HPLC (Methode F) gereinigt. Man erhielt 0.015 g der Titelverbindung als Feststoff.
LC-MS (Methode B); Rt = 4.87 min; m/z = 509 (M+H)+
1H-NMR (400 MHz, DMSO-d6): δ [ppm] = 0.40 - 0.47 (m, 2H), 0.62 - 0.69 (m, 2H), 1.29 - 1.40 (m, 1H), 2.37 - 2.46 (m, 2H, under DMSO), 2.51 - 2.63 (m, 2H), 3.23 - 3.33 (m, 2H, under HOD), 3.4 - 3.5 (m, 2H), 4.03 (d, 2H), 4.82 - 4.91 (m, 1H), 7.12 (s, 1H), 8.22 (dd, 1H), 8.38 - 8.49 (m, 3H), 8.75 (s, 1H), 10.71 (s, 1H).

### Beispiel 18

### rel-N-{6-Methoxy-2-[(1S,4S,5R)-2-methyl-2-azabicyclo[2.2.1]hept-5-yl]-2H-indazol-5-yl}-6-(trifluormethyl)pyridin-2-carboxamid

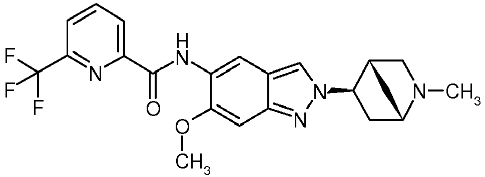

114 mg (0.265mmol) rel-N-{2-[(1S,4S,5R)-2-Azabicyclo[2.2.1]hept-5-yl]-6-methoxy-2H-indazol-5-yl}-6-(trifluormethyl)pyridin-2-carboxamid (Beispiel 14) in 0.5 ml Tetrahydrofuran und 0.5 ml Methanol wurden mit 0.065 ml 37%iger (Gewichtsprozent) wässriger Formaldehydlösung versetzt und man rührte die Mischung 0.5 h bei Raumtemperatur. 80 mg (0.376 mmol) Natriumtriacetoxyborohydrid wurden zugegeben und die Mischung wurde 1 h bei Raumtemperatur gerührt. Zu der Mischung wurde 1M Salzsäure gegeben und man reinigte mit einer SCX Kartusche mit 2M Ammoniak in Methanol. Nach Entfernen des Lösungsmittels und Trocknen wurden 104 mg (0.233mmol) der Titelverbindung erhalten.
LC-MS (Methode B): Rt = 3.38 min; m/z = 446 (M+H)+
¹H-NMR (400 MHz, DMSO-d6): δ = 1.59 (d, 1H), 1.92 (d, 1H), 2.10 - 2.17 (m, 1H), 2.25 - 2.28 (m, 4H), 2.30 - 2.40 (m, 1H), 2.59 - 2.60 (m, 1H), 2.67 (dd, 1H), 3.18 (s, 1H), 3.95 (s, 3H), 4.59 (dd, 1H), 7.15 (s, 1H), 8.19 (dd, 1H), 8.35 - 8.45(m, 3H), 8.65 (s, 1H), 10.47 (s, 1H).

### Beispiel 19

### rel-N-{6-Methoxy-2-[(1S,4S,5S)-2-methyl-2-azabicyclo[2.2.1]hept-5-yl]-2H-indazol-5-yl}-6-(trifluormethyl)pyridin-2-carboxamid

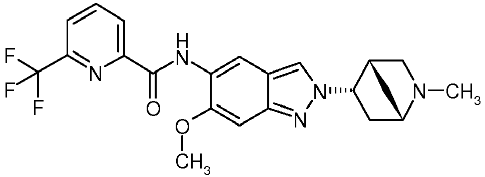

245 mg (0.568mmol) rel-N-{2-[(1S,4S,5S)-2-Azabicyclo[2.2.1]hept-5-yl]-6-methoxy-2H-indazol-5-yl}-6-(trifluormethyl)pyridin-2-carboxamid (Beispiel 15) in 2.0 ml Tetrahydrofuran und 2.0 ml Methanol wurden mit 0.139 ml 37%iger (Gewichtsprozent) wässriger Formaldehydlösung [50-00-0] versetzt und man rührte 0.5 h bei Raumtemperatur. 171 mg (0.807 mmol) Natriumtriacetoxyborhydrid wurden zugegeben und die Mischung wurde 1 h gerührt. Zu der Mischung wurde 1M Salzsäure gegeben und man reinigte mit einer SCX Kartusche mit 2M Ammoniak in Methanol. Nach Entfernen des Lösungsmittels und Trocknen wurden 237 mg (0.532 mmol) der Titelverbindung erhalten.
LC-MS (Methode B): Rt = 3.35 min; m/z = 446 (M+H)+
¹H-NMR (400 MHz, DMSO-d6): δ = 1.60 (d, 1H), 1.78 (dd, 1H), 1.93 - 2.08 (m, 2H), 2.22 (s, 3H), 2.34 - 2.40 (m, 1H), 2.50 - 2.53 (m, 1H), 2.86 (dd, 1H), 3.13 (s, 1H), 3.96 (s, 3H), 4.86 - 4.93 (m, 1H), 7.15 (s, 1H), 8.19 (dd, 1H), 8.37 - 8.46 (m, 3H), 8.67 (s, 1H), 10.48 (s, 1H).

### Beispiel 20 und Beispiel 21

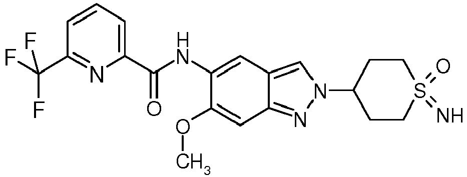

### N-[2-(1-Imino-1-oxidohexahydro-1λ⁴-thiopyran-4-yl)-6-methoxy-2H-indazol-5-yl]-6-(trifluormethyl)pyridin-2-carboxamid (Isomer 1, Beispiel 20)

### N-[2-(1-Imino-1-oxidohexahydro-1λ⁴-thiopyran-4-yl)-6-methoxy-2H-indazol-5-yl]-6-(trifluormethyl)pyridin-2-carboxamid (Isomer 2, Beispiel 21)

### Stufe A:

### N-(6-Methoxy-2-{1-oxido-1-[(trifluoracetyl)imino]hexahydro-1λ⁴-thlopyran-4-yl}-2H-indazol-5-yl)-6-(trifluormethyl)pyridin-2-carboxamid

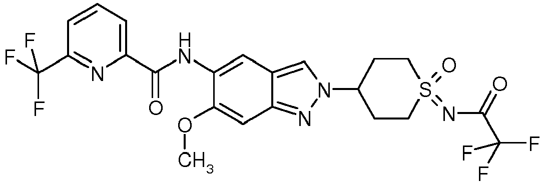

335 mg (0.74 mmol) Rac-N-[6-Methoxy-2-(1-oxidotetrahydro-2H-thiopyran-4-yl)-2H-indazol-5-yl]-6-(trifluormethyl)pyridin-2-carboxamid (Beispiel 12) in 12 ml Dichlormethan wurden mit 167 mg (1.48 mmol) 2,2,2-Trifluoroacetamid [354-38-1], 358 mg (1.1 mmol) Diacetoxy(phenyl)-lambda³-iodan [3240-34-4], 119 mg (3.0 mmol) Magnesiumoxid [1309-48-4] und 32.7 mg (0.074 mmol) Rhodium-(II)acetat Dimer [15956-28-2] versetzt und die Mischung wurde bei Raumtemperatur 18 h unter Argon gerührt. Man addierte 167 mg (1.48 mmol) 2,2,2-Trifluoroacetamid [354-38-1], 358 mg (1.1 mmol) Diacetoxy(phenyl)- λ³-iodan [3240-34-4], 119 mg (3.0 mmol) Magnesiumoxid [1309-48-4] und 32.7 mg (0.074 mmol) Rhodium-(II)acetat Dimer [15956-28-2] und rührte weitere 24 h. 50 ml Dichlormethan wurden zugesetzt und die Mischung wurde 1 h gerührt, dann filtriert (Whatman PTFE filter cup) und es wurde mit 50 ml Dichlormethan gewaschen. Das Lösungsmittel wurde in Vakuum entfernt und der Rückstand gereinigt (Biotage Isolera (50g Kieselgelsäule), Cyclohexan / Ethylacetat). Man erhielt 265 mg (0.47 mmol) N-(6-Methoxy-2-{1-oxido-1-[(trifluoracetyl)imino]hexahydro-1λ⁴-thiopyran-4-yl}-2H-indazol-5-yl)-6-(trifluormethyl)pyridin-2-carboxamid.
LC-MS (Methode A): Rt = 4.28 min; m/z = 564 (M+H)+
¹H-NMR (300 MHz, CDCl3): δ = 2.69 - 3.04 (m, 6H), 3.38 (t, 1H), 3.83 (s, 1H), 3.98 (d, 3H), 4.20 - 4.25 (m, 1H), 7.29 (s, 1H), 7.88 (d, 1H), 8.11 - 8.17 (m, 2H), 8.53 (d, 1H), 8.97 (s, 1H), 10.77 (d, 1H)

### Stufe B:

Zu 265 mg (0.471 mmol) N-(6-Methoxy-2-{1-oxido-1-[(trifluoracetyl)imino]hexahydro-1λ⁴-thiopyran-4-yl}-2H-indazol-5-yl)-6-(trifluormethyl)pyridin-2-carboxamid in 20 ml Methanol wurden 325 mg (2.4 mmol) Kaliumcarbonat gegeben und man rührte bei Raumtemperatur 2 h. Weitere 325 mg (2.4mmol) Kaliumcarbonat wurden zugegeben und man rührte 67 h bei Raumtemperatur. Methanol wurde entfernt, Wasser zugegeben und der entstandene Feststoff abfiltriert und gereinigt (Biotage Isolera (25g Kieselgelsäule), Methanol / Dichlormethan). Man erhielt zwei Isomere der Titelverbindung (Isomer 1 (Beispiel 20): 46.2mg (0.099 mmol) und Isomer 2 (Beispiel 21): 10 mg (0.021 mmol)).

### Beispiel 20

LC-MS (Methode B): Rt = 3.79 min; m/z = 468 (M+H)+
¹H-NMR (400 MHz, DMSO-d6): δ = 2.26 - 2.34 (m, 2H), 2.50 - 2.60 (m, 2H), 3.10 - 3.18 (m, 2H), 3.33 - 3.38 (m, 2H), 3.65 (s, 1H), 3.96 (s, 3H), 4.77 - 4.86 (m, 1H), 7.16 (s, 1H), 8.19 (dd, 1H), 8.45 - 8.33 - 8.45 (m, 3H), 8.67 (s, 1H), 10.48 (s, 1H).

### Beispiel 21

LC-MS (Methode B): Rt = 3.86 min; m/z = 468 (M+H)+
¹H-NMR (400 MHz, DMSO-d6): δ = 2.34 (dd, 2H), 2.50 - 2.58 (m, 2H), 3.12 - 3.27 (m, 4H), 3.80 (s, 1H), 3.96 (s, 3H), 4.75 - 4.84 (m, 1H), 7.17 (s, 1H), 8.19 (dd, 1H), 8.36 - 8.45 (m, 3H), 8.67 (s, 1H), 10.48 (s, 1H).

### Beispiel 22

### N-[6-Methoxy-2-(5-oxopyrrolidin-3-yl)-2H-indazol-5-yl]-6-(trifluormethyl)pyridin-2-carboxamid

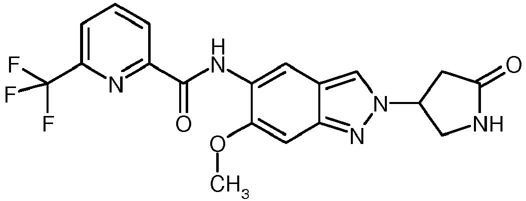

Eine Mischung aus 119 mg (0.483 mmol) 4-(5-Amino-6-methoxy-2H-indazol-2-yl)pyrrolidin-2-on (Intermediat 4I), 92 mg (0.483 mmol) 6-(Trifluormethyl)pyridin-2-carbonsäure und 193 mg (0.507 mmol) HATU in 5 ml DMF und 0.168 ml (0.966 mmol) N,N-Diisopropylethylamin wurde bei Raumtemperatur 18 h gerührt. Wasser und Ethylacetat wurden zugegeben, danach entfernte man zum Teil das Lösungsmittel im Vakuum, addierte Wasser und extrahierte mit einer Mischung aus Methanol/Dichlormethan. Man filtrierte und engte ein. Danach wurde das Rohprodukt aus heißem Ethanol umkristallisert. Man erhielt 64 mg der Titelverbindung.
¹H-NMR (400 MHz, DMSO-d6): d = 2.65 (dd, 1H), 2.83 (dd, 1H), 3.48 (dd, 1H), 3.82 (dd, 1H), 3.96 (s, 3H), 5.34 - 5.42 (m, 1H), 7.18 (s, 1H), 7.83 (s, 1H), 8.19 (dd, 1H), 8.37 - 8.46 (m, 3H), 8.67 (s, 1H), 10.48 (s, 1H).

### Beispiel 23

### 6-(Difluormethyl)-N-[6-methoxy-2-(tetrahydro-2H-pyran-4-yl)-2H-indazol-5-yl]pyridin-2-carboxamid

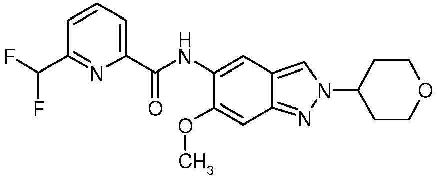

Eine Mischung aus 80 mg 6-Methoxy-2-(tetrahydro-2H-pyran-4-yl)-2H-indazol-5-amin (Intermediat 4A), 73 mg 6-(Difluormethyl)pyridin-2-carbonsäure [1256824-41-5] und 124 mg N-[3-(Dimethylamino)propyl]-N'-ethylcarbodiimid (EDC, CAS1892-57-5) und 50 mg 1H-Benzotriazol-l-olhydrat (1:1) (HOBt, CAS123333-53-9] in 2.5 ml Dimethylformamid wurde mit 135 Microliter Triethylamin versetzt und man rührte bei Raumtemperatur 17 h. Die Reaktion wurde mit Ethylacetat und Wasser versetzt, die organische Phase wurde abgetrennt und die wässrige Phase dreimal mit Ethylacetat extrahiert. Die vereinigten organischen Phasen wurden eingeengt und der Rückstand durch präparative HPLC gereinigt. Man erhielt 56 mg der Titelverbindung.
UPLC-MS (Methode C): Rt = 1.11 min; gefundene Masse 402.15.
¹H-NMR (400MHz, DMSO-d₆): δ [ppm]= 2.02 - 2.16 (m, 4H), 3.46 - 3.57 (m, 2H), 3.95 - 4.04 (m, 5H), 4.60 - 4.70 (m, 1H), 7.14 (t, 1H), 7.16 (s, 1H), 7.97-8.00 (m, 1H), 8.27 - 8.37 (m, 3H), 8.69 (s, 1H), 10.55 (s, 1H).

### Beispiel 24

### N-[6-Methoxy-2-(tetrahydro-2H-pyran-4-yl)-2H-indazol-5-yl]-6-(morpholin-4-yl)pyridin-2-carboxamid

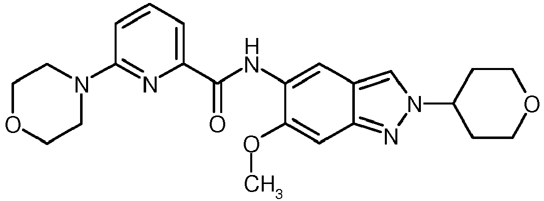

Analog zu Beispiel 23 wurden 30 mg 6-Methoxy-2-(tetrahydro-2H-pyran-4-yl)-2H-indazol-5-amin (Intermediat 4A) mit 33 mg 6-(Morpholin-4-yl)pyridin-2-carbonsäure umgesetzt. Nach Aufreinigung durch präparative HPLC erhielt man 9 mg der Titelverbindung.
UPLC-MS (Methode C): Rt = 1.08 min; gefundene Masse 437.21.
¹H-NMR (400MHz, DMSO-d₆): δ [ppm]= 2.03 - 2.16 (m, 4H), 3.48 - 3.57 (m, 2H), 3.57-3.65 (m, 4H), 3.76 - 3.85 (m, 4H), 3.97 (s, 3H), 3.98 - 4.05 (m, 2H), 4.61 - 4.70 (m, 1H), 7.13 - 7.19 (m, 2H), 7.46 (d, 1H), 7.82 (dd, 1H), 8.35 (s, 1H), 8.65 (s, 1H), 10.80 (s, 1H).

### Beispiel 25

### N-[6-Methoxy-2-(tetrahydro-2H-pyran-4-yl)-2H-indazol-5-yl]-2-methyl-1,3-thiazol-4-carboxamid

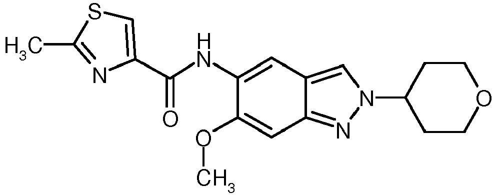

Analog zu Beispiel 23 wurden 30 mg 6-Methoxy-2-(tetrahydro-2H-pyran-4-yl)-2H-indazol-5-amin (Intermediat 4A) und 23 mg 2-Methyl-1,3-thiazol-4-carbonsäure umgesetzt. Nach Aufreinigung durch präparative HPLC erhielt man 19 mg der Titelverbindung.
UPLC-MS (Methode C): Rt = 1.03 min; gefundene Masse 372.13.
¹R-NMR (400MHz, DMSO-d6): δ [ppm]= 2.03 - 2.16 (m, 4H), 2.77 (s, 3H), 3.47 - 3.59 (m, 2H), 3.94 - 4.05 (m, 5H), 4.61 - 4.71 (m, 1H), 7.16 (s, 1H), 8.30 (s, 1H), 8.36 (s, 1H), 8.63 (s, 1H), 9.83 (s, 1H).

### Beispiel 26

### 6-Amino-N-[6-methoxy-2-(tetrahydro-2H-pyran-4-yl)-2H-indazol-5-yl]pyridin-2-carboxamid

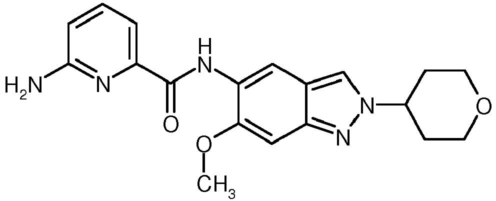

Analog zu Beispiel 23 wurden 30 mg 6-Methoxy-2-(tetrahydro-2H-pyran-4-yl)-2H-indazol-5-amin (Intermediat 4A) und 22 mg 6-Aminopyridin-2-carbonsäure umgesetzt. Nach Aufreinigung durch präparative HPLC erhielt man 23 mg der Titelverbindung.
UPLC-MS (Methode C): Rt = 0.87 min; gefundene Masse 367.16.
¹H-NMR (400MHz, DMSO-d6): δ [ppm]= 2.01 - 2.14 (m, 4H), 3.45 - 3.56 (m, 2H), 3.94 - 4.04 (m, 5H), 4.59 - 4.68 (m, 1H), 6.35 (breites Singulett, 2H), 6.66 - 6.71 (m, 1H), 7.11 (s, 1H), 7.27 - 7.31 (m, 1H), 7.59 (dd, 1H), 8.32 (s, 1H), 8.66 (s, 1H), 10.57 (s, 1H).

### Beispiel 27

### 2-Isopropyl-N-[6-methoxy-2-(tetrahydro-2H-pyran-4-yl)-2H-indazol-5-yl]pyrimidin-4-carboxamid

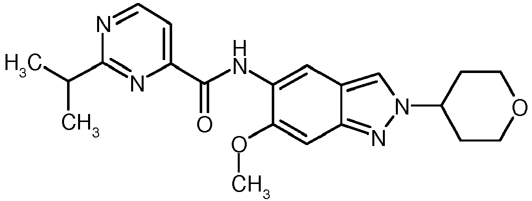

Analog zu Beispiel 23 wurden 30 mg 6-Methoxy-2-(tetrahydro-2H-pyran-4-yl)-2H-indazol-5-amin (Intermediat 4A) und 26 mg 2-Isopropylpyrimidin-4-carbonsäure umgesetzt. Zur Aufarbeitung wurde auf Wasser gegeben, der Niederschlag wurde abgesaugt, mit Wasser und Diethylether gewaschen und getrocknet. Man erhielt 33 mg der Titelverbindung.
UPLC-MS (Methode C): Rt = 1.19 min; gefundene Masse 395.00.
¹H-NMR (300MHz, DMSO-d6): δ [ppm]= 1.38 (d, 6H), 2.00 - 2.17 (m, 4H), 3.21 - 3.31 (m, Signal verdeckt durch DMSO-Signal), 3.45 - 3.59 (m, 2H), 3.94 - 4.06 (m, 5H), 4.60 - 4.71 (m, 1H), 7.18 (s, 1H), 7.96 (d, 1H), 8.38 (s, 1H), 8.68 (s, 1H), 9.08 (d, 1H), 10.81 (s, 1H).

### Beispiel 28

### 6-(2-Hydroxypropan-2-yl)-N-[6-methoxy-2-(tetrahydro-2H-pyran-4-yl)-2H-indazol-5-yl]pyridin-2-carboxamid

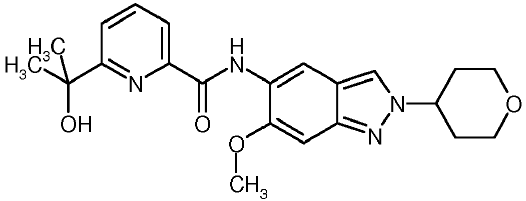

Analog zu Beispiel 23 wurden 80 mg 6-Methoxy-2-(tetrahydro-2H-pyran-4-yl)-2H-indazol-5-amin (Intermediat 4A) und 85 mg Kalium-6-(2-hydroxypropan-2-yl)pyridin-2-carboxylat (Intermediat V3-1) in THF umgesetzt. Nach Aufreinigung durch präparative HPLC erhielt man 62 mg der Titelverbindung.
UPLC-MS (Methode C): Rt = 0.99 min; gefundene Masse 410.20.
¹H-NMR (400MHz, DMSO-d₆): δ [ppm]= 1.56 (s, 6H), 2.02 - 2.15 (m, 4H), 3.47 - 3.56 (m, 2H), 3.95 - 4.03 (m, 5H), 4.58 - 4.70 (m, 1H), 5.43 (s, 1H), 7.15 (s, 1H), 7.92 (dd, 1H), 7.98 - 8.08 (m, 2H), 8.35 (s, 1H), 8.66 (s, 1H), 10.91 (s, 1H).

### Beispiel 29

### N-[6-Methoxy-2-(tetrahydrofuran-3-yl)-2H-indazol-5-yl]-6-(trifluormethyl)pyridin-2-carboxamid

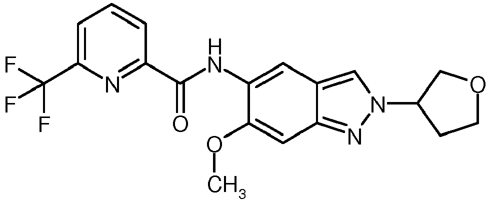

Eine Mischung aus 250 mg N-(6-Methoxy-1H-indazol-5-yl)-6-(trifluoromethyl)pyridin-2-carboxamid (Intermediat 5A), 174 mg 3-Iodtetrahydrofuran (1.5 Äquivalente) und 244 mg Kaliumcarbonat (3.0 Äquivalente) in 4 ml DMF wurde 16 h bei 100°C gerührt. Man addierte erneut 0.7 Äquivalente 3-Iodtetrahydrofuran und 1.5 Äquivalente Kaliumcarbonat und ließ 24 h bei 100°C rühren. Man versetzte mit Wasser, extrahierte dreimal mit Ethylacetat, wusch die vereinigten organischen Phasen mit Kochsalzlösung, filtrierte durch einen wasserabweisenden Filter und engte ein. Das Rohprodukt wurde per HPLC gereinigt. Man erhielt 49.3 mg der Titelverbindung.
¹H-NMR (400MHz, DMSO-d₆): δ [ppm]= 2.35 - 2.48 (m, 2H, verdeckt durch Lösungsmittelsignal), 3.89 (td, 1H), 3.97 - 4.12 (m, 6H), 5.25 - 5.32 (m, 1H), 7.18 (s, 1H), 8.21 (dd, 1H), 8.35 - 8.43 (m, 2H), 8.45 - 8.48 (m, 1H), 8.69 (s, 1H), 10.51 (s, 1H).

### Beispiel 30

### N-[6-Chlor-2-(tetrahydro-2H-pyran-4-yl)-2H-indazol-5-yl]-6-(trifluormethyl)pyridin-2-carboxamid

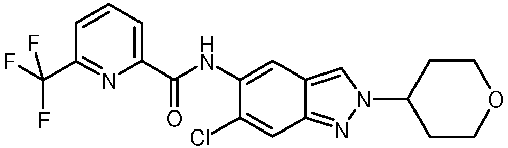

100 mg 6-Chlor-2-(tetrahydro-2H-pyran-4-yl)-2H-indazol-5-amin (Intermediat 4G) und 99 mg 6-(Trifluormethyl)pyridin-2-carbonsäure wurden analog zu Beispiel 23 in THF umgesetzt. Man erhielt nach Reinigung durch präparative HPLC 21 mg der Titelverbindung.
UPLC-MS (Methode C): Rt = 1.29 min; gefundene Masse 424.09.
¹H-NMR (300MHz, DMSO-d6): δ [ppm]= 2.03 - 2.19 (m, 4H), 3.43 - 3.59 (m, 2H), 3.96 - 4.06 (m, 2H), 4.76 (dt, 1H), 7.95 (s, 1H), 8.23 (dd, 1H), 8.37 - 8.48 (m, 2H), 8.57 (s, 1H), 8.63 (s, 1H), 10.52 (s, 1H).

### Beispiel 31

### N-[6-Chlor-2-(tetrahydro-2H-pyran-4-yl)-2H-indazol-5-yl]-6-(difluormethyl)pyridin-2-carboxamid

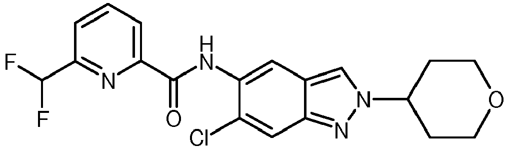

100 mg 6-Chlor-2-(tetrahydro-2H-pyran-4-yl)-2H-indazol-5-amin (Intermediat 4G) und 89 mg 6-(Difluormethyl)pyridin-2-carbonsäure wurden analog zu Beispiel 23 in THF umgesetzt. Zur Aufarbeitung wurde mit Wasser versetzt, der Niederschlag wurde abgesaugt, mit Wasser und Diethylether gewaschen und getrocknet. Man erhielt 138 mg der Titelverbindung.
UPLC-MS (Methode C): Rt = 1.22 min; gefundene Masse 406.00.
¹R-NMR (400MHz, DMSO-d6): δ [ppm]= 2.04 - 2.17 (m, 4H), 3.46 - 3.61 (m, 2H), 3.96 - 4.05 (m, 2H), 4.70 - 4.81 (m, 1H), 7.13 (t, 1H), 7.94 (s, 1H), 7.97 - 8.04 (m, 1H), 8.28 - 8.37 (m, 2H), 8.52 - 8.58 (m, 1H), 8.64 (s, 1H), 10.58 (s, 1H).

### Beispiel 32

### N-[6-Chlor-2-(1,1-dioxidotetrahydro-2H-thiopyran-4-yl)-2H-indazol-5-yl]-6-(2-hydroxypropan-2-yl)pyridin-2-carboxamid

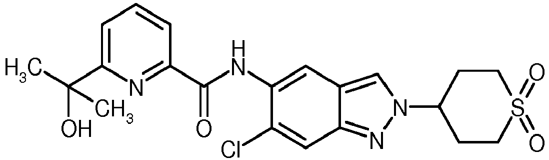

Eine Mischung aus 100 mg 6-Chlor-2-(1,1-dioxidotetrahydro-2H-thiopyran-4-yl)-2H-indazol-5-amin (Intermediat 4H), 110 mg Kalium-6-(2-hydroxypropan-2-yl)pyridin-2-carboxylat (Intermediat V3-1), 140 mg HATU und 63 Microliter N-Ethyl-N-isopropylpropan-2-amin in 2 ml DMF wurde 20 h bei Raumtemperatur gerührt. Man versetzte mit Wasser, filtrierte den ausgefallenen Feststoff ab, wusch dreimal mit Wasser und dreimal mit Diethylether und trocknete. Man erhielt 144 mg der Titelverbindung.
UPLC-MS (Methode C): Rt = 0.99 min; gefundene Masse 462.00.
¹H-NMR (400MHz, DMSO-d₆): δ [ppm]= 1.55 (s, 6H), 2.40-2.65 (m, zum Teil verdeckt durch Lösungsmittelsignal), 2.51 - 2.67 (m, 3H), 3.22 - 3.28 (m, zum Teil verdeckt durch Lösungsmittelsignal), 3.41 - 3.52 (m, 2H), 4.95 (tt, 1H), 5.46 (s, 1H), 7.92 - 8.10 (m, 4H), 8.57 (d, 1H), 8.74 (s, 1H), 10.88 (s, 1H).

### Beispiel 33

### Methyl-2-(tetrahydro-2H-pyran-4-yl)-5-({[6-(trifluormethyl)pyridin-2-yl]carbonyl}amino)-2H-indazol-6-carboxylat

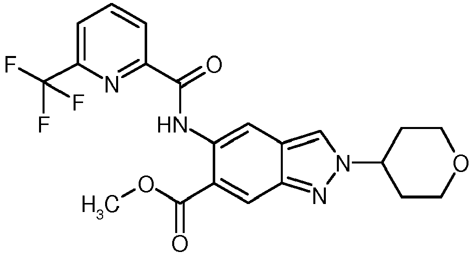

Eine Lösung aus 450 mg Methyl-5-amino-2-(tetrahydro-2H-pyran-4-yl)-2H-indazol-6-carboxylat (Intermediat 4J) in 10 ml DMF wurde mit 406 mg 6-(Trifluormethyl)pyridin-2-carbonsäure, 684 mg HATU und 307 Microliter N-Ethyl-N-isopropylpropan-2-amin versetzt und man rührte 24 h bei Raumtemperatur. Man versetzte mit Wasser, saugte den Niederschlag ab, wusch dreimal mit Wasser und dreimal mit Diethylether. Nach Trocknen wurden 677 mg der Titelverbindung erhalten.
UPLC-MS (Methode C): Rt = 1.27 min; gefundene Masse 448.00.
¹H-NMR (400MHz, DMSO-d₆): δ [ppm]= 2.07 - 2.24 (m, 4H), 3.49 - 3.63 (m, 2H), 3.97 (s, 3H), 3.99 - 4.11 (m, 2H), 4.78 - 4.92 (m, 1H), 8.18 - 8.28 (m, 1H), 8.37 - 8.55 (m, 3H), 8.64 (s, 1H), 9.08 (s, 1H), 12.55 (s, 1H).

### Beispiel 34

### N-[6-(2-Hydroxypropan-2-yl)-2-(tetrahydro-2H-pyran-4-yl)-2H-indazol-5-yl]-6-(trifluormethyl)pyridin-2-carboxamid

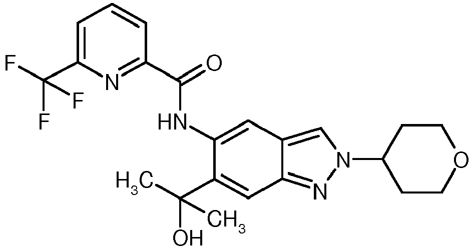

300 mg Methyl-2-(tetrahydro-2H-pyran-4-yl)-5-({[6-(trifluormethyl)pyridin-2-yl]carbonyl}amino)-2H-indazol-6-carboxylat (Beispiel 33) wurden in 5 ml THF vorgelegt. Man kühlte mit einem Eiswasser-Kältebad ab und addierte vorsichtig 1.1 ml 3M Methylmagnesiumbromidlösung (in Diethylether). Man ließ 1 h mit Kühlung durch das Kältebad rühren und rührte danach 4.5 h bei Raumtemperatur. Man versetzte mit gesättigter wässriger Ammoniumchloridlösung, extrahierte dreimal mit Ethylacetat, wusch die vereinigten organischen Phasen mit Kochsalzlösung, filtrierte durch einen wasserabweisenden Filter und engte ein. Der Rückstand wurde mit Diethylether versetzt und man ließ 10 min rühren. Man saugte ab, wusch den Feststoff dreimal mit Diethylether und trocknete. Es wurden 241 mg der Titelverbindung erhalten.
UPLC-MS (Methode C): Rt = 1.11 min; gefundene Masse 448.0.
¹H-NMR (400MHz, DMSO-d₆): δ [ppm]= 1.61 (s, 6H), 2.02 - 2.18 (m, 4H), 3.52 (td, 2H), 3.95 - 4.05 (m, 2H), 4.71 (tt, 1H), 5.93 (s, 1H), 7.58 (s, 1H), 8.15 (d, 1H), 8.33 - 8.47 (m, 3H), 8.72 (s, 1H), 12.36 (s, 1H).

### Beispiel 35

### Methyl-2-[(3S)-tetrahydrofuran-3-yl]-5-({[6-(trifluormethyl)pyridin-2-yl]carbonyl}amino)-2H-indazol-6-carboxylat

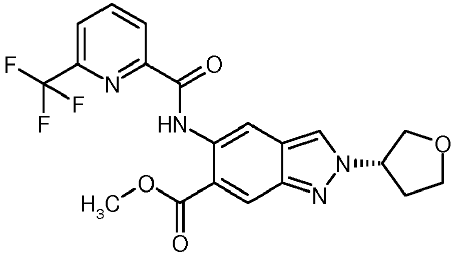

Eine Lösung aus 759 mg Methyl-5-amino-2-[(3S)-tetrahydrofuran-3-yl]-2H-indazol-6-carboxylat (Intermediat 4L) in 10 ml THF wurde mit 666 mg 6-(Trifluormethyl)pyridin-2-carbonsäure, 1.12 g O-(Benzotriazol-1-yl)-N,N,N',N'-tetramethyluroniumtetrafluoroborat (TBTU, CAS 125700-67-6), und 0.61 ml N-Ethyl-N-isopropylpropan-2-amin versetzt und man rührte 18 h bei Raumtemperatur. Man versetzte mit Wasser, extrahierte dreimal mit Ethylacetat, wusch mit Kochsalzlösung, filtrierte durch einen wasserabweisenden Filter und engte ein. Nach Reinigung des Rohproduktes durch Säulenchromatographie an Kieselgel (Isolera, Hexan/Ethylacetat) wurden 824 mg der Titelverbindung erhalten.
UPLC-MS (Methode C): Rt = 1.24 min; gefundene Masse 434.00.
¹H-NMR (400MHz, DMSO-d₆, ausgewählte Signale vom Rohprodukt): δ [ppm]= 2.38 - 2.59 (m, überlagert durch Lösungsmittelsignal), 3.85 - 3.98 (m, 4H), 4.03 - 4.14 (m, 3H), 5.38 - 5.46 (m, 1H), 8.17 - 8.22 (m, 1H), 8.35 - 8.41 (m, 1H), 8.44 - 8.48 (m, 2H), 8.58 (s, 1H), 9.05 (s, 1H), 12.51 (s, 1H).

### Beispiel 36

### N-{6-(2-Hydroxypropan-2-yl)-2-[(3S)-tetrahydrofuran-3-yl]-2H-indazol-5-yl}-6-(trifluormethyl)pyridin-2-carboxamid

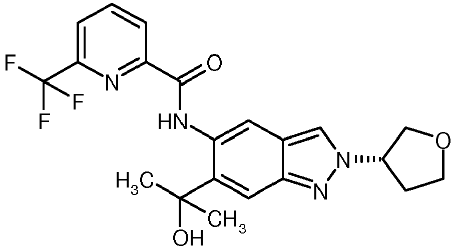

819 mg Methyl-2-[(3S)-tetrahydrofuran-3-yl]-5-({[6-(trifluormethyl)pyridin-2-yl]carbonyl}-amino)-2H-indazol-6-carboxylat (Beispiel 35) wurden analog zu Beispiel 34 mit 2.42 ml 3M Methylmagnesiumbromidlösung (in Diethylether) in 13 ml THF umgesetzt. Man erhielt 774 mg eines Rohproduktes, welches durch Säulenchromatographie an Kieselgel (Biotage Isolera, Hexan/Ethylacetat) gereinigt wurde. Nach einer weiteren Reinigung durch präparative HPLC wurden 431 mg der Titelverbindung (Analyse durch chirale HPLC: ee-Wert 98.5%) erhalten.

### Chirale Analytik:

| | | | |
|---|---|---|---|
| *System:* | Agilent 1260/ Agilent 1290 | | |
| *Säule:* | Chiralpak IB 3µm 100x4.6 mm | | |
| *Solvent:* | Hexan / 2-Propanol 5-50% B (v/v), 10 min +0,1 % DEA | | |
| *Fluss:* | 1.0 mL/min | | |
| *Temperatur:* | 25°C | | |
| *Lösung:* | 1.0 mg/mL EtOH/MeOH 1:1 | | |
| *Injektion:* | 5.0 µl | | |
| *Detektion:* | DAD 254 nm | | |

| **Peak** | Rt in min | Reinheit in % | |
|---|---|---|---|
| **1** | 9.52 | 99.2 | ee-Wert: 98.5 % (Titelverbindung) |
| **2** | 10.18 | 0.8 | Entspricht Beispiel 38 (Rt: 10.16) |

¹H-NMR (400MHz, DMSO-d₆): δ [ppm]= 1.63 (s, 6H), 2.37 - 2.49 (m, verdeckt durch Lösungsmittelsignal), 2.53 - 2.58 (m, 1H), 3.90 (td, 1H), 3.99 - 4.15 (m, 3H), 5.30 - 5.38 (m, 1H), 5.97 (s, 1H), 7.60 (s, 1H), 8.17 (dd, 1H), 8.34 - 8.48 (m, 3H), 8.73 (s, 1H), 12.38 (s, 1H).

### Beispiel 37

### Methyl-2-[(3R)-tetrahydrofuran-3-yl]-5-({[6-(trifluormethyl)pyridin-2-yl]carbonyl}amino)-2H-indazol-6-carboxylat

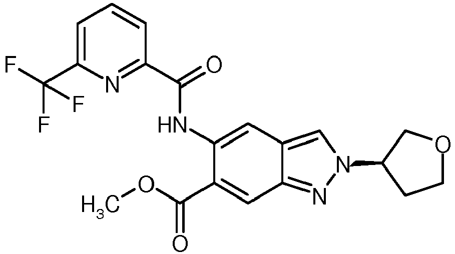

Analog zur Herstellung von Beispiel 35 wurden 552 mg Methyl-5-amino-2-[(3R)-tetrahydrofuran-3-yl]-2H-indazol-6-carboxylat (Intermediat 4M) in 10 ml THF mit 460 mg 6-(Trifluormethyl)pyridin-2-carbonsäure, 773 mg O-(Benzotriazol-1-yl)-N,N,N',N'-tetramethyluroniumtetrafluoroborat (TBTU, CAS 125700-67-6) und 0.42 ml N-Ethyl-N-isopropylpropan-2-amin umgesetzt und aufgereinigt. Es wurden 794 mg eines Feststoffes als Titelverbindung erhalten.
UPLC-MS (Methode C): Rt = 1.23 min; gefundene Masse 434.00.

### Beispiel 38

### N-{6-(2-Hydroxypropan-2-yl)-2-[(3R)-tetrahydrofuran-3-yl]-2H-indazol-5-yl}-6-(trifluormethyl)pyridin-2-carboxamid

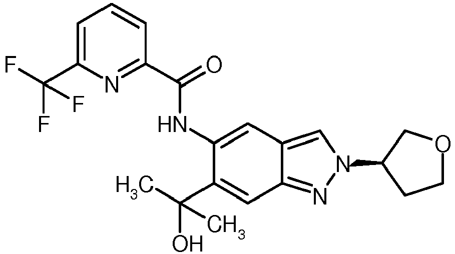

794 mg Methyl-2-[(3R)-tetrahydrofuran-3-yl]-5-({[6-(trifluormethyl)pyridin-2-yl]carbonyl}-amino)-2H-indazol-6-carboxylat (Beispiel 37) wurden analog zur Herstellung von Beispiel 36 mit 2.35 ml 3M Methylmagnesiumbromidlösung (in Diethylether) in 11 ml THF umgesetzt. Man erhielt 777 mg eines Rohproduktes, welches durch Säulenchromatographie an Kieselgel (Biotage Isolera, Hexan/Ethylacetat) gereinigt wurde. Nach einer weiteren Reinigung durch präparative HPLC wurden 394 mg der Titelverbindung (Analyse durch chirale HPLC: ee-Wert 99.1%) erhalten.

### Chirale Analytik:

| | | | |
|---|---|---|---|
| *System:* | Agilent 1260/ Agilent 1290 | | |
| *Säule:* | Chiralpak IB 3µm 100x4.6 mm | | |
| *Solvent:* | Hexan / 2-Propanol 5-50% B (v/v), 10 min +0,1 % DEA | | |
| *Fluss:* | 1.0 mL/min | | |
| *Temperatur:* | 25°C | | |
| *Lösung:* | 1.0 mg/mL EtOH/MeOH 1:1 | | |
| *Injektion:* | 5.0 µl | | |
| *Detektion:* | DAD 254 nm | | |

| **Peak** | Rt in min | Reinheit in % | |
|---|---|---|---|
| **1** | 9.57 | 0.4 | Entspricht Beispiel 36 (Rt: 9.52) |
| **2** | 10.16 | 99.6 | ee-Wert: 99.1 % (Titelverbindung) |

¹H-NMR (400MHz, DMSO-d₆): δ [ppm]= 1.62 (s, 6H), 2.37 - 2.49 (m, verdeckt durch Lösungsmittelsignal), 3.90 (td, 1H), 3.99 - 4.15 (m, 3H), 5.30 - 5.39 (m, 1H), 5.97 (s, 1H), 7.60 (s, 1H), 8.17 (dd, 1H), 8.34 - 8.49 (m, 3H), 8.73 (s, 1H), 12.38 (s, 1H).

### Beispiel 39

### Methyl-2-[(3S)-tetrahydrothiophen-3-yl]-5-({[6-(trifluormethyl)pyridin-2-yl]carbonyl}amino)-2H-indazol-6-carboxylat

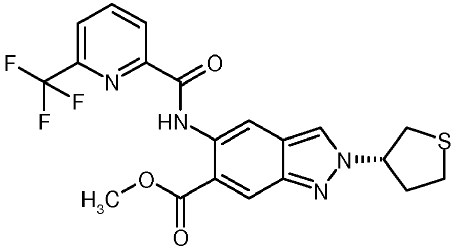

Analog zur Herstellung von Beispiel 35 wurden 91 mg Methyl-5-amino-2-[(3S)-tetrahydrothiophen-3-yl]-2H-indazol-6-carboxylat (Intermediat 4K) in 3 ml THF mit 72 mg 6-(Trifluormethyl)pyridin-2-carbonsäure, 120 mg O-(Benzotriazol-1-yl)-N,N,N',N'-tetramethyluroniumtetrafluoroborat (TBTU, CAS 125700-67-6) und 65 Microliter N-Ethyl-N-isopropylpropan-2-amin innerhalb von 23 h bei Raumtemperatur umgesetzt. Das Rohprodukt nach der wässrigen Aufarbeitung wurde mit Dimethylsulfoxid versetzt, der verbleibende Feststoff abfiltriert und dreimal mit Diethylether gewaschen und getrocknet. Man erhielt 50 mg der Titelverbindung.
UPLC-MS (Methode C): Rt = 1.38 min; gefundene Masse 450.00.
¹H-NMR (400MHz, DMSO-d₆): δ [ppm]= 2.60 - 2.73 (m, 1H), 2.96 - 3.07 (m, 2H), 3.35 - 3.46 (m, 2H), 3.97 (s, 3H), 5.41 (quin, 1H), 8.19 - 8.24 (m, 1H), 8.37 - 8.44 (m, 1H), 8.45 - 8.51 (m, 2H), 8.68 (s, 1H), 9.08 (s, 1H), 12.54 (s, 1H).

### Beispiel 40

### N-{6-(2-Hydroxypropan-2-yl)-2-[(3S)-tetrahydrothiophen-3-yl]-2H-indazol-5-yl}-6-(trifluormethyl)pyridin-2-carboxamid

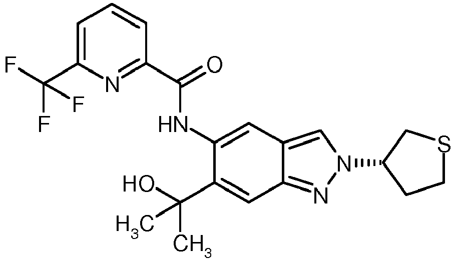

Eine Lösung aus 50 mg Methyl-2-[(3S)-tetrahydrothiophen-3-yl]-5-({[6-(trifluormethyl)pyridin-2-yl]carbonyl}amino)-2H-indazol-6-carboxylat (Beispiel 39) in 2 ml THF wurde mit einem Eiswasser-Kältebad abgekühlt und mit einer Methylmagnesiumbromid-Lösung (3M in Diethylether) versetzt. Man rührte 30 min mit Kühlung durch das Eisbad, dann wurde 69 h bei Raumtemperatur gerührt. Man versetzte mit gesättigter wässriger Ammoniumchloridlösung, extrahierte dreimal mit Ethylacetat, filtrierte durch einen wasserabweisenden Filter und engte ein. Man erhielt 51 mg der Titelverbindung als Rohprodukt.
UPLC-MS (Methode C): Rt = 1.23 min; gefundene Masse 450.00.

### Beispiel 41

### N-{2-[(3S)-1,1-Dioxidotetrahydrothiophen-3-yl]-6-(2-hydroxypropan-2-yl)-2H-indazol-5-yl}-6-(trifluormethyl)pyridin-2-carboxamid

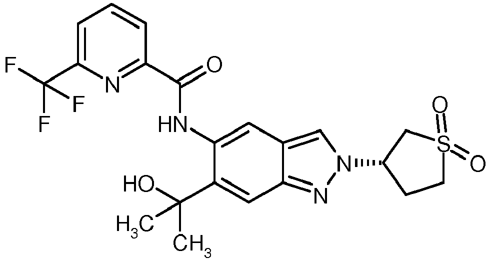

Eine Mischung aus 51 mg N-{6-(2-Hydroxypropan-2-yl)-2-[(3S)-tetrahydrothiophen-3-yl]-2H-indazol-5-yl}-6-(trifluormethyl)pyridin-2-carboxamid (Beispiel 40) in 3 ml Dichlormethan wurde mit einem Eiswasser-Kältebad abgekühlt. 56 mg 3-Chlor-perbenzoesäure (CAS937-14-4, ca. 77 prozentig) wurden portionsweise zugegeben und die Mischung wurde bei Raumtemperatur 19 h gerührt. Erneut wurden 50 mg der 3-Chlor-perbenzoesäure zugegeben und die Mischung wurde 26 h bei Raumtemperatur gerührt. Man engte ein und reinigte den Rückstand durch präparative HPLC. Es wurden 7 mg der Titelverbindung erhalten, welche dreimal mit Pentan und wenig Dichlormethan überschichtet wurden, wobei jeweils die Lösungsmittel abdekantiert wurden. Nach Trocknen erhielt man 7 mg der Titelverbindung.
UPLC-MS (Methode C): Rt = 1.08 min; gefundene Masse 482.00.
¹H-NMR (400MHz, CHLOROFORM-d): δ [ppm]= 1.33-2.34 (breites Signal, enhält Singulett bei 1.84 ppm), 2.88 (q, 2H), 3.23 - 3.34 (m, 1H), 3.62 - 3.76 (m, 2H), 3.81 - 3.89 (m, 1H), 5.39 (quin, 1H), 7.75 (s, 1H), 7.88 (d, 1H), 8.02 (s, 1H), 8.11 - 8.17 (m, 1H), 8.53 (d, 1H), 8.90 (s, 1H), 12.35 (s, 1H).

### Beispiel 42

### Methyl-2-(tetrahydro-2H-thiopyran-4-yl)-5-({[6-(trifluormethyl)pyridin-2-yl]carbonyl}amino)-2H-indazol-6-carboxylat

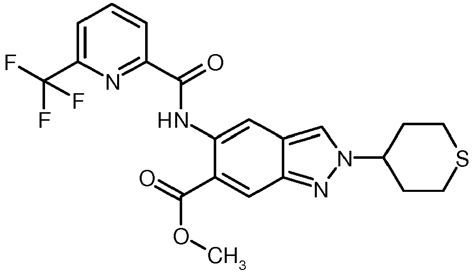

Analog zur Herstellung von Beispiel 35 wurde eine Lösung aus 992 mg Methyl-5-amino-2-(tetrahydro-2H-thiopyran-4-yl)-2H-indazol-6-carboxylat (Intermediat 4N) in 10 ml THF mit 694 mg 6-(Trifluormethyl)pyridin-2-carbonsäure, 1.08 g O-(Benzotriazol-1-yl)-N,N,N',N'-tetramethyluroniumtetrafluoroborat (TBTU, CAS 125700-67-6) und 0.59 ml N-Ethyl-N-isopropylpropan-2-amin umgesetzt. Nach Ausrühren des Rohproduktes aus Diethylether wurden 1.29 g der Titelverbindung erhalten.
UPLC-MS (Methode C): Rt = 1.39 min; gefundene Masse 464.00.

### Beispiel 43

### N-[6-(2-Hydroxypropan-2-yl)-2-(tetrahydro-2H-thiopyran-4-yl)-2H-indazol-5-yl]-6-(trifluormethyl)pyridin-2-carboxamid

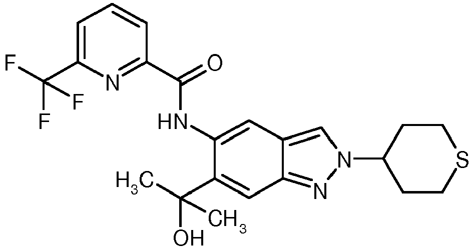

Analog zur Herstellung von Beispiel 34 wurden 1.29 g Methyl-2-(tetrahydro-2H-thiopyran-4-yl)-5-({[6-(trifluormethyl)pyridin-2-yl]carbonyl}amino)-2H-indazol-6-carboxylat (Beispiel 42) in 10 ml THF mit 3.1 ml 3M Methylmagnesiumbromidlösung (in Diethylether) umgesetzt. Das nach analoger wässriger Aufarbeitung erhaltene Rohprodukt wurde mit Diethylether ausgerührt. Es wurden 893 mg der Titelverbindung erhalten.
UPLC-MS (Methode C): Rt = 1.25 min; gefundene Masse 464.00.

### Beispiel 44

### N-[2-(1,1-Dioxidotetrahydro-2H-thiopyran-4-yl)-6-(2-hydroxypropan-2-yl)-2H-indazol-5-yl]-6-(trifluormethyl)pyridin-2-carboxamid

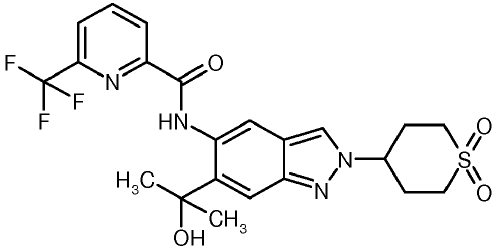

Eine Lösung aus 893 mg N-[6-(2-Hydroxypropan-2-yl)-2-(tetrahydro-2H-thiopyran-4-yl)-2H-indazol-5-yl]-6-(trifluormethyl)pyridin-2-carboxamid (Beispiel 43) in 15 ml Dichlormethan wurde mit einem Eiswasser-Kältebad abgekühlt und portionsweise mit 1.16 g 3-Chlor-perbenzoesäure (CAS937-14-4, ca. 77 prozentig) versetzt. Danach wurde 1 h gerührt im Eiswasser-Kältebad und 19 h bei Raumtemperatur gerührt. Man addierte Wasser, trennte die organische Phase ab und extrahierte dreimal mit Dichlormethan. Die vereinigten organischen Phasen wurden mit Kochsalzlösung gewaschen, durch einen wasserabweisenden Filter filtriert und eingeengt. Nach Reinigung des Rohproduktes durch präparative HPLC wurden 342 mg der Titelverbindung erhalten.
¹H-NMR (400MHz, DMSO-d₆): δ [ppm]= 1.63 (s, 6H), 2.38 - 2.66 (m, zum Teil verdeckt durch Lösungsmittelsignal), 3.25 - 3.54 (m, zum Teil verdeckt durch Lösungsmittelsignal), 4.93 (tt, 1H), 5.99 (s, 1H), 7.62 (s, 1H), 8.17 (dd, 1H), 8.37 (t, 1H), 8.42 - 8.50 (m, 2H), 8.74 (s, 1H), 12.40 (s, 1H).

### Beispiel 45

### Methyl-2-(piperidin-4-yl)-5-({[6-(trifluormethyl)pyridin-2-yl]carbonyl}amino)-2H-indazol-6-carboxylat

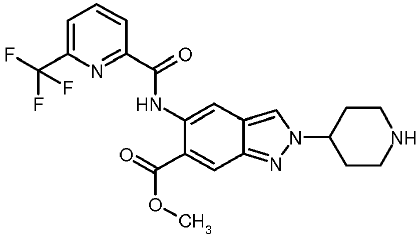

### Stufe A

### Methyl-2-[1-(tert-butoxycarbonyl)piperidin-4-yl]-5-({[6-(trifluormethyl)pyridin-2-yl]carbonyl}amino)-2H-indazol-6-carboxylat

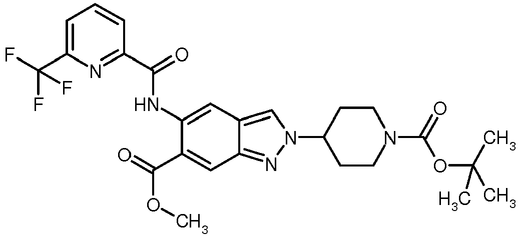

Analog zur Herstellung von Beispiel 35 wurde eine Lösung aus 5.52 g Methyl-5-amino-2-[1-(tert-butoxycarbonyl)piperidin-4-yl]-2H-indazol-6-carboxylat (Intermediat 40) in 30 ml THF mit 2.66 g 6-(Trifluormethyl)pyridin-2-carbonsäure, 3.58 g O-(Benzotriazol-1-yl)-N,N,N',N'-tetramethyluroniumtetrafluoroborat (TBTU, CAS 125700-67-6) und 1.9 ml N-Ethyl-N-isopropylpropan-2-amin umgesetzt. Nach analoger Aufarbeitung wurde das Rohprodukt durch säulenchromatographische Reinigung an Kieselgel (Isolera, Hexan/Ethylacetat) gereinigt. Man erhielt 3.85 g eines gelben Schaumes, der mit Diethylether versetzt wurde. Der verbleibende Feststoff wurde abgesaugt, dreimal mit Diethylether gewaschen und getrocknet. Es wurden 1.70 g der Titelverbindung erhalten.
UPLC-MS (Methode C): Rt = 1.46 min; gefundene Masse 547.00.

### Stufe B

1.70 g Methyl-2-[1-(tert-butoxycarbonyl)piperidin-4-yl]-5-({[6-(trifluormethyl)pyridin-2-yl]carbonyl}amino)-2H-indazol-6-carboxylat wurden in 20 ml Dichlormethan vorgelegt. 2.4 ml Trifluoressigsäure wurden zugegeben und die Mischung wurde 20 h bei Raumtemperatur gerührt. Danach wurde eingeengt, der Rückstand mit Ethylacetat verdünnt und vorsichtig gesättigte Natriumhydrogencarbonatlösung zugegeben. Dabei fiel ein Feststoff aus. Man entfernte Dichlormethan und Ethylacetat am Rotationsverdampfer, filtrierte den Feststoff ab, wusch den Feststoff zweimal mit Wasser und dreimal mit Diethylether und trocknete. Man erhielt 1.39 g Methyl-2-(piperidin-4-yl)-5-({[6-(trifluormethyl)pyridin-2-yl]carbonyl}amino)-2H-indazol-6-carboxylat als Feststoff.
UPLC-MS (Methode C): Rt = 0.93 min; gefundene Masse 447.00.

### Beispiel 46

### N-[6-(2-Hydroxypropan-2-yl)-2-(piperidin-4-yl)-2H-indazol-5-yl]-6-(trifluormethyl)pyridin-2-carboxamid

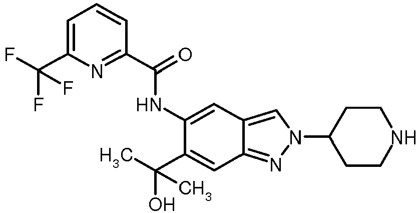

595 mg Methyl-2-(piperidin-4-yl)-5-({[6-(trifluormethyl)pyridin-2-yl]carbonyl}amino)-2H-indazol-6-carboxylat (Beispiel 45) wurden in 10 ml THF vorgelegt. Man kühlte mit einem Eiswasser-Kältebad ab und addierte vorsichtig 2.2 ml 3M Methylmagnesiumbromidlösung (in Diethylether). Man ließ 2 h mit Kühlung durch das Kältebad rühren und rührte danach 24 h bei Raumtemperatur. Erneut gab man 2.5 Äquivalente der Methylmagnesiumbromidlösung zu und ließ 93 h bei Raumtemperatur rühren. Man versetzte mit gesättigter wässriger Ammoniumchloridlösung, extrahierte dreimal mit Ethylacetat, wusch die vereinigten organischen Phasen mit Kochsalzlösung, filtrierte durch einen wasserabweisenden Filter und engte ein. Nach Reinigung des Rückstandes durch präparative HPLC wurden 81 mg der Titelverbindung erhalten.
UPLC-MS (Methode C): Rt = 1.02 min; gefundene Masse 447.19.
¹H-NMR (400MHz, DMSO-d₆): δ [ppm]= 1.62 (s, 6H), 1.88 - 2.11 (m, 4H), 2.39 (br. s., 1H), 2.65 (t, 2H), 3.09 (d, 2H), 4.45 - 4.56 (m, 1H), 5.97 (s, 1H), 7.59 (s, 1H), 8.17 (d, 1H), 8.34 - 8.49 (m, 3H), 8.72 (s, 1H), 12.38 (s, 1H).

### Beispiel 47

### N-[6-Methoxy-2-(tetrahydro-2H-pyran-4-yl)-2H-indazol-5-yl]-4-(trifluormethyl)-1,3-thiazol-2-carboxamid

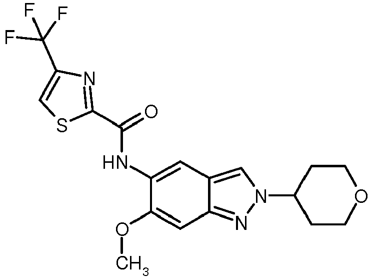

Eine Lösung aus 80 mg 6-Methoxy-2-(tetrahydro-2H-pyran-4-yl)-2H-indazol-5-amin (Intermediat 4A) in 2 ml THF wurde mit 77 mg 4-(Trifluormethyl)-1,3-thiazol-2-carbonsäure, 148 mg HATU und 68 Microliter N-Ethyl-N-isopropylpropan-2-amin versetzt und man rührte 21.5 h bei Raumtemperatur. Man versetzte mit Wasser, saugte den ausgefallenen Feststoff ab, wusch den Feststoff dreimal mit Wasser, dreimal mit Diethylether und trocknete. Man erhielt 121 mg der Titelverbindung.
UPLC-MS (Methode C): Rt = 1.22 min; gefundene Masse 426.00.
¹H-NMR (400MHz, DMSO-d₆): δ [ppm]= 2.03 - 2.16 (m, 4H), 3.48 - 3.57 (m, 2H), 3.94 - 4.04 (m, 5H), 4.63 - 4.72 (m, 1H), 7.19 (s, 1H), 8.41 (s, 1H), 8.43 (s, 1H), 8.90 (d, 1H), 9.78 (s, 1H).

### Beispiel 48

### N-[6-Methoxy-2-(tetrahydro-2H-pyran-4-yl)-2H-indazol-5-yl]-3-(pyridin-4-yl)-1,2,4-oxadiazol-5-carboxamid

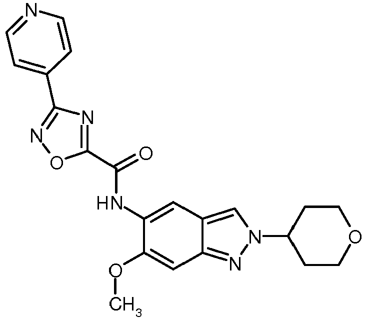

Eine Lösung aus 80 mg 6-Methoxy-2-(tetrahydro-2H-pyran-4-yl)-2H-indazol-5-amin (Intermediat 4A) in 2 ml THF wurde mit 77 mg 3-(Pyridin-4-yl)-1,2,4-oxadiazol-5-carbonsäure, 148 mg HATU und 68 Microliter N-Ethyl-N-isopropylpropan-2-amin versetzt und man rührte 21.5 h bei Raumtemperatur. Man versetzte mit Wasser, saugte den ausgefallenen Feststoff ab, wusch den Feststoff dreimal mit Wasser, dreimal mit Diethylether und trocknete. Man erhielt 99 mg der Titelverbindung.
UPLC-MS (Methode C): Rt = 0.98 min; gefundene Masse 420.00.
¹H-NMR (400MHz, DMSO-d₆): δ [ppm]= 2.04 - 2.16 (m, 4H), 2.53 (br. s., 1H), 3.48 - 3.58 (m, 2H), 3.95 - 4.05 (m, 5H), 4.64 - 4.75 (m, 1H), 7.21 (s, 1H), 8.03 - 8.07 (m, 2H), 8.39 (s, 1H), 8.44 (s, 1H), 8.86 - 8.91 (m, 2H), 10.11 (s, 1H).

### Beispiel 49

### N-[6-Methoxy-2-(tetrahydro-2H-pyran-4-yl)-2H-indazol-5-yl]-3-methyl-1,2,4-oxadiazol-5-carboxamid

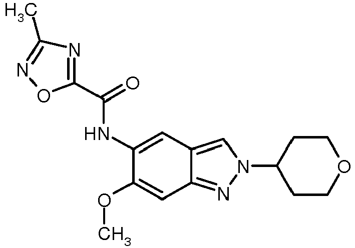

Eine Lösung aus 80 mg 6-Methoxy-2-(tetrahydro-2H-pyran-4-yl)-2H-indazol-5-amin (Intermediat 4A) in 2 ml THF wurde mit 50 mg (1.2 Äquivalente) 3-Methyl-1,2,4-oxadiazol-5-carbonsäure, 148 mg (1.2 Äquivalente) HATU und 68 Microliter N-Ethyl-N-isopropylpropan-2-amin versetzt und man rührte 21.5 h bei Raumtemperatur. Danach wurden erneut 0.6 Äquiv. HATU und 0.6 Äquiv. 3-Methyl-1,2,4-oxadiazol-5-carbonsäure zugegeben und die Mischung wurde 24 h bei Raumtemperatur gerührt. Man versetzte mit Wasser, saugte den ausgefallenen Feststoff ab, wusch den Feststoff dreimal mit Wasser, dreimal mit Diethylether und trocknete. Nach Reinigung des Feststoffes durch präparative HPLC wurden 25 mg der Titelverbindung als Feststoff erhalten.
UPLC-MS (Methode C): Rt = 0.96 min; gefundene Masse 357.14.
¹H-NMR (400MHz, DMSO-d₆): δ [ppm]= 2.03 - 2.16 (m, 4H), 2.49 (s, überlagert durch Lösungsmittelsignal), 3.47 - 3.58 (m, 2H), 3.91 - 4.05 (m, 5H), 4.63 - 4.73 (m, 1H), 7.18 (s, 1H), 8.41 (s, 1H), 8.37 (s, 1H), 9.89 (br. s., 1H).

### Beispiel 50

### N-[6-Chlor-2-(tetrahydro-2H-pyran-4-yl)-2H-indazol-5-yl]-4-(trifluormethyl)-1,3-thiazol-2-carboxamid

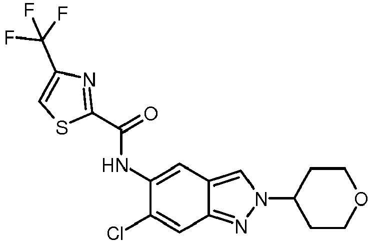

Eine Lösung aus 80 mg 6-Chlor-2-(tetrahydro-2H-pyran-4-yl)-2H-indazol-5-amin (Intermediat 4G) in 2 ml THF wurde mit 75 mg 4-(Trifluormethyl)-1,3-thiazol-2-carbonsäure, 145 mg HATU und 66 Microliter N-Ethyl-N-isopropylpropan-2-amin versetzt und man rührte 20 h bei Raumtemperatur. Man versetzte mit Wasser, extrahierte dreimal mit Ethylacetat, engte die vereinigten organischen Phasen ein und reinigte durch präparative HPLC. Man erhielt 84 mg der Titelverbindung als Feststoff.
UPLC-MS (Methode C): Rt = 1.27 min; gefundene Masse 430.05
¹H-NMR (400MHz, DMSO-d₆): δ [ppm]= 2.05 - 2.18 (m, 5H), 3.46 - 3.59 (m, 2H), 3.98 - 4.06 (m, 2H), 4.74 - 4.83 (m, 1H), 7.92 (s, 1H), 8.06 (s, 1H), 8.58 (d, 1H), 8.90 (d, 1H), 10.54 (s, 1H).

### Beispiel 51

### 1-(Difluormethyl)-N-[6-methoxy-2-(tetrahydro-2H-pyran-4-yl)-2H-indazol-5-yl]-1H-pyrazol-3-carboxamid

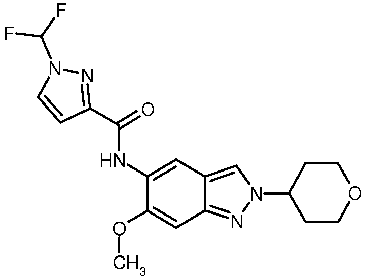

Eine Lösung aus 100 mg 6-Methoxy-2-(tetrahydro-2H-pyran-4-yl)-2H-indazol-5-amin (Intermediat 4A) in 2.5 ml THF wurde mit 50 mg 1-(Difluormethyl)-1H-pyrazol-3-carbonsäure, 185 mg HATU und 85 Microliter N-Ethyl-N-isopropylpropan-2-amin versetzt und man rührte 21.5 h bei Raumtemperatur. Man versetzte mit Wasser, extrahierte dreimal mit Ethylacetat und engte ein. Der Rückstand wurde durch präparative HPLC (Methode beinhaltete den Zusatz von Ammoniak) gereinigt. Es wurden 109 mg der Titelverbindung als Feststoff erhalten.
UPLC-MS (Methode D): Rt = 0.99 min; gefundene Masse 391.15.
¹H-NMR (500MHz, DMSO-d₆): δ [ppm]= 2.02 - 2.13 (m, 4H), 3.51 (td, 2H), 3.92 - 4.02 (m, 5H), 4.64 (tt, 1H), 7.02 (d, 1H), 7.15 (s, 1H), 7.96 (t, 1H), 8.34 - 8.36 (m, 1H), 8.44 (d, 1H), 8.50 (s, 1H), 9.39 (s, 1H).

### Beispiel 52

### N-{2-[1-(3-Hydroxy-3-methylbutyl)piperidin-4-yl]-6-methoxy-2H-indazol-5-yl}-6-(trifluormethyl)pyridin-2-carboxamid

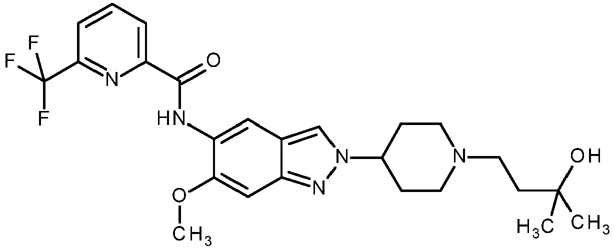

Analog zur Herstellung von Beispiel 9 wurden 200 mg N-[6-Methoxy-2-(piperidin-4-yl)-2H-indazol-5-yl]-6-(trifluormethyl)pyridin-2-carboxamid (Beispiel 2) mit 88 mg 4-Brom-2-methylbutan-2-ol und Kaliumcarbonat und Kaliumiodid umgesetzt. Nach Aufreinigung durch präparative HPLC wurden 38 mg der Titelverbindung erhalten.
UPLC-MS (Methode D): Rt = 0.92 min; gefundene Masse 505.23
¹H-NMR (400MHz, DMSO-d₆): δ [ppm]= 1.12 (s, 6H), 1.53 - 1.60 (m, 2H), 2.00 - 2.17 (m, 6H), 2.43 -2.49 (m), 3.03 (d, 2H), 3.99 (s, 3H), 4.40 (dt, 1H), 7.18 (s, 1H), 8.19 - 8.25 (m, 1H), 8.36 - 8.49 (m, 3H), 8.69 (s, 1H), 10.51 (s, 1H).

### Beispiel 53

### 2-(Piperidin-4-yl)-5-({[6-(trifluormethyl)pyridin-2-yl]carbonyl}amino)-2H-indazol-6-carboxamid

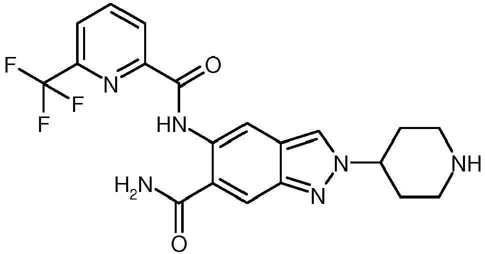

100 mg Methyl-2-(piperidin-4-yl)-5-({[6-(trifluormethyl)pyridin-2-yl]carbonyl}amino)-2H-indazol-6-carboxylat (Beispiel 45) wurden mit einer Ammoniaklösung (7M in Methanol) in einem geschlossenen Gefäß 20.5 h bei Raumtemperatur und 22 h bei 50°C gerührt. Nach Verdünnen mit Wasser fiel ein Feststoff aus. Man engte die Reaktionsmischung etwas am Rotationsverdampfer ein, filtrierte den Feststoff ab und wusch den Feststoff dreimal mit Wasser. Da die Waschphasen Anteile des Produktes enthielten, wurde der Feststoff und die Waschphase zusammen eingeengt und durch präparative HPLC (Methode beinhaltete Zusatz von Ammoniak) gereinigt. Nach Ausrühren des durch HPLC gereinigten Produktes aus Diethylether und Trocknen wurden 14 mg der Titelverbindung erhalten (leicht verunreinigt mit einer Nebenkomponente laut UPLC Analyse (89% Titelverbindung, 11% Nebenkomponente: UV-Detektor - TIC Smooth Spur)).
UPLC-MS (Methode D): Rt = 0.91 min; gefundene Masse 432.15.
1H-NMR (400MHz, DMSO-d6): δ [ppm]= 1.90 - 2.11 (m, 4H), 2.60 - 2.72 (m), 3.04-3.15 (m, 2H), 3.36 - 3.44 (m, 1H), 4.57 (tt, 1H), 7.89 (s, 1H), 8.17 (dd, 1H), 8.23 (s, 1H), 8.31 - 8.41 (m, 2H), 8.45 (d, 1H), 8.52 (s, 1H), 9.01 (s, 1H), 13.07 (s, 1H).

### Beispiel 54

### 2-(Piperidin-4-yl)-5-({[6-(trifluormethyl)pyridin-2-yl]carbonyl}amino)-2H-indazol-6-carbonsäure

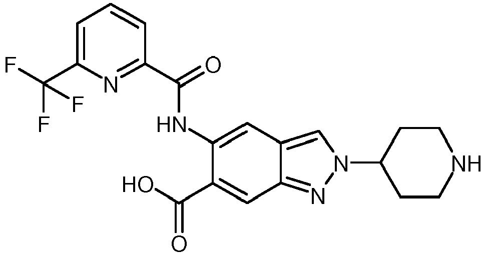

100 mg Methyl-2-(piperidin-4-yl)-5-({[6-(trifluormethyl)pyridin-2-yl]carbonyl}amino)-2H-indazol-6-carboxylat (Beispiel 45) wurden in 1 ml THF und 0.4 ml Methanol vorgelegt. Es wurde eine Lösung aus 94 mg Lithiumhydroxidmonohydrat in 0.8 ml Wasser zugegeben und die Mischung wurde 19 h bei Raumtemperatur gerührt. Es wurde mit Wasser verdünnt und mit 10 prozentiger wässriger Zitronensäurelösung auf pH = 7 gebracht. Der ausgefallene Feststoff wurde abfiltriert, dreimal mit Wasser gewaschen, im Vakuum getrocknet, mit Diethylether ausgerührt, abfiltriert, mit Diethylether gewaschen und erneut im Vakuum getrocknet. Es wurden 65 mg der Titelverbindung erhalten.
¹H-NMR (Analytik der Titelverbindung vor dem Ausrühren mit Diethylether, 400MHz, DMSO-d₆): δ [ppm]= 2.21 (d, 2H), 2.45 - 2.59 (Signale überlagert durch DMSO-Signal), 3.03 - 3.13 (m, 2H), 3.43-3.51 (Signal überlagert durch Wasser-Signal), 4.74 - 4.90 (m, 1H), 8.12 (dd, 1H), 8.33 (t, 1H), 8.37 - 8.43 (m, 2H), 8.62 (s, 1H), 9.01 (s, 1H), 15.09 (br. s., 1H).
UPLC-MS (Methode C): Rt = 0.84 min; gefundene Masse 433.00.

### Beispiel 55

### Methyl-2-(1-methylpiperidin-4-yl)-5-({[6-(trifluormethyl)pyridin-2-yl]carbonyl}amino)-2H-indazol-6-carboxylat

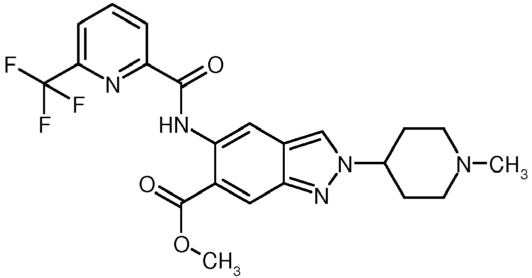

Zu 250 mg (0.56 mmol) Methyl-2-(piperidin-4-yl)-5-({[6-(trifluormethyl)pyridin-2-yl]carbonyl}amino)-2H-indazol-6-carboxylat (Beispiel 45) in 1.5 ml Tetrahydrofuran und 1.5 ml Methanol wurden 0.20 ml Formaldehydlösung (37 prozentig in Wasser) gegeben und die Mischung wurde 45 Minuten bei RT gerührt. Man addierte 200 mg Natriumtriacetoxyborhydrid und rührte 18 h bei RT. Die Reaktionsmischung wurde durch Zugabe von 10 prozentiger wässriger Zitronensäurelösung auf pH 4 angesäuert, man dampfte die leichter siedenden Lösungsmittel ab und verdünnte mit Wasser. Der ausgefallene Feststoff wurde abfiltriert und in einem Ethylacetat / Wassergemisch gelöst. Man versetzte mit gesättigter wässriger Natriumhydrogencarbonatlösung, extrahierte dreimal mit Ethylacetat, wusch mit gesättiger Kochsalzlösung, filtrierte durch einen wasserabweisenden Filter und engte ein. Man erhielt 191 mg der Titelverbindung.
¹H-NMR (400MHz, DMSO-d₆): δ [ppm]= 2.06 - 2.21 (m, 6H), 2.24 (s, 3H), 2.92 (d, 2H), 3.96 (s, 3H), 4.49 - 4.58 (m, 1H), 8.22 (dd, 1H), 8.40 (t, 1H), 8.45 - 8.50 (m, 2H), 8.61 - 8.64 (m, 1H), 9.07 (s, 1H), 12.56 (s, 1H).

### Beispiel 56

### 2-[1-(3-Hydroxy-3-methylbutyl)piperidin-4-yl]-5-({[6-(trifluormethyl)pyridin-2-yl]carbonyl}amino)-2H-indazol-6-carboxamid

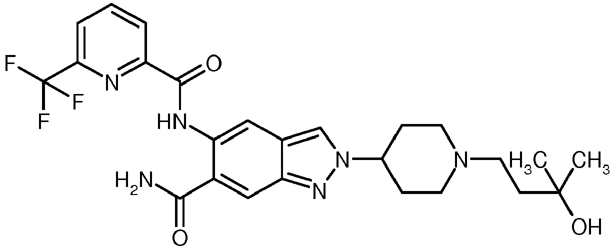

Ein Mischung aus 250 mg Methyl-2-(piperidin-4-yl)-5-({[6-(trifluormethyl)pyridin-2-yl]carbonyl}amino)-2H-indazol-6-carboxylat (Beispiel 45), 112 mg 4-Brom-2-methylbutan-2-ol und 463 mg Kaliumcarbonat und 139 mg Kaliumiodid in 3 ml DMSO wurde 19.5 h bei 80°C gerührt. Man addierte 2.8 ml 2M Natronlauge und rührte 3 h bei 50°C. Man säuerte mit 10 prozentiger wässriger Zitronensäurelösung auf pH = 4 an, filtrierte den ausgefallenen Feststoff ab, wusch dreimal mit Wasser und trocknete im Vakuum. Danach wurde der Feststoff aus Diethylether ausgerührt. Nach Trocknen wurden 135 mg eines Feststoffes erhalten. Eine Mischung aus 132 mg dieses Feststoffes, 130 mg HATU, 119 Microliter N-Ethyl-N-isopropylpropan-2-amin in 3 ml THF wurde 45 min bei Raumtemperatur gerührt. Man addierte 100 Microliter Ammoniaklösung (33 prozentig) und rührte 17 h bei Raumtemperatur, verdünnte mit Wasser, extrahierte viermal mit Ethylacetat, engte die vereinigten organischen Phasen ein und reinigte durch HPLC (Eluent enthielt Ameisensäure). Nach Gefriertrocknung wurden 42 mg der Titelverbindung erhalten.
UPLC-MS (Methode C): Rt = 0.73 min; gefundene Masse 518.23.
¹H-NMR (400MHz, DMSO-d₆, Anteile an Ameisensäure in Substanzprobe der Titelverbindung möglich): δ [ppm]= 1.11 (s, 6H), 1.54 - 1.60 (m, 2H), 2.06 - 2.20 (m, 6H), 3.05 (br d, 3H), 3.35 (br s, 2H), 4.49 - 4.58 (m, 1H), 7.88 (s, 1H), 8.14 - 8.23 (m, 2.6H), 8.32 - 8.39 (m, 2H), 8.42 - 8.46 (m, 1H), 8.54 (s, 1H), 9.00 (s, 1H), 13.07 (s, 1H).

### Beispiel 57

### 2-(1-Methylpiperidin-4-yl)-5-({[6-(trifluormethyl)pyridin-2-yl]carbonyl}amino)-2H-indazol-6-carboxamid

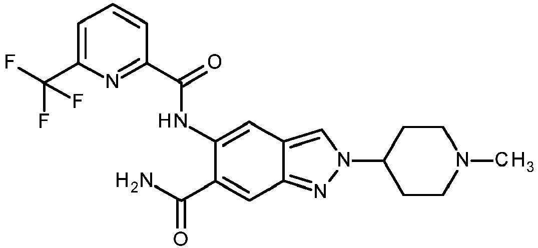

Eine Mischung aus 184 mg Methyl-2-(1-methylpiperidin-4-yl)-5-({[6-(trifluormethyl)pyridin-2-yl]carbonyl}amino)-2H-indazol-6-carboxylat (Beispiel 55) in 5.0 ml Ammoniaklösung (7M in Methanol) bei 50°C 47.5 h in einem Druckgefäß gerührt. Man verdünnte mit Wasser, extrahierte dreimal mit Ethylacetat und engte die vereinigten organischen Phasen ein. Der Rückstand wurde mit Dimethylsulfoxid versetzt, der verbleibende Feststoff abfiltriert und mit Diethylether gewaschen. In der Waschphase fiel erneut ein Feststoff aus, der abfiltriert wurde. Die Waschphase wurde eingeengt und durch HPLC aufgereinigt (Eluent enthielt Ameisensäure). Nach Gefriertrocknung wurden 10 mg der Titelverbindung erhalten.
UPLC-MS (Methode C): Rt = 0.71 min; gefundene Masse 446.17.
¹H-NMR (400MHz, DMSO-d₆): δ [ppm]= 2.08 - 2.21 (m, 6H), 2.24 (s, 3H), 2.87 - 2.97 (m, 2H), 4.45 - 4.55 (m, 1H), 7.88 (s, 1H), 8.15 - 8.19 (m, 1H), 8.23 (s, 1H), 8.31 - 8.40 (m, 2H), 8.42 - 8.46 (m, 1H), 8.54 (s, 1H), 9.00 (s, 1H), 13.07 (s, 1H).

### Beispiel 58

### N-{6-Methoxy-2-[1-(2-methoxyethyl)piperidin-4-yl]-2H-indazol-5-yl}-6-(trifluormethyl)pyridin-2-carboxamid

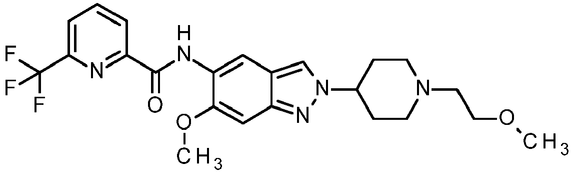

120 mg N-{2-[1-(2-Hydroxyethyl)piperidin-4-yl]-6-methoxy-2H-indazol-5-yl}-6-(trifluormethyl)-pyridin-2-carboxamid (Beispiel 13) in 5 ml THF wurden bei 0°C mit 23 mg Natriumhydrid (60 prozentig in Mineralöl) versetzt und die Mischung wurde 0.5 h gerührt. 0.1 ml einer Lösung aus 0.17 ml Methyliodid in 1.0 ml THF wurden zugegeben und man erwärmte innerhalb von 3 h auf Raumtemperatur. Man gab 2 ml DMF zu und rührte 3 h. Man addierte Wasser und Ethylacetat, trennte die organische Phase ab, extrahierte die wässrige Phase mit Ethylacetat, wusch mit gesättigter Kochsalzlösung, trocknete mit Natriumsulfat und engte ein. Nach Reinigung (Biotage Isolera, Kieselgel, dann präparative HPLC) wurden 13 mg der Titelverbindung erhalten.
¹H-NMR (400MHz, DMSO-d₆): δ [ppm]= 2.03 - 2.09 (m, 4H), 2.11 - 2.21 (m, 2H), 2.51 (dd, 2H), 2.98 (d, 2H), 3.22 (s, 3H), 3.44 (t, 2H), 3.95 (s, 3H), 4.29 - 4.39 (m, 1H), 7.14 (s, 1H), 8.18 - 8.20 (m, 1H), 8.34 - 8.45 (m, 3H), 8.66 (s, 1H), 10.47 (s, 1H).

### Bewertung der physiologischen Wirksamkeit

### IRAK4-Kinaseassay

Die IRAK4-inhibitorische Aktivität der erfindungsgemäßen Substanzen wurde in dem nachfolgend beschriebenen IRAK4-TR-FRET-Assay gemessen (TR-FRET = Time Resolved Fluorescence Resonance Energy Transfer).

Rekombinantes Fusionsprotein aus N-terminalem GST (Glutathion-S-Transferase) und humanem IRAK4, exprimiert in Bakulovirus-infizierten Insektenzellen (Hi5, BTI-TN-5B1-4, Zelllinie gekauft von Invitrogen, Katalog-Nr. B855-02) und gereinigt via Affinitätschromatographie, wurde als Enzym verwendet. Als Substrat für die Kinasereaktion wurde das biotinylierte Peptid Biotin-Ahx-KKARFSRFAGSSPSQASFAEPG (C-Terminus in Amid-Form) verwendet, das z.B. bei der Firma Biosyntan GmbH (Berlin-Buch) gekauft werden kann.
Für den Assay wurden 11 verschiedene Konzentrationen im Bereich von 20 µM bis 0,073 nM aus einer 2 mM Lösung der Testsubstanz in DMSO hergestellt. 50 nl der jeweiligen Lösung wurden in eine schwarze low-volume 384well-Mikrotiterplatte (Greiner Bio-One, Frickenhausen, Deutschland) pipettiert, 2 µl einer Lösung von IRAK4 in Assaypuffer [50 mM HEPES pH 7.5, 5 mM MgC12, 1.0 mM Dithiothreitol, 30 µM aktiviertes Natriumorthovanadat, 0,1 % (w/v) bovines gamma-Globulin (BGG) 0,04% (v/v) Nonidet-P40 (Sigma)] hinzugegeben und die Mischung für 15 min inkubiert, um eine Vorbindung der Substanzen an das Enzym vor der Kinasereaktion zu ermöglichen. Dann wurde die Kinasereaktion gestartet durch Zugabe von 3 µl einer Lösung von Adenosine-tri-phosphat (ATP, 1,67 mM =Endkonzentration in 5 µl Assayvolumen ist 1 mM) und Peptidsubstrat (0,83 µM =Endkonzentration in 5 µl Assayvolumen ist 0,5 µM) in Assaypuffer und die resultierende Mischung für die Reaktionszeit von 45 min bei 22°C inkubiert. Die Konzentration des IRAK4 wurde an die jeweilige Aktivität des Enzyms angepasst und so eingestellt, dass der Assay im linearen Bereich arbeitete. Typische Konzentrationen lagen in der Größenordnung von etwa 0,2 nM. Die Reaktion wurde gestoppt durch Zugabe von 5 µl einer Lösung von TR-FRET-Detektionsreagenzien [0,1 µM Streptavidin-XL665 (Cisbio Bioassays; Frankreich, Katalog-Nr. 610SAXLG) und 1,5 nM Anti-phosho-Serin Antikörper [Merck Millipore, "STK Antibody", Katalog-Nr. 35-002] und 0,6 nM LANCE EU-W1024-markierter anti-Maus-IgG-Antikörper (Perkin-Elmer, Produkt-Nr. AD0077, alternativ kann ein Terbium-Kryptat-markierter anti-Maus-IgG-Antikörper von Cisbio Bioassays verwendet werden) in wässriger EDTA-Lösung (100 mM EDTA, 0.4 % [w/v] bovines Serumalbumin [BSA] in 25 mM HEPES pH 7,5].

Die resultierende Mischung wurde 1 h bei 22°C inkubiert, um die Bildung eines Komplexes aus dem biotinylierten phosphorylierten Substrat und den Detektionsreagentien zu ermöglichen. Anschließend wurde die Menge des phosphorylierten Substrates ausgewertet durch eine Messung des Resonanz-Energietransfers vom Europium-Chelat markierten anti-Maus-IgG-Antikörper zum Streptavidin-XL665. Hierzu wurden in einem TR-FRET-Meßgerät, z.B. einem Rubystar (BMG Labtechnologies, Offenburg, Germany) oder einem Viewlux (Perkin-Elmer), die Fluoreszenz-Emissionen bei 620 nm and 665 nm nach Anregung bei 350 nm gemessen. Das Verhältnis der Emissionen bei 665 nm und 622 nm wurde als Maß für die Menge des phosphorylierten Substrates genommen. Die Daten wurden normalisiert (Enzymreaktion ohne Testsubstanz = 0 % Inhibition, alle anderen Assaykomponenten aber kein Enzym = 100 % Inhibition). Üblicherweise wurden die Testsubstanzen auf denselben Mikrotiterplatten bei 11 verschiedenen Konzentrationen im Bereich von 20 µM bis 0,073 nM getestet (20 µM, 5,7 µM, 1,6 µM, 0,47 µM, 0,13 µM, 38 nM, 11 nM, 3,1 nM, 0,89 nM, 0,25 nM und 0,073 nM). Die Verdünnungsreihen wurden vor dem Assay hergestellt (2 mM bis 7,3 nM in 100 % DMSO) durch serielle Verdünnungen. Die IC50-Werte wurden kalkuliert mit einem 4-Parameter-Fit.

**Tabelle 1: IC₅₀-Werte der Beispielverbindungen im IRAK4 Kinase Assay**

| Beispiel | IC₅₀ [nM] |
|---|---|
| 1 | 3 |
| 2 | 1 |
| 3 | 7 |
| 4 | 1 |
| 5 | 2 |
| 6 | 2 |
| 8 | 8 |
| 9 | 1 |
| 10 | 3 |
| 11 | 3 |
| 12 | 2 |
| 13 | 1 |
| 17 | 20 |
| 18 | 2 |
| 19 | 9 |
| 20 | 4 |
| 21 | 3 |
| 22 | 13 |
| 23 | 1 |
| 24 | 2 |
| 25 | 10 |
| 26 | 6 |
| 27 | 111 |
| 28 | 3 |
| 29 | 10 |
| 30 | 47 |
| 31 | 22 |
| 32 | 34 |
| 33 | 11 |
| 34 | 6 |
| 36 | 28 |
| 38 | 31 |
| 41 | 16 |
| 44 | 5 |
| 46 | 3 |
| 47 | 65 |
| 48 | 691 |
| 49 | 3782 |
| 50 | 496 |
| 51 | 6 |
| 52 | 1 |
| 53 | 1 |
| 54 | 75 |
| 55 | 1 |
| 56 | 4 |
| 57 | 2 |
| 58 | 2 |

### TNF-α Ausschüttung in THP-1 Zellen

Mithilfe dieses Tests können Substanzen auf ihre Fähigkeit hin getestet werden, TNF-α (Tumornekrosefaktor-alpha) Ausschüttung in THP-1 Zellen (humane monozytische akute Leukämie-Zellinie) zu inhibieren. TNF-α ist ein Zytokin, welches in inflammatorischen Prozessen beteiligt ist. Die TNF-α Ausschüttung wird in diesem Test ausgelöst durch Inkubation mit bakteriellem Lipopolysaccharid (LPS).

THP-1 Zellen werden in kontinuierlicher Suspensions-Zellkultur [RPMI 1460 Medium mit L-Glutamax (Gibco, Kat-Nr. 61870-044) supplementiert mit foetalem Kälberserum (FCS) 10% (Invitrogen, Kat-Nr. 10082-147), 1% Penicillin/Streptomycin (Gibco BRL, Kat-Nr. 15140-114)] gehalten und sollten eine Zellkonzentration von 1x10⁶ Zellen/ml nicht überschreiten. Der Assay erfolgte im Zellkulturmedium (RPMI 1460 Medium mit L-Glutamax supplemetiert mit FCS 10%).
Jeweils 2-2.5 µl der Zellsuspension (entspricht 4000 Zellen) wurden pro well in eine 384-well Testplatte Greiner Kat-Nr. 784076) dispensiert, in der sich jeweils 40-50 nl Substanz in 100% DMSO gelöst befanden. Hierbei wurden jeweils 10 verschiedene Konzentrationen im Bereich von 20 µM bis 0,073 nM je Substanz eingesetzt. Die Zellen wurden 15 min bei Raumtemperatur inkubiert. Anschließend wurden 2-2.5 µl pro well 0.1 µg/ml LPS (Sigma, *Escherichia coli* 055:B5, Kat-Nr. L5418) gelöst in Zellkulturmedium dispensiert (finale Konzentration 0.05 µg/ml). Als Neutralkontrolle wurden Zellen mit 0.05 µg/ml LPS und 1% DMSO und als Inhibitorkontrolle nur mit 1% DMSO behandelt.
Die Platten wurden 30 sec bei 80g zentrifugiert und 17 h bei 37°C, 5% CO₂ und 95% Luftfeuchtigkeit inkubiert. Zur Bestimmung der Menge an TNF-α wurde der TNF-alpha HTRF Detection Kit (Cisbio, Kat-Nr. 62TNFPEB/C) verwendet. Hierzu wurden jeweils 2 µl der Detektionslösung, bestehend aus Anti-TNF-α-XL665 Konjugat und Anti-TNF-α-Kryptat Konjugat gelöst nach Anweisung des Herstellers im Rekonstitutionspuffer, für den HTRF (Homogeneous Time-Resolved Fluorescence) Test zugegeben. Nach Zugabe wurde entweder 3 h bei Raumtemperatur oder über Nacht bei 4°C inkubiert. Anschließend wurden die Signale mit einem HTRF-fähigen wie dem BMG PheraStar bei 620/665nm Messgerät ausgelesen.

Die Wirkung der Substanzen wird als Verhältnis zwischen Neutral- und Inhibitorkontrolle in Prozent ausgedrückt. Die IC₅₀-Werte wurden mit einem 4-Parameter-Fit kalkuliert.

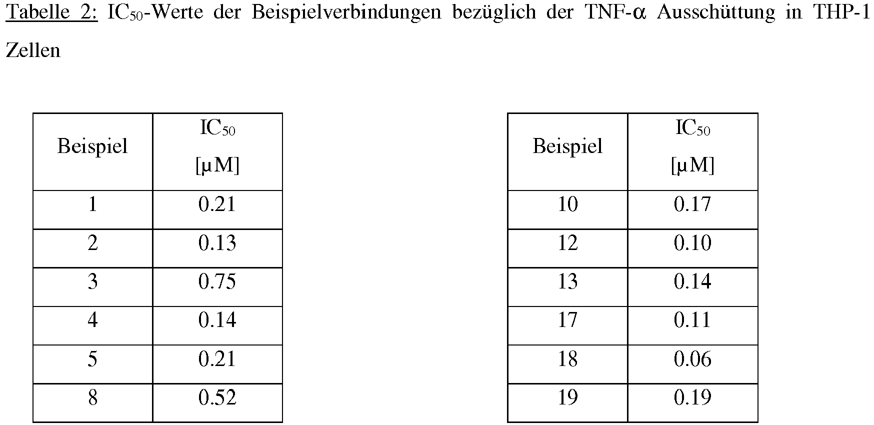

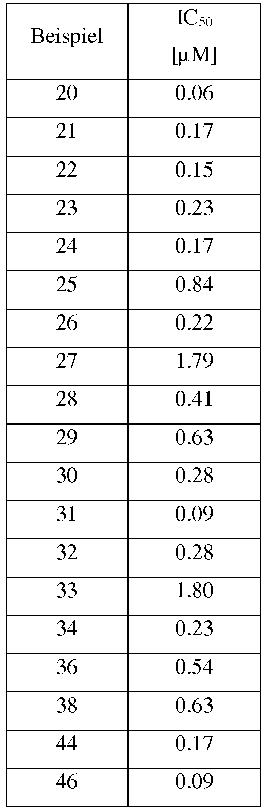

### In vitro LPS (Lipopolysaccharide)-induzierte Zytokinproduktion in humanen PBMCs (peripheral blood mononuclear cells)

Die Wirkung der erfindungsgemäßen Verbindungen der allgemeinen Formel (I) auf die induzierte Zytokinproduktion in humanen PBMCs wurde untersucht. Hierbei erfolgte die Induktion der Zytokinproduktion durch LPS, einen TLR4 Liganden, welcher zur Aktivierung des IRAK4-vermittelten Signalweges führt.
Die Gewinnung der humanen PBMCs erfolgte aus anti-koaguliertem humanem Vollbut. Hierzu wurde in Leucosep-Röhrchen 15 ml Ficoll-Paque (Biochrom, Kat-Nr. L6115) vorgelegt und 20 ml Humanblut hinzugefügt. Nach Zentrifugation des Blutes bei 800g für 15 min bei Raumtemperatur wurde Plasma inklusive der Thrombozyten abgenommen und verworfen. Die PBMCs wurden in Zentrifugenröhrchen überführt und mit PBS (phosphatgepufferte Salzlösung) (Gibco, Kat-Nr. 14190) aufgefüllt. Die Zellsuspension wurde bei 250g für 10 min bei Raumtemperatur zentrifugiert und der Überstand verworfen. Die Resuspension der PBMCs erfolgte in Komplettmedium (RPMI 1640, ohne L-Glutamin (PAA, Kat-Nr. E15-039), 10% FCS; 50 U/ml Penicillin, 50 µg/ml Streptomycin (PAA, Kat-Nr. P11-010) und 1% L-Glutamin (Sigma, Kat-Nr. G7513)).
Auch der Assay erfolgte in Komplettmedium. Die PBMCs wurden in 96-well Platten mit einer Zelldichte von 2.5x10⁵ Zellen/well ausgesät. Die erfindungsgemäßen Verbindungen wurden in einem konstanten Volumen von 100% DMSO seriell verdünnt und in dem Assay mit 8 verschiedenen Konzentrationen im Bereich von 10 µM bis 3 nM so eingesetzt, so dass die finale DMSO Konzentration 0.4% DMSO betrug. Die Zellen wurden damit für 30 min vor der eigentlichen Stimulation vorinkubiert. Zur Induktion der Zytokinsekretion erfolgte eine Stimulation mit 0.1 µg/ml LPS (Sigma, *Escherichia coli* 0128:B12, Kat-Nr. L2887) für 24 Stunden. Die Bestimmung der Zellviabilität erfolgte unter Verwendung des CellTiter-Glo Luminescent Assay (Promega, Kat-Nr. G7571 (G755/G756A)) nach Anweisung des Herstellers. Die Bestimmung der Menge an sekretiertem TNF-α im Zellkulturüberstand erfolgte mittels Human ProInflammatory 9-Plex Tissue Culture Kit (MSD, Kat.-Nr. K15007B) nach Anweisung des Herstellers. Exemplarisch sind die Beispielverbindung 44, 46 und die Beispielverbindung 52 mit einer Aktivität ≤ 1µM genannt.

### Zellproliferations-Messung

Die antiproliferative Aktivität der erfindungsgemäßen Verbindungen der allgemeinen Formel (I) wurde in humanen ABC-DLBCL Zellen *in vitro* untersucht (siehe Tabelle 3). Hierzu wurden 4000 TMD-8 Zellen (beide von ATCC) in 30 µL/Kavität in Wachstumsmedium ((RPMI (Biochrom: FG 1215), 20% FCS (Biochrom: S 0615)) in eine 384- Kavitäten Platte (Perkin Elmer, weiß) überführt und über Nacht bei 37°C inkubiert. Nach 24h wurden Zellen einer Platte (0h Platte) mit 30 µL/Kavität CTG Lösung behandelt (Promega Cell Titer Glo (Katalog # G755B und G756B)), für 10 min bei Raumtemperatur inkubiert und die Lumineszenz mittels eines VICTOR V (Perkin Elmer) gemessen um die Zellviablilität zu Beginn der Behandlung zu bestimmen. Die Zellen der Testplatte wurden mit den erfindungsgemäßen Verbindungen der allgemeinen Formel (I) behandelt und für 72 Stunden bei 37°C inkubiert. Die Verbindungen wurden in einer 7-stufigen, 3-fachen Verdünnungsreihe mittels eines HP D300 Digital Dispensers zu den Zellen gegeben. Als Kontrolle wurden Zellen mit Vehikel (DMSO) behandelt. Nach 72h wurden die Zellen mit 30 µL/Kavität CTG Lösung behandelt (Promega Cell Titer Glo (Katalog # G755B und G756B)), für 10 min bei Raumtemperatur inkubiert und die Lumineszenz mittels eines VICTOR V (Perkin Elmer) gemessen um die Zellviablilität zum Ende der Behandlung zu bestimmen. Die prozentuale Beeinflussung des Zellwachstums und die daraus abgeleitete IC50 wurden unter Zuhilfenahme der Werte der 0h Platte (=maximale Inhibition) und der DMSO Kontrolle (= minimale Inhibition) für jede Testsubstanz bestimmt. Die IC₅₀-Werte wurden mit einem 4-Parameter-Fit kalkuliert.

**Tabelle 3: IC₅₀-Werte der Beispielverbindungen bezüglich Proliferationsinhibition in TMD-8 Zellen**

| Beispiel | IC₅₀ [mol/1] |
|---|---|
| 2 | 8.61E-06 |
| 1 | 2.82E-05 |
| 4 | 4.08E-06 |
| 5 | 1.55E-05 |
| 23 | 8.52E-06 |
| 13 | 5.85E-06 |
| 24 | 2.81E-05 |
| 28 | 3.51E-06 |
| 10 | 2.33E-05 |
| 12 | 1.15E-05 |
| 21 | 1.48E-05 |
| 20 | 1.61E-05 |
| 18 | 3.45E-06 |
| 44 | 1.88E-05 |
| 46 | 1.41E-05 |

### NF-kB-Reporter Messung

Die Wirkung der erfindungsgemäßen Verbindungen der allgemeinen Formel (I) auf den NF-kB Signalweg wurde in humanen DLBCL Zellen in vitro untersucht (siehe Tabelle 4). 10,000 TMD-8-NF-kB-luc Reporter-Zellen wurden in 30 µL/Kavität in Wachstumsmedium ((RPMI (Biochrom: FG 1215), 20% FCS (Biochrom: S 0615)) in einer 384- Kavitäten Platte (Perkin Elmer, weiß) überführt und über Nacht bei 37°C inkubiert. Nach 24h wurden die Zellen mit den Testsubstanzen behandelt und für 6h bei 37°C inkubiert. Die Testsubstanzen wurden in einer 7-stufigen, 3-fachen Verdünnungsreihe mittels eines HP D300 Digital Dispensers zu den Zellen gegeben. Als Kontrolle wurden Zellen mit dem Vehikel (DMSO) behandelt. Nach 6 h wurden die Zellen mit 30 µL/Kavität One-Glo Lösung (Promega E6110) behandelt, für 10 min bei Raumtemperatur inkubiert und die Lumineszenz mittels eines VICTOR V (Perkin Elmer) gemessen, um die NF-kB Reporter Aktivität am Ende der Behandlung zu bestimmen. Die prozentuale Beeinflussung der NF-kB Reporter Aktivität und die daraus abgeleitete IC₅₀ wurden unter Zuhilfenahme der Werte eines NF-kB-pathway inhibitors ++(I-kappa B kinase inhibitor) (-)-7-[2-(Cyclopropylmethoxy)-6-hydroxyphenyl]-5-[(3S)-3-iperidinyl]-1,4-dihydro-2H-pyrido[2,3-d][1,3]oxazin-2-on (CAS-Nummer 600734-02-9, siehe WO 2003076447) (= maximale Inhibition) sowie der DMSO Kontrolle (= minimale Inhibition) für jede Testsubstanz bestimmt. Die IC₅₀-Werte wurden mit einem 4-Parameter-Fit kalkuliert.

**Tabelle 4: IC₅₀-Werte der Beispielverbindungen bezüglich der Inhibition der NF-kB Aktivität in TMD-8-NF-kB-luc Zellen**

| Beispiel | IC₅₀ [mol/l] |
|---|---|
| 2 | 9.45E-06 |
| 1 | 1.56E-05 |
| 4 | 7.41E-06 |
| 5 | 5.10E-06 |
| 23 | 3.84E-06 |
| 13 | 5.10E-06 |
| 24 | 2.26E-05 |
| 28 | 2.68E-06 |
| 10 | 2.89E-05 |
| 12 | 6.81E-06 |
| 21 | 5.19E-06 |
| 20 | 2.18E-05 |
| 18 | 5.52E-06 |
| 44 | 1.46E-05 |
| 46 | 1.34E-05 |

### In vivo IL-1β-vermitteltes Inflammationsmodel

Zur Erfassung der potentiellen Wirksamkeit der erfindungsgemäßen Verbindungen der allgemeinen Formel (I) in IL-1β-vermittelten Erkrankungen wird weiblichen Balb/c Mäusen (ca. 8 Wochen, Charles River Laboratories, Deutschland) IL-1β i.p. appliziert und die Wirkung der erfindungsgemäßen Verbindungen auf die IL-1β-vermittelte Zytokinsekretion untersucht. Die Gruppengröße beträgt jeweils 5 Tiere. Die Kontrollgruppe wird mit den Vehikeln, welche zur Lösung der Substanz und des IL-1β eingesetzt werden, behandelt. Den Substanzbehandlungsgruppen und der Positivkontrollgruppe werden jeweils 90 µg IL-1β /kg Körpergewicht (R&D, Kat-Nr.401-ML/CF) i.p verabreicht. Die Substanz bzw. dessen Vehikel in der Positivkontrollgruppe werden vor der IL-1β Verabreichung appliziert. Die Bestimmung von TNF-α im Plasma nach finaler Blutentnahme erfolgt 2 Stunden nach Verabreichung des IL-1β mittels des Mouse Prolnflammatory 7-Plex Tissue Culture Kit (MSD, Kat.-Nr. K15012B) nach Anweisung des Herstellers.

### In vivo Adjuvanz-induziertes Arthritismodell

Zur Ermittlung der anti-inflammatorischen Aktivität der erfindungsgemäßen Verbindungen der allgemeinen Formel (I) werden diese in einem Arthritismodel hinsichtlich ihrer *in vivo* Wirksamkeit untersucht. Hierzu wird männlichen Lewis-Ratten (ca. 100-125g, Charles River Laboratories, Deutschland) je 100µl einer Complete-Freund'schen Adjuvanz (CFA)-Lösung (M. tuberculosis H37Ra [Difo Lab, Kat.-Nr. -231141] gelöst in Incomplete Freund'schen Adjuvanz [Difco Lab, Kat.-Nr. -263910]) subkutan in die Schwanzwurzel an Tag 0 appliziert. Die Gruppengröße beträgt jeweils n= 8 Ratten. Es wird sowohl eine gesunde Kontrollgruppe als auch eine Erkrankungs-Kontrollgruppe im Experiment mitgeführt. Beide Kontrollgruppen werden jeweils nur mit dem Vehikel der Prüfsubstanz p.o. behandelt. Die Behandlung mit unterschiedlichen Dosierungen der Prüfsubstanz wird präventiv, d.h. ab Tag 0, per oraler Gabe durchgeführt. An Tag 0 wird zudem die Ausgangssituation der Tiere bezüglich des Disease Activity Scores (Bewertung des Schweregrads der Arthritis anhand eines Punktesystems) bestimmt. In diesem Punktesystem (Score) werden je nach Ausmaß der Gelenkentzündung Punkte von 0 bis 4 für das Vorhandensein eines Erythems inklusive einer Gelenkschwellung (0= keine; 1=leicht; 2= moderat; 3=deutlich; 4=massiv) für beide Hinterpfoten vergeben und addiert. Zur Ermittlung der anti-entzündlichenWirksamkeit der Verbindungen wird ab Tag 8, an dem die Tiere erstmals Zeichen einer Arthritis zeigen, und weiterfolgend 3 Mal in der Woche der Erkrankungsstatus der Tiere mittels Disease Activity Score bis zum Ende (Tag 20) bewertet. Die statistische Analyse erfolgt unter Verwendung der einfaktoriellen Varianzanalyse (ANOVA; Analysis of Variance) und dem Vergleich zur Kontrollgruppe mittels multipler Vergleichsanalyse (Dunnett-Test).
Die s.c. Applikation von CFA in Ratten führt zu einer akuten Arthritis mit deutlicher Gelenkentzündung in Ratten.

## Patentansprüche

1. Verbindungen der allgemeinen Formel (I) 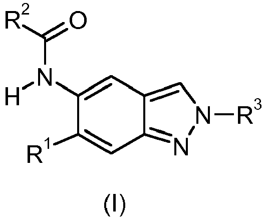 worin
R¹ für Halogen, Cyano, C(=O)OH, C(=O)OR^{a}, C(=O)NH₂, C(=O)N(H)R^{a}, C(=O)N(R^{a})R^{b}, C(=O)R^{d}, Hydroxy oder C₁-C₆-Alkyl steht, wobei der C₁-C₆-Alkylrest gegebenenfalls ein- oder mehrfach, gleich oder verschieden substituiert sein kann mit
Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- bis sechsfach mit Fluor substituiertem C₁-C₆-Alkoxy oder C₃-C₇-Cycloalkoxy, einem gegebenenfalls ein- bis dreifach, gleich oder verschieden mit R^{c} substituiertem 4- bis 7-gliedrigem Heterocycloalkyl,
oder für C₁-C₆-Alkoxy steht, wobei der C₁-C₆-Alkoxyrest gegebenenfalls ein- oder mehrfach, gleich oder verschieden substituiert sein kann mit
Hydroxy, Halogen, Cyano, C(=O)OH, C(=O)OR^{a}, S(=O)₂-C₁-C₆-Alkyl, NH₂, NHR^{a}, N(R^{a})R^{b}, einem gegebenenfalls ein- bis vierfach mit Fluor substituiertem C₃-C₇-Cycloalkyl, einem gegebenenfalls ein- bis fünffach mit Fluor substituiertem C₁-C₆-Alkoxy, einem gegebenenfalls ein- bis vierfach mit Fluor substituiertem C₃-C₇-Cycloalkoxy, einem gegebenenfalls ein- oder mehrfach, gleich oder verschieden mit R^{c} substituiertem 4- bis 7-gliedrigem Heterocycloalkyl,
oder für C₃-C₇-Cycloalkyloxy oder 4- bis 7-gliedriges Heterocycloalkyloxy steht, worin C₃-C₇-Cycloalkyloxy und 4- bis 7-gliedriges Heterocycloalkyloxy gegebenenfalls ein- oder mehrfach, gleich oder verschieden substituiert sein können mit Hydroxy, Fluor, Cyano, C₁-C₆-Alkyl oder C₁-C₆-Alkoxy;
R^{a} für C₁-C₆-Alkyl, C₃-C₇-Cycloalkyl, 4- bis 7-gliedriges Heterocycloalkyl steht,
worin C₁-C₆-Alkyl, C₃-C₇-Cycloalkyl und 4- bis 7-gliedriges Heterocycloalkyl gegebenenfalls ein- oder mehrfach, gleich oder verschieden substituiert sein können mit Fluor, Hydroxy, Cyano, C₁-C₄-Alkyl, C₁-C₄-Alkoxy oder C₃-C₇-Cycloalkyl;
R^{b} für C₁-C₆-Alkyl oder C₃-C₇-Cycloalkyl steht;
oder R^{a} und R^{b} gemeinsam mit dem Stickstoffatom einen 5- oder 6-gliedrigen Heterocyclus bilden, welcher gegebenenfalls ein- oder zweifach, gleich oder verschieden substituiert sein kann mit Hydroxy, Halogen, Cyano oder C₁-C₆-Alkyl;
R^{c} für Hydroxy, Fluor, Chlor, Cyano, C₁-C₄-Alkyl oder C₁-C₄-Alkoxy steht;
R^{d} für Wasserstoff, C₃-C₇-Cycloalkyl oder für C₁-C₆-Alkyl steht, welches gegebenenfalls mit einer Hydroxygruppe substituiert sein kann;
R² für 5-gliedriges Heteroaryl steht, welches einfach mit R⁴ und einfach mit R⁵ substituiert sein kann oder
R² für 6-gliedriges Heteroaryl steht, welches einfach mit R⁴ und ein- oder zweifach, gleich oder verschieden mit R⁵ substituiert sein kann,
R³ für eine Gruppe steht, ausgewählt aus: 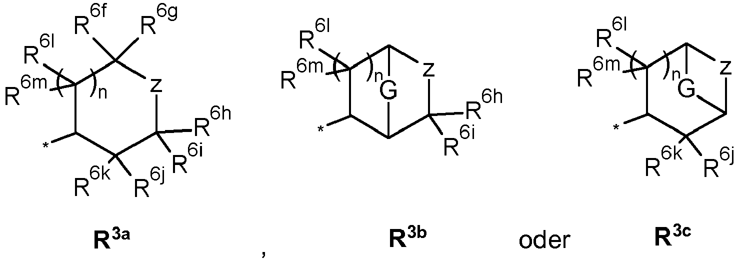
wobei * für die Verknüpfungsstelle der Gruppe mit dem Rest des Moleküls steht;
R⁴ für Wasserstoff, Halogen, Hydroxy, C(=O)OH, Cyano, NH₂, NHR^{a}, N(R^{a})R^{b}, C(=O)R^{a}, N(H)C(=O)R^{a}, C(=O)NH₂, C(=O)N(H)R^{a}, C(=O)N(R^{a})R^{b}, S(=O)R^{a}, S(=O)₂R^{a}, S(=O)₂NH₂, S(=O)₂NHR^{a} oder S(=O)₂N(R^{a})R^{b} steht,
oder für C₁-C₆-Alkyl steht, wobei C₁-C₆-Alkyl gegebenenfalls mit ein bis fünf Fluoratomen substituiert sein kann und gegebenfalls ein- oder zweifach, gleich oder verschieden substituiert sein kann mit Hydroxy, Brom, Chlor, Cyano, C(=O)OH, S(=O)₂-C₁-C₆-Alkyl, NH₂, NHR^{a}, N(R^{a})R^{b}, C₃-C₇-Cycloalkyl, C₁-C₄-Alkoxy, C₃-C₇-Cycloalkoxy, Trifluormethoxy,
oder für C₁-C₆-Alkoxy steht, wobei C₁-C₆-Alkoxy gegebenenfalls mit ein bis fünf Fluoratomen substituiert sein kann und gegebenenfalls ein- oder zweifach, gleich oder verschieden substituiert sein kann mit Hydroxy, Chlor, Brom, Cyano, C(=O)OH, S(=O)₂-C₁-C₆-Alkyl, NH₂, NHR^{a}, N(R^{a})R^{b}, C₃-C₇-Cycloalkyl, C₁-C₄-Alkoxy, C₃-C₇-Cycloalkoxy, Trifluormethoxy,
oder für C₃-C₇-Cycloalkyl oder für C₃-C₇-Cycloalkyloxy steht, wobei C₃-C₇-Cycloalkyl und C₃-C₇-Cycloalkyloxy gegebenenfalls mit ein bis vier Fluoratomen substituiert sein können und gegebenenfalls ein- oder zweifach, gleich oder verschieden substituiert sein können mit Hydroxy, Chlor, Brom, Cyano, C(=O)R^{d}, C(=O)OH, C₁-C₆-Alkyl oder C₁-C₄-Alkoxy,
oder für 4-7-gliedriges Heterocycloalkyl steht, welches gegebenenfalls mit ein bis vier Fluoratomen substituiert sein kann und gegebenenfalls ein- oder zweifach, gleich oder verschieden substituiert sein können mit Hydroxy, Chlor, Brom, Cyano, NH₂, NHR^{a}, N(R^{a})R^{b}, C(=O)R^{d}, C(=O)OH, C₁-C₆-Alkyl, Trifluormethyl, 2,2,2-Trifluorethyl, Cyclopropyl, Cyclopropylmethyl oder C₁-C₄-Alkoxy,
oder für Phenyl oder 5- oder 6-gliedriges Heteroaryl steht, worin Phenyl und 5- oder 6-gliedriges Heteroaryl gegebenenfalls ein- bis zweimal, gleich oder verschieden substituiert sein können mit Fluor, Chlor, Brom, Hydroxy, Cyano, C(=O)OH, S(=O)₂-C₁-C₄-Alkyl, NH₂, NHR^{a}, N(R^{a})R^{b}, N(H)C(=O)R^{a}, C₁-C₄-Alkoxy, Trifluormethoxy oder C₁-C₄-Alkyl, wobei C₁-C₄-Alkyl gegebenenfalls ein- bis dreifach substituiert sein kann mit Fluor;
R⁵ für Wasserstoff, Halogen, Hydroxy, Cyano, C₁-C₄-Alkoxy, Trifluormethoxy oder C₁-C₆-Alkyl steht, worin C₁-C₆-Alkyl gegebenenfalls mit ein bis fünf Fluoratomen substituiert sein kann,
R^{6f} für Wasserstoff, Fluor, C(=O)OH, C(=O)NH₂, Trifluormethyl, Hydroxymethyl, Methoxymethyl, Cyano oder C₁-C₆-Alkyl steht,
R^{6g} für Wasserstoff, Fluor oder C₁-C₆-Alkyl steht oder R^{6f} und R^{6g} bilden gemeinsam mit dem Kohlenstoffatom, an welches sie gebunden sind, ein C₃-C₇-Cycloalkyl oder
R^{6f} und R^{6g} stehen gemeinsam für eine Oxo-Gruppe und
R^{6h} für Wasserstoff, Trifluormethyl oder C₁-C₆-Alkyl steht sowie
R⁶ⁱ für Wasserstoff oder C₁-C₆-Alkyl steht oder
R^{6h} und R⁶ⁱ stehen gemeinsam für eine Oxo-Gruppe,
R^{6j} für Wasserstoff, Fluor, NH₂, N(H)R^{a}, N(R^{a})R^{b}, C₁-C₆-Alkyl, Hydroxy, Cyano, C₁-C₄-Alkoxy, C(=O)OH, C(=O)NH₂, C(=O)N(H)R^{a}, C(=O)N(R^{a})R^{b}, Hydroxymethyl, Dimethylaminomethyl, Trifluormethyl steht,
R^{6k} für Wasserstoff, Fluor oder C₁-C₆-Alkyl steht oder
R^{6j} und R^{6k} bilden gemeinsam mit dem Kohlenstoffatom ein C₃-C₇-Cycloalkyl,
R⁶¹ für Wasserstoff oder Methyl steht,
R^{6m} für Wasserstoff oder Methyl steht,
G für -CH₂- oder -CH2CH2- steht,
n in der Formel R^{3a} für 0, 1 oder 2 steht;
n in der Formeln R^{3b} für 1 oder 2 steht.
n in der Formel R^{3c} für 0 oder 1 steht
z für eine Gruppe steht ausgewählt aus NR⁷, O, S, S(=O), S(=O)₂, S(=O)(=NH);
R⁷ für Wasserstoff, C(=O)R^{e}, C(=O)OR^{a}, C(=O)NH₂, C(=O)N(H)R^{a}, C(=O)N(R^{a})R^{b}, S(=O)₂R^{a}, S(=O)₂NH₂, S(=O)₂N(R^{a})H, S(=O)₂N(R^{a})R^{b}, S(=O)₂NHC(=O)CH₃, S(=O)₂NHC(=O)CH₂CH₃ oder C₁-C₆-Alkyl steht, wobei
C₁-C₆-Alkyl gegebenenfalls ein- bis fünfmal mit Fluoratomen substituiert sein kann und ein- bis zweifach, gleich oder verschieden substituiert sein kann mit Hydroxy, Chlor, Brom, Cyano, C(=O)R^{a}, C(=O)OH, C(=O)NH₂, C(=O)N(H)R^{a}, C(=O)N(R^{a})R^{b}, S(=O)₂-C₁-C₆-Alkyl, NH₂, NHR^{a}, N(R^{a})R^{b}, Morpholin-4-yl, 4-Methylpiperazin-1-yl, C₃-C₇-Cycloalkyl, C₁-C₄-Alkoxy oder C₃-C₇-Cycloalkoxy,
oder für C₃-C₇-Cycloalkyl, welches gegebenenfalls ein- bis viermal mit Fluoratomen substutiert sein kann und gegebenenfalls ein- bis zweifach, gleich oder verschieden substituiert sein kann mit Hydroxy, Methyl, Ethyl, Trifluormethyl oder Cyano,
oder für ein über ein Kohlenstoffatom an den Rest des Moleküls gebundenes 4-7-gliedriges Heterocycloalkyl oder für 4-7-gliedriges Heterocycloalkyl-C₁-C₄-Alkyl steht, welche gegebenenfalls ein- bis sechsmal mit Fluoratomen substituiert sein können und ein- bis dreifach, gleich oder verschieden substituiert sein können mit Hydroxy, Chlor, Brom, Cyano, C₁-C₆-Alkyl, C₁-C₆-Alkoxy, Trifluormethyl, 2,2,2-Trifluorethyl, Trifluormethoxy, Cyclopropyl, Cyclopropylmethyl,
R^{e} für C₁-C₆-Alkyl steht, wobei C₁-C₆-Alkyl gegebenenfalls ein- bis dreifach, gleich oder verschieden substituiert sein kann mit Hydroxy, Fluor, Chlor, Cyano, C(=O)R^{a}, C(=O)OH, NH₂, NHR^{a}, N(R^{a})R^{b}, C₃-C₇-Cycloalkyl, C₁-C₄-Alkoxy, Trifluormethoxy oder C₃-C₇-Cycloalkoxy oder
R^{e} für C₃-C₇-Cycloalkyl steht, wobei C₃-C₇-Cycloalkyl gegebenfalls ein- bis vierfach substituiert sein kann mit Fluor und gegebenfalls einfach mit Hydroxy.

2. Verbindungen gemäß Anspruch 1, worin R¹ Hydroxymethyl, 1-Hydroxyethyl und 2-Hydroxypropan-2-yl, bevorzugt 2-Hydroxypropan-2-yl ist.

3. Verbindungen gemäß Anspruch 1, worin
R² ein Pyridin-2-ylrest ist, der an der 6-Position mit C₁-C₆-Alkyl subsituiert ist, wobei C₁-C₆-Alkyl gegebenfalls mit bis zu 5 Fluoratomen substituiert ist oder
ein Pyridin-2-ylrest ist, der an der 6-Position mit Cyano, Chlor, Cyclopropyl, Cyclopropylmethyl, NH₂, NH(C₁-C₄-Alkyl), N(C₁-C₄-Alkyl)₂, C₁-C₄-Alkoxy, 2,2,2-Trifluorethoxy, 2-Hydroxypropan-2-yl, Morpholin-4-yl, 4-Methylpiperazin-1-yl oder Piperazin-1-yl substituiert ist.

4. Verbindungen gemäß Anspruch 1 und 3, worin
R² ein Pyridin-2-ylrest ist, der an der 6-Position mit Trifluormethyl, Difluormethyl, Methyl, 2,2,2-Trifluorethyl, 1,1-Difluorethyl, Ethyl, Isopropyl, tert-Butyl, Cyano, Chlor, Cyclopropyl, Cyclopropylmethyl, NH₂, NH(C₁-C₄-Alkyl), N(C₁-C₄-Alkyl)₂, C₁-C₄-Alkoxy, 2,2,2-Trifluorethoxy, 2-Hydroxypropan-2-yl, Morpholin-4-yl, 4-Methylpiperazin-1-yl oder Piperazin-1-yl substituiert ist.

5. Verbindungen gemäß Anspruch 1 und 4, worin R² für 6-(Trifluormethyl)pyridin-2-yl, 6-(Difluormethyl)pyridin-2-yl, 6-(1,1-Difluorethyl)pyridin-2-yl, 6-Amino-pyridin-2-yl und 6-(2-Hydroxypropan-2-yl)-pyridin-2-yl, bevorzugt für 6-(Trifluormethyl)pyridin-2-yl steht.

6. Verbindungen gemäß einem der vorangegangenen Ansprüche, worin
R¹ für C(=O)NH₂, 2-Hydroxypropan-2-yl oder Methoxy steht,
R² für 6-(Trifluormethyl)pyridin-2-yl, 6-(Difluormethyl)pyridin-2-yl, 6-Amino-pyridin-2-yl, 4-(Trifluormethyl)-1,3-thiazol-2-yl, 1-(Difluormethyl)-1H-pyrazol-3-yl,
R³ für Tetrahydro-2H-pyran-4-yl, 1,1-Dioxidotetrahydro-2H-thiopyran-4-yl, Tetrahydrofuran-3-yl, (3S)-Tetrahydrofuran-3-yl, (3R)-Tetrahydrofuran-3-yl oder (3S)-1,1-Dioxidotetra-hydrothiophen-3-yl steht oder
R³ steht für Piperidin-4-yl, 1-(2,2,2-Trifluorethyl)piperidin-4-yl, 1-Methylpiperidin-4-yl, 1-Glycoloylpiperidin-4-yl, 1'-Methyl-1,4'-bipiperidin-4-yl, 1-(Acetylsulfamoyl)-piperidin-4-yl, [2-(Dimethylamino)ethyl]piperidin-4-yl, 1-(Oxetan-3-yl)piperidin-4-yl, 1-(2-Hydroxyethyl)piperidin-4-yl oder 1-(3-Hydroxy-3-methylbutyl)piperidin-4-yl steht.

7. Verbindungen nach einem der Ansprüche 1 bis 6, nämlich
(1) N-[6-Methoxy-2-(tetrahydro-2H-pyran-4-yl)-2H-indazol-5-yl]-6-(trifluormethyl)pyridin-2-carboxamid
(2) N-[6-Methoxy-2-(piperidin-4-yl)-2H-indazol-5-yl]-6-(trifluormethyl)pyridin-2-carboxamid
(3) N-{6-Methoxy-2-[1-(2,2,2-trifluorethyl)piperidin-4-yl]-2H-indazol-5-yl}-6-(trifluormethyl)pyridin-2-carboxamid
(4) N-[6-Methoxy-2-(1-methylpiperidin-4-yl)-2H-indazol-5-yl]-6-(trifluormethyl)pyridin-2-carboxamid
(5) N-[2-(1-Glycoloylpiperidin-4-yl)-6-methoxy-2H-indazol-5-yl]-6-(trifluormethyl)pyridin-2-carboxamid
(6) N-[6-Methoxy-2-(1'-methyl-1,4'-bipiperidin-4-yl)-2H-indazol-5-yl]-6-(trifluoromethyl)-pyridin-2-carboxamid
(7) N-[6-Methoxy-2-(1-sulfamoylpiperidin-4-yl)-2H-indazol-5-yl]-6-(trifluormethyl)pyridin-2-carboxamid
(8) N- {2-[1-(Acetylsulfamoyl)piperidin-4-yl]-6-methoxy-2H-indazol-5-yl} -6-(trifluormethyl)pyridin-2-carboxamid
(9) N-(2-{1-[2-(Dimethylamino)ethyl]piperidin-4-yl}-6-methoxy-2H-indazol-5-yl)-6-(trifluormethyl)pyridin-2-carboxamid
(10) N-{6-Methoxy-2-[1-(oxetan-3-yl)piperidin-4-yl]-2H-indazol-5-yl}-6-(trifluormethyl)pyridin-2-carboxamid
(11) N-[2-(1,1-Dioxidotetrahydro-2H-thiopyran-4-yl)-6-methoxy-2H-indazol-5-yl]-6-(trifluormethyl)pyridin-2-carboxamid
(12) N-[6-Methoxy-2-(1-oxidotetrahydro-2H-thiopyran-4-yl)-2H-indazol-5-yl]-6-(trifluormethyl)pyridin-2-carboxamid
(13) N-{2-[1-(2-Hydroxyethyl)piperidin-4-yl]-6-methoxy-2H-indazol-5-yl}-6-(trifluormethyl)pyridin-2-carboxamid
(14)rel-N-{2-[(1R,4R,5S)-2-Azabicyclo[2.2.1]hept-5-yl]-6-methoxy-2H-indazol-5-yl}-6-(trifluormethyl)pyridin-2-carboxamid
(15) rel-N-{2-[(1R,4R,5R)-2-Azabicyclo[2.2.1]hept-5-yl]-6-methoxy-2H-indazol-5-yl}-6-(trifluormethyl)pyridin-2-carboxamid
(16) N-[2-(1,1-Dioxidotetrahydro-2H-thiopyran-4-yl)-6-hydroxy-2H-indazol-5-yl]-6-(trifluormethyl)pyridin-2-carboxamid
(17) N-[6-(Cyclopropylmethoxy)-2-(1,1-dioxidotetrahydro-2H-thiopyran-4-yl)-2H-indazo1-5-yl]-6-(trifluormethyl)pyridin-2-carboxamid
(18) rel-N-{6-Methoxy-2-[(1R,4R,5S)-2-methyl-2-azabicyclo[2.2.1]hept-5-yl]-2H-indazol-5-yl}-6-(trifluormethyl)pyridin-2-carboxamid
(19) rel-N-{6-Methoxy-2-[(1R,4R,5R)-2-methyl-2-azabicyclo[2.2.1]hept-5-yl]-2H-indazol-5-yl}-6-(trifluormethyl)pyridin-2-carboxamid
(20) N-[2-(1-Imino-1-oxidohexahydro-1λ⁴-thiopyran-4-yl)-6-methoxy-2H-indazol-5-yl]-6-(trifluormethyl)pyridin-2-carboxamid (Isomer 1)
(21) N-[2-(1-Imino-1-oxidohexahydro-1λ⁴-thiopyran-4-yl)-6-methoxy-2H-mdazol-5-yl]-6-(trifluormethyl)pyridin-2-carboxamid (Isomer 2)
(22) N-[6-Methoxy-2-(5-oxopyrrolidin-3-yl)-2H-indazol-5-yl]-6-(trifluormethyl)pyridin-2-carboxamid
(23) 6-(Difluormethyl)-N-[6-methoxy-2-(tetrahydro-2H-pyran-4-yl)-2H-indazol-5-yl]pyridin-2-carboxamid
(24) N-[6-Methoxy-2-(tetrahydro-2H-pyran-4-yl)-2H-indazol-5-yl]-6-(morpholin-4-yl)pyridin-2-carboxamid
(25) N-[6-Methoxy-2-(tetrahydro-2H-pyran-4-yl)-2H-indazol-5-yl]-2-methyl-1,3-thiazol-4-carboxamid
(26) 6-Amino-N-[6-methoxy-2-(tetrahydro-2H-pyran-4-yl)-2H-indazol-5-yl]pyridin-2-carboxamid
(27) 2-Isopropyl-N-[6-methoxy-2-(tetrahydro-2H-pyran-4-yl)-2H-indazol-5-yl]pyrimidin-4-carboxamid
(28) 6-(2-Hydroxypropan-2-yl)-N-[6-methoxy-2-(tetrahydro-2H-pyran-4-yl)-2H-indazol-5-yl]pyridin-2-carboxamid
(29) N-[6-Methoxy-2-(tetrahydrofuran-3-yl)-2H-indazol-5-yl]-6-(trifluormethyl)pyridin-2-carboxamid
(30) N-[6-Chlor-2-(tetrahydro-2H-pyran-4-yl)-2H-indazol-5-yl]-6-(trifluormethyl)pyridin-2-carboxamid
(31) N-[6-Chlor-2-(tetrahydro-2H-pyran-4-yl)-2H-indazol-5-yl]-6-(difluormethyl)pyridin-2-carboxamid
(32) N-[6-Chlor-2-(1,1-dioxidotetrahydro-2H-thiopyran-4-yl)-2H-indazol-5-yl]-6-(2-hydroxypropan-2-yl)pyridin-2-carboxamid
(33) Methyl-2-(tetrahydro-2H-pyran-4-yl)-5-({[6-(trifluormethyl)pyridin-2-yl]carbonyl}amino)-2H-indazol-6-carboxylat
(34) N-[6-(2-Hydroxypropan-2-yl)-2-(tetrahydro-2H-pyran-4-yl)-2H-indazol-5-yl]-6-(trifluormethyl)pyridin-2-carboxamid
(35) Methyl-2-[(3S)-tetrahydrofuran-3-yl]-5-({[6-(trifluormethyl)pyridin-2-yl]carbonyl}amino)-2H-indazol-6-carboxylat
(36) N-{6-(2-Hydroxypropan-2-yl)-2-[(3S)-tetrahydrofuran-3-yl]-2H-indazol-5-yl}-6-(trifluormethyl)pyridin-2-carboxamid
(37) Methyl-2-[(3R)-tetrahydrofuran-3-yl]-5-({[6-(trifluormethyl)pyridin-2-yl]carbonyl}amino)-2H-indazol-6-carboxylat
(38) N-{6-(2-Hydroxypropan-2-yl)-2-[(3R)-tetrahydrofuran-3-yl]-2H-indazol-5-yl}-6-(trifluormethyl)pyridin-2-carboxamid
(39) Methyl-2-[(3S)-tetrahydrothiophen-3-yl]-5-({[6-(trifluormethyl)pyridin-2-yl]carbonyl}-amino)-2H-indazol-6-carboxylat
(40) N-{6-(2-Hydroxypropan-2-yl)-2-[(3S)-tetrahydrothiophen-3-yl]-2H-indazol-5-yl}-6-(trifluormethyl)pyridin-2-carboxamid
(41) N-{2-[(3S)-1,1-Dioxidotetrahydrothiophen-3-yl]-6-(2-hydroxypropan-2-yl)-2H-indazol-5-yl}-6-(trifluormethyl)pyridin-2-carboxamid
(42) Methyl-2-(tetrahydro-2H-thiopyran-4-yl)-5-({[6-(trifluormethyl)pyridin-2-yl]carbonyl}-amino)-2H-indazol-6-carboxylat
(43) N-[6-(2-Hydroxypropan-2-yl)-2-(tetrahydro-2H-thiopyran-4-yl)-2H-indazol-5-yl]-6-(trifluormethyl)pyridin-2-carboxamid
(44) N-[2-(1,1-Dioxidotetrahydro-2H-thiopyran-4-yl)-6-(2-hydroxypropan-2-yl)-2H-indazol-5-yl]-6-(trifluormethyl)pyridin-2-carboxamid
(45) Methyl-2-(piperidin-4-yl)-5-({[6-(trifluormethyl)pyridin-2-yl]carbonyl}amino)-2H-indazol-6-carboxylat
(46) N-[6-(2-Hydroxypropan-2-yl)-2-(piperidin-4-yl)-2H-indazol-5-yl]-6-(trifluormethyl)pyridin-2-carboxamid
(47) N-[6-Methoxy-2-(tetrahydro-2H-pyran-4-yl)-2H-indazol-5-yl]-4-(trifluormethyl)-1,3-thiazol-2-carboxamid
(48) N-[6-Methoxy-2-(tetrahydro-2H-pyran-4-yl)-2H-indazol-5-yl]-3-(pyridin-4-yl)-1,2,4-oxadiazol-5-carboxamid
(49) N-[6-Methoxy-2-(tetrahydro-2H-pyran-4-yl)-2H-indazol-5-yl]-3-methyl-1,2,4-oxadiazol-5-carboxamid
(50) N-[6-Chlor-2-(tetrahydro-2H-pyran-4-yl)-2H-indazol-5-yl]-4-(trifluormethyl)-1,3-thiazol-2-carboxamid
(51) 1-(Difluormethyl)-N-[6-methoxy-2-(tetrahydro-2H-pyran-4-yl)-2H-indazol-5-yl]-1H-pyrazol-3-carboxamid
(52) N-{2-[1-(3-Hydroxy-3-methylbutyl)piperidin-4-yl]-6-methoxy-2H-indazol-5-yl}-6-(trifluormethyl)pyridin-2-carboxamid
(53) 2-(Piperidin-4-yl)-5-({[6-(trifluormethyl)pyridin-2-yl]carbonyl}amino)-2H-indazol-6-carboxamid
(54) 2-(Piperidin-4-yl)-5-({[6-(trifluormethyl)pyridin-2-yl]carbonyl}amino)-2H-indazol-6-carbonsäure
(55) Methyl-2-(1-methylpiperidin-4-yl)-5-({[6-(trifluormethyl)pyridin-2-yl]carbonyl}amino)-2H-indazol-6-carboxylat
(56)2-[1-(3-Hydroxy-3-methylbutyl)piperidin-4-yl]-5-({[6-(trifluormethyl)pyridin-2-yl]carbonyl}amino)-2H-indazol-6-carboxamid
(57) 2-(1-Methylpiperidin-4-yl)-5-({[6-(trifluormethyl)pyridin-2-yl]carbonyl}amino)-2H-indazol-6-carboxamid
(58) N-{6-Methoxy-2-[1-(2-methoxyethyl)piperidin-4-yl]-2H-indazol-5-yl}-6-(trifluormethyl)pyridin-2-carboxamid

8. Verbindung der allgemeinen Formel (I), wie in einem der Ansprüche 1 bis 7 definiert, zur Behandlung und/oder Prophylaxe von Krankheiten.

9. Verbindung der allgemeinen Formel (I), wie in einem der Ansprüche 1 bis 7 definiert, zur Verwendung in einem Verfahren zur Behandlung und/oder Prophylaxe von Tumorerkrankungen, dermatologischen Erkrankungen, gynäkologischen Erkrankungen, kardiovaskulären Erkrankungen, pulmonalen Erkrankungen, ophthalmologischen Erkrankungen, neurologischen Erkrankungen, Stoffwechselerkrankungen, Lebererkrankungen, inflammatorischen Erkrankungen, Autoimmunerkrankungen und Schmerz.

10. Verbindung der allgemeinen Formel (I), wie in einem Ansprüche 1 bis 7 definiert, zur Verwendung in einem Verfahren zur Behandlung und/oder Prophylaxe von Lymphomen, Makuladegeneration, Psoriasis, Lupus erythematodes, Multipler Sklerosis, COPD, Gicht, NASH, Leberfibrose, Insulinresistenz, des metabolischen Syndroms, von Spondyloarthritiden und rheumatoider Arthritis, Endometriose sowie Endometriose-assoziierten Schmerzen und anderen Endometriose-assoziierten Symptomen wie Dysmenorrhoe, Dyspareunie, Dysurie und Dyschezie.

11. Verbindung der allgemeinen Formel (I), wie in einem der Ansprüche 1 bis 7 definiert, zur Verwendung in einem Verfahren zur Behandlung und/oder Prophylaxe von Schmerz inklusive akutem, chronischem, entzündlichem und neuropathischem Schmerz, vorzugsweise von Hyperalgesie, Allodynie, Schmerz bei Arthritis (wie Osteoarthritis, rheumatoider Arthritis und Spondyloarthritis), prämenstruellem Schmerz, Endometriose-assoziiertem Schmerz, postoperativem Schmerz, Schmerz bei interstitieller Zystitis, CRPS (komplexes regionales Schmerzsyndrom), Trigeminusneuralgie, Schmerz bei Prostatitis, Schmerzen verursacht durch Rückenmarksverletzungen, entzündungsinduziertem Schmerz, Kreuzschmerzen, Krebsschmerzen, Chemotherapie-assoziiertem Schmerz, HIV behandlungsinduzierter Neuropathie, Verbrennungs-induziertem Schmerz und chronischem Schmerz.

12. Verwendung einer Verbindung der allgemeinen Formel (I), wie in einem der Ansprüche 1 bis 7 definiert, zur Herstellung eines Arzneimittels.

13. Verwendung gemäß Anspruch 12, wobei das Arzneimittel zur Behandlung und/oder Prophylaxe von Tumorerkrankungen, dermatologischen Erkrankungen, gynäkologischen Erkrankungen, kardiovaskulären Erkrankungen, pulmonalen Erkrankungen, ophthalmologischen Erkrankungen, neurologischen Erkrankungen, Stoffwechselerkrankungen, Lebererkrankungen, inflammatorischen Erkrankungen, Autoimmunerkrankungen und Schmerz verwendet wird.

14. Verwendung gemäß der Ansprüche 12 und 13, wobei das Arzneimittel zur Behandlung und/oder Prophylaxe von Lymphomen, Makuladegeneration, Psoriasis, Lupus erythematodes, Multipler Sklerosis, COPD, Gicht, NASH, Leberfibrose, Insulinresistenz, metabolisches Syndrom, Spondyloarthritiden und rheumatoider Arthritis, Endometriose sowie Endometriose-assoziierten Schmerzen und anderen Endometriose-assoziierten Symptomen wie Dysmenorrhoe, Dyspareunie, Dysurie und Dyschezie verwendet wird.

15. Verwendung gemäß der Ansprüche 12 und 13, wobei das Arzneimittel zur Behandlung und/oder Prophylaxe von Schmerz inklusive akutem, chronischem, entzündlichem und neuropathischem Schmerz, vorzugsweise von Hyperalgesie, Allodynie, Schmerz bei Arthritis (wie Osteoarthritis, rheumatoider Arthritis und Spondyloarthritis), prämenstruellem Schmerz, Endometriose-assoziiertem Schmerz, postoperativem Schmerz, Schmerz bei interstitieller Zystitis, CRPS (komplexes regionales Schmerzsyndrom), Trigeminusneuralgie, Schmerz bei Prostatitis, Schmerzen verursacht durch Rückenmarksverletzungen, entzündungsinduziertem Schmerz, Kreuzschmerzen, Krebsschmerzen, Chemotherapie-assoziiertem Schmerz, HIV behandlungsinduzierter Neuropathie, Verbrennungs-induziertem Schmerz und chronischem Schmerz verwendet wird.

16. Arzneimittel enthaltend eine Verbindung der Formel (I), wie in einem der Ansprüche 12 bis 13 definiert, in Kombination mit einem inerten, nicht-toxischen, pharmazeutisch geeigneten Hilfsstoff.

## Claims

1. Compounds of the general formula (I) 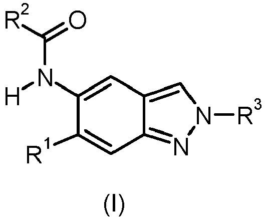 in which
R¹ is halogen, cyano, C(=O)OH, C(=O)OR^{a}, C(=O)NH₂, C(=O)N(H)R^{a}, C(=O)N(R^{a})R^{b}, C(=O)R^{d}, hydroxyl or C₁-C₆-alkyl, where the C₁-C₆-alkyl radical can optionally be mono- or polysubstituted identically or differently by
hydroxyl, halogen, cyano, C(=O)OH, C(=O)OR^{a}, S(=O)₂-C₁-C₆-alkyl, NH₂, NHR^{a}, N(R^{a})R^{b}, an optionally mono- to hexa-fluorine-substituted C₁-C₆-alkoxy or C₃-C₇-cycloalkoxy, a 4- to 7-membered heterocycloalkyl optionally mono- to trisubstituted identically or differently by R^{c},
or is C₁-C₆-alkoxy, where the C₁-C₆-alkoxy radical may optionally be mono- or polysubstituted identically or differently by
hydroxyl, halogen, cyano, C(=O)OH, C(=O)OR^{a}, S(=O)₂-C₁-C₆-alkyl, NH₂, NHR^{a}, N(R^{a})R^{b}, an optionally mono- to tetra-fluorine-substituted C₃-C₇-cycloalkyl, an optionally mono- to penta-fluorine-substituted C₁-C₆-alkoxy, an optionally mono- to tetra-fluorine-substituted C₃-C₇-cycloalkoxy, a 4- to 7-membered heterocycloalkyl optionally mono- or polysubstituted identically or differently by R^{c},
or is C₃-C₇-cycloalkyloxy or 4- to 7-membered heterocycloalkyloxy in which C₃-C₇-cycloalkyloxy and 4- to 7-membered heterocycloalkyloxy may optionally be mono- or polysubstituted identically or differently by hydroxyl, fluorine, cyano, C₁-C₆-alkyl or C₁-Cₑ-alkoxy;
R^{a} is C₁-C₆-alkyl, C₃-C₇-cycloalkyl, 4- to 7-membered heterocycloalkyl,
in which C₁-C₆-alkyl, C₃-C₇-cycloalkyl and 4- to 7-membered heterocycloalkyl may optionally be mono- or polysubstituted identically or differently by fluorine, hydroxyl, cyano, C₁-C₄-alkyl, C₁-C₄-alkoxy or C₃-C₇-cycloalkyl;
R^{b} is C₁-C₆-alkyl or C₃-C₇-cycloalkyl;
or R^{a} and R^{b} together with the nitrogen atom form a 5- or 6-membered heterocycle which may optionally be mono- or disubstituted identically or differently by hydroxyl, halogen, cyano, or C₁-C₆-alkyl;
R^{c} is hydroxyl, fluorine, chlorine, cyano, C₁-C₄-alkyl or C₁-C₄-alkoxy;
R^{d} is hydrogen, C₃-C₇-cycloalkyl, or C₁-C₆-alkyl which may optionally be substituted by a hydroxyl group;
R² is 5-membered heteroaryl which may be monosubstituted by R⁴ and monosubstituted by R⁵ or
R² is 6-membered heteroaryl which may be monosubstituted by R⁴ and mono- or disubstituted identically or differently by R⁵,
R³ is a group selected from: 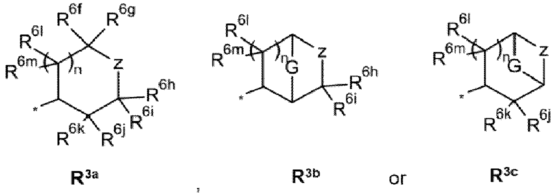
where * represents the bonding site of the group to the rest of the molecule;
R⁴ is hydrogen, halogen, hydroxyl, C(=O)OH, cyano, NH₂, NHR^{a}, N(R^{a})R^{b}, C(=O)R^{a}, N(H)C(=O)R^{a}, C(=O)NH₂, C(=O)N(H)R^{a}, C(=O)N(R^{a})R^{b}, S(=O)R^{a}, S(=O)₂R^{a}, S(=O)₂NH₂, S(=O)₂NHR^{a} or S(=O)₂N(R^{a})R^{b},
or is C₁-C₆-alkyl, where C₁-C₆-alkyl may optionally be substituted by one to five fluorine atoms and may optionally be mono- or disubstituted identically or differently by hydroxyl, bromine, chlorine, cyano, C(=O)OH, S(=O)₂-C₁-C₆-alkyl, NH₂, NHR^{a}, N(R^{a})R^{b}, C₃-C₇-cycloalkyl, C₁-C₄-alkoxy, C₃-C₇-cycloalkoxy, trifluoromethoxy,
or is C₁-C₆-alkoxy, where C₁-C₆-alkoxy may optionally be substituted by one to five fluorine atoms and may optionally be mono- or disubstituted identically or differently by hydroxyl, chlorine, bromine, cyano, C(=O)OH, S(=O)₂-C₁-C₆-alkyl, NH₂, NHR^{a}, N(R^{a})R^{b}, C₃-C₇-cycloalkyl, C₁-C₄-alkoxy, C₃-C₇-cycloalkoxy, trifluoromethoxy,
or is C₃-C₇-cycloalkyl or is C₃-C₇-cycloalkyloxy, where C₃-C₇-cycloalkyl and C₃-C₇-cycloalkyloxy may optionally be substituted by one to four fluorine atoms and may optionally be mono- or disubstituted identically or differently by hydroxyl, chlorine, bromine, cyano, C(=O)R^{d}, C(=O)OH, C₁-C₆-alkyl or C₁-C₄-alkoxy,
or is 4-7-membered heterocycloalkyl which may optionally be substituted by one to four fluorine atoms and may optionally be mono- or disubstituted identically or differently by hydroxyl, chlorine, bromine, cyano, NH₂, NHR^{a}, N(R^{a})R^{b}, C(=O)R^{d}, C(=O)OH, C₁-C₆-alkyl, trifluoromethyl, 2,2,2-trifluoroethyl, cyclopropyl, cyclopropylmethyl or C₁-C₄-alkoxy,
or is phenyl or 5- or 6-membered heteroaryl, in which phenyl and 5- or 6-membered heteroaryl may optionally be mono- to disubstituted identically or differently by fluorine, chlorine, bromine, hydroxyl, cyano, C(=O)OH, S(=O)₂-C₁-C₄-alkyl, NH₂, NHR^{a}, N(R^{a})R^{b}, N(H)C(=O)R^{a}, C₁-C₄-alkoxy, trifluoromethoxy or C₁-C₄-alkyl, where C₁-C₄-alkyl may optionally be mono- to trisubstituted by fluorine;
R⁵ is hydrogen, halogen, hydroxyl, cyano, C₁-C₄-alkoxy, trifluoromethoxy or C₁-C₆-alkyl, in which C₁-C₆-alkyl may optionally be substituted by one to five fluorine atoms,
R^{6f} is hydrogen, fluorine, C(=O)OH, C(=O)NH₂, trifluoromethyl, hydroxymethyl, methoxymethyl, cyano or C₁-C₆-alkyl,
R^{6g} is hydrogen, fluorine or C₁-C₆-alkyl or R^{6f} and R^{6g} together with the carbon atom to which they are bonded form a C₃-C₇-cycloalkyl or
R^{6f} and R^{6g} together are an oxo group and
R^{6h} is hydrogen, trifluoromethyl or C₁-C₆-alkyl and
R⁶ⁱ is hydrogen or C₁-C₆-alkyl or
R^{6h} and R⁶ⁱ together are an oxo group,
R^{6j} is hydrogen, fluorine, NH₂, N(H)R^{a}, N(R^{a})R^{b}, C₁-C₆-alkyl, hydroxyl, cyano, C₁-C₄-alkoxy, C(=O)OH, C(=O)NH₂, C(=O)N(H) R^{a}, C(=O)N(R^{a})R^{b}, hydroxymethyl, dimethylaminomethyl, trifluoromethyl,
R^{6k} is hydrogen, fluorine or C₁-C₆-alkyl or
R^{6j} and R^{6k} together with the carbon atom form a C₃-C₇-cycloalkyl,
R^{6l} is hydrogen or methyl,
R^{6m} is hydrogen or methyl,
G is -CH₂- or -CH₂CH₂-,
n in the formula R^{3a} is 0, 1 or 2,
n in the formula R^{3b} is 1 or 2,
n in the formula R^{3e} is 0 or 1,
z is a group selected from NR⁷, O, S, S(=O), S(=O)₂, S(=O)(=NH);
R⁷ is hydrogen, C(=O)R^{e}, C(=O)OR^{a}, C(=O)NH₂, C(=O)N(H)R^{a}, C(=O)N(R^{a})R^{b}, S(=O)₂R^{a}, S(=O)₂NH₂, S(=O)₂N(R^{a})H, S(=O)₂N(R^{a})R^{b}, S(=O)₂NHC(=O)CH₃, S(=O)₂NHC (=O)CH₂CH₃ or C₁-C₆-alkyl, where C₁-C₆-alkyl may optionally be mono- to pentasubstituted by fluorine atoms and mono- to disubstituted identically or differently by hydroxyl, chlorine, bromine, cyano, C(=O)R^{a}, C(=O)OH, C(=O)NH₂, C(=O)N(H)R^{a}, C(=O)N(R^{a})R^{b}, S(=O)₂-C₁-C₆-alkyl, NH₂, NHR^{a}, N(R^{a})R^{b}, morpholin-4-yl, 4-methylpiperazin-1-yl, C₃-C₇-cycloalkyl, C₁-C₄-alkoxy or C₃-C₇-cycloalkoxy,
or is C₃-C₇-cycloalkyl which may optionally be mono- to tetrasubstituted by fluorine atoms and may optionally be mono- to disubstituted identically or differently by hydroxyl, methyl, ethyl, trifluoromethyl or cyano,
or is a 4-7-membered heterocycloalkyl bonded to the rest of the molecule by a carbon atom or is 4-7-membered heterocycloalkyl-C₁-C₄-alkyl which may optionally be mono- to hexasubstituted by fluorine atoms and mono- to trisubstituted identically or differently by hydroxyl, chlorine, bromine, cyano, C₁-C₆-alkyl, C₁-C₆-alkoxy, trifluoromethyl, 2,2,2-trifluoroethyl, trifluoromethoxy, cyclopropyl, cyclopropylmethyl,
R^{e} is C₁-C₆-alkyl, where C₁-C₆-alkyl may optionally be mono- to trisubstituted identically or differently by hydroxyl, fluorine, chlorine, cyano, C(=O)R^{a}, C(=O)OH, NH₂, NHR^{a}, N(R^{a})R^{b}, C₃-C₇-cycloalkyl, C₁-C₄-alkoxy, trifluoromethoxy or C₃-C₇-cycloalkoxy or
R^{e} is C₃-C₇-cycloalkyl, where C₃-C₇-cycloalkyl may optionally be mono- to tetrasubstituted by fluorine and may optionally be monosubstituted by hydroxyl.

2. Compounds according to Claim 1, in which R¹ is hydroxymethyl, 1-hydroxyethyl and 2-hydroxypropan-2-yl, preferably 2-hydroxypropan-2-yl.

3. Compounds according to Claim 1, in which
R² is a pyridin-2-yl radical substituted at the 6 position by C₁-C₆-alkyl, where C₁-C₆-alkyl is optionally substituted by up to 5 fluorine atoms or
is a pyridin-2-yl radical substituted at the 6 position by cyano, chlorine, cyclopropyl, cyclopropylmethyl, NH₂, NH(C₁-C₄-alkyl), N(C₁-C₄-alkyl)₂, C₁-C₄-alkoxy, 2,2,2-trifluoroethoxy, 2-hydroxypropan-2-yl, morpholin-4-yl, 4-methylpiperazin-1-yl or piperazin-1-yl.

4. Compounds according to Claim 1 and 3, in which
R² is a pyridin-2-yl radical substituted at the 6 position by trifluoromethyl, difluoromethyl, methyl, 2,2,2-trifluoroethyl, 1,1-difluoroethyl, ethyl, isopropyl, tert-butyl, cyano, chlorine, cyclopropyl, cyclopropylmethyl, NH₂, NH(C₁-C₄-alkyl), N(C₁-C₄-alkyl)₂, C₁-C₄-alkoxy, 2,2,2-trifluoroethoxy, 2-hydroxypropan-2-yl, morpholin-4-yl, 4-methylpiperazin-1-yl or piperazin-1-yl.

5. Compounds according to Claim 1 and 4, in which R² is 6-(trifluoromethyl)pyridin-2-yl, 6-(difluoromethyl)-pyridin-2-yl, 6-(1,1-difluoroethyl)pyridin-2-yl, 6-aminopyridin-2-yl and 6-(2-hydroxypropan-2-yl)-pyridin-2-yl, preferably 6-(trifluoromethyl)pyridin-2-yl.

6. Compounds according to any of the preceding claims, in which
R¹ is C(=O)NH₂, 2-hydroxypropan-2-yl or methoxy,
R² is 6-(trifluoromethyl)pyridin-2-yl, 6-(difluoromethyl)-pyridin-2-yl, 6-aminopyridin-2-yl, 4-(trifluoromethyl)-1,3-thiazol-2-yl, 1-(difluoromethyl)-1H-pyrazol-3-yl,
R³ is tetrahydro-2H-pyran-4-yl, 1,1-dioxidotetra-hydro-2H-thiopyran-4-yl, tetrahydrofuran-3-yl, (3S)-tetrahydrofuran-3-yl, (3R)-tetrahydrofuran-3-yl or (3S)-1,1-dioxidotetrahydrothio-phen-3-yl or
R³ is piperidin-4-yl, 1-(2,2,2-trifluoroethyl)-piperidin-4-yl, 1-methylpiperidin-4-yl, 1-glycoloylpiperidin-4-yl,1'-methyl-1,4'-bi-piperidin-4-yl, 1-(acetylsulphamoyl)piperidin-4-yl, [2-(dimethylamino)ethyl]piperidin-4-yl, 1-(oxetan-3-yl)piperidin-4-yl, 1-(2-hydroxyethyl)piperidin-4-yl or 1-(3-hydroxy-3-methylbutyl)piperidin-4-yl.

7. Compounds according to any of Claims 1 to 6, as follows:
(1) N-[6-methoxy-2-(tetrahydro-2H-pyran-4-yl)-2H-indazol-5-yl]-6-(trifluoromethyl)pyridine-2-carboxamide
(2) N-[6-methoxy-2-(piperidin-4-yl)-2H-indazol-5-yl]-6-(trifluoromethyl)pyridine-2-carboxamide
(3) N-{6-methoxy-2-[1-(2,2,2-trifluoroethyl)piperidin-4-yl]-2H-indazol-5-yl}-6-(trifluoromethyl)pyridine-2-carboxamide
(4) N-[6-methoxy-2-(1-methylpiperidin-4-yl)-2H-indazol-5-yl]-6-(trifluoromethyl)pyridine-2-carboxamide
(5) N-[2-(1-glycoloylpiperidin-4-yl)-6-methoxy-2H-indazol-5-yl]-6-(trifluoromethyl)pyridine-2-carboxamide
(6) N-[6-methoxy-2-(1'-methyl-1,4'-bipiperidin-4-yl)-2H-indazol-5-yl]-6-(trifluoromethyl)-pyridine-2-carboxamide
(7) N-[6-methoxy-2-(1-sulphamoylpiperidin-4-yl)-2H-indazol-5-yl]-6-(trifluoromethyl)pyridine-2-carboxamide
(8) N-{2-[1-(acetylsulphamoyl)piperidin-4-yl]-6-methoxy-2H-indazol-5-yl}-6-(trifluoromethyl)-pyridine-2-carboxamide
(9) N-(2-{1-[2-(dimethylamino)ethyl]piperidin-4-yl}-6-methoxy-2H-indazol-5-yl)-6-(trifluoromethyl)pyridine-2-carboxamide
(10) N-{6-methoxy-2-[1-(oxetan-3-yl)piperidin-4-yl]-2H-indazol-5-yl}-6-(trifluoromethyl)pyridine-2-carboxamide
(11) N-[2-(1,1-dioxidotetrahydro-2H-thiopyran-4-yl)-6-methoxy-2H-indazol-5-yl]-6-(trifluoromethyl)--pyridine-2-carboxamide
(12) N-[6-methoxy-2-(1-oxidotetrahydro-2H-thiopyran-4-yl)-2H-indazol-5-yl]-6-(trifluoromethyl)-pyridine-2-carboxamide
(13) N-{2-[1-(2-hydroxyethyl)piperidin-4-yl]-6-methoxy-2H-indazol-5-yl}-6-(trifluoromethyl)-pyridine-2-carboxamide
(14) rel-N-{2-[(1R,4R,5S)-2-azabicyclo[2.2.1]hept-5-yl]-6-methoxy-2H-indazol-5-yl}-6-(trifluoromethyl)pyridine-2-carboxamide
(15) rel-N-{2-[(1R,4R,5R)-2-azabicyclo[2.2.1]hept-5-yl]-6-methoxy-2H-indazol-5-yl}-6-(trifluoromethyl)pyridine-2-carboxamide
(16) N-[2-(1,1-dioxidotetrahydro-2H-thiopyran-4-yl)-6-hydroxy-2H-indazol-5-yl]-6-(trifluoromethyl)-pyridine-2-carboxamide
(17) N-[6-(cyclopropylmethoxy)-2-(1,1-dioxidotetrahydro-2H-thiopyran-4-yl)-2H-indazol-5-yl]-6-(trifluoromethyl)pyridine-2-carboxamide
(18) rel-N-{6-methoxy-2-[(1R,4R,5S)-2-methyl-2-azabicyclo[2.2.1]hept-S-yl]-2H-indazol-S-yl}-6-(trifluoromethyl)pyridine-2-carboxamide
(19) rel-N-{6-methoxy-2-[(1R,4R,5R)-2-methyl-2-azabicyclo[2.2.1]hept-5-yl]-2H-indazol-5-yl}-6-(trifluoromethyl)pyridine-2-carboxamide
(20) N-[2-(1-imino-1-oxidohexahydro-1λ⁴-thiopyran-4-yl)-6-methoxy-2H-indazol-5-yl]-6-(trifluoromethyl)pyridine-2-carboxamide (isomer 1)
(21) N-[2-(1-imino-1-oxidohexahydro-1λ⁴-thiopyran-4-yl)-6-methoxy-2H-indazol-5-yl]-6-(trifluoromethyl)pyridine-2-carboxamide (isomer 2)
(22) N-[6-methoxy-2-(5-oxopyrrolidin-3-yl)-2H-indazol-5-yl]-6-(trifluoromethyl)pyridine-2-carboxamide
(23) 6-(difluoromethyl)-N-[6-methoxy-2-(tetrahydro-2H-pyran-4-yl)-2H-indazol-5-yl]pyridine-2-carboxamide
(24) N-[6-methoxy-2-(tetrahydro-2H-pyran-4-yl)-2H-indazol-5-yl]-6-(morpholin-4-yl)pyridine-2-carboxamide
(25) N-[6-methoxy-2-(tetrahydro-2H-pyran-4-yl)-2H-indazol-5-yl]-2-methyl-1,3-thiazole-4-carboxamide
(26) 6-amino-N-[6-methoxy-2-(tetrahydro-2H-pyran-4-yl)-2H-indazol-5-yl]pyridine-2-carboxamide
(27) 2-isopropyl-N-[6-methoxy-2-(tetrahydro-2H-pyran-4-yl)-2H-indazol-5-yl]pyrimidine-4-carboxamide
(28) 6-(2-hydroxypropan-2-yl)-N-[6-methoxy-2-(tetrahydro-2H-pyran-4-yl)-2H-indazol-5-yl]pyridine-2-carboxamide
(29) N-[6-methoxy-2-(tetrahydrofuran-3-yl)-2H-indazol-5-yl]-6-(trifluoromethyl)pyridine-2-carboxamide
(30) N-[6-chloro-2-(tetrahydro-2H-pyran-4-yl)-2H-indazol-5-yl]-6-(trifluoromethyl)pyridine-2-carboxamide
(31) N-[6-chloro-2-(tetrahydro-2H-pyran-4-yl)-2H-indazol-5-yl]-6-(difluoromethyl)pyridine-2-carboxamide
(32) N-[6-chloro-2-(1,1-dioxidotetrahydro-2H-thiopyran-4-yl)-2H-indazol-5-yl]-6-(2-hydroxypropan-2-yl)pyridine-2-carboxamide
(33) methyl 2-(tetrahydro-2H-pyran-4-yl)-5-({[6-(trifluoromethyl)pyridin-2-yl]carbonyl}amino)-2H-indazole-6-carboxylate
(34) N-[6-(2-hydroxypropan-2-yl)-2-(tetrahydro-2H-pyran-4-yl)-2H-indazol-5-yl]-6-(trifluoromethyl)pyridine-2-carboxamide
(35) methyl 2-[(3S)-tetrahydrofuran-3-yl]-5-({[6-(trifluoromethyl)pyridin-2-yl]carbonyl}amino)-2H-indazole-6-carboxylate
(36) N-{6-(2-hydroxypropan-2-yl)-2-[(3S)-tetrahydrofuran-3-yl]-2H-indazol-5-yl}-6-(trifluoromethyl)pyridine-2-carboxamide
(37) methyl 2-[(3R)-tetrahydrofuran-3-yl]-5-({[6-(trifluoromethyl)pyridin-2-yl]carbonyl}amino)-2H-indazole-6-carboxylate
(38) N-{6-(2-hydroxypropan-2-yl)-2-[(3R)-tetrahydrofuran-3-yl]-2H-indazol-5-yl}-6-(trifluoromethyl)pyridine-2-carboxamide
(39) methyl 2-[(3S)-tetrahydrothiophen-3-yl]-5-({[6-(trifluoromethyl)pyridin-2-yl]carbonyl}amino)-2H-indazole-6-carboxylate
(40) N-{6-(2-hydroxypropan-2-yl)-2-[(3S)-tetrahydrothiophen-3-yl]-2H-indazol-5-yl}-6-(trifluoromethyl)pyridine-2-carboxamide
(41) N-{2-[(3S)-1,1-dioxidotetrahydrothiophen-3-yl]-6-(2-hydroxypropan-2-yl)-2H-indazol-5-yl}-6-(trifluoromethyl)pyridine-2-carboxamide
(42) methyl 2-(tetrahydro-2H-thiopyran-4-yl)-5-({[6-(trifluoromethyl)pyridin-2-yl]carbonyl}amino)-2H-indazole-6-carboxylate
(43) N-[6-(2-hydroxypropan-2-yl)-2-(tetrahydro-2H-thiopyran-4-yl)-2H-indazol-5-yl]-6-(trifluoromethyl)pyridine-2-carboxamide
(44) N-[2-(1,1-dioxidotetrahydro-2H-thiopyran-4-yl)-6-(2-hydroxypropan-2-yl)-2H-indazol-5-yl]-6-(trifluoromethyl)pyridine-2-carboxamide
(45) methyl 2-(piperidin-4-yl)-5-({[6-(trifluoromethyl)pyridin-2-yl]carbonyl}amino)-2H-indazole-6-carboxylate
(46) N-[6-(2-hydroxypropan-2-yl)-2-(piperidin-4-yl)-2H-indazol-5-yl]-6-(trifluoromethyl)pyridine-2-carboxamide
(47) N-[6-methoxy-2-(tetrahydro-2H-pyran-4-yl)-2H-indazol-5-yl]-4-(trifluoromethyl)-1,3-thiazole-2-carboxamide
(48) N-[6-methoxy-2-(tetrahydro-2H-pyran-4-yl)-2H-indazol-5-yl]-3-(pyridin-4-yl)-1,2,4-oxadiazole-5-carboxamide
(49) N-[6-methoxy-2-(tetrahydro-2H-pyran-4-yl)-2H-indazol-5-yl]-3-methyl-1,2,4-oxadiazole-5-carboxamide
(50) N-[6-chloro-2-(tetrahydro-2H-pyran-4-yl)-2H-indazol-5-yl]-4-(trifluoromethyl)-1,3-thiazole-2-carboxamide
(51) 1-(difluoromethyl)-N-[6-methoxy-2-(tetrahydro-2H-pyran-4-yl)-2H-indazol-5-yl]-1H-pyrazole-3-carboxamide
(52) N-{2-[1-(3-hydroxy-3-methylbutyl)piperidin-4-yl]-6-methoxy-2H-indazol-5-yl}-6-(trifluoromethyl)pyridine-2-carboxamide
(53) 2-(piperidin-4-yl)-5-({[6-(trifluoromethyl)-pyridin-2-yl]carbonyl}amino)-2H-indazole-6-carboxamide
(54) 2-(piperidin-4-yl)-5-({[6-(trifluoromethyl)-pyridin-2-yl]carbonyl}amino)-2H-indazole-6-carboxylic acid
(55) methyl 2-(1-methylpiperidin-4-yl)-5-({[6-(trifluoromethyl)pyridin-2-yl]carbonyl}amino)-2H-indazole-6-carboxylate
(56) 2-[1-(3-hydroxy-3-methylbutyl)piperidin-4-yl]-5-({[6-(trifluoromethyl)pyridin-2-yl]carbonyl}-amino)-2H-indazole-6-carboxamide
(57) 2-(1-methylpiperidin-4-yl)-5-({[6-(trifluoromethyl)pyridin-2-yl]carbonyl}amino)-2H-indazole-6-carboxamide
(58) N-{6-methoxy-2-[1-(2-methoxyethyl)piperidin-4-yl]-2H-indazol-5-yl}-6-(trifluoromethyl)-pyridine-2-carboxamide

8. Compound of the general formula (I) as defined in any of Claims 1 to 7 for treatment and/or prophylaxis of diseases.

9. Compound of the general formula (I) as defined in any of Claims 1 to 7 for use in a method for treatment and/or prophylaxis of neoplastic disorders, dermatological disorders, gynaecological disorders, cardiovascular disorders, pulmonary disorders, ophthalmological disorders, neurological disorders, metabolic disorders, hepatic disorders, inflammatory disorders, autoimmune disorders and pain.

10. Compound of the general formula (I) as defined in any of Claims 1 to 7 for use in a method for treatment and/or prophylaxis of lymphoma, macular degeneration, psoriasis, lupus erythematosus, multiple sclerosis, COPD, gout, NASH, hepatic fibrosis, insulin resistance, metabolic syndrome, spondyloarthritis and rheumatoid arthritis, endometriosis and endometriosis-related pain and other endometriosis-associated symptoms such as dysmenorrhoea, dyspareunia, dysuria and dyschezia.

11. Compound of the general formula (I) as defined in any of Claims 1 to 7 for use in a method for treatment and/or prophylaxis of pain including acute, chronic, inflammatory and neuropathic pain, preferably of hyperalgesia, allodynia, pain from arthritis (such as osteoarthritis, rheumatoid arthritis and spondyloarthritis), premenstrual pain, endometriosis-associated pain, post-operative pain, pain from interstitial cystitis, CRPS (complex regional pain syndrome), trigeminal neuralgia, pain from prostatitis, pain caused by spinal cord injuries, inflammation-induced pain, lower back pain, cancer pain, chemotherapy-associated pain, HIV treatment-induced neuropathy, burn-induced pain and chronic pain.

12. Use of a compound of the general formula (I) as defined in any of Claims 1 to 7 for production of a medicament.

13. Use according to Claim 12, wherein the medicament is used for treatment and/or prophylaxis of neoplastic disorders, dermatological disorders, gynaecological disorders, cardiovascular disorders, pulmonary disorders, ophthalmological disorders, neurological disorders, metabolic disorders, hepatic disorders, inflammatory disorders, autoimmune disorders and pain.

14. Use according to Claims 12 and 13, wherein the medicament is used for treatment and/or prophylaxis of lymphoma, macular degeneration, psoriasis, lupus erythematosus, multiple sclerosis, COPD, gout, NASH, hepatic fibrosis, insulin resistance, metabolic syndrome, spondyloarthritis and rheumatoid arthritis, endometriosis and endometriosis-related pain and other endometriosis-associated symptoms such as dysmenorrhoea, dyspareunia, dysuria and dyschezia.

15. Use according to Claims 12 and 13, wherein the medicament is used for treatment and/or prophylaxis of pain including acute, chronic, inflammatory and neuropathic pain, preferably of hyperalgesia, allodynia, pain from arthritis (such as osteoarthritis, rheumatoid arthritis and spondyloarthritis), premenstrual pain, endometriosis-associated pain, post-operative pain, pain from interstitial cystitis, CRPS (complex regional pain syndrome), trigeminal neuralgia, pain from prostatitis, pain caused by spinal cord injuries, inflammation-induced pain, lower back pain, cancer pain, chemotherapy-associated pain, HIV treatment-induced neuropathy, burn-induced pain and chronic pain.

16. Medicament comprising a compound of the formula (I) as defined in any of Claims 12 to 13 in combination with an inert, non-toxic, pharmaceutically suitable excipient.

## Revendications

1. Composés de formule générale (I) 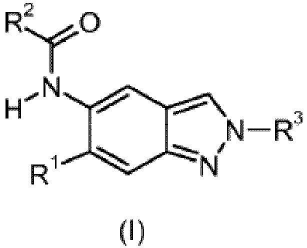 dans laquelle
R¹ représente halogène, cyano, C(=O)OH, C(=O)OR^{a}, C(=O)NH₂, C(=O)N(H)R^{a}, C(=O)N(R^{a})R^{b}, C(=O)R^{d}, hydroxy ou C₁-C₆-alkyle, le radical C₁-C₆-alkyle pouvant éventuellement être substitué une ou plusieurs fois, de manière identique ou différente par
hydroxy, halogène, cyano, C(=O)OH, C(=O)OR^{a}, S(=O)₂-C₁-C₆-alkyle, NH₂, NHR^{a}, N(R^{a})R^{b}, un C₁-C₆-alcoxy ou C₃-C₇-cycloalcoxy éventuellement substitué une à six fois par fluor, un hétérocycloalkyle à 4 à 7 chaînons éventuellement substitué une à trois fois, de manière identique ou différente par R^{c},
ou représente C₁-C₆-alcoxy, le radical C₁-C₆-alcoxy pouvant éventuellement être substitué une ou plusieurs fois, de manière identique ou différente par
hydroxy, halogène, cyano, C(=O)OH, C(=O)OR^{a}, S(=O)₂-C₁-C₆-alkyle, NH₂, NHR^{a}, N(R^{a})R^{b}, un C₃-C₇-cycloalkyle éventuellement substitué une à quatre fois par fluor, un C₁-C₆-alcoxy éventuellement substitué une à cinq fois par fluor, un C₃-C₇-cycloalcoxy éventuellement substitué une à quatre fois par fluor, un hétérocycloalkyle à 4 à 7 chaînons éventuellement substitué une ou plusieurs fois, de manière identique ou différente par R^{c},
ou représente C₃-C₇-cycloalkyloxy ou hétérocycloalkyloxy à 4 à 7 chaînons, où C₃-C₇-cycloalkyloxy et hétérocycloalkyloxy à 4 à 7 chaînons peuvent éventuellement être substitués une ou plusieurs fois, de manière identique ou différente par hydroxy, fluor, cyano, C₁-C₆-alkyle ou C₁-C₆-alcoxy ;
R^{a} représente C₁-C₆-alkyle, C₃-C₇-cycloalkyle, hétérocycloalkyle à 4 à 7 chaînons,
où C₁-C₆-alkyle, C₃-C₇-cycloalkyle et hétérocycloalkyle à 4 à 7 chaînons peuvent éventuellement être substitués une ou plusieurs fois, de manière identique ou différente par fluor, hydroxy, cyano, C₁-C₄-alkyle, C₁-C₄-alcoxy ou C₃-C₇-cycloalkyle ;
R^{b} représente C₁-C₆-alkyle ou C₃-C₇-cycloalkyle ;
ou R^{a} et R^{b} forment conjointement avec l'atome d'azote un hétérocycle à 5 ou 6 chaînons, qui peut éventuellement être substitué une ou deux fois, de manière identique ou différente par hydroxy, halogène, cyano ou C₁-C₆-alkyle ;
R^{c} représente hydroxy, fluor, chlore, cyano, C₁-C₄-alkyle ou C₁-C₄-alcoxy ;
R^{d} représente hydrogène, C₃-C₇-cycloalkyle ou représente C₁-C₆-alkyle, qui peut éventuellement être substitué par un groupe hydroxy ;
R² représente hétéroaryle à 5 chaînons, qui peut être substitué une fois par R⁴ et une fois par R⁵ ou
R² représente hétéroaryle à 6 chaînons, qui peut être substitué une fois par R⁴ et une ou deux fois, de manière identique ou différente, par R⁵,
R³ représente un groupe choisi parmi : 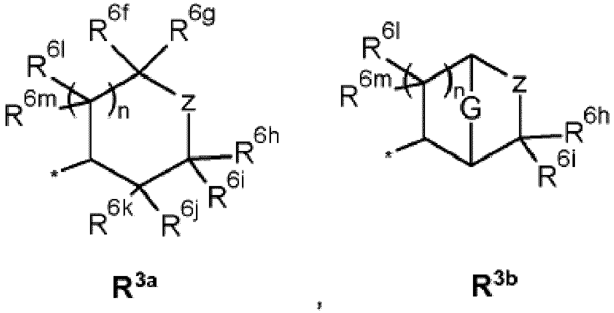 et 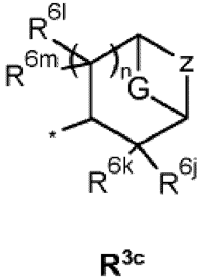
* représentant le site de liaison du groupe avec le reste de la molécule ;
R⁴ représente hydrogène, halogène, hydroxy, C(=O)OH, cyano, NH₂, NHR^{a}, N(R^{a})R^{b}, C(=O)R^{a}, N(H)C(=O)R^{a}, C(=O)NH₂, C(=O)N(H)R^{a}, C(=O)N(R^{a})R^{b}, S(=O)R^{a}, S(=O)₂R^{a}, S(=O)₂NH₂, S(=O)₂NHR^{a} ou S(=O)₂N(R^{a})R^{b},
ou represente C₁-C₆-alkyle, C₁-C₆-alkyle pouvant éventuellement être substitué par un à cinq atomes de fluor et pouvant éventuellement être substitué une ou deux fois, de manière identique ou différente par hydroxy, brome, chlore, cyano, C(=O)OH, S(=O)₂-C₁-C₆-alkyle, NH₂,
NHR^{a}, N(R^{a})R^{b}, C₃-C₇-cycloalkyle, C₁-C₄-alcoxy, C₃-C₇-cycloalcoxy, trifluorométhoxy,
ou représente C₁-C₆-alcoxy, C₁-C₆-alcoxy pouvant éventuellement être substitué par un à cinq atomes de fluor et pouvant éventuellement être substitué une ou deux fois, de manière identique ou différente par hydroxy, chlore, brome, cyano, C(=O)OH, S(=O)₂-C₁-C₆-alkyle, NH₂, NHR^{a}, N(R^{a})R^{b}, C₃-C₇-cycloalkyle, C₁-C₄-alcoxy, C₃-C₇-cycloalcoxy, trifluorométhoxy,
ou représente C₃-C₇-cycloalkyle ou C₃-C₇-cycloalkyloxy, C₃-C₇-cycloalkyle et C₃-C₇-cycloalkyloxy pouvant éventuellement être substitués par un à quatre atomes de fluor et pouvant éventuellement être substitués une ou deux fois, de manière identique ou différente par hydroxy, chlore, brome, cyano, C(=O)R^{d}, C(=O)OH, C₁-C₆-alkyle ou C₁-C₄-alcoxy,
ou représente hétérocycloalkyle à 4 à 7 chaînons, qui peut éventuellement être substitué par un à quatre atomes de fluor et peut éventuellement être substitué une ou deux fois, de manière identique ou différente par hydroxy, chlore, brome, cyano, NH₂, NHR^{a}, N(R^{a})R^{b}, C(=O)R^{d}, C(=O)OH, C₁-C₆-alkyle, trifluorométhyle, 2,2,2-trifluoroéthyle, cyclopropyle, cyclopropylméthyle ou C₁-C₄-alcoxy,
ou représente phényle ou hétéroaryle à 5 ou 6 chaînons, où phényle et hétéroaryle à 5 ou 6 chaînons peuvent être éventuellement substitués une à deux fois, de manière identique ou différente, par fluor, chlore, brome, hydroxy, cyano, C(=O)OH, S(=O)₂-C₁-C₄-alkyle, NH₂, NHR^{a}, N(R^{a})R^{b}, N(H) C(=O)R^{a}, C₁-C₄-alcoxy, trifluorométhoxy ou C₁-C₄-alkyle, C₁-C₄-alkyle pouvant éventuellement être substitué une à trois fois par fluor ;
R⁵ représente hydrogène, halogène, hydroxy, cyano, C₁-C₄-alcoxy, trifluorométhoxy ou C₁-C₆-alkyle, où C₁-C₆-alkyle peut éventuellement être substitué par un à cinq atomes de fluor,
R^{6f} représente hydrogène, fluor, C(=O)OH, C(=O)NH₂, trifluorométhyle, hydroxyméthyle, méthoxyméthyle, cyano ou C₁-C₆-alkyle,
R^{6g} représente hydrogène, fluor ou C₁-C₆-alkyle, ou R^{6f} et R^{6g} forment conjointement avec l'atome de carbone auquel ils sont liés, un C₃-C₇-cycloalkyle ou
R^{6f} et R^{6g} représentent ensemble un groupe oxo et
R^{6h} représente hydrogène, trifluorométhyle ou C₁-C₆-alkyle ainsi que
R⁶ⁱ représente hydrogène ou C₁-C₆-alkyle ou
R^{6h} et R⁶ⁱ représentent ensemble un groupe oxo,
R^{6j} représente hydrogène, fluor, NH₂, N(H)R^{a}, N(R^{a})R^{b}, C₁-C₆-alkyle, hydroxy, cyano, C₁-C₄-alcoxy, C(=O)OH, C(=O)NH₂, C(=O)N(H)R^{a}, C(=O)N(R^{a})R^{b}, hydroxyméthyle, diméthylaminométhyle, trifluorométhyle,
R^{6k} représente hydrogène, fluor ou C₁-C₆-alkyle ou
R^{6j} et R^{6k} forment conjointement avec l'atome de carbone un C₃-C₇-cycloalkyle,
R⁶¹ représente hydrogène ou méthyle,
R^{6m} représente hydrogène ou méthyle,
G représente -CH₂- ou -CH₂CH₂-,
n représente 0, 1 ou 2 dans la formule R^{3a},
n représente 1 ou 2 dans la formule R^{3b},
n représente 0 ou 1 dans la formule R^{3c},
z représente un groupe choisi parmi NR⁷, O, S, S(=O), S(=O)₂, S(=O) (=NH) ;
R⁷ représente hydrogène, C(=O)R^{e}, C(=O)OR^{a}, C(=O)NH₂, C(=O)N(H)R^{a}, C(=O)N(R^{a})R^{b}, S(=O)₂R^{a}, S(=O)₂NH₂, S(=O)₂N(R^{a})H, S(=O)₂N(R^{a})R^{b}, S(=O)₂NHC(=O)CH₃, S(=O)₂NHC(=O)CH₂CH₃ ou C₁-C₆-alkyle,
C₁-C₆-alkyle pouvant éventuellement être substitué une à cinq fois par des atomes de fluor et pouvant être substitué une à deux fois, de manière identique ou différente, par hydroxy, chlore, brome, cyano, C(=O)R^{a}, C(=O)OH, C(=O)NH₂, C(=O)N(H)R^{a}, C(=O)N(R^{a})R^{b}, S(=O)₂-C₁-C₆-alkyle, NH₂, NHR^{a}, N(R^{a})R^{b}, morpholin-4-yle, 4-méthylpipérazin-1-yle, C₃-C₇-cycloalkyle, C₁-C₄-alcoxy ou C₃-C₇-cycloalcoxy,
ou représente C₃-C₇-cycloalkyle, qui peut éventuellement être substitué une à quatre fois par des atomes de fluor et peut éventuellement être substitué une à deux fois, de manière identique ou différente, par hydroxy, méthyle, éthyle, trifluorométhyle ou cyano,
ou représente un hétérocycloalkyle à 4 à 7 chaînons lié au reste de la molécule par l'intermédiaire d'un atome de carbone ou représente hétérocycloalkyle à 4 à 7 chaînons-C₁-C₄-alkyle, qui peuvent éventuellement être substitués une à six fois par des atomes de fluor et peuvent être substitués une à trois fois, de manière identique ou différente par hydroxy, chlore, brome, cyano, C₁-C₆-alkyle, C₁-C₆-alcoxy, trifluorométhyle, 2,2,2-trifluoroéthyle, trifluorométhoxy, cyclopropyle, cyclopropylméthyle,
R^{e} représente C₁-C₆-alkyle, C₁-C₆-alkyle pouvant éventuellement être substitué une à trois fois, de manière identique ou différente par hydroxy, fluor, chlore, cyano, C(=O)R^{a}, C(=O)OH, NH₂, NHR^{a}, N(R^{a})R^{b}, C₃-C₇-cycloalkyle, C₁-C₄-alcoxy, trifluorométhoxy ou C₃-C₇-cycloalcoxy ou
R^{e} représente C₃-C₇-cycloalkyle, C₃-C₇-cycloalkyle pouvant éventuellement être substitué une à quatre fois par fluor et éventuellement une fois par hydroxy.

2. Composés selon la revendication 1, dans lesquels R¹ est hydroxyméthyle, 1-hydroxyéthyle et 2-hydroxypropan-2-yle, préférablement 2-hydroxypropan-2-yle.

3. Composés selon la revendication 1, dans lesquels R
² est un radical pyridin-2-yle, qui est substitué au niveau de la position 6 par C₁-C₆-alkyle, C₁-C₆-alkyle étant éventuellement substitué par jusqu'à 5 atomes de fluor ou
est un radical pyridin-2-yle, qui est substitué au niveau de la position 6 par cyano, chlore, cyclopropyle, cyclopropylméthyle, NH₂, NH(C₁-C₄-alkyle), N(C₁-C₄-alkyle)₂, C₁-C₄-alcoxy, 2,2,2-trifluoroéthoxy, 2-hydroxypropan-2-yle, morpholin-4-yle, 4-méthylpipérazin-1-yle ou pipérazin-1-yle.

4. Composés selon les revendications 1 et 3, dans lesquels
R² est un radical pyridin-2-yle, qui est substitué au niveau de la position 6 par trifluorométhyle, difluorométhyle, méthyle, 2,2,2-trifluoroéthyle, 1,1-difluoroéthyle, éthyle, isopropyle, tert-butyle, cyano, chlore, cyclopropyle, cyclopropylméthyle, NH₂, NH(C₁-C₄-alkyle), N(C₁-C₄-alkyle)₂, C₁-C₄-alcoxy, 2,2,2-trifluoroéthoxy, 2-hydroxypropan-2-yle, morpholin-4-yle, 4-méthylpipérazin-1-yle ou pipérazin-1-yle.

5. Composés selon les revendications 1 et 4, dans lesquels R² représente 6-(trifluorométhyl)pyridin-2-yle, 6-(difluorométhyl)pyridin-2-yle, 6-(1,1-difluoroéthyl)pyridin-2-yle, 6-aminopyridin-2-yle et 6-(2-hydroxypropan-2-yl)pyridin-2-yle, préférablement 6-(trifluorométhyl)pyridin-2-yle.

6. Composés selon l'une quelconque des revendications précédentes, dans lesquels
R¹ représente C(=O)NH₂, 2-hydroxypropan-2-yle ou méthoxy,
R² représente 6-(trifluorométhyl)pyridin-2-yle, 6-(difluorométhyl)pyridin-2-yle, 6-aminopyridin-2-yle, 4-(trifluorométhyl)-1,3-thiazol-2-yle, 1-(difluorométhyl)-1H-pyrazol-3-yle,
R³ représente tétrahydro-2H-pyran-4-yle, 1,1-dioxydotétrahydro-2H-thiopyran-4-yle, tétrahydrofuran-3-yle, (3S)-tétrahydrofuran-3-yle, (3R)-tétrahydrofuran-3-yle ou (3S)-1,1-dioxydotétrahydrothiophén-3-yle ou
R³ représente pipéridin-4-yle, 1-(2,2,2-trifluoroéthyl)pipéridin-4-yle, 1-méthylpipéridin-4-yle, 1-glycoloylpipéridin-4-yle, 1'-méthyl-1,4'-bipipéridin-4-yle, 1-(acétylsulfamoyl)pipéridin-4-yle, [2-(diméthylamino)éthyl]pipéridin-4-yle, 1-(oxétan-3-yl)pipéridin-4-yle, 1-(2-hydroxyéthyl)pipéridin-4-yl ou 1-(3-hydroxy-3-méthylbutyl)pipéridin-4-yle.

7. Composés selon l'une quelconque des revendications 1 à 6, c'est-à-dire
(1) N-[6-méthoxy-2-(tétrahydro-2H-pyran-4-yl)-2H-indazol-5-yl]-6-(trifluorométhyl)pyridine-2-carboxamide
(2) N-[6-méthoxy-2-(pipéridin-4-yl)-2H-indazol-5-yl]-6-(trifluorométhyl)pyridine-2-carboxamide
(3) N-{6-méthoxy-2-[1-(2,2,2-trifluoroéthyl)pipéridin-4-yl]-2H-indazol-5-yl}-6-(trifluorométhyl)pyridine-2-carboxamide
(4) N-[6-méthoxy-2-(1-méthylpipéridin-4-yl)-2H-indazol-5-yl]-6-(trifluorométhyl)pyridine-2-carboxamide
(5) N-[2-(1-glycoloylpipéridin-4-yl)-6-méthoxy-2H-indazol-5-yl]-6-(trifluorométhyl)pyridine-2-carboxamide
(6) N-[6-méthoxy-2-(1'-méthyl-1,4'-bipipéridin-4-yl)-2H-indazol-5-yl]-6-(trifluorométhyl)pyridine-2-carboxamide
(7) N-[6-méthoxy-2-(1-sulfamoylpipéridin-4-yl)-2H-indazol-5-yl]-6-(trifluorométhyl)pyridine-2-carboxamide
(8) N-{2-[1-(acétylsulfamoyl)pipéridin-4-yl]-6-méthoxy-2H-indazol-5-yl}-6-(trifluorométhyl)pyridine-2-carboxamide
(9) N-(2-{1-[2-(diméthylamino)éthyl]pipéridin-4-yl}-6-méthoxy-2H-indazol-5-yl)-6-(trifluorométhyl)pyridine-2-carboxamide
(10) N-{6-méthoxy-2-[1-(oxétan-3-yl)pipéridin-4-yl]-2H-indazol-5-yl}-6-(trifluorométhyl)pyridine-2-carboxamide
(11) N-[2-(1,1-dioxydotétrahydro-2H-thiopyran-4-yl)-6-méthoxy-2H-indazol-5-yl]-6-(trifluorométhyl)pyridine-2-carboxamide
(12) N-[6-méthoxy-2-(1-oxydotétrahydro-2H-thiopyran-4-yl)-2H-indazol-5-yl]-6-(trifluorométhyl)pyridine-2-carboxamide
(13) N-{2-[1-(2-hydroxyéthyl)pipéridin-4-yl]-6-méthoxy-2H-indazol-5-yl}-6-(trifluorométhyl)pyridine-2-carboxamide
(14) rel-N-{2-[(1R,4R,5S)-2-azabicyclo[2.2.1]hept-5-yl]-6-méthoxy-2H-indazol-5-yl}-6-(trifluorométhyl)pyridine-2-carboxamide
(15) rel-N-{2-[(1R,4R,5R)-2-azabicyclo[2.2.1]hept-5-yl]-6-méthoxy-2H-indazol-5-yl}-6-(trifluorométhyl)pyridine-2-carboxamide
(16) N-[2-(1,1-dioxydotétrahydro-2H-thiopyran-4-yl)-6-hydroxy-2H-indazol-5-yl]-6-(trifluorométhyl)pyridine-2-carboxamide
(17) N-[6-(cyclopropylméthoxy)-2-(1,1-dioxydotétrahydro-2H-thiopyran-4-yl)-2H-indazol-5-yl]-6-(trifluorométhyl)pyridine-2-carboxamide
(18) rel-N-{6-méthoxy-2-[(1R,4R,5S)-2-méthyl-2-azabicyclo[2.2.1]hept-5-yl]-2H-indazol-5-yl}-6-(trifluorométhyl)pyridine-2-carboxamide
(19) rel-N-{6-méthoxy-2-[(1R,4R,5R)-2-méthyl-2-azabicyclo[2.2.1]hept-5-yl]-2H-indazol-5-yl}-6-(trifluorométhyl)pyridine-2-carboxamide
(20) N-[2-(1-imino-1-oxydohexahydro-1λ⁴-thiopyran-4-yl)-6-méthoxy-2H-indazol-5-yl]-6-(trifluorométhyl)pyridine-2-carboxamide (isomère 1)
(21) N-[2-(1-imino-1-oxydohexahydro-1λ⁴-thiopyran-4-yl)-6-méthoxy-2H-indazol-5-yl]-6-(trifluorométhyl)pyridine-2-carboxamide (isomère 2)
(22) N-[6-méthoxy-2-(5-oxopyrrolidin-3-yl)-2H-indazol-5-yl]-6-(trifluorométhyl)pyridine-2-carboxamide
(23) 6-(difluorométhyl)-N-[6-méthoxy-2-(tétrahydro-2H-pyran-4-yl)-2H-indazol-5-yl]pyridine-2-carboxamide
(24) N-[6-méthoxy-2-(tétrahydro-2H-pyran-4-yl)-2H-indazol-5-yl]-6-(morpholin-4-yl)pyridine-2-carboxamide
(25) N-[6-méthoxy-2-(tétrahydro-2H-pyran-4-yl)-2H-indazol-5-yl]-2-méthyl-1,3-thiazole-4-carboxamide
(26) 6-amino-N-[6-méthoxy-2-(tétrahydro-2H-pyran-4-yl)-2H-indazol-5-yl]pyridine-2-carboxamide
(27) 2-isopropyl-N-[6-méthoxy-2-(tétrahydro-2H-pyran-4-yl)-2H-indazol-5-yl]pyrimidine-4-carboxamide
(28) 6-(2-hydroxypropan-2-yl)-N-[6-méthoxy-2-(tétrahydro-2H-pyran-4-yl)-2H-indazol-5-yl]pyridine-2-carboxamide
(29) N-[6-méthoxy-2-(tétrahydrofuran-3-yl)-2H-indazol-5-yl]-6-(trifluorométhyl)pyridine-2-carboxamide
(30) N-[6-chloro-2-(tétrahydro-2H-pyran-4-yl)-2H-indazol-5-yl]-6-(trifluorométhyl)pyridine-2-carboxamide
(31) N-[6-chloro-2-(tétrahydro-2H-pyran-4-yl)-2H-indazol-5-yl]-6-(difluorométhyl)pyridine-2-carboxamide
(32) N-[6-chloro-2-(1,1-dioxydotétrahydro-2H-thiopyran-4-yl)-2H-indazol-5-yl]-6-(2-hydroxypropan-2-yl)pyridine-2-carboxamide
(33) 2-(tétrahydro-2H-pyran-4-yl)-5-({[6-(trifluorométhyl)pyridin-2-yl]carbonyl}amino)-2H-indazole-6-carboxylate de méthyle
(34) N-[6-(2-hydroxypropan-2-yl)-2-(tétrahydro-2H-pyran-4-yl)-2H-indazol-5-yl]-6-(trifluorométhyl)pyridine-2-carboxamide
(35) 2-[(3S)-tétrahydrofuran-3-yl]-5-({[6-(trifluorométhyl)pyridin-2-yl]carbonyl}amino)-2H-indazole-6-carboxylate de méthyle
(36) N-{6-(2-hydroxypropan-2-yl)-2-[(3S)-tétrahydrofuran-3-yl]-2H-indazol-5-yl}-6-(trifluorométhyl)pyridine-2-carboxamide
(37) 2-[(3R)-tétrahydrofuran-3-yl]-5-({[6-(trifluorométhyl)pyridin-2-yl]carbonyl}amino)-2H-indazole-6-carboxylate de méthyle
(38) N-{6-(2-hydroxypropan-2-yl)-2-[(3R)-tétrahydrofuran-3-yl]-2H-indazol-5-yl}-6-(trifluorométhyl)pyridine-2-carboxamide
(39) 2-[(3S)-tétrahydrothiophén-3-yl]-5-({[6-(trifluorométhyl)pyridin-2-yl]carbonyl}amino)-2H-indazole-6-carboxylate de méthyle
(40) N-{6-(2-hydroxypropan-2-yl)-2-[(3S)-tétrahydrothiophén-3-yl]-2H-indazol-5-yl}-6-(trifluorométhyl)pyridine-2-carboxamide
(41) N-{2-[(3S)-1,1-dioxydotétrahydrothiophén-3-yl]-6-(2-hydroxypropan-2-yl)-2H-indazol-5-yl}-6-(trifluorométhyl)pyridine-2-carboxamide
(42) 2-(tétrahydro-2H-thiopyran-4-yl)-5-({[6-(trifluorométhyl)pyridin-2-yl]carbonyl}amino)-2H-indazole-6-carboxylate de méthyle
(43) N-[6-(2-hydroxypropan-2-yl)-2-(tétrahydro-2H-thiopyran-4-yl)-2H-indazol-5-yl]-6-(trifluorométhyl)pyridine-2-carboxamide
(44) N-[2-(1,1-dioxydotétrahydro-2H-thiopyran-4-yl)-6-(2-hydroxypropan-2-yl)-2H-indazol-5-yl]-6-(trifluorométhyl)pyridine-2-carboxamide
(45) 2-(pipéridin-4-yl)-5-({[6-(trifluorométhyl)pyridin-2-yl]carbonyl}amino)-2H-indazole-6-carboxylate de méthyle
(46) N-[6-(2-hydroxypropan-2-yl)-2-(pipéridin-4-yl)-2H-indazol-5-yl]-6-(trifluorométhyl)pyridine-2-carboxamide
(47) N-[6-méthoxy-2-(tétrahydro-2H-pyran-4-yl)-2H-indazol-5-yl]-4-(trifluorométhyl)-1,3-thiazole-2-carboxamide
(48) N-[6-méthoxy-2-(tétrahydro-2H-pyran-4-yl)-2H-indazol-5-yl]-3-(pyridin-4-yl)-1,2,4-oxadiazole-5-carboxamide
(49) N-[6-méthoxy-2-(tétrahydro-2H-pyran-4-yl)-2H-indazol-5-yl]-3-méthyl-1,2,4-oxadiazole-5-carboxamide
(50) N-[6-chloro-2-(tétrahydro-2H-pyran-4-yl)-2H-indazol-5-yl]-4-(trifluorométhyl)-1,3-thiazole-2-carboxamide
(51) 1-(difluorométhyl)-N-[6-méthoxy-2-(tétrahydro-2H-pyran-4-yl)-2H-indazol-5-yl]-1H-pyrazole-3-carboxamide
(52) N-{2-[1-(3-hydroxy-3-méthylbutyl)pipéridin-4-yl]-6-méthoxy-2H-indazol-5-yl}-6-(trifluorométhyl)pyridine-2-carboxamide
(53) 2-(pipéridin-4-yl)-5-({[6-(trifluorométhyl)pyridin-2-yl]carbonyl}amino)-2H-indazole-6-carboxamide
(54) acide 2-(pipéridin-4-yl)-5-({[6-(trifluorométhyl)pyridin-2-yl]carbonyl}amino)-2H-indazole-6-carboxylique
(55) 2-(1-méthylpipéridin-4-yl)-5-({[6-(trifluorométhyl)pyridin-2-yl]carbonyl}amino)-2H-indazole-6-carboxylate de méthyle
(56) 2-[1-(3-hydroxy-3-méthylbutyl)pipéridin-4-yl]-5-({[6-(trifluorométhyl)pyridin-2-yl]carbonyl}amino)-2H-indazole-6-carboxamide
(57) 2-(1-méthylpipéridin-4-yl)-5-({[6-(trifluorométhyl)pyridin-2-yl]carbonyl}amino)-2H-indazole-6-carboxamide
(58) N-{6-méthoxy-2-[1-(2-méthoxyéthyl)pipéridin-4-yl]-2H-indazol-5-yl}-6-(trifluorométhyl)pyridine-2-carboxamide.

8. Composé de formule générale (I), tel que défini dans l'une quelconque des revendications 1 à 7, pour le traitement et/ou la prophylaxie de maladies.

9. Composé de formule générale (I), tel que défini dans l'une quelconque des revendications 1 à 7, pour une utilisation dans un procédé pour le traitement et/ou la prophylaxie de maladies tumorales, de maladies dermatologiques, de maladies gynécologiques, de maladies cardiovasculaires, de maladies pulmonaires, de maladies ophtalmologiques, de maladies neurologiques, de maladies métaboliques, de maladies du foie, de maladies inflammatoires, de maladies auto-immunes et d'une douleur.

10. Composé de formule générale (I), tel que défini dans l'une quelconque des revendications 1 à 7, pour une utilisation dans un procédé pour le traitement et/ou la prophylaxie de lymphomes, de la dégénération maculaire, du psoriasis, du lupus érythémateux, de la sclérose en plaques, de la BPCO, de la goutte, de la SHNA, d'une fibrose du foie, de la résistance à l'insuline, du syndrome métabolique, de spondylarthrites et de l'arthrite rhumatoïde, de l'endométriose ainsi que de douleurs associées à l'endométriose et d'autres symptômes associés à l'endométriose comme la dysménorrhée, la dyspareunie, la dysurie et la dyschésie.

11. Composé de formule générale (I), tel que défini dans l'une quelconque des revendications 1 à 7, pour une utilisation dans un procédé pour le traitement et/ou la prophylaxie d'une douleur y compris une douleur aiguë, chronique, inflammatoire et neuropathique, de préférence de l'hyperalgie, de l'allodynie, d'une douleur due à l'arthrite (comme l'ostéoarthrite, l'arthrite rhumatoïde et la spondylarthrite), d'une douleur prémenstruelle, d'une douleur associée à l'endométriose, d'une douleur postopératoire, d'une douleur due à une cystite interstitielle, du CRPS (syndrome de douleur complexe régional), d'une névralgie du trijumeau, d'une douleur due à une prostatite, de douleurs causées par des blessures à la moelle épinière, d'une douleur induite par une inflammation, de lombalgies, de douleurs cancéreuses, d'une douleur associée à une chimiothérapie, d'une neuropathie induite par un traitement du VIH, d'une douleur induite par une brûlure et d'une douleur chronique.

12. Utilisation d'un composé de formule générale (I), tel que défini dans l'une quelconque des revendications 1 à 7, pour la préparation d'un médicament.

13. Utilisation selon la revendication 12, le médicament étant utilisé pour le traitement et/ou la prophylaxie de maladies tumorales, de maladies dermatologiques, de maladies gynécologiques, de maladies cardiovasculaires, de maladies pulmonaires, de maladies ophtalmologiques, de maladies neurologiques, de maladies métaboliques, de maladies du foie, de maladies inflammatoires, de maladies auto-immunes et d'une douleur.

14. Utilisation selon les revendications 12 et 13, le médicament étant utilisé pour le traitement et/ou la prophylaxie de lymphomes, de la dégénération maculaire, du psoriasis, du lupus érythémateux, de la sclérose en plaques, de la BPCO, de la goutte, de la SHNA, d'une fibrose du foie, de la résistance à l'insuline, du syndrome métabolique, de spondylarthrites et de l'arthrite rhumatoïde, de l'endométriose ainsi que de douleurs associées à l'endométriose et d'autres symptômes associés à l'endométriose comme la dysménorrhée, la dyspareunie, la dysurie et la dyschésie.

15. Utilisation selon les revendications 12 et 13, le médicament étant utilisé pour le traitement et/ou la prophylaxie d'une douleur y compris une douleur aiguë, chronique, inflammatoire et neuropathique, de préférence de l'hyperalgie, de l'allodynie, d'une douleur due à l'arthrite (comme l'ostéoarthrite, l'arthrite rhumatoïde et la spondylarthrite), d'une douleur prémenstruelle, d'une douleur associée à l'endométriose, d'une douleur postopératoire, d'une douleur due à une cystite interstitielle, du CRPS (syndrome de douleur complexe régional), d'une névralgie du trijumeau, d'une douleur due à une prostatite, de douleurs causées par des blessures à la moelle épinière, d'une douleur induite par une inflammation, de lombalgies, de douleurs cancéreuses, d'une douleur associée à une chimiothérapie, d'une neuropathie induite par un traitement du VIH, d'une douleur induite par une brûlure et d'une douleur chronique.

16. Médicament contenant un composé de formule (I), tel que défini dans l'une quelconque des revendications 12 et 13, en combinaison avec un auxiliaire inerte, non toxique, pharmaceutiquement acceptable.
